(19) Europäisches Patentamt
European Patent Office
Office européen des brevets

(11) **EP 4 534 540 A1**

(12) **EUROPEAN PATENT APPLICATION**
published in accordance with Art. 153(4) EPC

(43) Date of publication:
**09.04.2025 Bulletin 2025/15**

(21) Application number: **23815312.6**

(22) Date of filing: **02.06.2023**

(51) International Patent Classification (IPC):
***C07D 495/04*** *(2006.01)* ***C07D 519/00*** *(2006.01)*
***A61K 31/519*** *(2006.01)* ***A61P 11/00*** *(2006.01)*

(52) Cooperative Patent Classification (CPC):
**A61K 31/519; A61P 11/00; C07D 495/04; C07D 519/00**

(86) International application number:
**PCT/CN2023/097944**

(87) International publication number:
**WO 2023/232135 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**02.06.2022 CN 202210625926**
**22.06.2022 CN 202210714663**
**19.07.2022 CN 202210848130**
**09.08.2022 CN 202210952925**
**02.11.2022 CN 202211362508**
**24.11.2022 CN 202211482574**
**12.01.2023 CN 202310038878**
**24.02.2023 CN 202310162024**
**20.03.2023 CN 202310271298**
**06.04.2023 CN 202310357142**
**23.04.2023 CN 202310440428**

(71) Applicant: **Xizang Haisco Pharmaceutical Co., Ltd.**
**Lhoka, Tibet 856099 (CN)**

(72) Inventors:
- **LI, Yao**
  **Lhoka, Tibet 856099 (CN)**
- **CHEN, Lei**
  **Lhoka, Tibet 856099 (CN)**
- **SHI, Zongjun**
  **Lhoka, Tibet 856099 (CN)**
- **REN, Lei**
  **Lhoka, Tibet 856099 (CN)**
- **HUANG, Shuai**
  **Lhoka, Tibet 856099 (CN)**
- **YANG, Shuang**
  **Lhoka, Tibet 856099 (CN)**
- **HE, Tiancheng**
  **Lhoka, Tibet 856099 (CN)**
- **WANG, Jie**
  **Lhoka, Tibet 856099 (CN)**
- **CHENG, Fengkai**
  **Lhoka, Tibet 856099 (CN)**
- **ZHANG, Chen**
  **Lhoka, Tibet 856099 (CN)**
- **YAN, Pangke**
  **Lhoka, Tibet 856099 (CN)**

(74) Representative: **IP TRUST SERVICES**
**Europole - Le Grenat**
**3, avenue Doyen Louis Weil**
**38000 Grenoble (FR)**

# (54) PDE4B INHIBITOR AND USE THEREOF

(57) Disclosed are a compound as represented by formula I; a stereoisomer or a pharmaceutically acceptable salt thereof, or a pharmaceutical composition containing same; and the use thereof as a PDE4B inhibitor in the preparation of a drug for the treatment of related diseases. Each group as shown in formula (I) is as defined in the description.

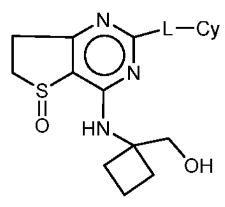

(I)

EP 4 534 540 A1

## Description

### Technical Field

The present invention belongs to the field of medicine and in particular relates to a small molecule compound and a stereoisomer or pharmaceutically acceptable salt thereof, which have a selective inhibitory activity against PDE4B, and the use thereof in the preparation of a medicament for treating a related disease.

### Background Art

PDE4 inhibitors produce antidepressant effects in humans and animals by enhancing cAMP signalling in the brain. PDE4 inhibitors also play an important role in the treatment of other central nervous system diseases, including diseases such as Alzheimer's disease, Parkinson's disease, schizophrenia, stroke, and Huntington's chorea.

In addition, the research and development of PDE4 inhibitors have also made great progress in the treatment of respiratory diseases such as asthma and chronic obstructive pulmonary disease. The principle of research and development of such drugs stems from the role of PDE4 in inhibiting the function of a range of inflammatory cells and resident cells, which is believed to be associated with the pathogenesis of such diseases. A large number of clinical studies have shown that cyclic adenosine monophosphate (cAMP) can block the proliferation and chemotaxis of inflammatory cells and inhibit the release of inflammatory and cytotoxic mediators in lungs. In addition, PDE4 is especially abundant in immune cells, inflammatory cells, and smooth muscle cells.

PDE4 inhibitors play an anti-inflammatory role mainly by inhibiting the hydrolysis function of PDE4 so as to increase the cAMP level *in vivo,* thereby inhibiting the release of inflammatory factors and at the same time promoting the production of anti-inflammatory mediators. Roflumilast is used clinically for the treatment of COPD with a significant anti-inflammatory effect and can inhibit the release of inflammatory mediators such as TNF-$\alpha$, interleukins, and chemokines from monocytes, macrophages, T cells, etc. However, such inhibitors generally have serious side effects such as nausea and vomiting, which limits the clinical application of PDE4 inhibitors. A large number of studies have shown that phosphodiesterase 4 subtype B (PDE4B), which is associated with inflammatory responses in the human body, participates in the release of various inflammatory mediators *in vivo,* while subtype D is closely related to the production of side effects such as nausea and vomiting, which provides a new idea for finding PDE4 inhibitors with low side effects, i.e., designing PDE4B inhibitors that may have reduced side effects, thereby promoting further clinical application.

Phosphodiesterase 4 is highly selective for cAMP and has four subtypes, namely PDE4A, 4B, 4C, and 4D, with at least 25 splice variants. The protein sequences of the catalytic domains of the four subtypes of PDE4 are highly homologous; therefore, inhibitors that act on the catalytic domain do not exhibit subtype selectivity. Most of the classical PDE4 inhibitors have been reported to act on the catalytic domain. A new mode of action of PDE4 inhibitors has been reported in recent years, in which the inhibitor acts on both the catalytic domain and a regulatory sequence, so that the regulatory sequence can stabilize the closed conformation of the protein, thereby preventing cAMP from entering, thus exerting an inhibitory effect. However, it has been found from research that there are amino acid differences between PDE4B and 4D in this regulatory sequence, so designing inhibitors based on such differences can produce subtype selectivity. Therefore, based on the difference of the two amino acids PDE4B Leu674/PDE4D Gln594 in CR3 (Conserved Region 3) of the downstream regulatory sequence, it is expected to achieve the selectivity to subtype B and reduce the side effects of the inhibitors while maintaining the activity.

A selective PDE4B inhibitor with a good activity, a high safety, less side effects, and superior pharmacokinetics has been found, which has good clinical development prospects and can be used for treating COPD, cancer, or other proliferative diseases or conditions.

### Summary of the Invention

The present invention provides a small molecule compound with PDE4B inhibitory activity, or a stereoisomer or pharmaceutically acceptable salt thereof, wherein the compound is as represented by formula (I),

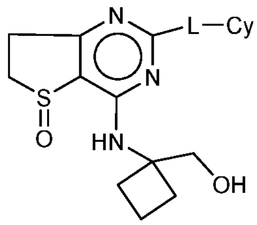

I

wherein Cy is selected from Cy1, Cy2, a 5-membered monocyclic heteroaryl, or an 8- to 10-membered bicyclic heteroaryl, wherein the monocyclic heteroaryl and the bicyclic heteroaryl are optionally further substituted with 1-3 R; in some embodiments, Cy is selected from Cy1 or Cy2; in some embodiments, Cy is selected from Cy1; in some embodiments, Cy is selected from 5-membered monocyclic heteroaryl, 5-membered heteroaryl fused benzene ring, 5-membered heteroaryl fused 5-membered heteroaryl, 5-membered heteroaryl fused 6-membered heteroaryl, 5-membered heteroaryl fused 4-membered cycloalkyl, 5-membered heteroaryl fused 5-membered cycloalkyl, 5-membered heteroaryl fused 6-membered cycloalkyl, 5-membered heteroaryl fused 4-membered heterocycloalkyl, 5-membered heteroaryl fused 5-membered heterocycloalkyl, 5-membered heteroaryl fused 6-membered heterocycloalkyl, 6-membered heteroaryl fused benzene ring, 6-membered heteroaryl fused 5-membered heteroaryl, 6-membered heteroaryl fused 6-membered heteroaryl, 6-membered heteroaryl fused 4-membered cycloalkyl, 6-membered heteroaryl fused 5-membered cycloalkyl, 6-membered heteroaryl fused 6-membered cycloalkyl, 6-membered heteroaryl fused 4-membered heterocycloalkyl, 6-membered heteroaryl fused 5-membered heterocycloalkyl, and 6-membered heteroaryl fused 6-membered heterocycloalkyl; in some embodiments, Cy is selected from the following structures:

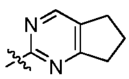 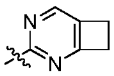 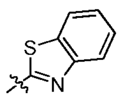 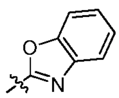 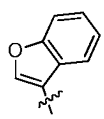 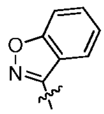 ;

in some embodiments, Cy is selected from the following structures:

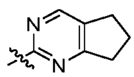 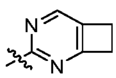 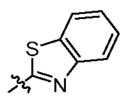 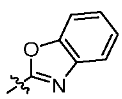 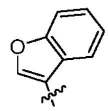 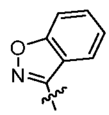 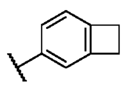 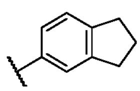

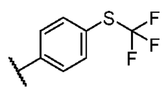 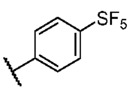 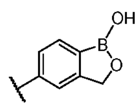 ;

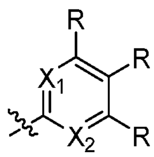 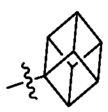

Cy1 , Cy2 ;

Cy2 is optionally substituted with 1-3 R; in some embodiments, Cy2 is unsubstituted;

L is -$(L_1)$n-$(L_2)$m-;

in all the solutions of the present invention, the site of attachment of L to Cy is arbitrary; in some embodiments, the $L_2$ end is a site of attachment to Cy; in some embodiments, the $L_1$ end is a site of attachment to Cy;

$L_1$ is -$NR_1$-, -$CR_2$=N-, -$CR_2$=$CR_2$-, or -$CR_2R_2$-;

in all the solutions of the present invention, the site of attachment of $L_1$ is arbitrary, for example, N atom and C atom in -$CR_2$=N- connected to $L_2$ can both be the site of attachment to $L_2$;

$L_2$ is 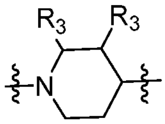, 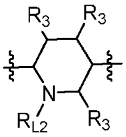, 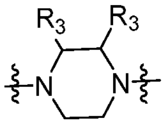, 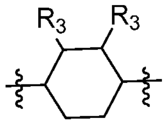, 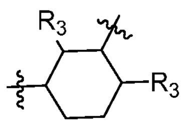,

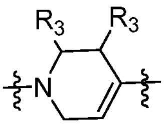, 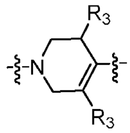, 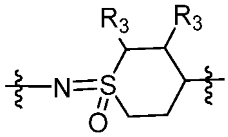, 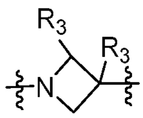,

$C_{1-4}$ alkylene, $C_{2-4}$ alkenylene, $C_{2-4}$ alkynylene, CO, O, $NR_{L2}$, $C_{3-5}$ cycloalkyl, 6- to 9-membered aryl, 5- or 6-membered heteroaryl containing 1-3 heteroatoms selected from N, S and O, 5-membered monoheterocyclic alkyl containing 1-3 heteroatoms selected from N, S and O, 7- or 8-membered monoheterocyclic alkyl containing 1-3 heteroatoms selected from N, S and O, 5- to 10-membered fused-heterocyclic alkyl containing 1-3 heteroatoms selected from N, S and O, 6- to 10-membered bridged-heterocyclic alkyl containing 1-3 heteroatoms selected from N, S and O, or 7- to 12-membered spiro-heterocyclic alkyl containing 1-3 heteroatoms selected from N, S and O, wherein the alkylene, alkenylene, alkynylene, cycloalkyl, aryl, heteroaryl, monoheterocyclic alkyl, fused-heterocyclic alkyl, bridged-heterocyclic alkyl, and spiro-heterocyclic alkyl are optionally substituted with 1-3 groups selected from halogen, =O, CN, $C_{1-4}$ alkyl, $C_{1-4}$ alkoxy, OH, and $NH_2$; in some embodiments, $L_2$

is 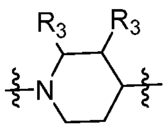, 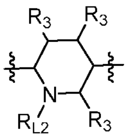, 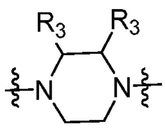, 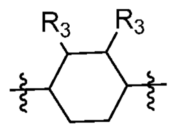, 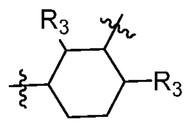,

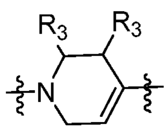, 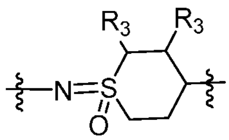, 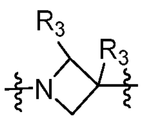,

$C_{1-4}$ alkylene, $C_{2-4}$ alkenylene, $C_{2-4}$ alkynylene, CO, O, $NR_{L2}$, $C_{3-5}$ cycloalkyl, 6- to 9-membered aryl, 5- or 6-membered heteroaryl containing 1-3 heteroatoms selected from N, S and O, 5-membered monoheterocyclic alkyl containing 1-3 heteroatoms selected from N, S and O, 7- or 8-membered monoheterocyclic alkyl containing 1-3 heteroatoms selected from N, S and O, 5- to 10-membered fused-heterocyclic alkyl containing 1-3 heteroatoms selected from N, S and O, 6- to 10-membered bridged-heterocyclic alkyl containing 1-3 heteroatoms selected from N, S and O, or 7- to 12-membered spiro-heterocyclic alkyl containing 1-3 heteroatoms selected from N, S and O, wherein the alkylene, alkenylene, alkynylene, cycloalkyl, aryl, heteroaryl, monoheterocyclic alkyl, fused-heterocyclic alkyl, bridged-heterocyclic alkyl, and spiro-heterocyclic alkyl are optionally substituted with 1-3 groups selected from halogen, =O, CN, $C_{1-4}$ alkyl, $C_{1-4}$ alkoxy, OH, and $NH_2$; in some embodiments, each $L_2$ is independently selected from CO, O, $NR_{L2}$, $C_{1-4}$ alkylene, $C_{2-4}$ alkenylene, $C_{2-4}$ alkynylene,

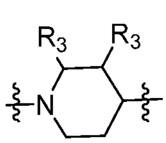, 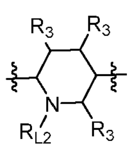, 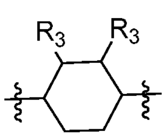, 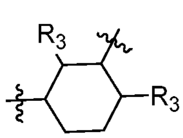,

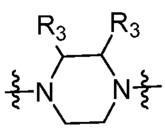, 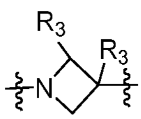,

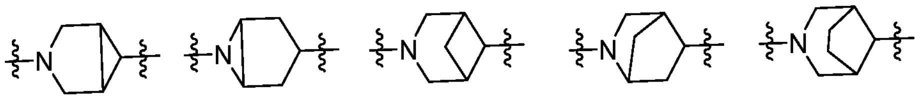,

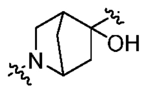, 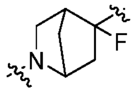,

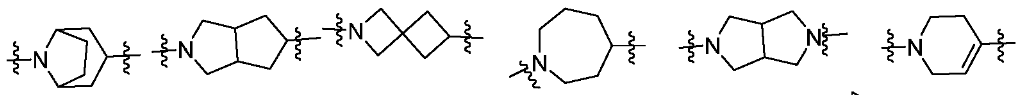

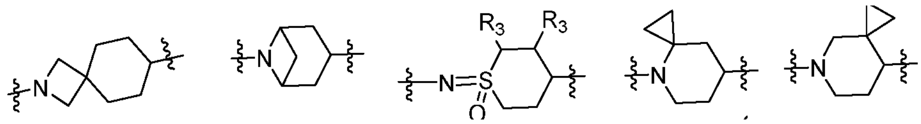

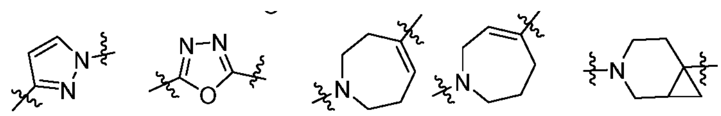

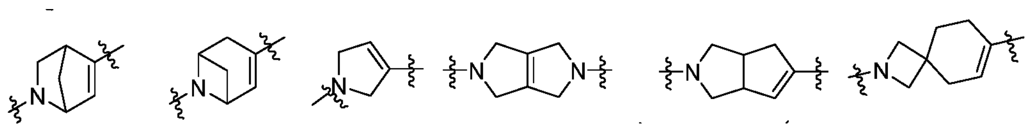

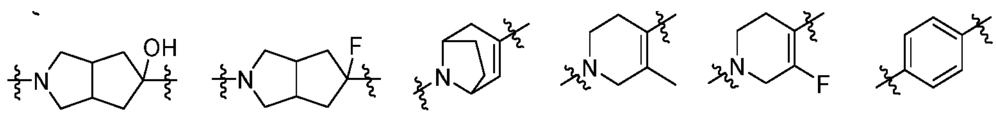,

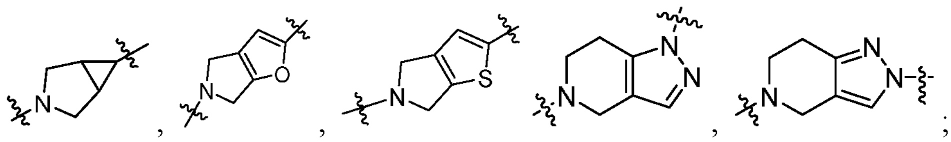;

in some embodiments, each $L_2$ is independently selected from CO, O, $NR_{L2}$, $C_{1-4}$ alkylene, $C_{2-4}$ alkenylene, $C_{2-4}$ alkynylene,

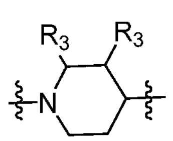, 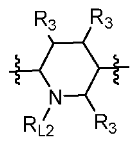, 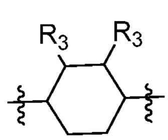,

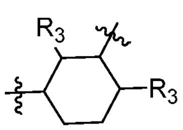, 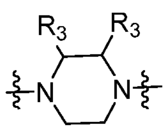, 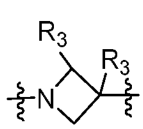,

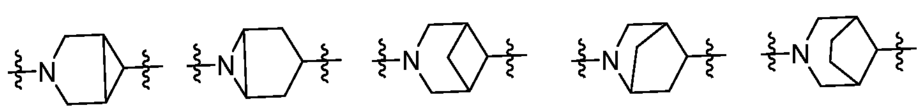.

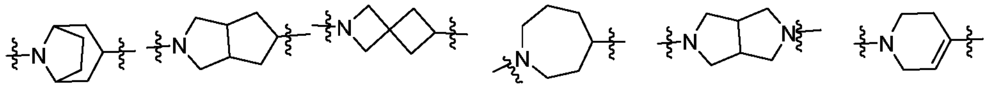

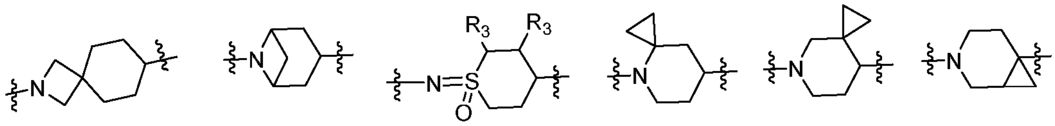

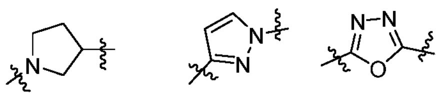

;

in some embodiments, each $L_2$ may also be independently selected from CO, O, $NR_{L2}$, $C_{1-4}$ alkylene, $C_{2-4}$ alkenylene, $C_{2-4}$ alkynylene,

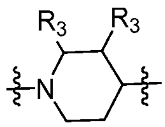,
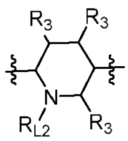,

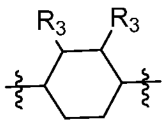,
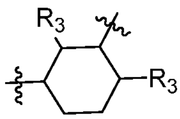,
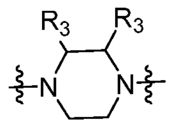,
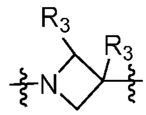,

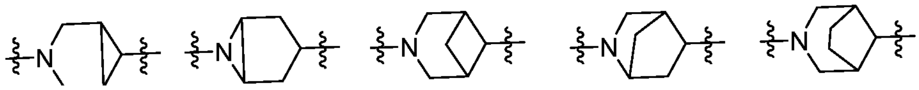

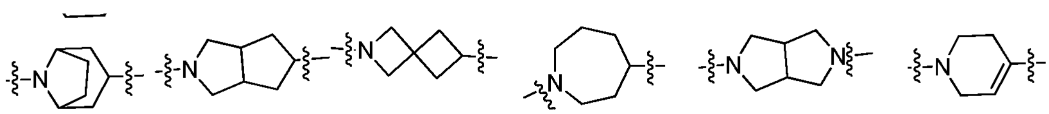

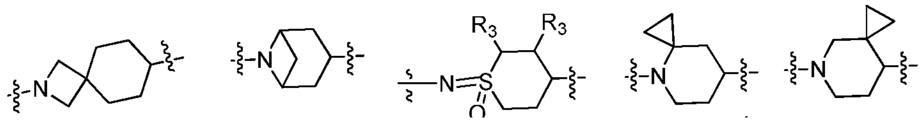

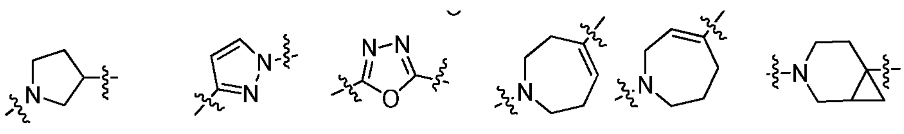

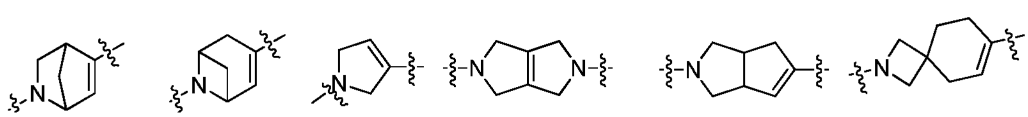

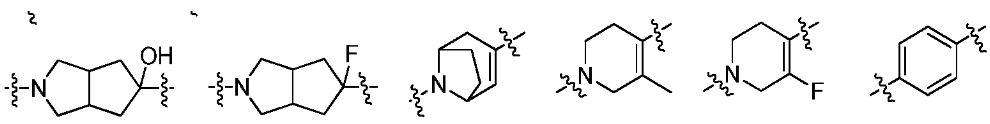

,

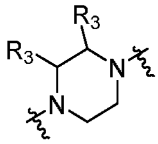, 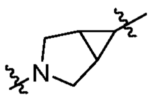, 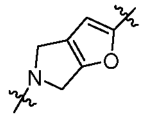, and 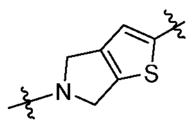;

in some embodiments, each $L_2$ is independently selected from CO, O, $NR_{L2}$, $C_{1-4}$ alkylene, $C_{2-4}$ alkenylene, $C_{2-4}$ alkynylene,

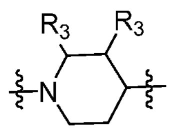, 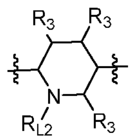,

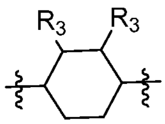, 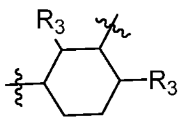, 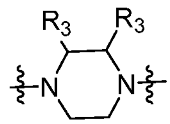, 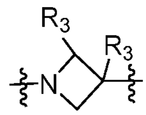,

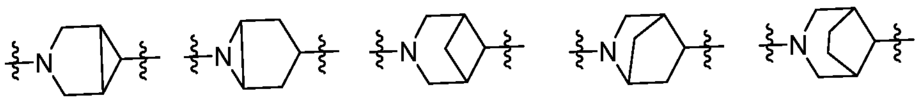,

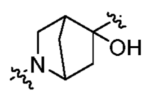, 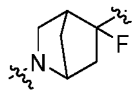,

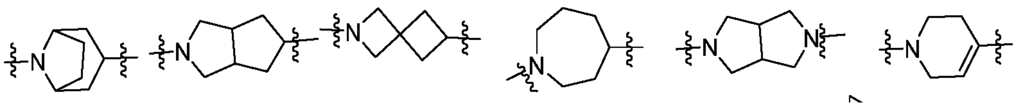

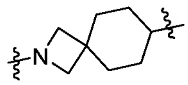 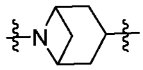 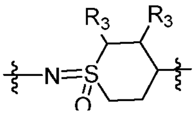 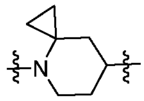 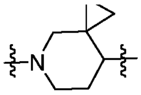

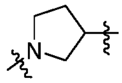 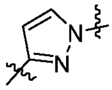 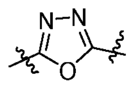 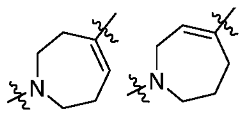 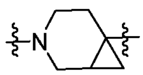

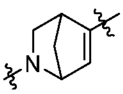 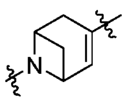 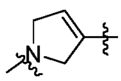 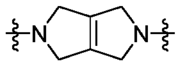 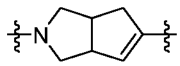 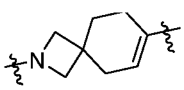

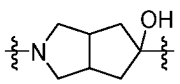 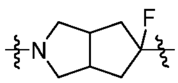 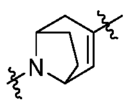 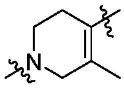 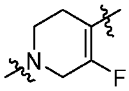 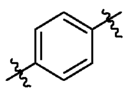,

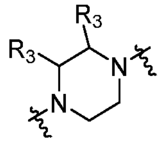, 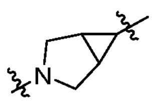, 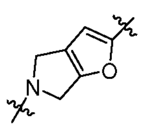, and 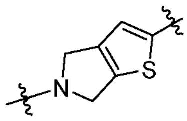;

in all the solutions of the present invention, the sites of attachment of $L_2$ to the left and right groups are arbitrary, for example, in $L_2$ (

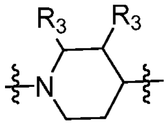

) as one end, both N atom and C atom can be used as sites of attachment to Cy;

$R_{L2}$ is H, $C_{1-4}$ alkyl, or $C_{3-6}$ cycloalkyl; in some embodiments, $R_{L2}$ is H, $C_{1-4}$ alkyl, or $C_{3-6}$ cycloalkyl; in some embodiments, $R_{L2}$ is H, methyl, ethyl, propyl, or isopropyl;

L is selected from one of the following structures:

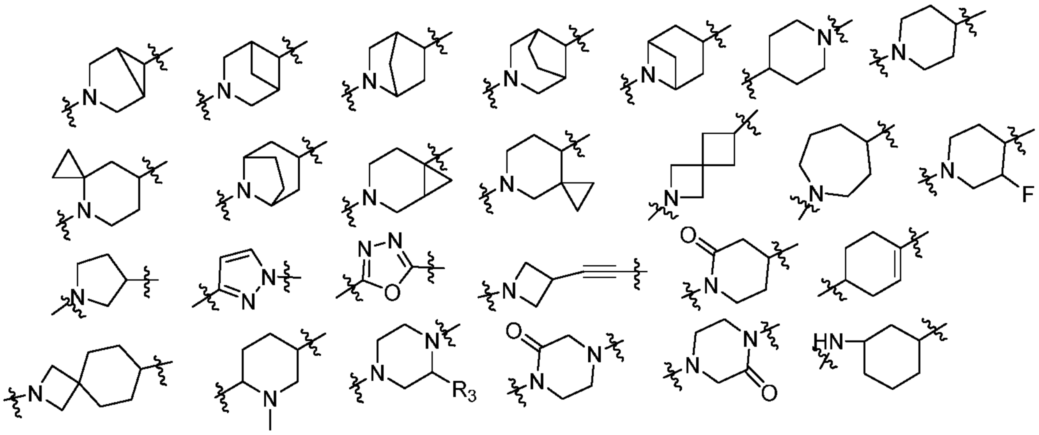

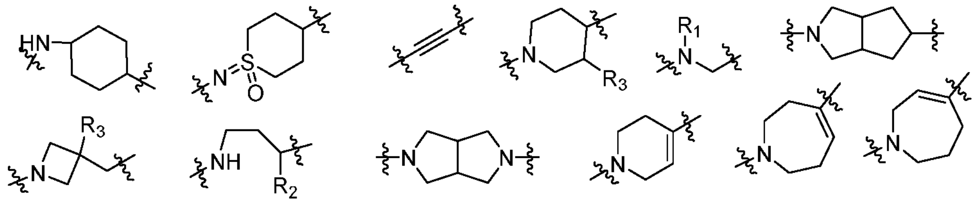

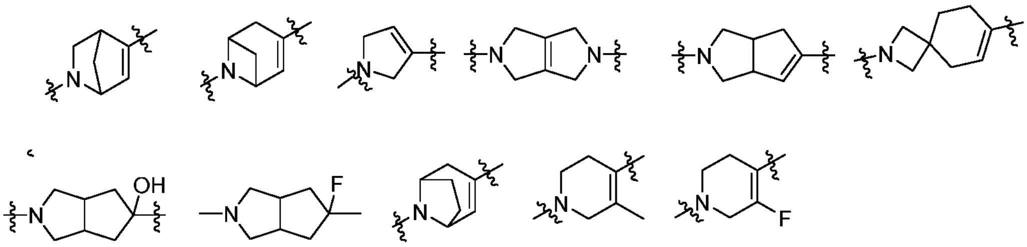,

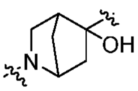, 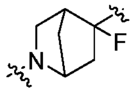, 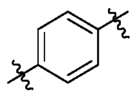, 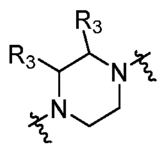, 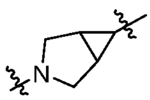, 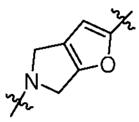,

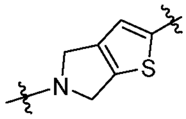, 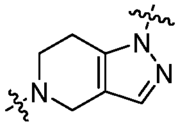, 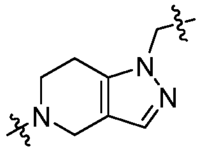, and 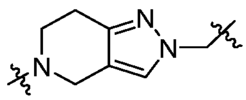;

in some embodiments, L is selected from

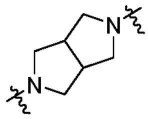, 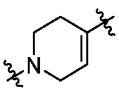, 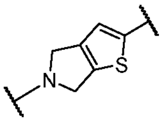,

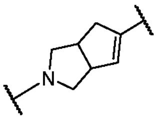, 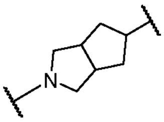, 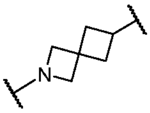, 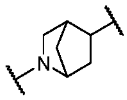, 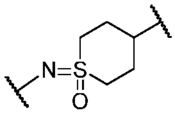, 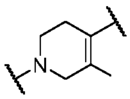,

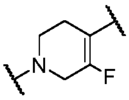, 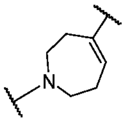, 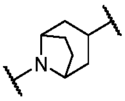, 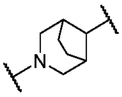, 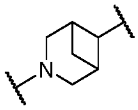, 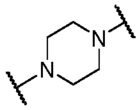, 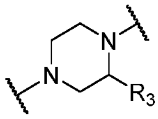,

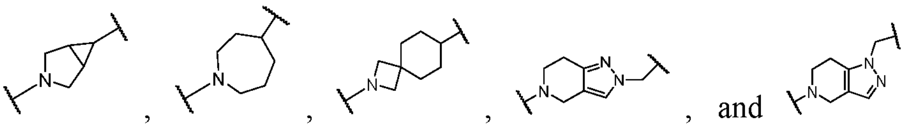

, , , , and ;

in some embodiments, L is selected from

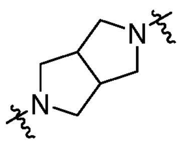, 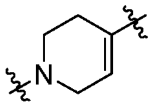, 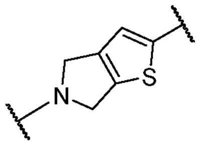,

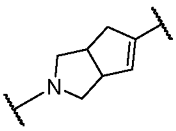, 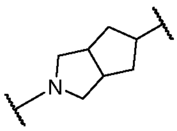, 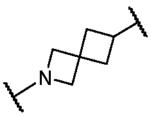, 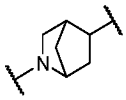, 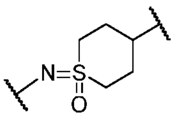, 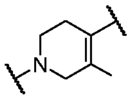,

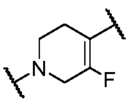, 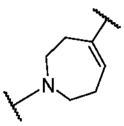, 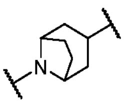, 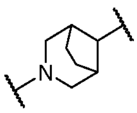, 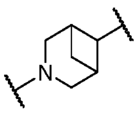, 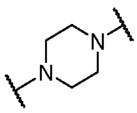, 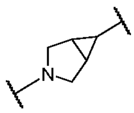,

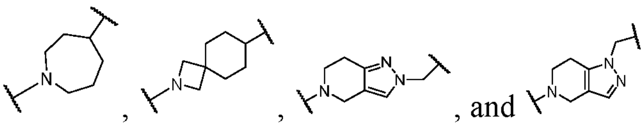

, , , and ;

in some embodiments, L is selected from

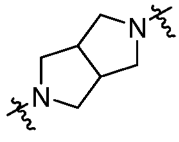 or 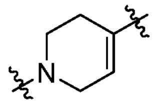 ;

in some embodiments, L is selected from

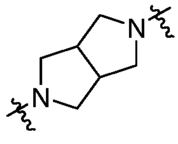 or 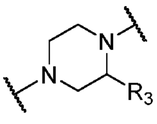 ;

in some embodiments,

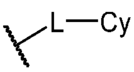

is selected from the following structures:

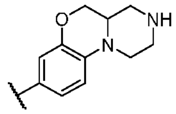 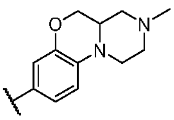 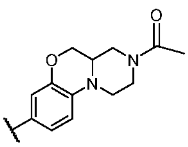 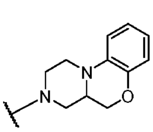 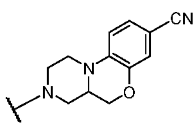 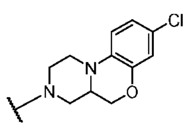

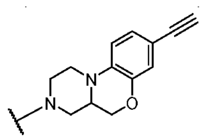 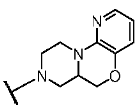 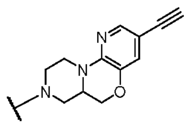 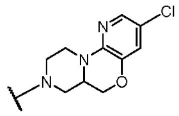 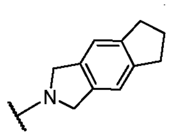 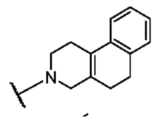

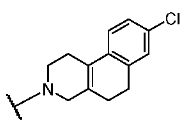 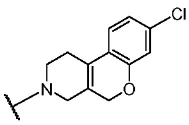 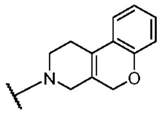 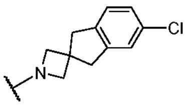 or 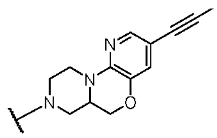 ,

or selected from

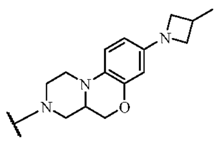 ;

$X_1$ and $X_2$ are independently $CR_{X1}$ or N; in some embodiments, $X_1$ and $X_2$ are $CR_{X1}$; in some embodiments, $X_1$ and $X_2$ are N;

each $R_{X1}$ is independently H, halogen, $C_{1-4}$ alkyl, $C_{1-4}$ haloalkyl, $C_{2-4}$ alkenyl, $C_{2-4}$ alkynyl, or $-SO_2NH_2$, or $R_{X1}$ and $R_1$, $R_{X1}$ and $R_2$, or $R_{X1}$ and $R_3$, together with the atoms to which they are attached, form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$; in some embodiments, each $R_{X1}$ is independently H, halogen, $C_{1-4}$ alkyl, or $C_{1-4}$ haloalkyl, or $R_{X1}$ and $R_1$, $R_{X1}$ and $R_2$, or $R_{X1}$ and $R_3$ together with the atoms to which they are attached form $C_{3-6}$ cycloalkyl, 5- to 7-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$; in some embodiments, each $R_{X1}$ is

independently H, halogen, $C_{1-4}$ alkyl, or $C_{1-4}$ haloalkyl, or $R_{X1}$ and $R_1$, $R_{X1}$ and $R_2$, or $R_{X1}$ and $R_3$ together with the atoms to which they are attached form $C_{3-6}$ cycloalkyl, 5- to 7-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$; in some embodiments, each $R_{X1}$ is independently H, F, Cl, Br, I, methyl, ethyl, propyl, or halomethyl, haloethyl, or halopropyl, or $R_{X1}$ and $R_1$, $R_{X1}$ and $R_2$, or $R_{X1}$ and $R_3$ together with the atoms to which they are attached form $C_5$ cycloalkyl, $C_6$ cycloalkyl, 5-membered heterocycloalkyl or 6-membered heterocycloalkyl containing 1-3 heteroatoms selected from N, S and O, pyrrolyl, imidazolyl, furyl, pyranyl, morpholinyl, etc., which are optionally substituted with 1-3 groups selected from halogen, =O, CN, $C_{1-4}$ alkyl, $C_{1-4}$ alkoxy, OH, and $NH_2$; in some embodiments, each $R_{X1}$ is independently H, F, Cl, methyl, or ethyl, or $R_{X1}$ and $R_3$ together with the atoms to which they are attached form $C_5$ cycloalkyl, $C_6$ cycloalkyl, 5-membered heterocycloalkyl containing 1-3 heteroatoms selected from N, S and O, or 6-membered heterocycloalkyl containing 1-3 heteroatoms selected from N, S and O;

each R is independently H, halogen, cyano, $C_{1-4}$ alkyl, $C_{2-4}$ alkenyl, $C_{2-4}$ alkynyl, $C_{3-8}$ cycloalkyl, 4- to 7-membered heterocycloalkyl containing 1-3 heteroatoms selected from N, S, O, Si and P, $-SO_2NH_2$, $-NHSO_2NH_2$, $-NHCONH_2$, $-NHCOC_{1-4}$ alkyl, $-CONHC_{1-4}$ alkyl, $-NHSO_2C_{1-4}$ alkyl, $-SO_2NHC_{1-4}$ alkyl, $SO_2C_{1-4}$ alkyl, $-P(O)(C_{1-4}$ alkyl$)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, $-C(O)C_{1-4}$ alkyl, and $NH_2$; or two adjacent R, or R and $R_{X1}$ together with the atoms to which they are attached form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, $-C(O)C_{1-4}$ alkyl, and $NH_2$; in some embodiments, each R is independently H, halogen, cyano, $C_{1-4}$ alkyl, $C_{2-4}$ alkenyl, $C_{2-4}$ alkynyl, $C_{3-8}$ cycloalkyl, 4- to 7-membered heterocycloalkyl containing 1-3 heteroatoms selected from N, S, O, Si and P, $-SO_2NH_2$, $-NHSO_2NH_2$, $-NHCONH_2$, $-NHCOC_{1-4}$ alkyl, $-CONHC_{1-4}$ alkyl, $-NHSO_2C_{1-4}$ alkyl, $-SO_2NHC_{1-4}$ alkyl, $SO_2C_{1-4}$ alkyl, $-P(O)(C_{1-4}$ alkyl$)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$; in some embodiments, each R is independently H, halogen, cyano, $C_{1-4}$ alkyl, $C_{2-4}$ alkenyl, $C_{2-4}$ alkynyl, $C_{3-6}$ cycloalkyl, 4- to 6-membered heterocycloalkyl containing 1-3 heteroatoms selected from N, S, O, Si and P, $-SO_2NH_2$, $-NHSO_2NH_2$, $-NHSO_2C_{1-4}$ alkyl, $-SO_2NHC_{1-4}$ alkyl, $SO_2C_{1-4}$ alkyl, $-P(O)(C_{1-2}$ alkyl$)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; in some embodiments, each R is independently H, halogen, cyano, $C_{1-4}$ alkyl, $C_{2-4}$ alkynyl, $C_{3-6}$ cycloalkyl, 4- to 6-membered heterocycloalkyl containing 1-3 heteroatoms selected from N, S, O, Si and P, $-SO_2NH_2$, $-NHSO_2C_{1-4}$ alkyl, $-SO_2NHC_{1-4}$ alkyl, $SO_2C_{1-4}$ alkyl, $-P(O)(CH_3)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; in some embodiments, each R is independently H, halogen, $C_{1-4}$ alkyl, $C_{2-4}$ alkenyl, $C_{2-4}$ alkynyl, $-SO_2NH_2$, $-NHSO_2NH_2$, $-NHCONH_2$, $-NHCOC_{1-4}$ alkyl, $-CONHC_{1-4}$ alkyl, $-NHSO_2C_{1-4}$ alkyl, $-SO_2NHC_{1-4}$ alkyl, $SO_2C_{1-4}$ alkyl, $SCF_3$, $SF_5$, or $C_{1-4}$ haloalkyl, or two adjacent R, or R and $R_{X1}$ together with the atoms to which they are attached form $C_{3-8}$ cycloalkyl, 5- to 10-membered heterocycloalkyl containing 1-3 heteroatoms selected from N, S, O, P and B, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$ or $-C(O)C_{1-4}$ alkyl; in some embodiments, each R is independently H, halogen, $C_{1-4}$ alkyl, $C_{2-4}$ alkenyl, $C_{2-4}$ alkynyl, $-SO_2NH_2$, $-NHSO_2NH_2$, $-NHSO_2C_{1-4}$ alkyl, $-SO_2NHC_{1-4}$ alkyl, $SO_2C_{1-4}$ alkyl, $SCF_3$, $SF_5$, or $C_{1-4}$ haloalkyl, or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; in some embodiments, each R is independently H, halogen, $C_{1-4}$ alkyl, $C_{2-4}$ alkynyl, $-SO_2NH_2$, $-NHSO_2C_{1-4}$ alkyl, $-SO_2NHC_{1-4}$ alkyl, $SO_2C_{1-4}$ alkyl, $SCF_3$, $SF_5$, or $C_{1-4}$ haloalkyl, or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; in some embodiments, each R is independently H, F, Cl, Br, I, methyl, ethyl, propyl, methenyl, ethynyl, propynyl, $-SO_2NH_2$, $-NHSO_2C_{1-4}$ alkyl (such as

methyl, ethyl, or propyl), $-SO_2NHC_{1-4}$ alkyl (such as methyl, ethyl, or propyl), $SO_2C_{1-4}$ alkyl (such as methyl, ethyl, or propyl), $SCF_3$, $SF_5$, or $C_{1-4}$ haloalkyl (such as methyl, ethyl, or propyl), or two adjacent R, or R and $R_{X1}$ together with the atoms to which they are attached form cyclobutenyl, cyclopentenyl, cyclohexenyl, cyclopentadienyl, cyclohexadienyl, etc., which are optionally substituted with 1-3 groups selected from halogen, =O, CN, $C_{1-4}$ alkyl, $C_{1-4}$ alkoxy, OH, and $NH_2$, or - $C(O)C_{1-4}$ alkyl;

$R_1$ is H, $C_{1-4}$ alkyl, $C_{1-4}$ haloalkyl, or $C_{3-6}$ cycloalkyl; in some embodiments, $R_1$ is H, $C_{1-4}$ alkyl, or $C_{1-4}$ haloalkyl; in some embodiments, $R_1$ is H, methyl, ethyl, propyl, isopropyl, or halogenated methyl, ethyl, propyl, or isopropyl, wherein the halogen includes F, Cl, Br, and I;

$R_2$ is H, $C_{1-4}$ alkyl, $C_{1-4}$ haloalkyl, $C_{1-4}$ alkoxy, $-NHC_{1-4}$ alkyl, or $C_{3-6}$ cycloalkyl; in some embodiments, $R_2$ is H, $C_{1-4}$ alkyl, $C_{1-4}$ haloalkyl, or $C_{1-4}$ alkoxy; in some embodiments, $R_2$ is H, $C_{1-4}$ alkyl, or $C_{1-4}$ haloalkyl; in some embodiments, $R_2$ is H, methyl, ethyl, or propyl;

$R_3$ is H, halogen, =O, CN, $C_{1-4}$ alkyl, or $C_{1-4}$ alkoxy; in some embodiments, $R_3$ is H, F, Cl, =O, CN, $C_{1-3}$ alkyl, or $C_{1-3}$ alkoxy; in some embodiments, $R_3$ is H, halogen, =O, CN, or $C_{1-4}$ alkyl; in some embodiments, $R_3$ is H, halogen, =O, CN, methyl, ethyl, or propyl; in some embodiments, $R_3$ is H, F, Cl, =O, CN, or $C_{1-3}$ alkyl;

n is 0, 1, 2, 3, or 4; in some embodiments, n is 0, 1, 2, or 3; in some embodiments, n is 0, 1, or 2;

m is 0, 1, 2, or 3; in some embodiments, m is 0, 1, or 2; in some embodiments, n is 0 or 1;

provided that

the compound is not

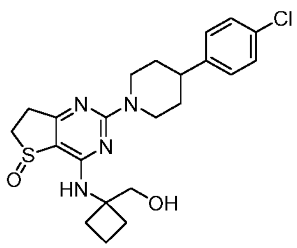

or

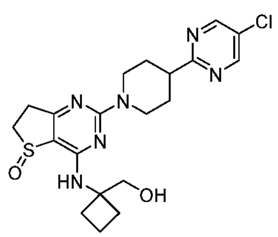

.

Specific solution 1 provided by the present invention relates to a compound represented by formula I or a stereoisomer or pharmaceutically acceptable salt thereof,

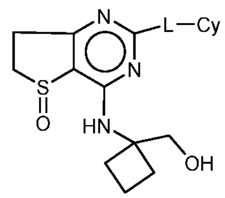

I

wherein Cy is selected from Cy1, Cy2, a 5-membered monocyclic heteroaryl, or an 8- to 10-membered bicyclic heteroaryl, wherein the monocyclic heteroaryl and the bicyclic heteroaryl are optionally further substituted with 1-3 R; furthermore, Cy is selected from Cy1 or Cy2;

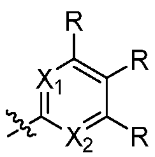

Cy1 ,

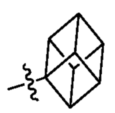

Cy2 ;

Cy2 is optionally substituted with 1-3 R;

L is $-(L_1)n-(L_2)m-$;

$L_1$ is $-NR_1-$, $-CR_2=N-$, $-CR_2=CR_2-$, or $-CR_2R_2-$;

$L_2$ is 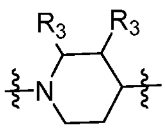, 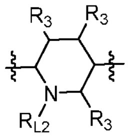, 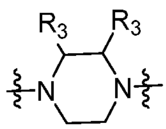, 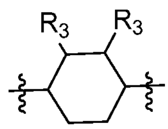, 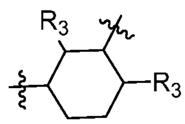,

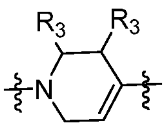, 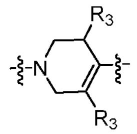, 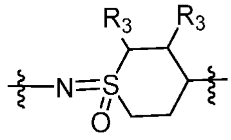, 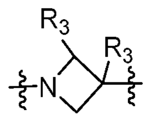,

$C_{1-4}$ alkylene, $C_{2-4}$ alkenylene, $C_{2-4}$ alkynylene, CO, O, $NR_{L2}$, $C_{3-5}$ cycloalkyl, 6- to 9-membered aryl, 5- or 6-membered heteroaryl containing 1-3 heteroatoms selected from N, S and O, 5-membered monoheterocyclic alkyl containing 1-3 heteroatoms selected from N, S and O, 7- or 8-membered monoheterocyclic alkyl containing 1-3 heteroatoms selected from N, S and O, 5- to 10-membered fused-heterocyclic alkyl containing 1-3 heteroatoms selected from N, S and O, 6- to 10-membered bridged-heterocyclic alkyl containing 1-3 heteroatoms selected from N, S and O, or 7- to 12-membered spiro-heterocyclic alkyl containing 1-3 heteroatoms selected from N, S and O, wherein the alkylene, alkenylene, alkynylene, cycloalkyl, aryl, heteroaryl, monoheterocyclic alkyl, fused-heterocyclic alkyl, bridged-heterocyclic alkyl, and spiro-heterocyclic alkyl are optionally substituted with 1-3 groups selected from halogen, =O, CN, $C_{1-4}$ alkyl, $C_{1-4}$ alkoxy, OH, and $NH_2$;

$R_{L2}$ is H, $C_{1-4}$ alkyl, or $C_{3-6}$ cycloalkyl;

$X_1$ and $X_2$ are independently $CR_{X1}$ or N;

each $R_{X1}$ is independently H, halogen, $C_{1-4}$ alkyl, $C_{1-4}$ haloalkyl, $C_{2-4}$ alkenyl, $C_{2-4}$ alkynyl, or $-SO_2NH_2$, or $R_{X1}$ and $R_1$, $R_{X1}$ and $R_2$, or $R_{X1}$ and $R_3$, together with the atoms to which they are attached, form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

each R is independently H, halogen, cyano, $C_{1-4}$ alkyl, $C_{2-4}$ alkenyl, $C_{2-4}$ alkynyl, $C_{3-8}$ cycloalkyl, 4- to 7-membered heterocycloalkyl containing 1-3 heteroatoms selected from N, S, O, Si and P, $-SO_2NH_2$, $-NHSO_2NH_2$, $-NHCONH_2$, $-NHCOC_{1-4}$ alkyl, $-CONHC_{1-4}$ alkyl, $-NHSO_2C_{1-4}$ alkyl, $-SO_2NHC_{1-4}$ alkyl, $SO_2C_{1-4}$ alkyl, $-P(O)(C_{1-4}$ alkyl$)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, $-C(O)C_{1-4}$ alkyl, and $NH_2$; or two adjacent R, or R and $R_{X1}$ together with the atoms to which they are attached form $C_{3-8}$ cycloalkyl, 5- to 10-membered heterocycloalkyl containing 1-3 heteroatoms selected from N, S, O, P and B, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$, or $-C(O)C_{1-4}$ alkyl; or each R is independently H, halogen, $C_{1-4}$ alkyl, $C_{2-4}$ alkenyl, $C_{2-4}$ alkynyl, $-SO_2NH_2$, $-NHSO_2NH_2$, $-NHCONH_2$, $-NHCOC_{1-4}$ alkyl, $-CONHC_{1-4}$ alkyl, $-NHSO_2C_{1-4}$ alkyl, $-SO_2NHC_{1-4}$ alkyl, $SO_2C_{1-4}$ alkyl, $SCF_3$, $SF_5$, or $C_{1-4}$ haloalkyl, or two adjacent R, or R and $R_{X1}$ together with the atoms to which they are attached form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$, or $-C(O)C_{1-4}$ alkyl;

$R_1$ is H, $C_{1-4}$ alkyl, $C_{1-4}$ haloalkyl, or $C_{3-6}$ cycloalkyl;

$R_2$ is H, $C_{1-4}$ alkyl, $C_{1-4}$ haloalkyl, $C_{1-4}$ alkoxy, $-NHC_{1-4}$ alkyl, or $C_{3-6}$ cycloalkyl;

$R_3$ is H, halogen, =O, CN, $C_{1-4}$ alkyl, or $C_{1-4}$ alkoxy;

n is 0, 1, 2, 3, or 4;

m is 0, 1, 2, or 3;

provided that

the compound is not

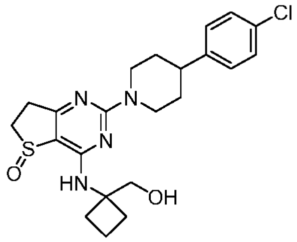

or

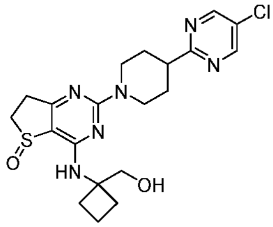

.

Furthermore, A compound as represented by formula (I) or a stereoisomer or pharmaceutically acceptable salt thereof,

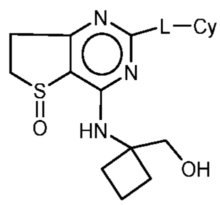

I

wherein Cy is selected from Cy1 or Cy2;

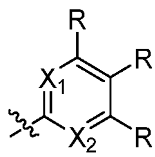

Cy1 ,

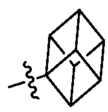

Cy2 ;

Cy2 is optionally substituted with 1-3 R;

L is $-(L_1)n-(L_2)m-$;

$L_1$ is $-NR_1-$, $-CR_2=N-$, $-CR_2=CR_2-$, or $-CR_2R_2-$;

$L_2$ is 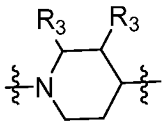 , 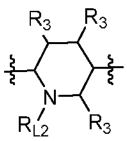 , 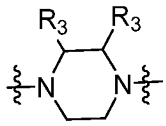 , 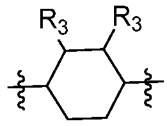 , 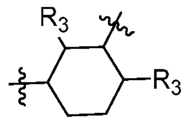 ,

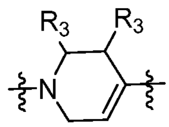 , 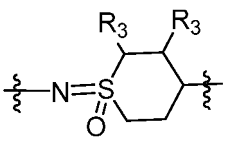 , 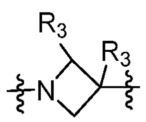 ,

$C_{1-4}$ alkylene, $C_{2-4}$ alkenylene, $C_{2-4}$ alkynylene, CO, O, $NR_{L2}$, $C_{3-5}$ cycloalkyl, 5- or 6-membered heteroaryl containing 1-3 heteroatoms selected from N, S and O, 5-membered monoheterocyclic alkyl containing 1-3 heteroatoms selected from N, S and O, 7- or 8-membered monoheterocyclic alkyl containing 1-3 heteroatoms selected from N, S and O, 5- to 10-membered fused-heterocyclic alkyl containing 1-3 heteroatoms selected from N, S and O, 6- to 10-membered bridged-heterocyclic alkyl containing 1-3 heteroatoms selected from N, S and O, or 7- to 12-membered spiro-heterocyclic alkyl containing 1-3 heteroatoms selected from N, S and O, wherein the alkylene, alkenylene, alkynylene, cycloalkyl, heteroaryl, monoheterocyclic alkyl, fused-heterocyclic alkyl, bridged-heterocyclic alkyl, and spiro-heterocyclic alkyl are optionally substituted with 1-3 groups selected from halogen, =O, CN, $C_{1-4}$ alkyl, $C_{1-4}$ alkoxy, OH, and $NH_2$; or $L_2$ is

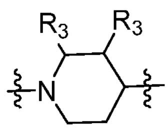, 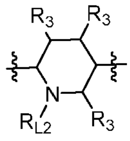, 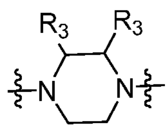,

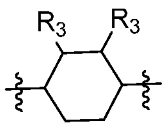, 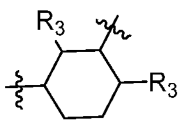, 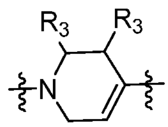, 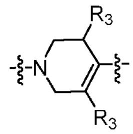, 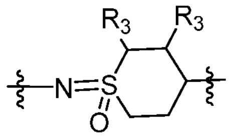,

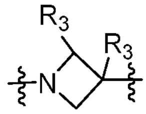,

$C_{1-4}$ alkylene, $C_{2-4}$ alkenylene, $C_{2-4}$ alkynylene, CO, O, $NR_{L2}$, $C_{3-5}$ cycloalkyl, 5- or 6-membered heteroaryl containing 1-3 heteroatoms selected from N, S and O, 5-membered monoheterocyclic alkyl containing 1-3 heteroatoms selected from N, S and O, 7- or 8-membered monoheterocyclic alkyl containing 1-3 heteroatoms selected from N, S and O, 5- to 10-membered fused-heterocyclic alkyl containing 1-3 heteroatoms selected from N, S and O, 6- to 10-membered bridged-heterocyclic alkyl containing 1-3 heteroatoms selected from N, S and O, or 7- to 12-membered spiro-heterocyclic alkyl containing 1-3 heteroatoms selected from N, S and O, wherein the alkylene, alkenylene, alkynylene, cycloalkyl, heteroaryl, monoheterocyclic alkyl, fused-heterocyclic alkyl, bridged-heterocyclic alkyl, and spiro-heterocyclic alkyl are optionally substituted with 1-3 groups selected from halogen, =O, CN, $C_{1-4}$ alkyl, $C_{1-4}$ alkoxy, OH, and $NH_2$;
$R_{L2}$ is H, $C_{1-4}$ alkyl, or $C_{3-6}$ cycloalkyl;
$X_1$ and $X_2$ are independently $CR_{X1}$ or N;
each $R_{X1}$ is independently H, halogen, $C_{1-4}$ alkyl, $C_{1-4}$ haloalkyl, $C_{2-4}$ alkenyl, $C_{2-4}$ alkynyl, or $-SO_2NH_2$, or $R_{X1}$ and $R_1$, $R_{X1}$ and $R_2$, or $R_{X1}$ and $R_3$, together with the atoms to which they are attached, form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;
each R is independently H, halogen, $C_{1-4}$ alkyl, $C_{2-4}$ alkenyl, $C_{2-4}$ alkynyl, - $SO_2NH_2$, $-NHSO_2NH_2$, $-NHCONH_2$, $-NHCOC_{1-4}$ alkyl, $-CONHC_{1-4}$ alkyl, - $NHSO_2C_{1-4}$ alkyl, $-SO_2NHC_{1-4}$ alkyl, $SO_2C_{1-4}$ alkyl, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, $-C(O)C_{1-4}$ alkyl, and $NH_2$; or two adjacent R, or R and $R_{X1}$ together with the atoms to which they are attached form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$, or - $C(O)C_{1-4}$ alkyl;
$R_1$ is H, $C_{1-4}$ alkyl, $C_{1-4}$ haloalkyl, or $C_{3-6}$ cycloalkyl;
$R_2$ is H, $C_{1-4}$ alkyl, $C_{1-4}$ haloalkyl, $C_{1-4}$ alkoxy, $-NHC_{1-4}$ alkyl, or $C_{3-6}$ cycloalkyl;
$R_3$ is H, halogen, =O, CN, $C_{1-4}$ alkyl, or $C_{1-4}$ alkoxy;
n is 0, 1, 2, 3, or 4;
m is 0, 1, 2, or 3;
provided that
the compound is not

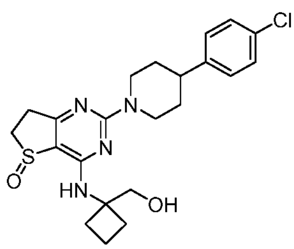 or 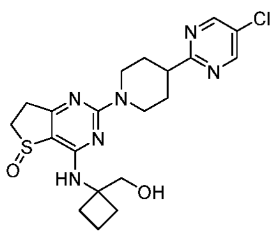.

Specific solution 2 of the present invention relates to the compound or the stereoisomer or pharmaceutically acceptable salt thereof according to solution 1,

wherein each $L_2$ is independently selected from CO, O, $NR_{L2}$, $C_{1-4}$ alkylene, $C_{2-4}$ alkenylene, $C_{2-4}$ alkynylene,

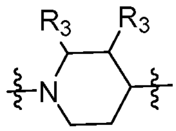, 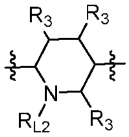, 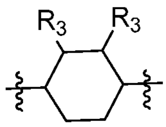,

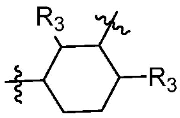, 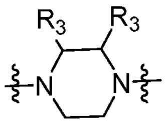, 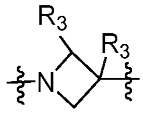,

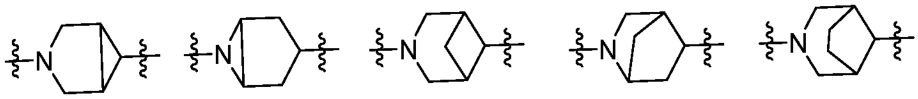,

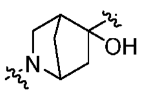, 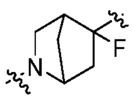,

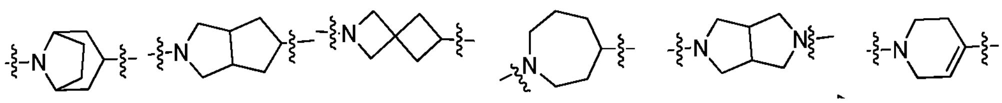

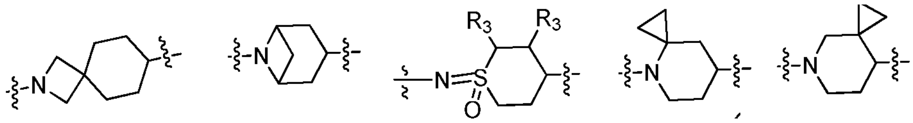

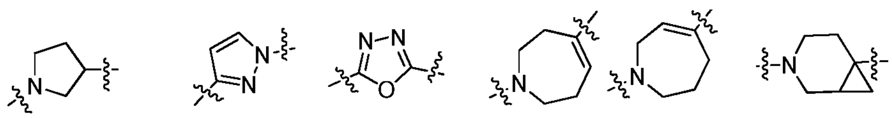

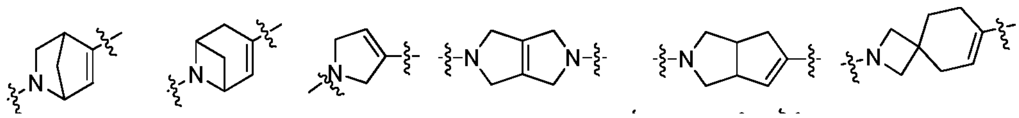

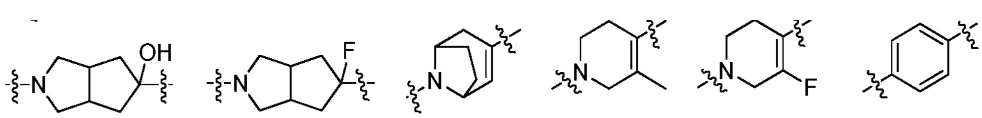,

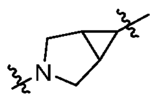, 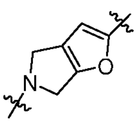, 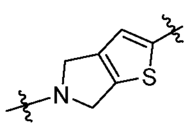, 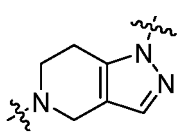, 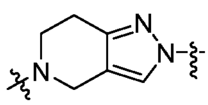;

or each $L_2$ is independently selected from CO, O, $NR_{L2}$, $C_{1-4}$ alkylene, $C_{2-4}$ alkenylene, $C_{2-4}$ alkynylene,

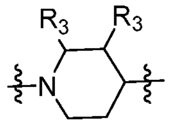, 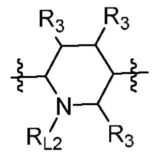, 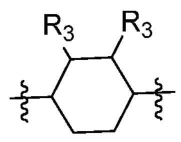,

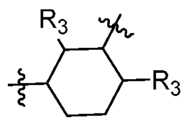, 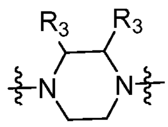, 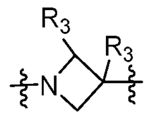,

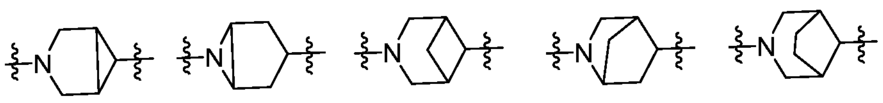

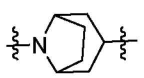 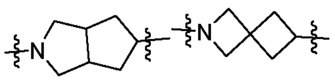 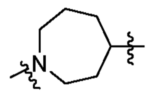 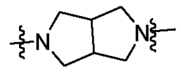 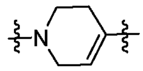

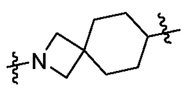 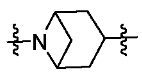 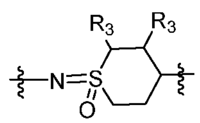 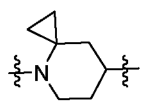 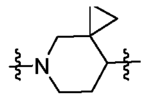 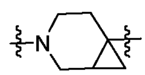

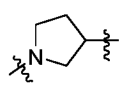 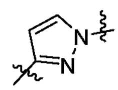 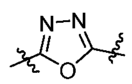;

or each $L_2$ is independently selected from CO, O, $NR_{L2}$, $C_{1-4}$ alkylene, $C_{2-4}$ alkenylene, $C_{2-4}$ alkynylene,

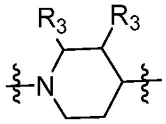, 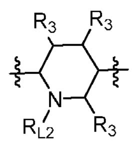, 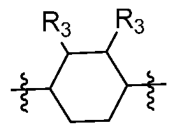, 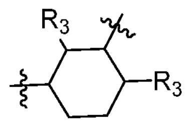,

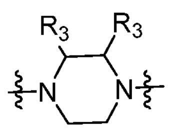, 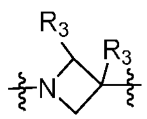,

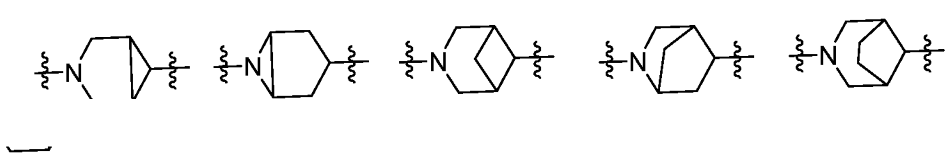

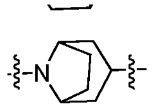 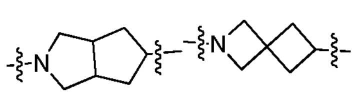 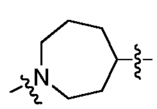 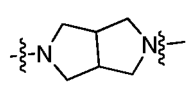 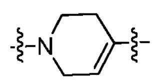

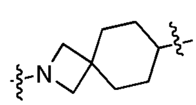 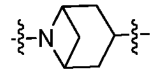 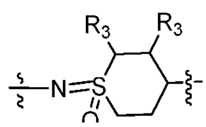 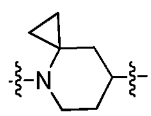 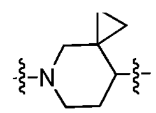

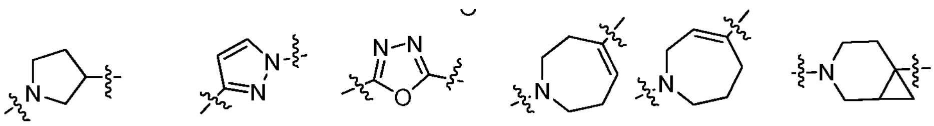

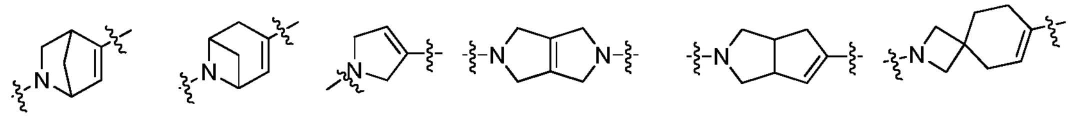

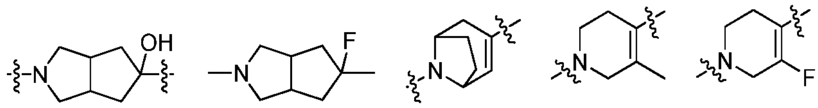

;

or each $L_2$ is independently selected from CO, O, $NR_{L2}$, $C_{1-4}$ alkylene, $C_{2-4}$ alkenylene, $C_{2-4}$ alkynylene,

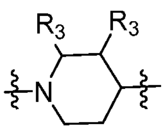, 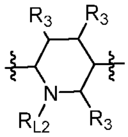, 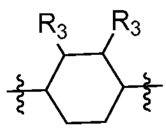, 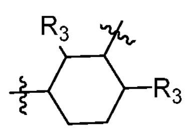,

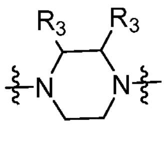,

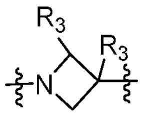,

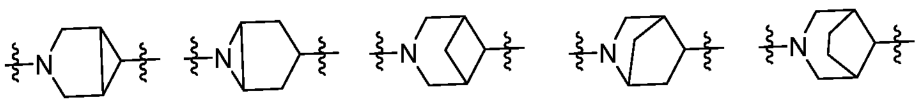,

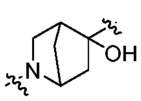, 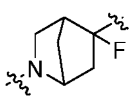,

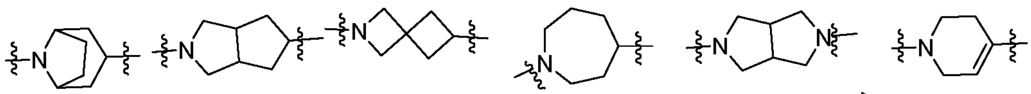

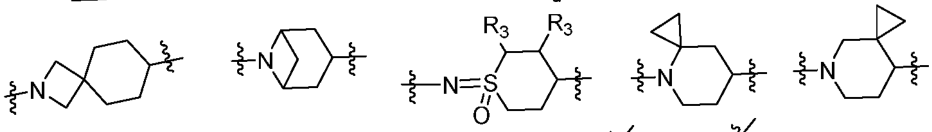

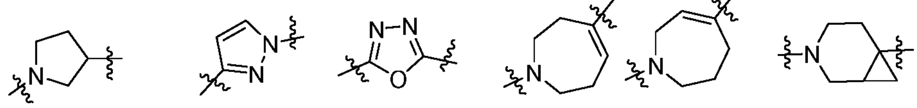

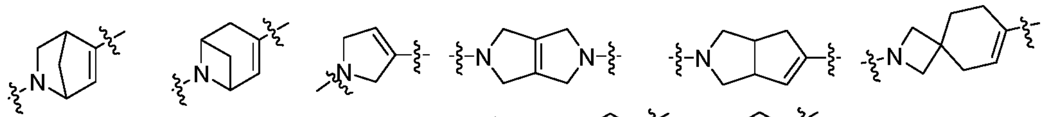

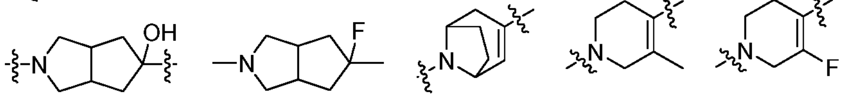

;

or each $L_2$ is independently selected from CO, O, $NR_{L2}$, $C_{1-4}$ alkylene, $C_{2-4}$ alkenylene, $C_{2-4}$ alkynylene,

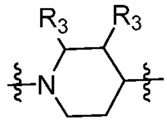, 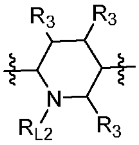, 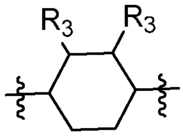,

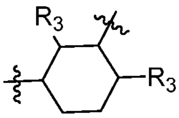, 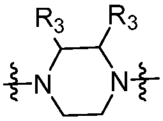, 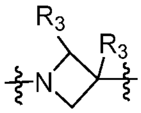,

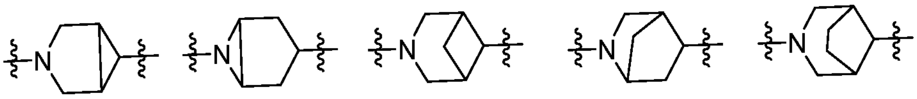,

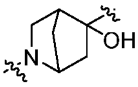, 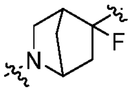,

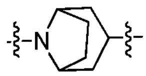 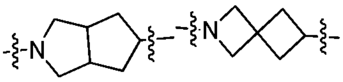 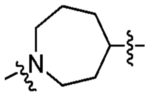 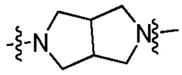 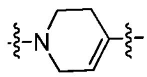

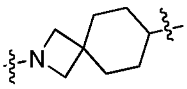 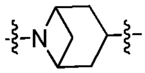 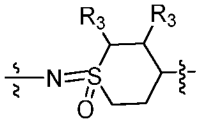 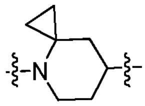 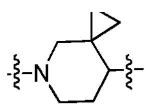

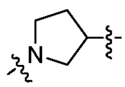 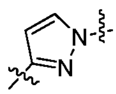 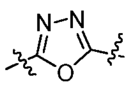 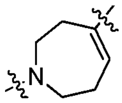 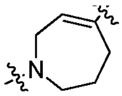 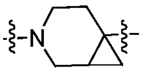

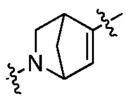 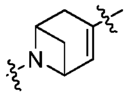 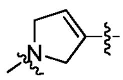 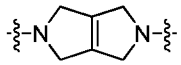 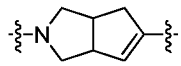 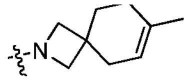

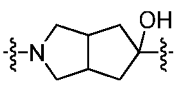 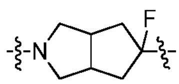 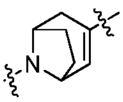 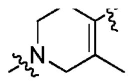 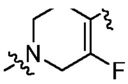 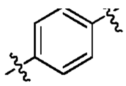,

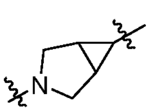, 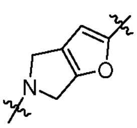, and 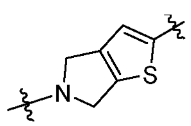;

$R_{L2}$ is H, $C_{1-4}$ alkyl, or $C_{3-6}$ cycloalkyl;

each $R_{X1}$ is independently H, halogen, $C_{1-4}$ alkyl, or $C_{1-4}$ haloalkyl, or $R_{X1}$ and $R_1$, $R_{X1}$ and $R_2$, or $R_{X1}$ and $R_3$ together with the atoms to which they are attached form $C_{3-6}$ cycloalkyl, 5- to 7-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

each R is independently H, halogen, cyano, $C_{1-4}$ alkyl, $C_{2-4}$ alkenyl, $C_{2-4}$ alkynyl, $C_{3-6}$ cycloalkyl, 4- to 6-membered heterocycloalkyl containing 1-3 heteroatoms selected from N, S, O, Si and P, $-SO_2NH_2$, $-NHSO_2NH_2$, $-NHSO_2C_{1-4}$ alkyl, $-SO_2NHC_{1-4}$ alkyl, $SO_2C_{1-4}$ alkyl, $-P(O)(C_{1-2}\ alkyl)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; or each R is independently H, halogen, $C_{1-4}$ alkyl, $C_{2-4}$ alkenyl, $C_{2-4}$ alkynyl, - $SO_2NH_2$, $-NHSO_2NH_2$, $-NHSO_2C_{1-4}$ alkyl, $-SO_2NHC_{1-4}$ alkyl, $SO_2C_{1-4}$ alkyl, $SCF_3$, $SF_5$, or $C_{1-4}$ haloalkyl, or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form 5- to 10-membered heterocycloalkyl containing 1-3 heteroatoms selected from N, S, O, P and B, wherein the heterocycloalkyl is optionally substituted with 1-3 groups selected from halogen, =O, CN, $C_{1-4}$ alkyl, $C_{1-4}$ alkoxy, OH, and $NH_2$;
$R_1$ is H, $C_{1-4}$ alkyl, or $C_{1-4}$ haloalkyl;
$R_2$ is H, $C_{1-4}$ alkyl, $C_{1-4}$ haloalkyl, or $C_{1-4}$ alkoxy;
$R_3$ is H, halogen, =O, CN, or $C_{1-4}$ alkyl;
n is 0, 1, 2, 3, or 4;
m is 0, 1, or 2.

Solution 3 of the present invention relates to the compound or the stereoisomer or pharmaceutically acceptable salt thereof as in solution 1 or 2, wherein the compound has the structure of the following formula II,

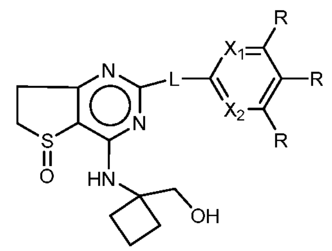

II

wherein L is selected from one of the following structures:

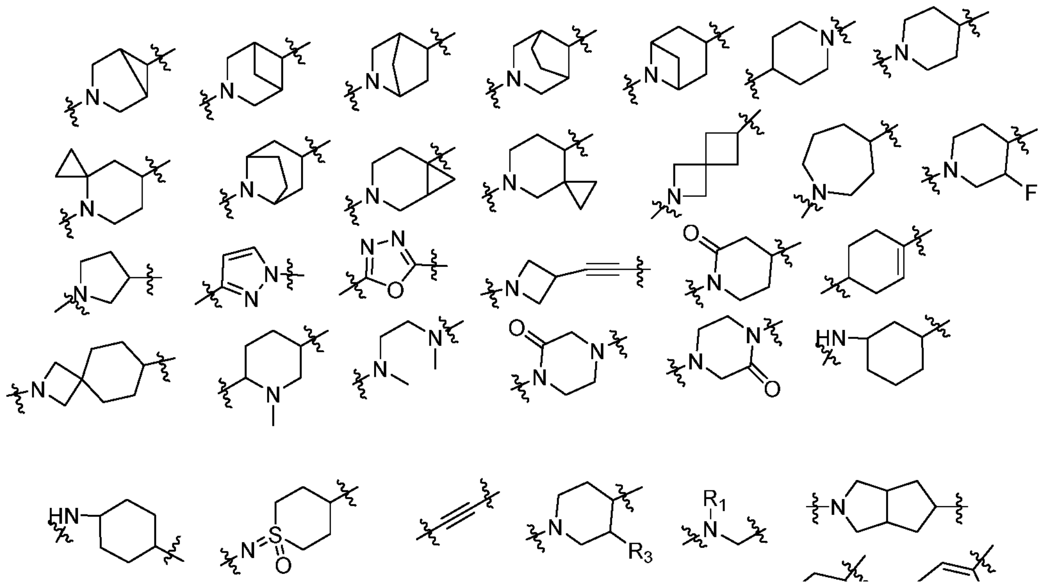

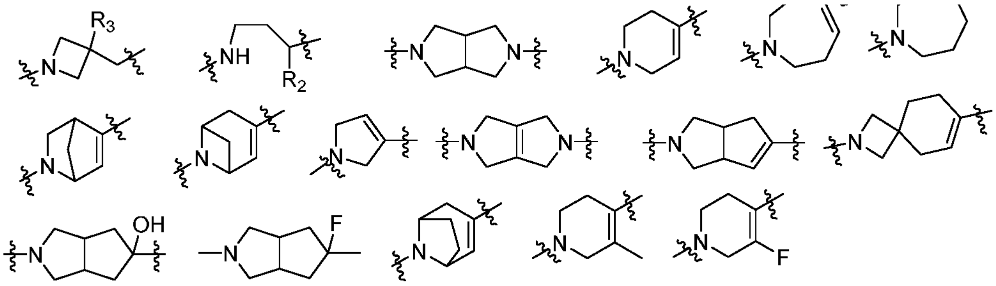

,

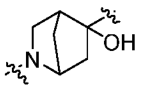, 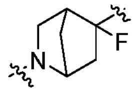, 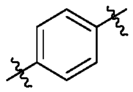, 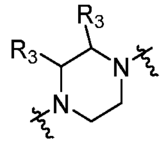, 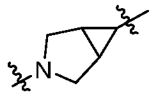, 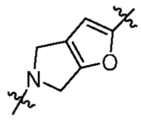,

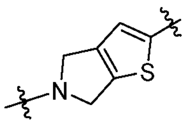, 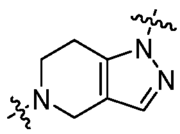, 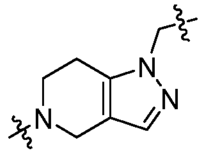, and 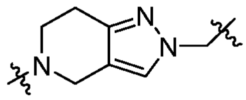;

or L is selected from one of the following structures:

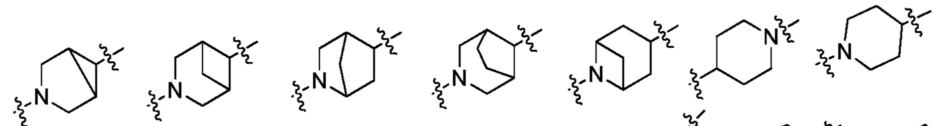

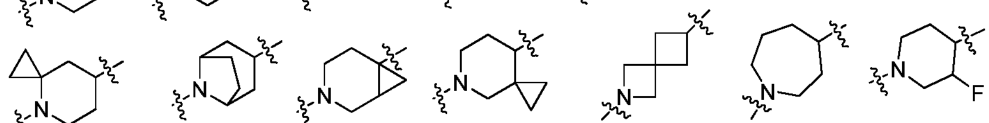

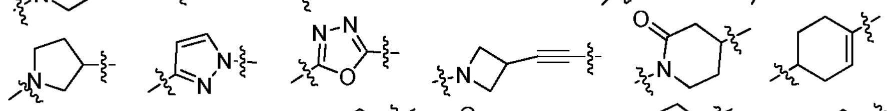

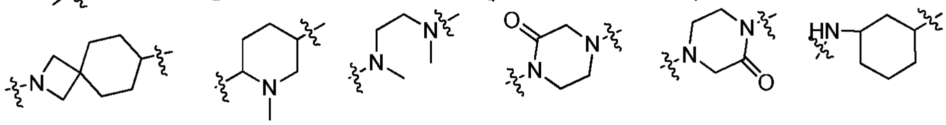

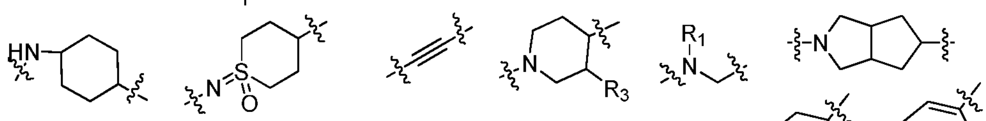

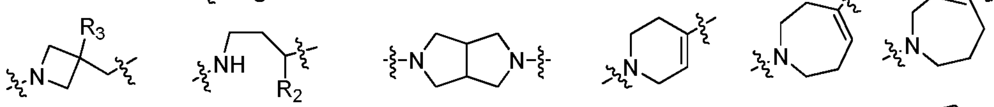

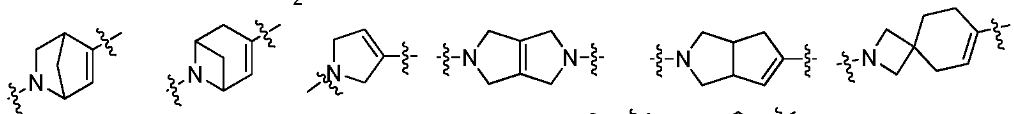

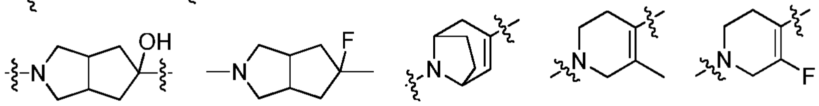

,

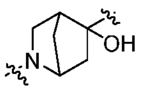, 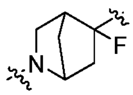, 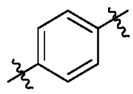, 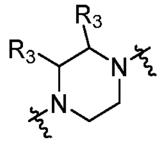, 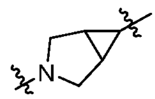, 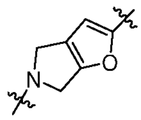,

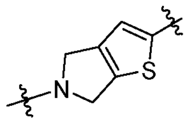, and 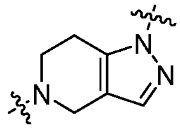;

or L is selected from one of the following structures:

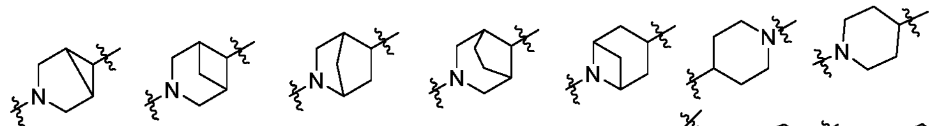
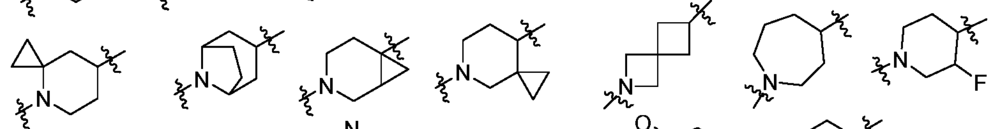
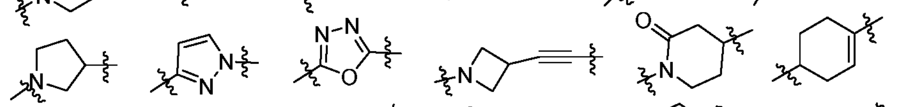
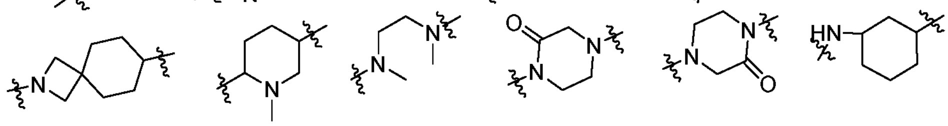
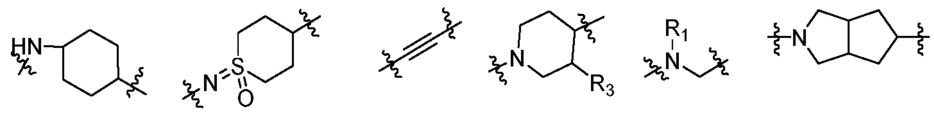
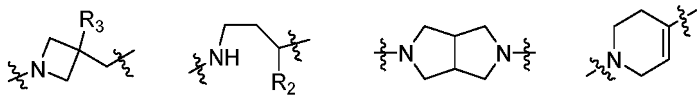;

or L is selected from one of the following structures:

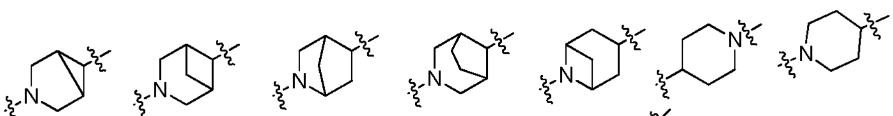
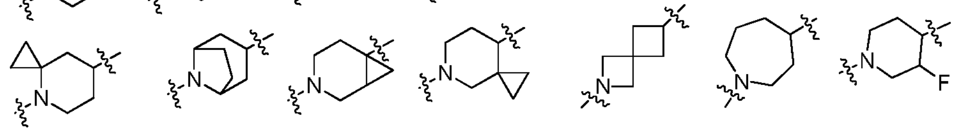
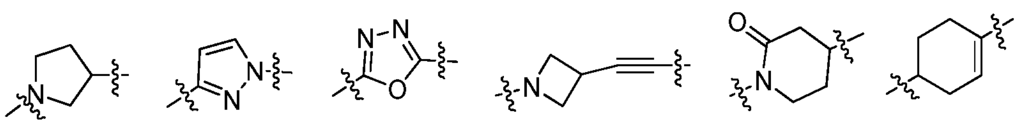
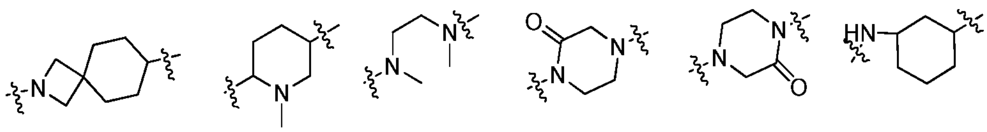

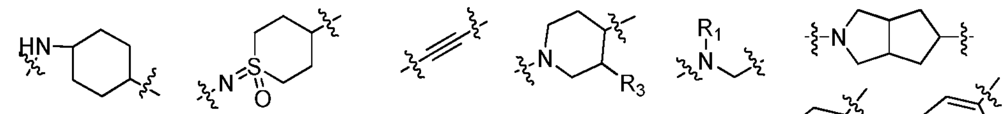

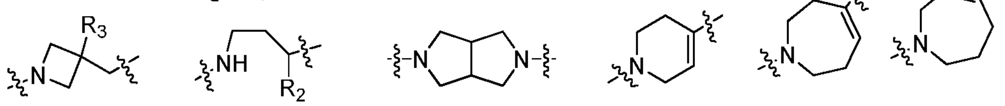

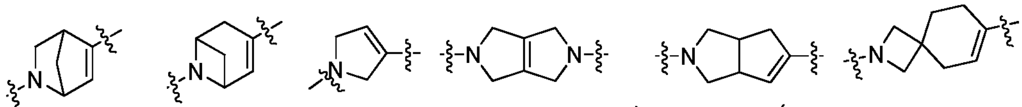

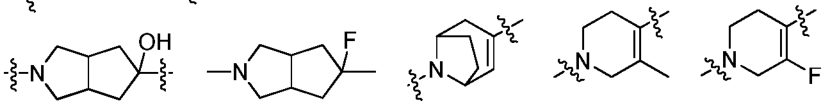

;

or L is selected from one of the following structures:

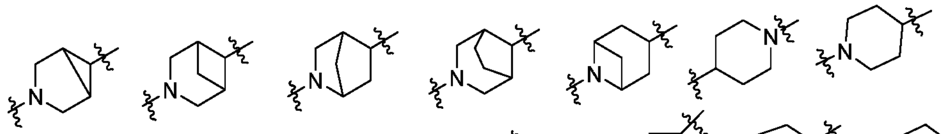

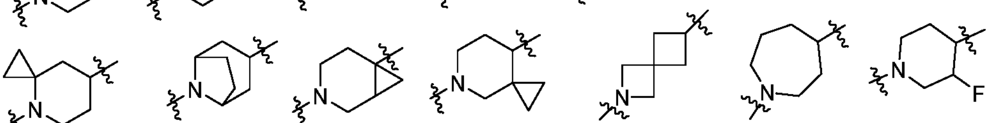

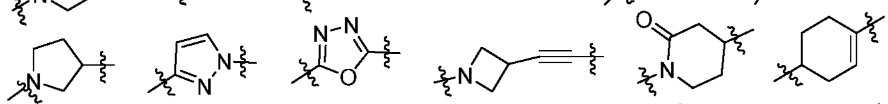

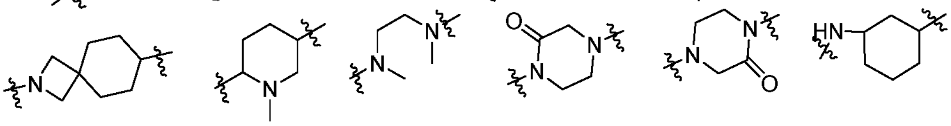

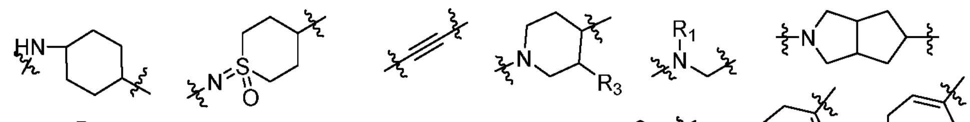

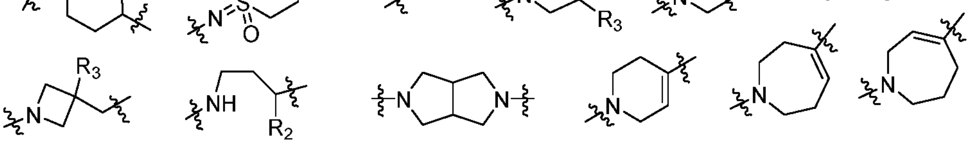

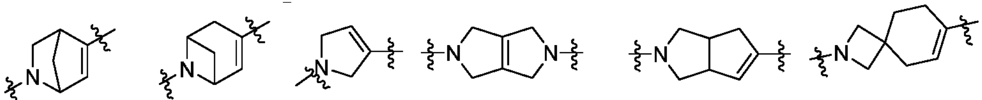

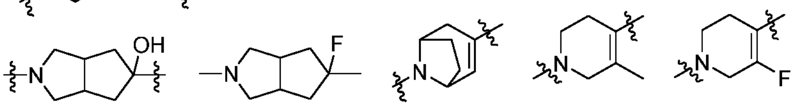

,

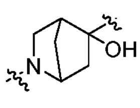, and 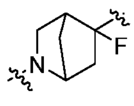;

or L is selected from one of the following structures:

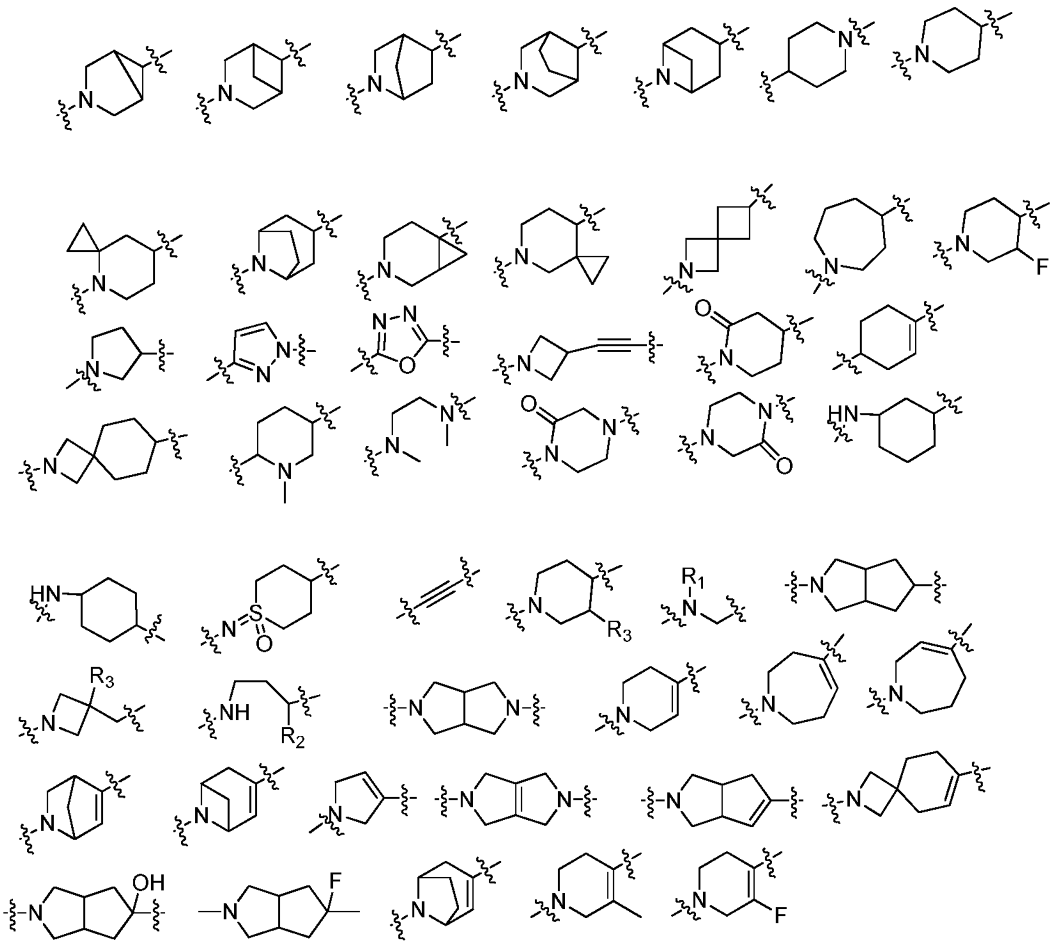

,

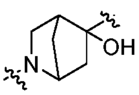 , 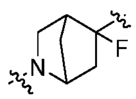 , 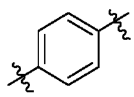 , 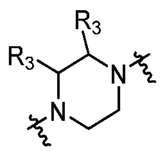 , 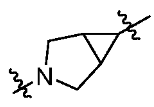 , 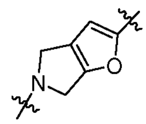 ,

and

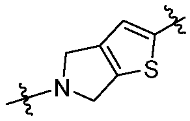 ;

$X_1$ and $X_2$ are independently $CR_{X1}$ or N;

each $R_{X1}$ is independently H, halogen, $C_{1-4}$ alkyl, or $C_{1-4}$ haloalkyl, or $R_{X1}$ and $R_1$, $R_{X1}$ and $R_2$, or $R_{X1}$ and $R_3$ together with the atoms to which they are attached form $C_{3-6}$ cycloalkyl, 5- to 7-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

each R is independently H, halogen, cyano, $C_{1-4}$ alkyl, $C_{2-4}$ alkynyl, $C_{3-6}$ cycloalkyl, 4- to 6-membered heterocycloalkyl containing 1-3 heteroatoms selected from N, S, O, Si and P, $-SO_2NH_2$, $-NHSO_2C_{1-4}$ alkyl, $-SO_2NHC_{1-4}$ alkyl, $SO_2C_{1-4}$ alkyl, $-P(O)(CH_3)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; or each R is independently H, halogen, $C_{1-4}$ alkyl, $C_{2-4}$ alkynyl, $-SO_2NH_2$, $-NHSO_2C_{1-4}$ alkyl, $-SO_2NHC_{1-4}$ alkyl,

$SO_2C_{1-4}$ alkyl, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form $C_{3-6}$ cycloalkyl, wherein the cycloalkyl is optionally substituted with 1-3 groups selected from halogen, =O, CN, $C_{1-4}$ alkyl, $C_{1-4}$ alkoxy, OH, and $NH_2$; or two adjacent R, or R and $R_{X1}$ together with the atoms to which they are attached form 5- to 10-membered heterocycloalkyl containing 1-3 heteroatoms selected from N, S, O, P and B, wherein the heterocycloalkyl is optionally substituted with 1-3 groups selected from halogen, =O, CN, $C_{1-4}$ alkyl, $C_{1-4}$ alkoxy, OH, and $NH_2$;

$R_1$ is H, or $C_{1-4}$ alkyl, or $C_{1-4}$ haloalkyl;

$R_2$ is H, $C_{1-4}$ alkyl, or $C_{1-4}$ haloalkyl; and

$R_3$ is H, halogen, =O, CN, or $C_{1-4}$ alkyl.

Solution 4 of the present invention relates to the compound or the stereoisomer or pharmaceutically acceptable salt thereof according to solution 1 or 2, wherein

Cy is selected from 5-membered monocyclic heteroaryl, 5-membered heteroaryl fused benzene ring, 5-membered heteroaryl fused 5-membered heteroaryl, 5-membered heteroaryl fused 6-membered heteroaryl, 5-membered heteroaryl fused 4-membered cycloalkyl, 5-membered heteroaryl fused 5-membered cycloalkyl, 5-membered heteroaryl fused 6-membered cycloalkyl, 5-membered heteroaryl fused 4-membered heterocycloalkyl, 5-membered heteroaryl fused 5-membered heterocycloalkyl, 5-membered heteroaryl fused 6-membered heterocycloalkyl, 6-membered heteroaryl fused benzene ring, 6-membered heteroaryl fused 5-membered heteroaryl, 6-membered heteroaryl fused 6-membered heteroaryl, 6-membered heteroaryl fused 4-membered cycloalkyl, 6-membered heteroaryl fused 5-membered cycloalkyl, 6-membered heteroaryl fused 6-membered cycloalkyl, 6-membered heteroaryl fused 4-membered heterocycloalkyl, 6-membered heteroaryl fused 5-membered heterocycloalkyl, and 6-membered heteroaryl fused 6-membered heterocycloalkyl;

or Cy is selected from the following structures that are optionally further substituted with 1-3 R:

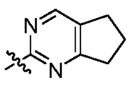 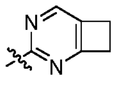 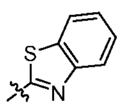 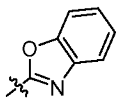 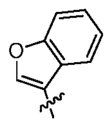 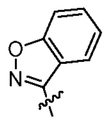 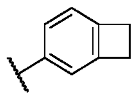 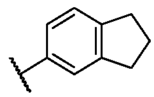

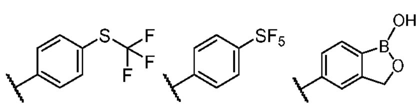 .

Furthermore, in the compound or the stereoisomer or pharmaceutically acceptable salt thereof according to solution 1 or 2,

Cy is selected from 5-membered monocyclic heteroaryl, 5-membered heteroaryl fused benzene ring, 5-membered heteroaryl fused 5-membered heteroaryl, 5-membered heteroaryl fused 6-membered heteroaryl, 5-membered heteroaryl fused 4-membered cycloalkyl, 5-membered heteroaryl fused 5-membered cycloalkyl, 5-membered heteroaryl fused 6-membered cycloalkyl, 5-membered heteroaryl fused 4-membered heterocycloalkyl, 5-membered heteroaryl fused 5-membered heterocycloalkyl, 5-membered heteroaryl fused 6-membered heterocycloalkyl, 6-membered heteroaryl fused benzene ring, 6-membered heteroaryl fused 5-membered heteroaryl, 6-membered heteroaryl fused 6-membered heteroaryl, 6-membered heteroaryl fused 4-membered cycloalkyl, 6-membered heteroaryl fused 5-membered cycloalkyl, 6-membered heteroaryl fused 6-membered cycloalkyl, 6-membered heteroaryl fused 4-membered heterocycloalkyl, 6-membered heteroaryl fused 5-membered heterocycloalkyl, and 6-membered heteroaryl fused 6-membered heterocycloalkyl;

or

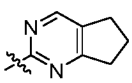 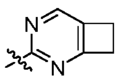 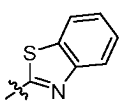 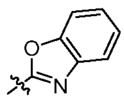 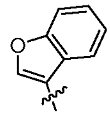 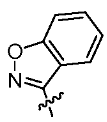 .

Solution 5 of the present invention relates to the compound or the stereoisomer or pharmaceutically acceptable salt thereof according to solution 3, wherein

L is selected from

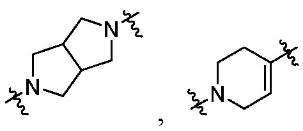, 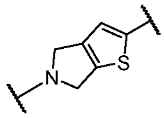, 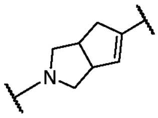,

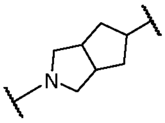, 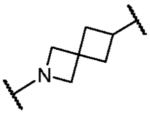, 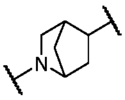, 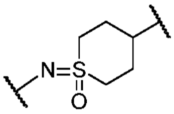, 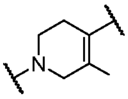, 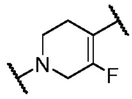,

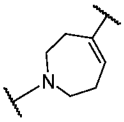, 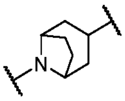, 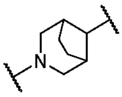, 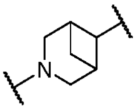, 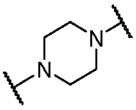, 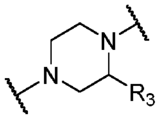, 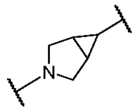,

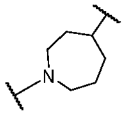, 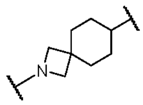, 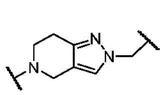, and 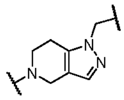;

$X_1$ and $X_2$ are independently $CR_{X1}$ or N;
each $R_{X1}$ is independently H, F, Cl, methyl, or ethyl, or $R_{X1}$ and $R_3$ together with the atoms to which they are attached form $C_5$ cycloalkyl, $C_6$ cycloalkyl, 5-membered heterocycloalkyl containing 1-3 heteroatoms selected from N, S and O, or 6-membered heterocycloalkyl containing 1-3 heteroatoms selected from N, S and O;
$R_3$ is H, F, Cl, =O, CN, or $C_{1-3}$ alkyl; and
each R is independently H, F, Cl, methyl, ethyl, acetylene, or propyne, or two adjacent R together with the atoms to which they are attached form cyclopropenyl, cyclobutenyl, cyclopentenyl, or cyclohexenyl, wherein the cyclopropenyl, cyclobutenyl, cyclopentenyl, and cyclohexenyl are optionally substituted with 1-3 groups selected from F, Cl, =O, CN, methyl, ethyl, methoxy, ethoxy, OH, and $NH_2$;
furthermore, in the compound or the stereoisomer or pharmaceutically acceptable salt thereof according to solution 3, L is selected from

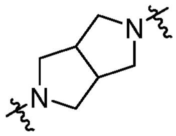,

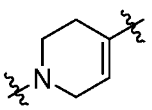, 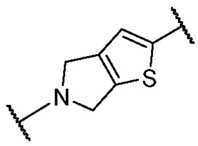, 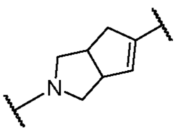, 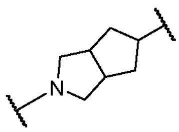, 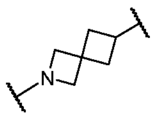, 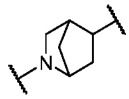,

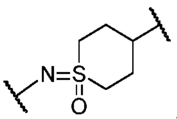, 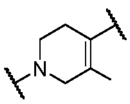, 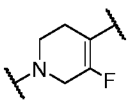, 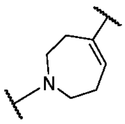, 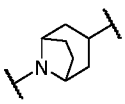, 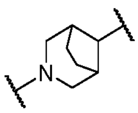, 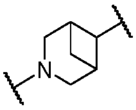,

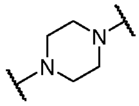, 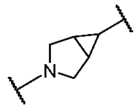, 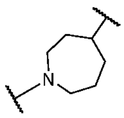, 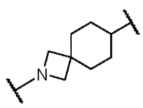, 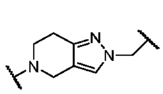, and 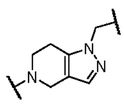;

$X_1$ and $X_2$ are independently $CR_{X1}$ or N;
each $R_{X1}$ is independently H, F, Cl, methyl, or ethyl; and
each R is independently H, F, Cl, methyl, ethyl, acetylene, or propyne, or two adjacent R together with the atoms to which they are attached form cyclopropenyl, cyclobutenyl, cyclopentenyl, or cyclohexenyl, wherein the cyclopropenyl, cyclobutenyl, cyclopentenyl, and cyclohexenyl are optionally substituted with 1-3 groups selected from F, Cl, =O, CN, methyl, ethyl, methoxy, ethoxy, OH, and $NH_2$.

Furthermore, in the compound or the stereoisomer or pharmaceutically acceptable salt thereof according to solution 3,

L is selected from

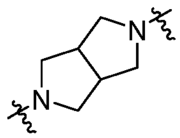 or 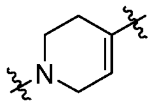 ;

$X_1$ and $X_2$ are independently $CR_{X1}$ or N;
each $R_{X1}$ is independently H, F, Cl, methyl, or ethyl; and
each R is independently H, F, Cl, methyl, ethyl, acetylene, or propyne, or two adjacent R together with the atoms to which they are attached form cyclopropenyl, cyclobutenyl, cyclopentenyl, or cyclohexenyl, wherein the cyclopropenyl, cyclobutenyl, cyclopentenyl, and cyclohexenyl are optionally substituted with 1-3 groups selected from F, Cl, =O, CN, methyl, ethyl, methoxy, ethoxy, OH, and $NH_2$.

Solution 6 of the present invention relates to the compound or the stereoisomer or pharmaceutically acceptable salt thereof according to solution 1, wherein

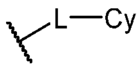

is selected from the following structures:

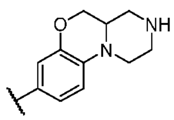 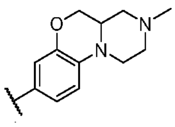 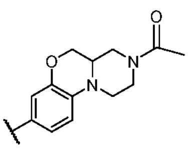 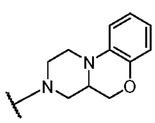 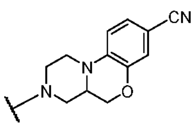 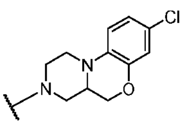

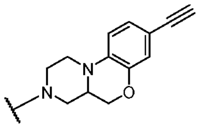 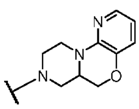 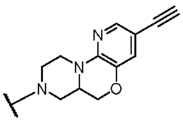 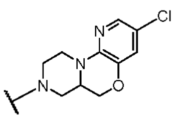 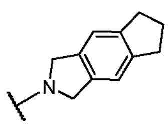 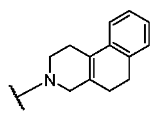

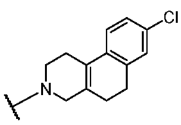 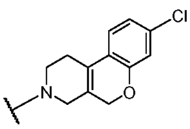 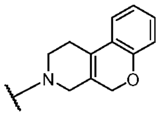 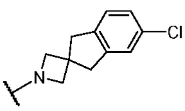 , and 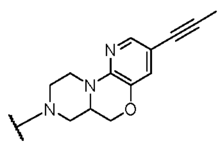 ,

or selected from

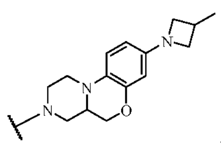 .

In solution 7 of the present invention, the compound of the present invention is selected from a compound with one of the structures in Table 1 below, or a stereoisomer or pharmaceutically acceptable salt thereof:

Table 1.

| | | | | |
|---|---|---|---|---|
| 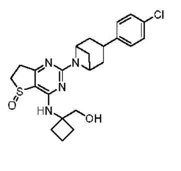 | 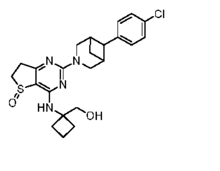 | 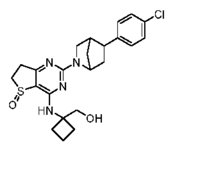 | 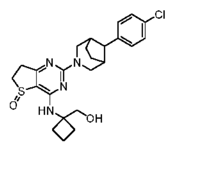 | 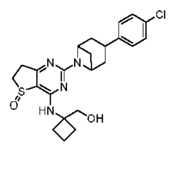 |
| 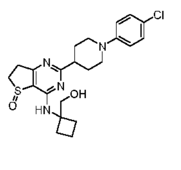 | 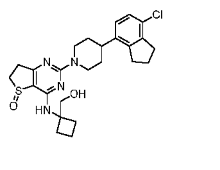 | 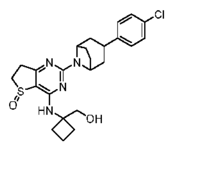 | 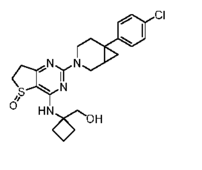 | 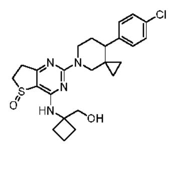 |
| 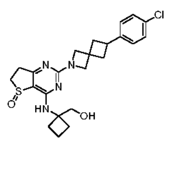 | 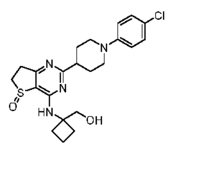 | 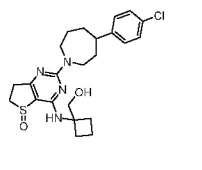 | 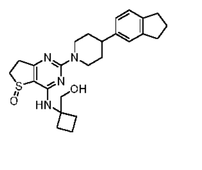 | 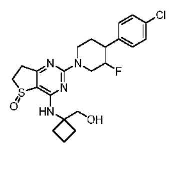 |
| 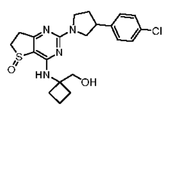 | 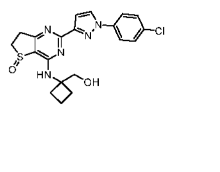 | 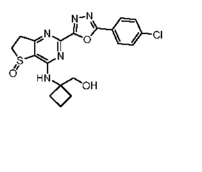 | 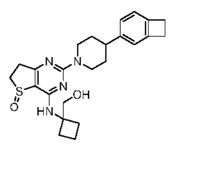 | 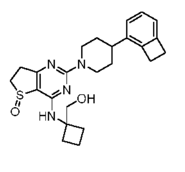 |
| 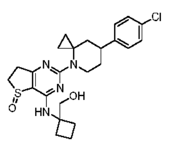 | 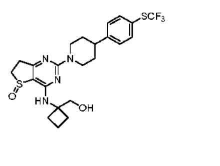 | 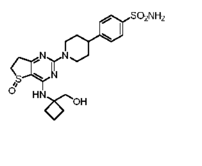 | 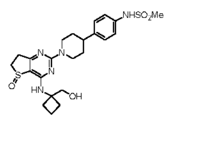 | 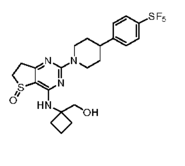 |
| 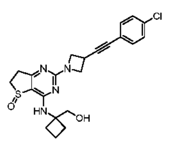 | 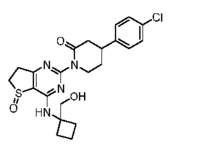 | 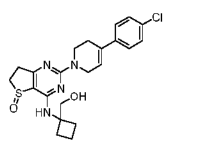 | 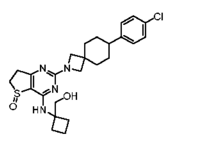 | 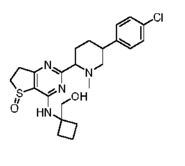 |
| 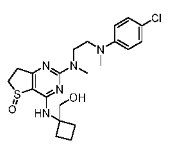 | 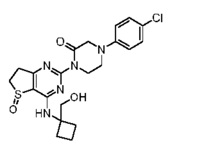 | 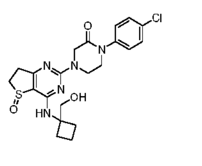 | 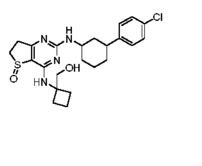 | 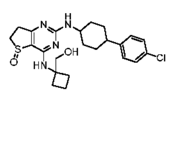 |

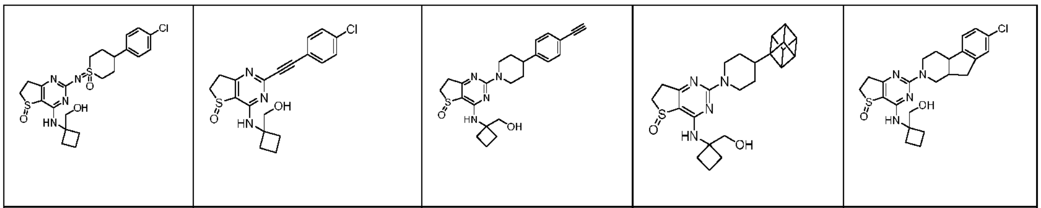

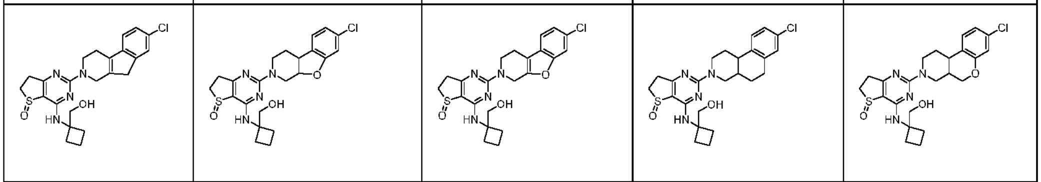

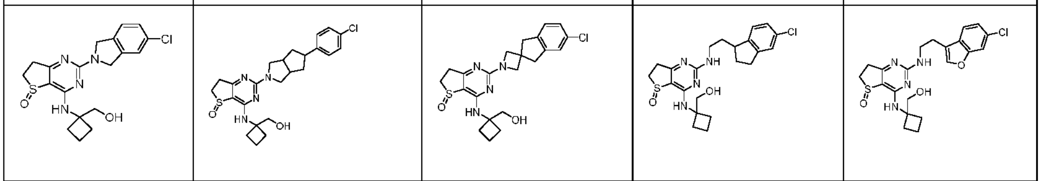

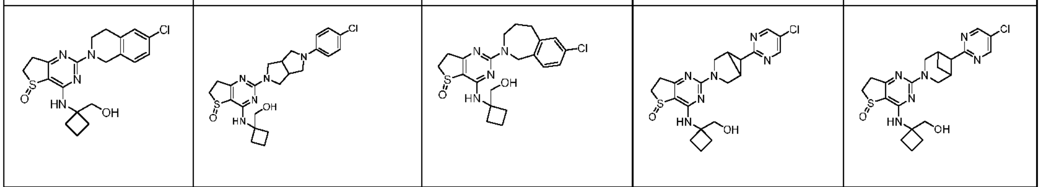

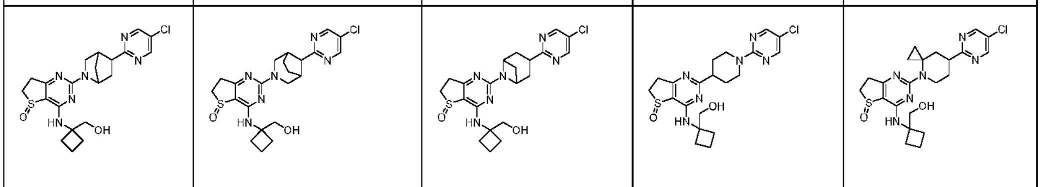

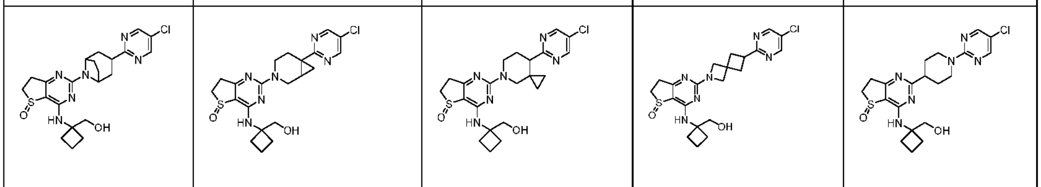

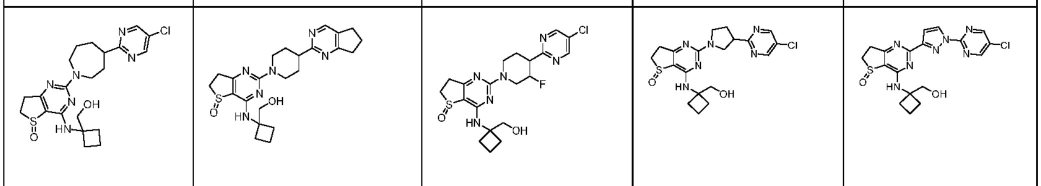

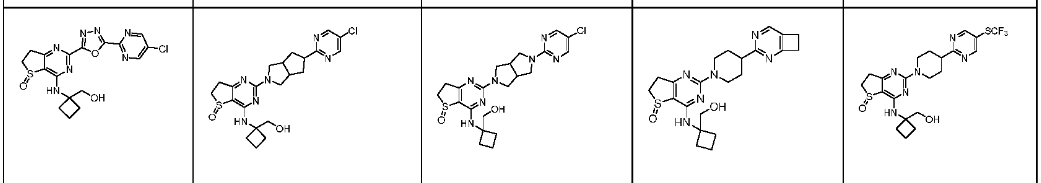

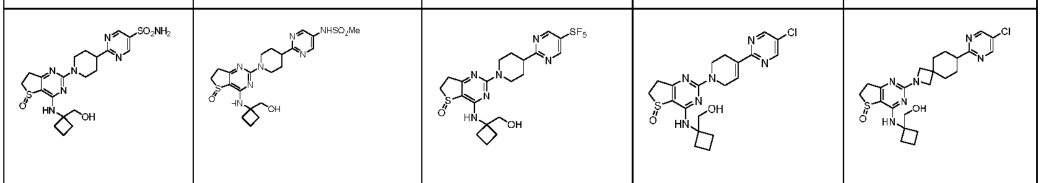

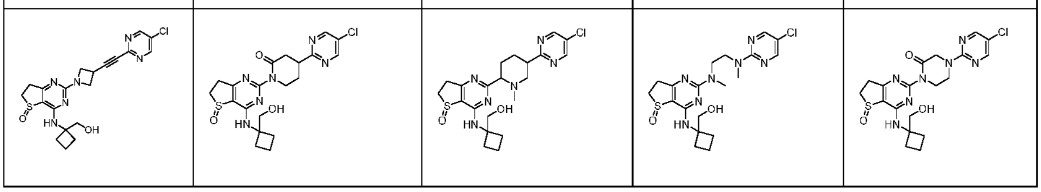

| | | | | |
|---|---|---|---|---|
| 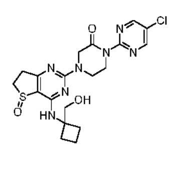 | 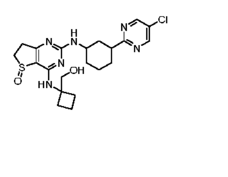 | 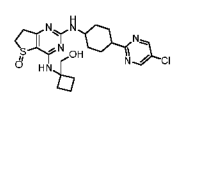 | 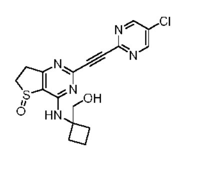 | 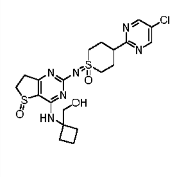 |
| 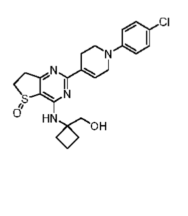 | 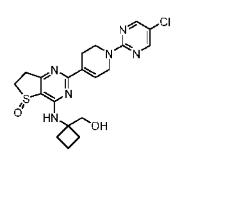 | 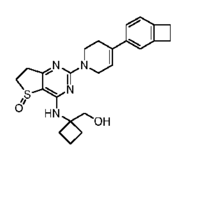 | 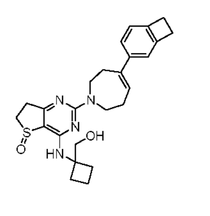 | 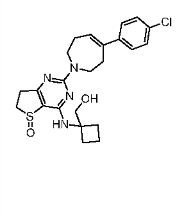 |
| 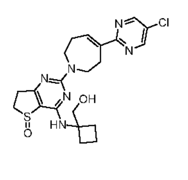 | 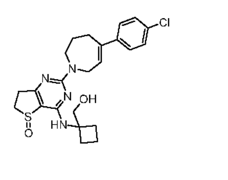 | 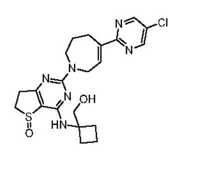 | 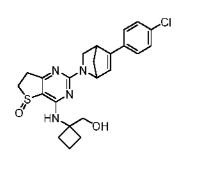 | 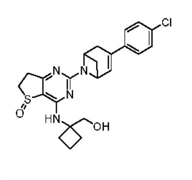 |
| 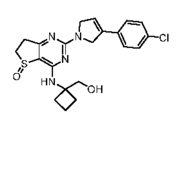 | 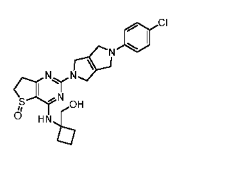 | 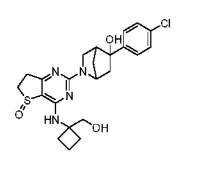 | 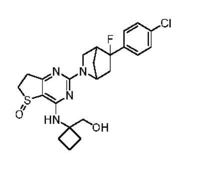 | 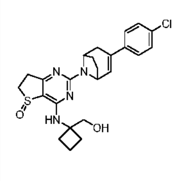 |
| 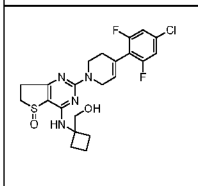 | 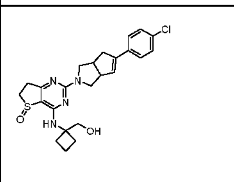 | 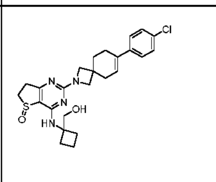 | 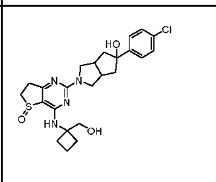 | 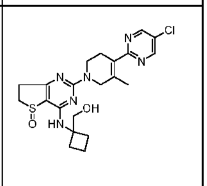 |
| 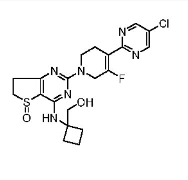 | 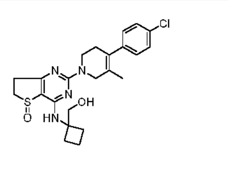 | 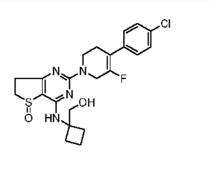 | 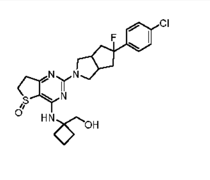 | 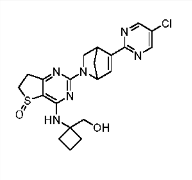 |
| 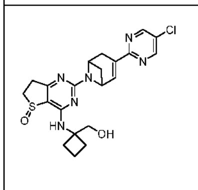 | 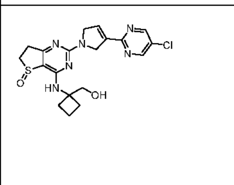 | 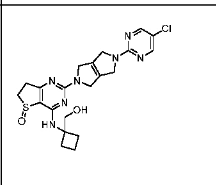 | 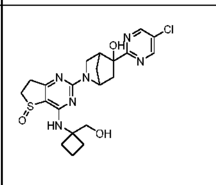 | 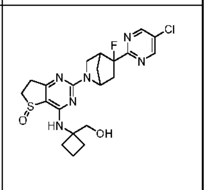 |
| 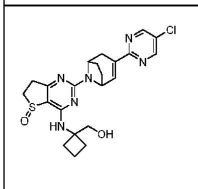 | 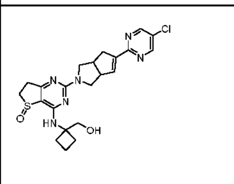 | 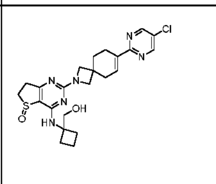 | 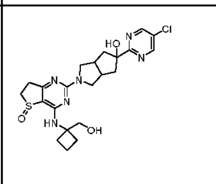 | 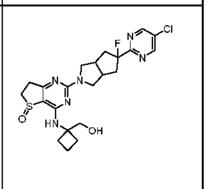 |

| | | | | |
|---|---|---|---|---|
| 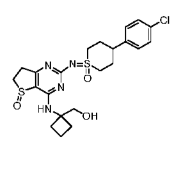 | 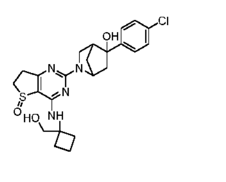 | 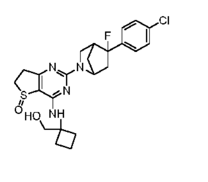 | 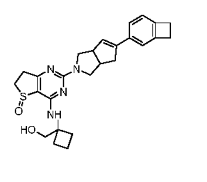 | 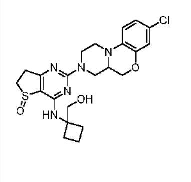 |
| 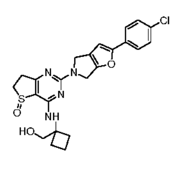 | 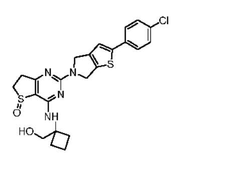 | 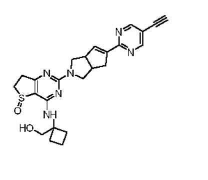 | 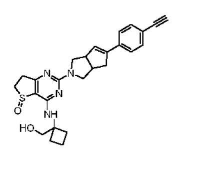 | 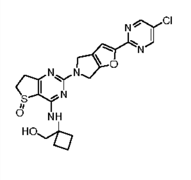 |
| 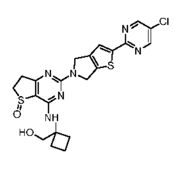 | 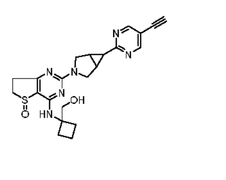 | 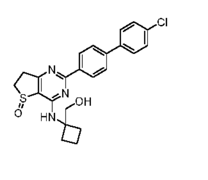 | 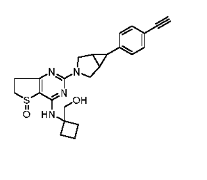 | 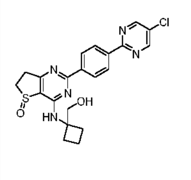 |
| 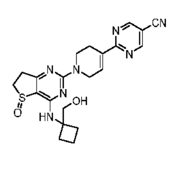 | 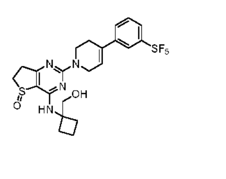 | 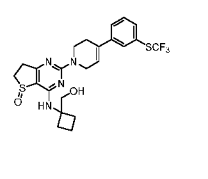 | 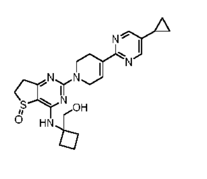 | 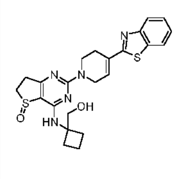 |
| 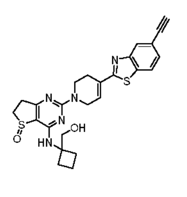 | 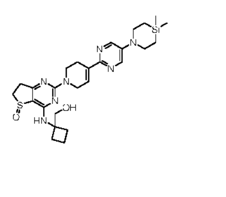 | 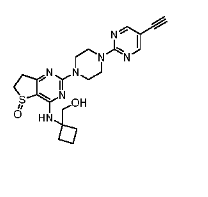 | 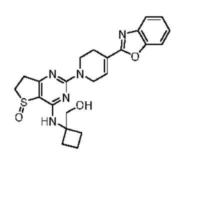 | 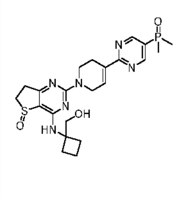 |
| 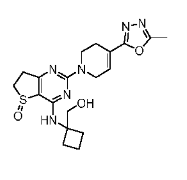 | 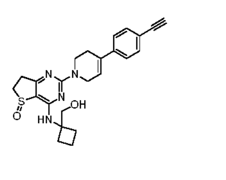 | 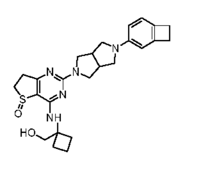 | 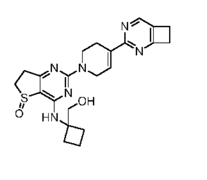 | 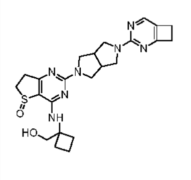 |
| 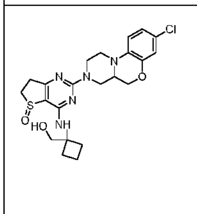 | 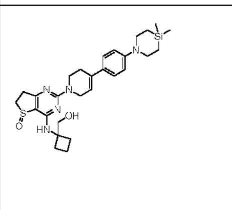 | 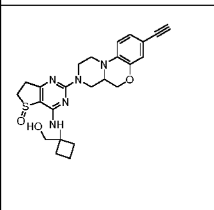 | 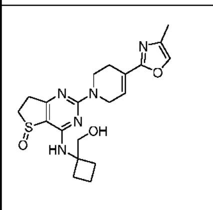 | 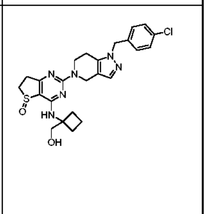 |
| 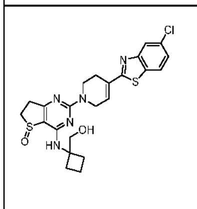 | 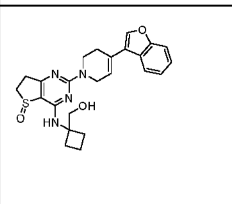 | 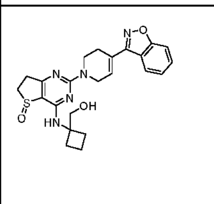 | 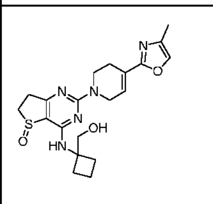 | 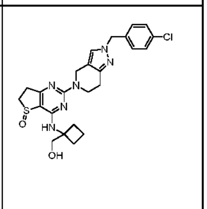 |
| | | | | |

| | | | | |
|---|---|---|---|---|
| 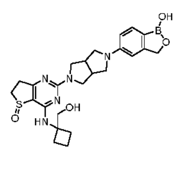 | 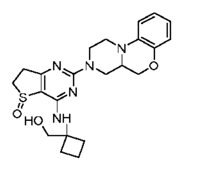 | 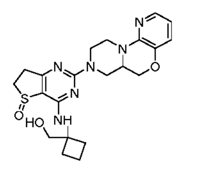 | 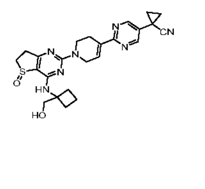 | 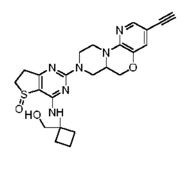 |
| 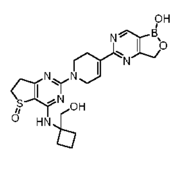 | 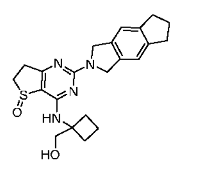 | 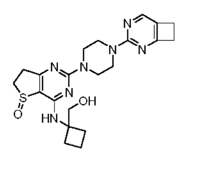 | 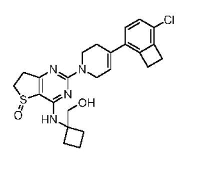 | 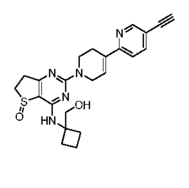 |
| 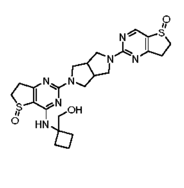 | 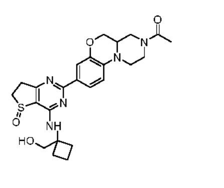 | 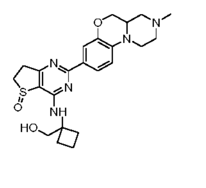 | 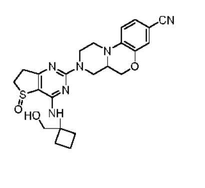 | 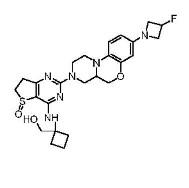 |
| 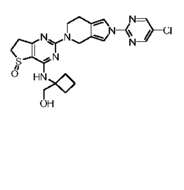 | 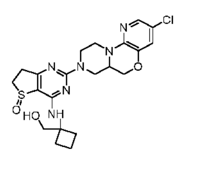 | 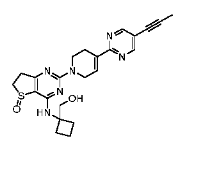 | 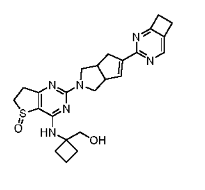 | 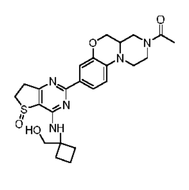 |
| 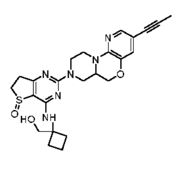 | 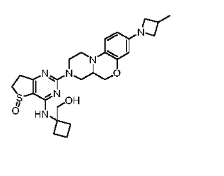 | 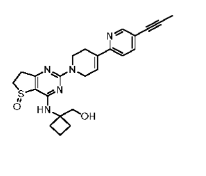 | 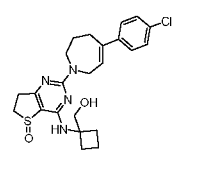 | . |

In solution 8 of the present invention, the compound of the present invention is selected from a compound with one of the structures in Table 2 below, or a stereoisomer or pharmaceutically acceptable salt thereof,

Table 2.

| | | | | |
|---|---|---|---|---|
| 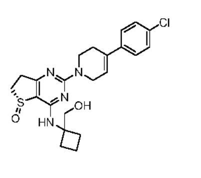 | 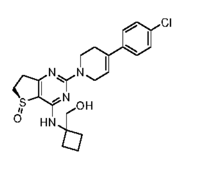 | 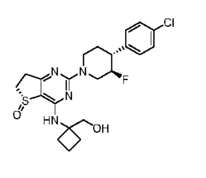 | 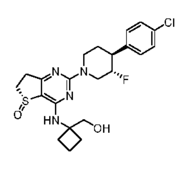 | 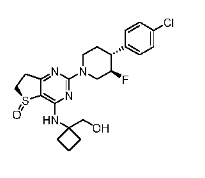 |
| 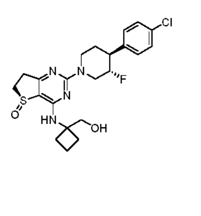 | 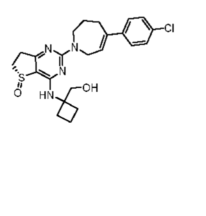 | 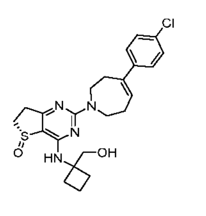 | 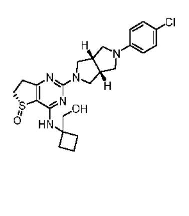 | 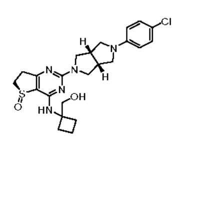 |
| 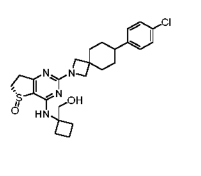 | 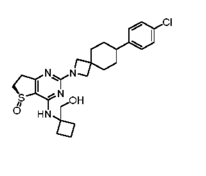 | 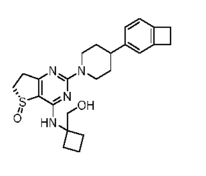 | 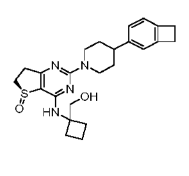 | 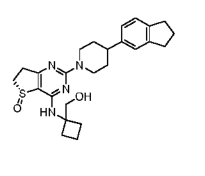 |

| | | | | |
|---|---|---|---|---|
| 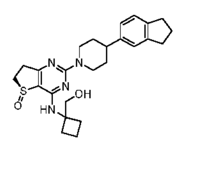 | 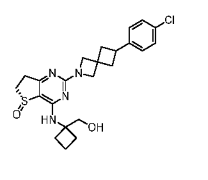 | 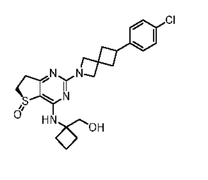 | 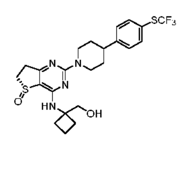 | 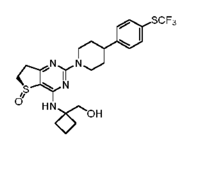 |
| 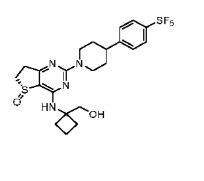 | 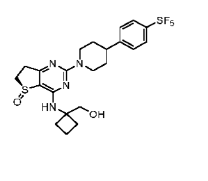 | 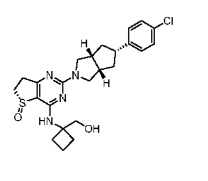 | 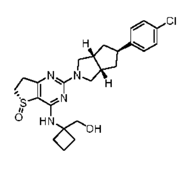 | 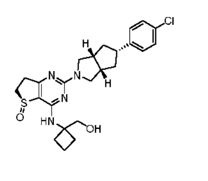 |
| 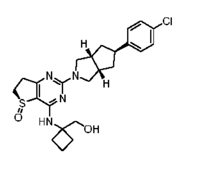 | 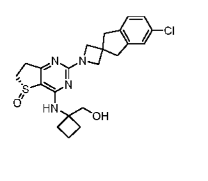 | 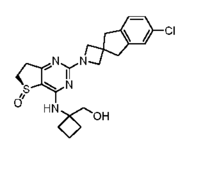 | 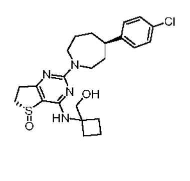 | 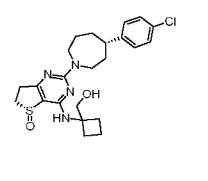 |
| 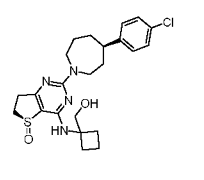 | 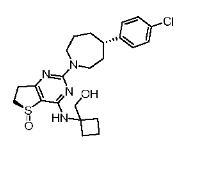 | 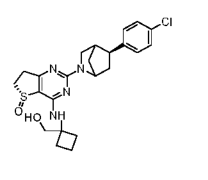 | 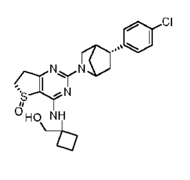 | 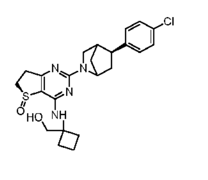 |
| 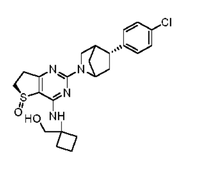 | 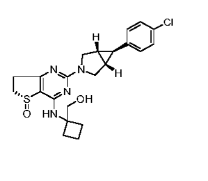 | 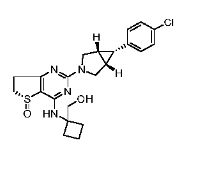 | 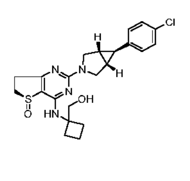 | 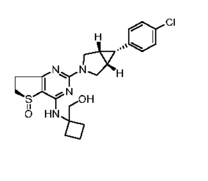 |
| 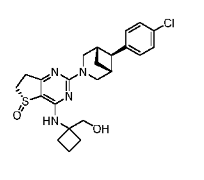 | 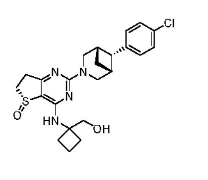 | 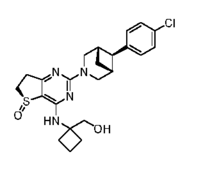 | 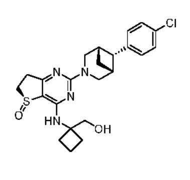 | 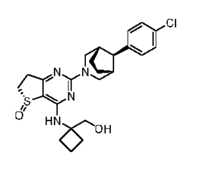 |
| 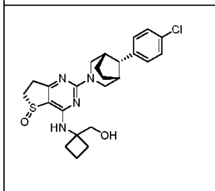 | 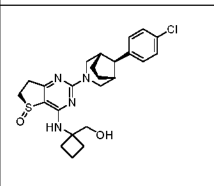 | 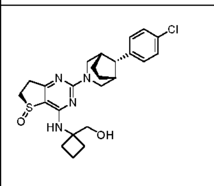 | 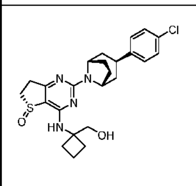 | 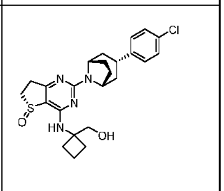 |
| 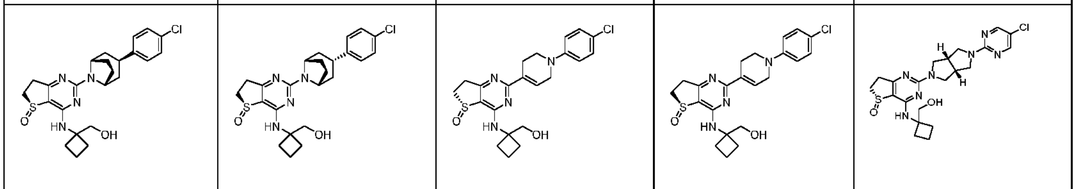 | | | | |
| 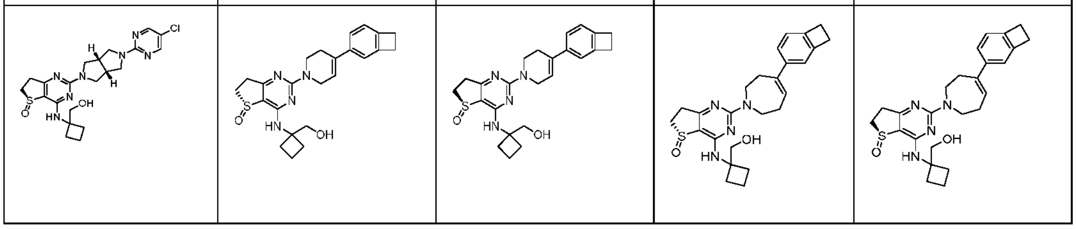 | | | | |

| | | | | |
|---|---|---|---|---|
| 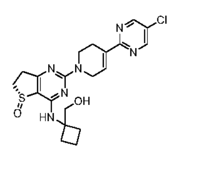 | 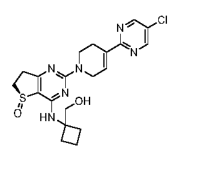 | 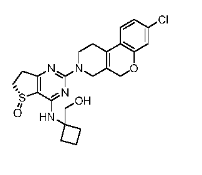 | 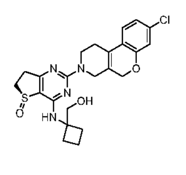 | 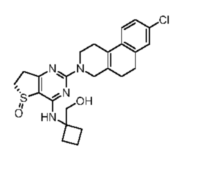 |
| 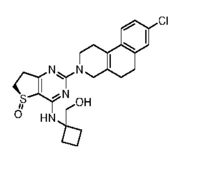 | 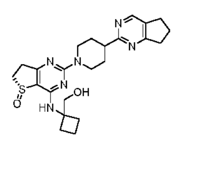 | 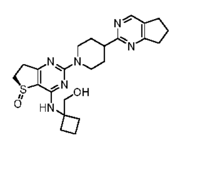 | 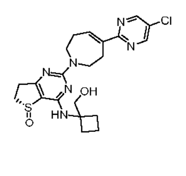 | 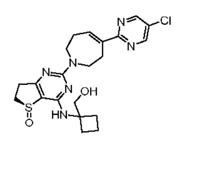 |
| 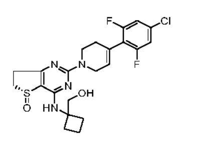 | 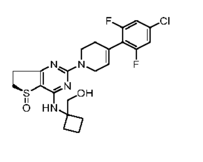 | 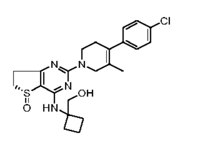 | 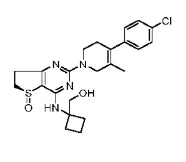 | 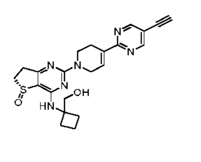 |
| 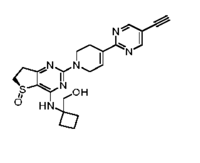 | 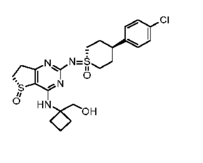 | 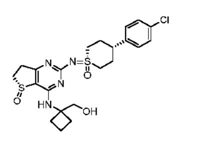 | 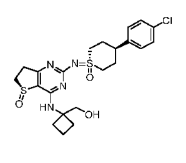 | 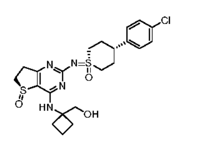 |
| 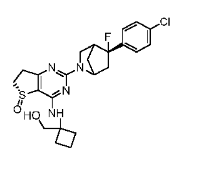 | 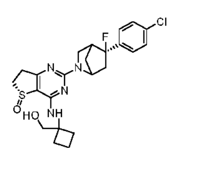 | 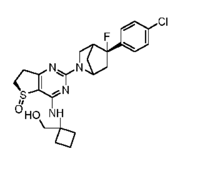 | 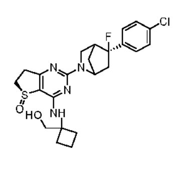 | 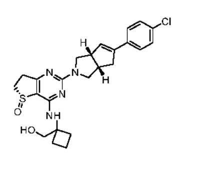 |
| 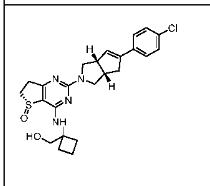 | 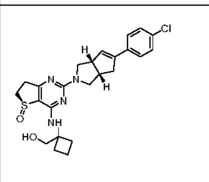 | 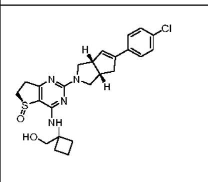 | 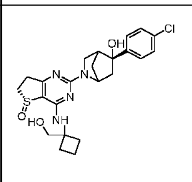 | 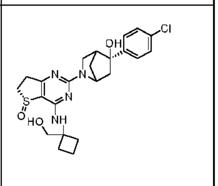 |
| 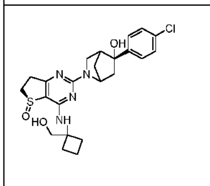 | 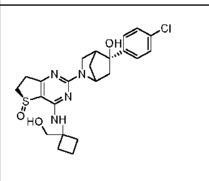 | 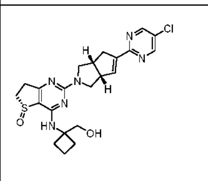 | 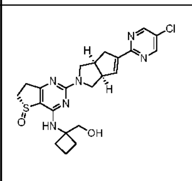 | 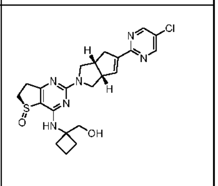 |
| 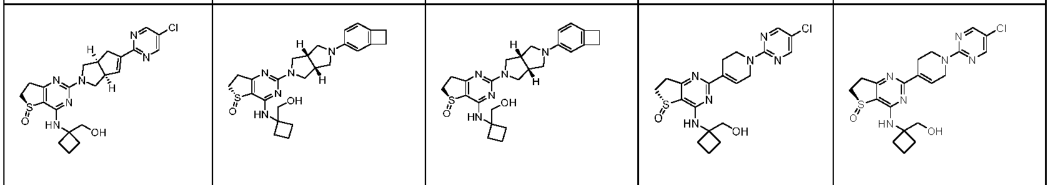 | | | | |
| 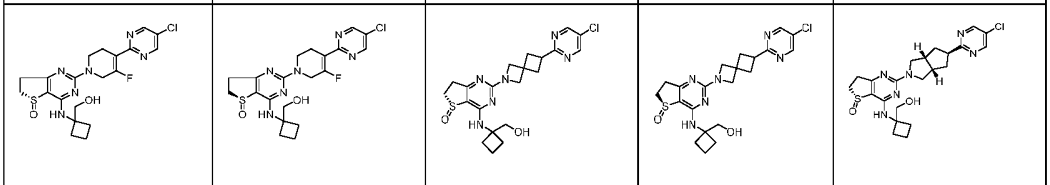 | | | | |
| 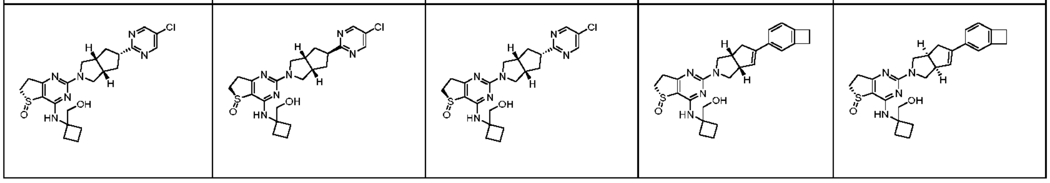 | | | | |

| | | | | |
|---|---|---|---|---|
| 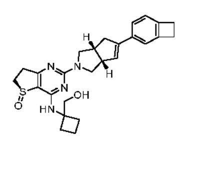 | 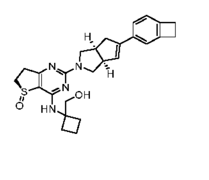 | 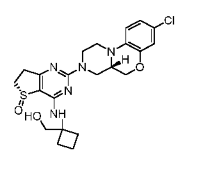 | 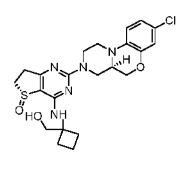 | 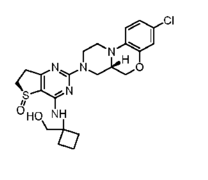 |
| 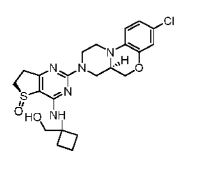 | 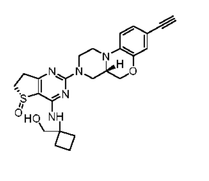 | 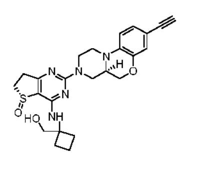 | 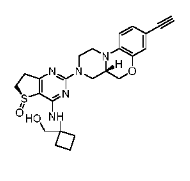 | 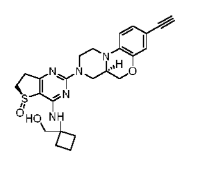 |
| 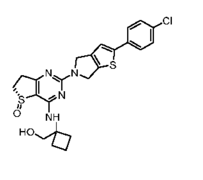 | 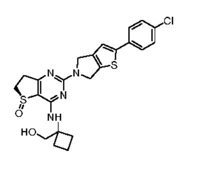 | 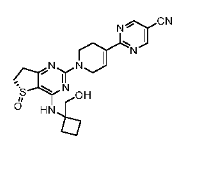 | 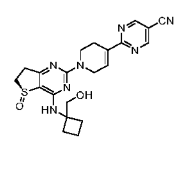 | 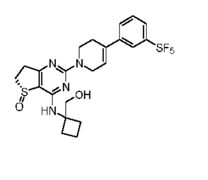 |
| 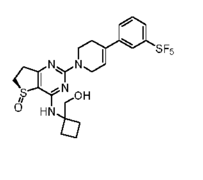 | 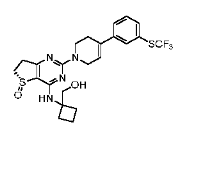 | 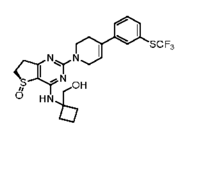 | 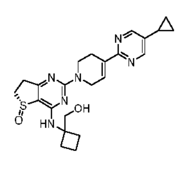 | 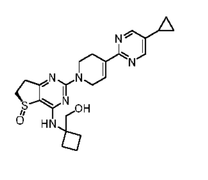 |
| 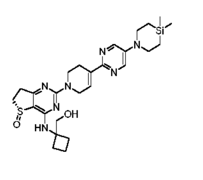 | 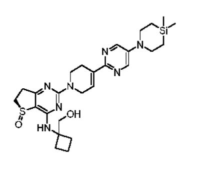 | 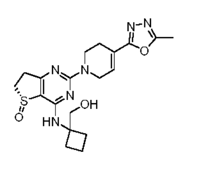 | 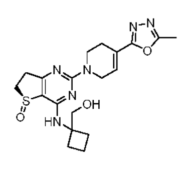 | 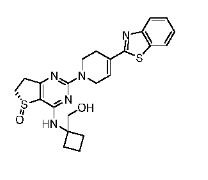 |
| 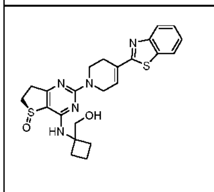 | 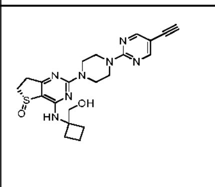 | 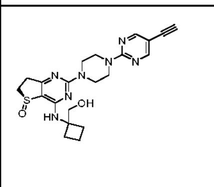 | 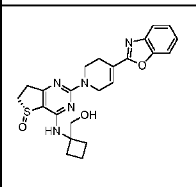 | 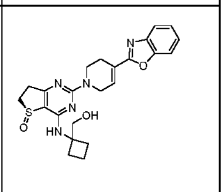 |
| 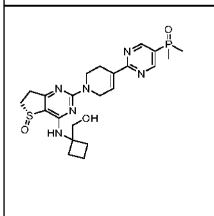 | 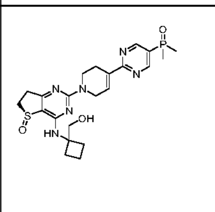 | 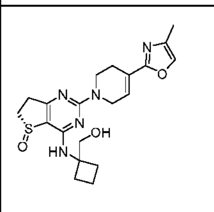 | 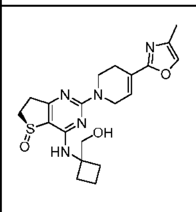 | 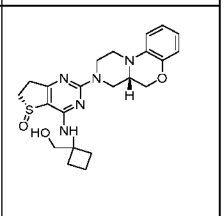 |
| 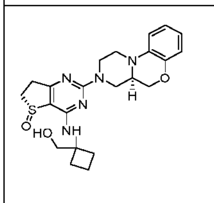 | 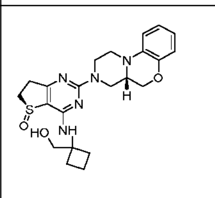 | 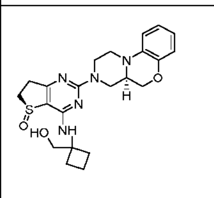 | 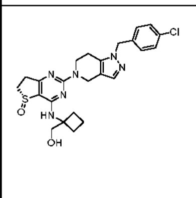 | 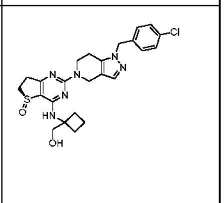 |
| | | | | |

| | | | | |
|---|---|---|---|---|
| 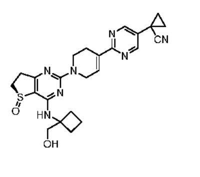 | 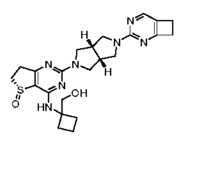 | 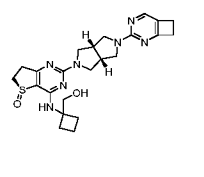 | 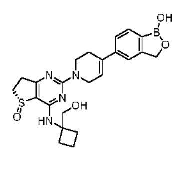 | 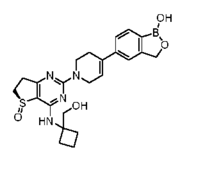 |
| 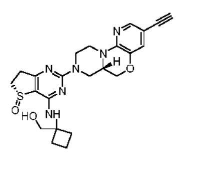 | 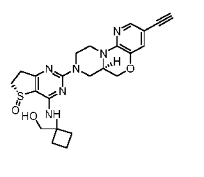 | 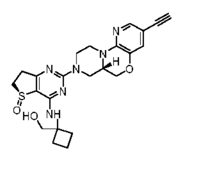 | 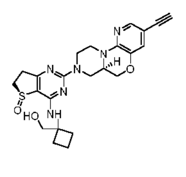 | 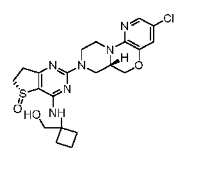 |
| 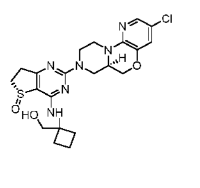 | 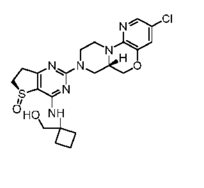 | 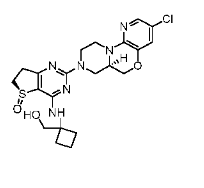 | 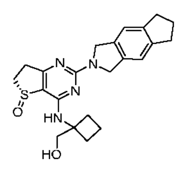 | 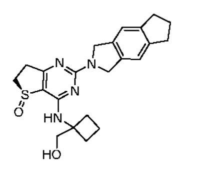 |
| 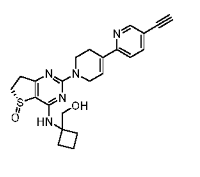 | 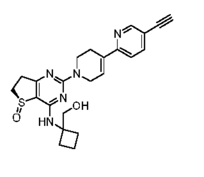 | 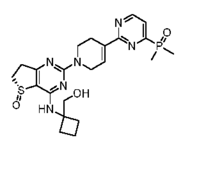 | 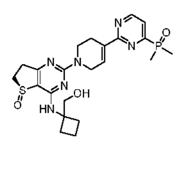 | 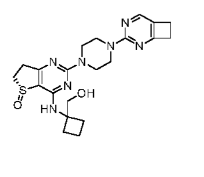 |
| 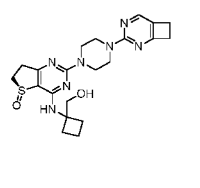 | 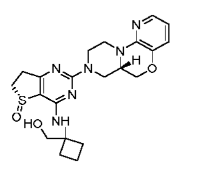 | 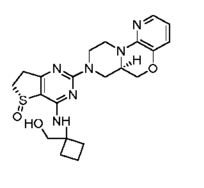 | 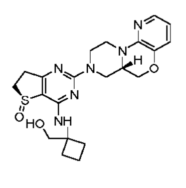 | 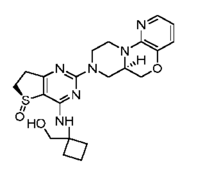 |
| 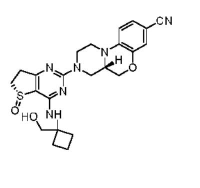 | 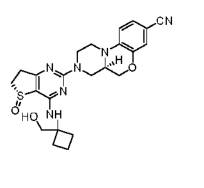 | 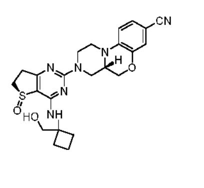 | 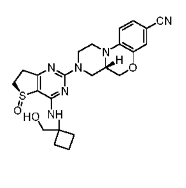 | 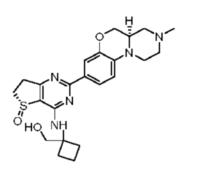 |
| 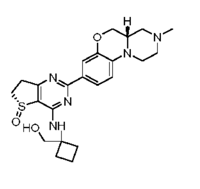 | 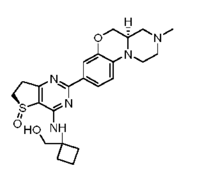 | 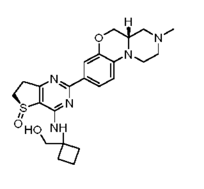 | 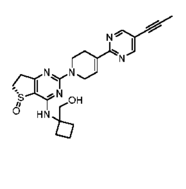 | 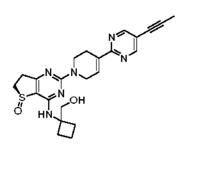 |
| 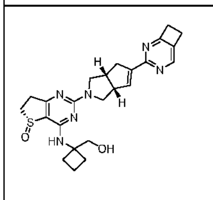 | 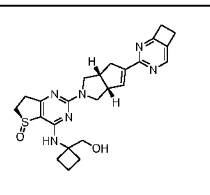 | 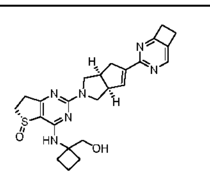 | 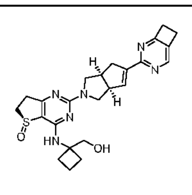 | 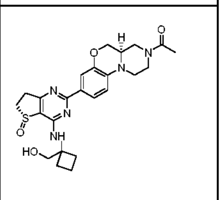 |
| | | | | |

| | | | | |
|---|---|---|---|---|
| 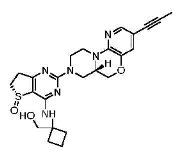 | 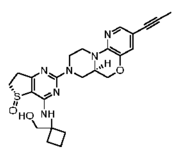 | 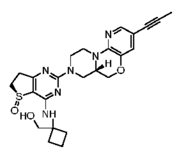 | 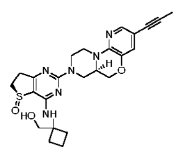 | 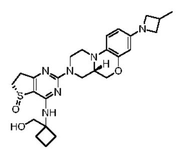 |
| 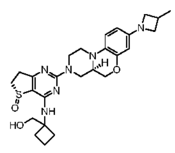 | 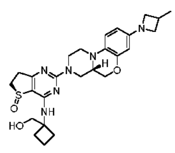 | 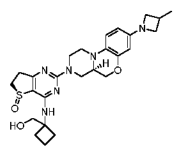 | 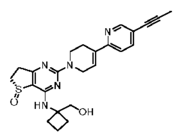 | 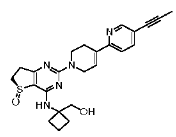 |
| 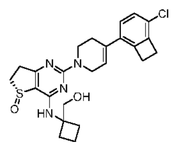 | 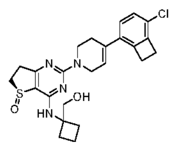 | . | | |

The present invention further provides a composition or pharmaceutical preparation comprising the compound or the stereoisomer or pharmaceutically acceptable salt thereof according to any one of the preceding solutions, and a pharmaceutically acceptable carrier and/or auxiliary material. The pharmaceutical composition can be in a unit preparation form (the unit preparation is also referred to as "preparation specification").

Furthermore, the composition or pharmaceutical preparation of the present invention comprises 1-1500 mg of the compound or the stereoisomer or pharmaceutically acceptable salt thereof according to any one of the preceding solutions, and a pharmaceutically acceptable carrier and/or auxiliary material.

The present invention further provides the use of the compound or the stereoisomer or pharmaceutically acceptable salt thereof according to any one of the preceding solutions in the preparation of a medicament for treating/preventing a PDE4B-mediated disease. Furthermore, the PDE4B-mediated disease is cancer, COPD, idiopathic pulmonary fibrosis, or interstitial lung disease.

The present invention further provides a method for treating a disease in a mammal, which comprises administering to a subject a therapeutically effective amount of the compound or the stereoisomer or pharmaceutically acceptable salt thereof as shown in any one of the preceding solutions, wherein the disease is preferably cancer, COPD, idiopathic pulmonary fibrosis, or interstitial lung disease, and the therapeutically effective amount is preferably 1-1500 mg. In some embodiments, the mammal mentioned in the present invention includes human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is reduction and/or remission of signs, symptoms, or causes of the disease, or any other desired change in the biological system. For example, an "effective amount" with reference to therapeutic use is an amount of the composition comprising the compound disclosed in the present application that is required to provide clinically significant reduction of the symptoms of the disease. Examples of the therapeutically effective amount include, but are not limited to, 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, and 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, and 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, and 50-100 mg; and 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg;

in some embodiments, the pharmaceutical composition or preparation of the present invention contains the above-mentioned therapeutically effective amount of the compound, or the stereoisomer, solvate, or pharmaceutically acceptable salt thereof according to the present invention; and

the present invention relates to a pharmaceutical composition or pharmaceutical preparation, comprising a therapeutically effective amount of the compound or the stereoisomer or pharmaceutically acceptable salt thereof according to the present invention, and a carrier and/or excipient. The pharmaceutical composition can be in a unit preparation form (the amount of the active drug in the unit preparation is also referred to as "preparation specification"). In some embodiments, the pharmaceutical composition comprises, without limitation, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1500 mg of the compound or the stereoisomer or pharmaceutically acceptable salt thereof according to the present invention.

A method for treating a disease in a mammal is provided, comprising administering to a subject a therapeutically effective amount of the compound or the stereoisomer or pharmaceutically acceptable salt thereof according to the present invention, and a pharmaceutically acceptable carrier and/or excipient, wherein the therapeutically effective amount is preferably 1-1500 mg, and the disease is preferably cancer, COPD, idiopathic pulmonary fibrosis, or interstitial lung disease.

A method for treating a disease in a mammal is provided, comprising administering to a subject a drug, i.e., the compound or the stereoisomer or pharmaceutically acceptable salt thereof according to the present invention, and a pharmaceutically acceptable carrier and/or excipient in a daily dose of 1-1500 mg/day, wherein the daily dose may be a single dose or divided doses; in some embodiments, the daily dose includes, but is not limited to, 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, and 200-400 mg/day; in some embodiments, the daily dose includes, but is not limited to, 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, and 1500 mg/day.

The present invention relates to a kit, which may comprise a composition in a single-dose or multi-dose form, wherein the kit comprises the compound or the stereoisomer or pharmaceutically acceptable salt thereof according to the present invention, and the amount of the compound or the stereoisomer or pharmaceutically acceptable salt thereof according to the present invention is the same as that in the above-mentioned pharmaceutical composition.

In the present invention, the amount of the compound or the stereoisomer or pharmaceutically acceptable salt thereof according to the present invention is calculated in the form of a free base in each case.

The term "preparation specification" refers to the weight of the active drug contained in each vial, tablet, or other unit preparation.

## Synthesis route

Patent documents such as WO 2013026797 A1 have introduced a method for preparing a PDE4B inhibitor, and those skilled in the art would have been able to prepare the compounds of the present invention in conjunction with this document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many of standard chemical supply plants (such as those listed above) provide custom synthesis services.

## Terminology

Unless otherwise specified, the terms of the present invention have the following meanings.

The term "halogen" herein refers to F, Cl, Br, I, or isotopes thereof.

The term "halo" or "substituted with halogen" means that a hydrogen atom is replaced with one or more groups selected from F, Cl, Br, I, or isotopes thereof, wherein the upper limit of the number of halogen substituents is equal to the sum of the number of hydrogens that can be replaced in the group to be substituted. Without particular limitation, the

number of halogen substituents is any integer between 1 and the upper limit, and when the number of halogen substituents is greater than 1, the group to be substituted can be substituted with the same or different halogen.

The term "deuterated" or "deuterated product" refers to the case where a hydrogen atom on alkyl, cycloalkyl, alkylene, aryl, heteroaryl, mercapto, heterocycloalkyl, alkenyl, alkynyl and other groups is replaced with at least one isotope deuterium, wherein the upper limit of the number of deuterium substituents is equal to the sum of the number of hydrogens that can be replaced in the group to be substituted. Without particular limitation, the number of deuterium substituents is any integer between 1 and the upper limit, preferably 1-20 deuterium atoms, more preferably 1-10 deuterium atoms, more preferably 1-6 deuterium atoms, further preferably 1-3 deuterium atoms.

The term "alkyl" refers to a monovalent linear or branched saturated aliphatic hydrocarbon group. Unless otherwise specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples include methyl, ethyl, n-propyl, isopropyl, n-butyl, sec-butyl, neobutyl, tert-butyl, n-pentyl, isoamyl, neopentyl, n-hexyl, and various branched isomers thereof.

The term "alkylene" refers to a divalent linear or branched chain divalent saturated alkyl, and examples of alkylene include, but are not limited to, methylene, ethylene, propylene, butylene, etc.

The term "cycloalkyl" refers to a monovalent non-aromatic, partially unsaturated or fully saturated, substituted or unsubstituted carbocyclic hydrocarbon group. Unless otherwise specified, it generally has 3 to 12 carbon atoms, preferably 3-10 carbon atoms, more preferably 3-6 carbon atoms, and further preferably 3-4 carbon atoms. Non-limiting examples include cyclopropyl, cyclobutyl, cyclopentyl, cyclohexyl,

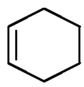,

cycloheptyl, cyclopropenyl, cyclobutenyl, cyclopentenyl, cyclohexenyl, etc.

The term "cycloalkylene" refers to a divalent group of "cycloalkyl", and non-limiting examples include cyclopropylene, cyclobutylene, etc.

The term "heterocyclic" or "heterocyclyl" refers to a substituted or unsubstituted aromatic ring or a substituted or unsubstituted, saturated or partially unsaturated non-aromatic ring, which comprises 1 to 3 heteroatoms selected from N, O or S unless otherwise defined, including monocyclic heterocyclic ring, bridged bicyclic heterocyclic ring, bicyclic fused heterocyclic ring, bicyclic spiro heterocyclic ring, etc. Unless otherwise specified, it is a 3- to 12-membered heterocyclic ring, more preferably 4- to 12-membered heterocyclic ring, more preferably 4- to 10-membered heterocyclic ring, further preferably 4- to 7-membered heterocyclic ring. The definition thereof comprises heterocycloalkyl and heteroaryl. The N and S in the heterocyclyl ring may be oxidized to various oxidation states. Heterocyclyl can be connected to a heteroatom or a carbon atom, and non-limiting examples of heterocyclyl include oxiranyl, aziridinyl, oxetanyl, azetidinyl, 1,3-dioxolanyl, 1,4-dioxolanyl, 1,3-dioxanyl, azepanyl, pyridinyl, furyl, thienyl, pyranyl, N-alkylpyrrolyl, pyrimidinyl, pyrazinyl, pyrazolyl, pyridazinyl, imidazolyl, piperidinyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridinyl, pyrrolopyridinyl, benzodihydrofuryl, azabicyclo[3.2.1]octyl, azabicyclo[5.2.0]nonanyl, oxatricyclo[5.3.1.1]dodecyl, azaadamantyl, oxaspiro[3.3]heptyl,

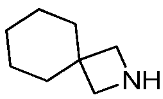, 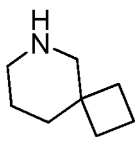, 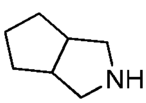, 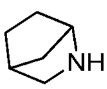, 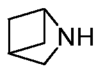,

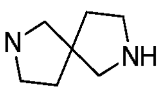, 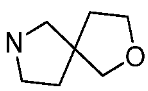, 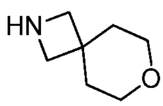, 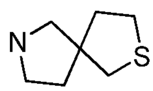, 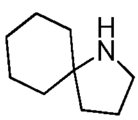, 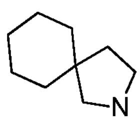,

etc.

The term "heterocyclylene" is a divalent group corresponding to "heterocyclyl", and non-limiting examples include imidazolylene, piperidinylene, aziridinylene, etc.

The term "carbocyclic" or "carbocyclyl" refers to a substituted or unsubstituted, saturated or unsaturated, aromatic or non-aromatic carbocyclic group, including a monocyclic carbocyclic ring, a bridged bicyclic ring, a fused

bicyclic ring, a spiro bicyclic ring, etc. Unless otherwise specified, it contains 3 to 12 carbon atoms, preferably 3-10 carbon atoms, further preferably 3-6 carbon atoms. The definition thereof comprises cycloalkyl and aryl. In non-limiting embodiments, monocyclic carbocyclic rings include cyclopropyl, cyclobutyl, cyclopentyl, cyclohexyl, cycloheptyl or phenyl,

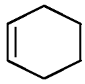,

*etc.,* bicyclic bridged rings include

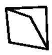, 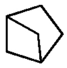, 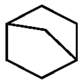,

*etc.,* bicyclic fused rings include

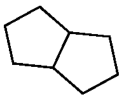, 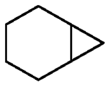,

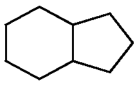, 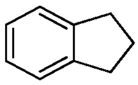, 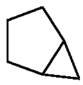,

*etc.,* and bicyclic spiro rings include

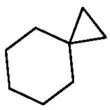,

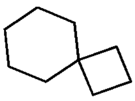, 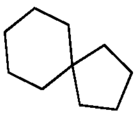, 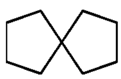, 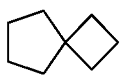, 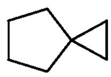,

*etc.*

The term "aryl" refers to an aromatic carbocyclic ring. Non-limiting examples include phenyl, naphthyl, etc.

The term "alkynyl" refers to a linear or branched monovalent unsaturated hydrocarbon group containing one or more carbon-carbon triple bonds. Unless otherwise specified, the alkynyl contains 2-6 carbon atoms, preferably contains 2-4 carbon atoms, and non-limiting examples include ethynyl, propynyl, propargyl, etc.

The term "alkenyl" refers to a linear or branched monovalent unsaturated hydrocarbon group containing one or more carbon-carbon double bonds. Unless otherwise specified, the alkenyl contains 2-6 carbon atoms, preferably contains 2-4 carbon atoms, and non-limiting examples are ethenyl, propenyl, allyl, 2-butenyl, 1-butenyl, etc.

The term "alkoxy" or "alkyloxy" refers to -O-alkyl. Without particular limitation, alkoxy or alkyloxy is -O-$C_{1-8}$ alkyl, preferably -O-$C_{1-6}$ alkyl, more preferably -O-$C_{1-4}$ alkyl, and further preferably -O-$C_{1-2}$ alkyl. Non-limiting examples include methoxy, ethoxy, n-propoxy, isopropoxy, n-butoxy, secbutoxy, tert-butoxy, n-pentoxy, n-hexyloxy, cyclopropoxy, cyclobutoxy, etc.

The term "haloalkoxy" refers to -O-haloalkyl. Without particular limitation, the haloalkoxy is -O-halo $C_{1-8}$ alkyl, preferably -O-halo $C_{1-6}$ alkyl, more preferably -O-halo $C_{1-4}$ alkyl, and further preferably -O-halo $C_{1-2}$ alkyl. Non-limiting examples include monofluoromethoxy, difluoromethoxy, trifluoromethoxy, difluoroethyloxy, *etc.*

The term "$C_{1-4}$ alkylacyl" refers to $C_{1-4}$ alkyl-C(O)-. Non-limiting examples include formyl, acetyl, and propionyl.

The term "$C_{1-4}$ alkylsulfonyl" refers to $C_{1-4}$ alkyl-$S(O)_2$-. Non-limiting examples include methylsulfonyl, ethylsulfonyl, and propylsulfonyl.

The term "heteroaromatic ring" or "heteroaryl" refers to an aromatic heterocyclic ring. Non-limiting examples

include pyrazolyl, pyrimidinyl, thiazolyl, pyridinyl, furyl, etc.

The term "heterocycloalkyl" refers to a non-aromatic, partially unsaturated or fully saturated heterocyclic ring, which generally has 4 to 12 ring members, preferably 4 to 10 ring members, more preferably 4 to 7 ring members, and further preferably 5 or 6 ring members. In addition to carbon atoms, heterocyclic alkyl groups also comprise 1-3 heteroatoms selected from N, S and O as ring members. Non-limiting examples include azetidinyl, morpholinyl, piperazinyl, piperidinyl, tetrahydropyranyl, oxetanyl, etc.

The term "alkylamino" or "alkamino" refers to amino substituted with one or two alkyl, and is also written as -N-(alkyl)$_2$ or -NH-alkyl, wherein the latter is also known as monoalkylamino. Non-limiting examples include dimethylamino, monomethylamino, diethylamino, monoethylamino, etc.

The term "optional" or "optionally" means that the events or circumstances described subsequent thereto may but not necessarily occur, and the description includes occasions where the events or circumstances occur or do not occur. For example, the term "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or cocrystals thereof as described herein and other components including physiologically/pharmaceutically acceptable carriers and/or excipients.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, auxiliary material, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrating agent, an absorbent, a preservative, a surfactant, a colorant, a flavouring agent, and a sweetening agent. Examples of pharmaceutically acceptable excipients include, but are not limited to: (1) sugars, such as lactose, glucose, and sucrose; (2) starch, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose, and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer’s solution; (19) ethanol; (20) pH buffer solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers, and conformational isomers.

The term "solvate" refers to a substance formed by the compound or the salt thereof according to the present invention and a stoichiometric or nonstoichiometric amount of a solvent by bonding via an intermolecular non-covalent force. When the solvent is water, the solvate is a hydrate.

The term "co-crystal" refers to a crystal formed by the combination of an active pharmaceutical ingredient (API) and a co-crystal former (CCF) under the action of hydrogen bonding or other non-covalent bonding. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

Detailed Description of Embodiments

The content of the present invention is described in detail by means of the following examples. In examples in which no specific conditions are indicated, experimental methods are carried out under conventional conditions. The listed examples are intended to better illustrate the content of the present invention but should not be construed as limiting the content of the present invention. Nonessential improvements and adjustments made to the embodiments by those skilled

in the art according to the above Summary of the Invention still fall within the scope of protection of the present invention.

Dess-Martin reagent; 1,1,1-Triacetoxy-1,1-dihydro-1,2-benziodoxol-3-(1H)-one
DIPEA: N,N-diisopropylethylamine
NMP; N-methylpyrrolidone
TBDMSCl: Tert-butyl dimethylsilyl chloride
DMAP: 4-Dimethylaminopyridine
HATU: 2-(7-azabenzotriazole)-N,N,N',N'-tetramethyluronium hexafluorophosphate
$T_3P$: Propylphosphonic cyclic anhydride

**Example 1:**

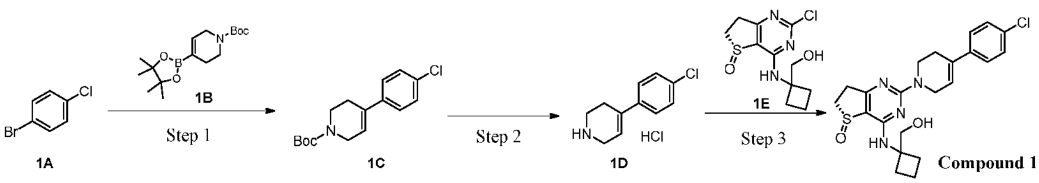


Step 1: Substrate **1A** (5.0 g, 26.11 mmol) was added to a 250 mL single-mouth flask and dissolved with dioxane (40 mL), and a sodium carbonate solution (2 M, 40 mL), **1B** (9.7 g, 31.33 mmol), and [1,1'-bis(diphenylphosphino) ferrocene]palladium dichloride (2.1 g, 2.61 mmol) were added and stirred at 100 degrees Celsius overnight under nitrogen protection. The reaction liquid was diluted by adding water (40 mL) and suction-filtered with diatomite, the filtrate was extracted with ethyl acetate (2 × 50 mL), and the organic phases were combined, dried over anhydrous sodium sulphate, concentrated, and subjected to column chromatography (petroleum ether : ethyl acetate (v/v) = 10 : 1) to obtain the title compound **1C** (7.2 g, 94%).
LC-MS (ESI): m/z = 238.1 $[M-56+H]^+$.
Step 2: Substrate **1C** (0.6 g, 2.04 mmol) was added to a 50 mL single-mouth flask, dissolved with methanol (5 mL) and hydrogen chloride dioxane (4 M, 5 mL), and stirred at room temperature for 3 h, and the reaction liquid was concentrated to obtain the title compound **1D** (0.47 g, 100%).
LC-MS (ESI): m/z = 194.1 $[M+H]^+$.
Step 3: Substrate **1E** (0.15 g, 0.52 mmol) (compound **1E** was synthesized according to the method of Patent WO 2013026797) was added to a 50 mL single-mouth flask and dissolved with dioxane (10 mL). **1C** (0.14 g, 0.62 mmol) and diisopropylethylamine (0.2 g, 1.55 mmol) were added, and the mixture was reacted at 100 degrees Celsius overnight. The reaction liquid was concentrated, purified by HPLC, and freeze-dried to obtain the title compound **1** (120 mg, 52%).
Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 30-80%; c. flow rate: 15 mL/min; d. elution time: 15 min; retention time: 7.0 min.
$^1H$ NMR (400 MHz, DMSO-*d*6) δ 7.49-7.46 (m, 2H), 7.42-7.37 (m, 2H), 7.35 (s, 1H), 6.31 (t, 1H), 4.83 (t, 1H), 4.35 (s, 2H), 3.97 (t, 2H), 3.75 (d, 2H), 3.45-3.39 (m, 1H), 3.26-3.16 (m, 1H), 2.99-2.81 (m, 2H), 2.51-2.49 (m, 2H), 2.43-2.27 (m, 2H), 2.23-2.16 (m, 2H), 1.87-1.71 (m, 2H).
LC-MS (ESI): m/z = 445.2 $[M+H]^+$.

**Example 2**

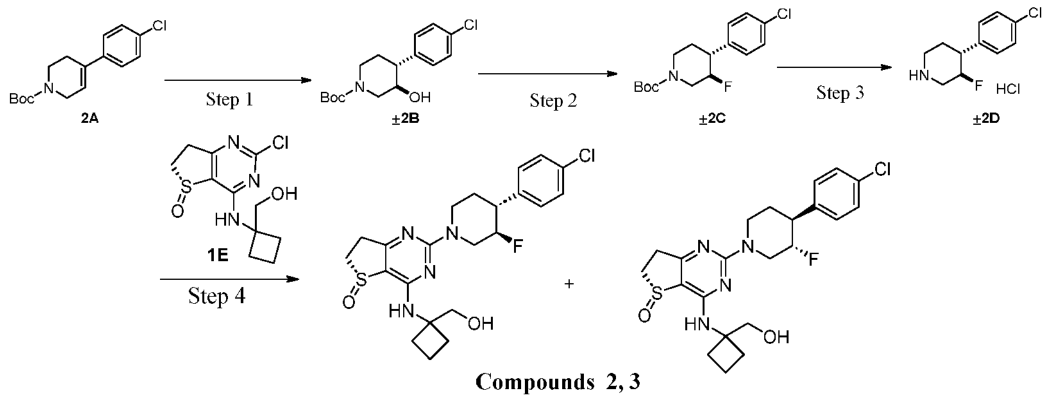


**Compounds 2, 3**

Step 1: Sodium borohydride (1.9 g, 51.05 mmol) and anhydrous tetrahydrofuran (50 mL) were added to a 500 mL single-mouth flask. Boron trifluoride diethyl etherate (7.25 g, 51.05 mmol) was added dropwise in an icewater bath. After the dropwise addition was complete, the mixture was stirred in the ice bath for 0.5 h, and a solution of **2A** (5.0 g, 17.02 mmol) in tetrahydrofuran (20 mL) was further added dropwise in the ice bath, naturally warmed to room temperature, and stirred overnight. Water (19 mL), ethanol (19 mL), and a sodium hydroxide solution (10%, 103 mL) were successively added to the reaction liquid. Finally, hydrogen peroxide (9.4 mL, 51.05 mmol) was added and stirred at 70 degrees Celsius overnight. The reaction liquid was adjusted to pH = 5-6 with HCl (2 M) and extracted with dichloromethane (3 × 100 mL), the organic phases were combined, dried over anhydrous sodium sulphate, concentrated, and subjected to column chromatography (petroleum ether : ethyl acetate (v/v) = 4:1) to obtain the title compound ±**2B** (± represented a racemate with the shown configuration) (4.0 g, 75% g).

LC-MS (ESI): m/z = 256.1 $[M-56+H]^+$.

Step 2: ±**2B** (1.9 g, 6.09 mmol) was added to a 100 mL single-mouth flask and dissolved with anhydrous dichloromethane (20 mL). Bis(2-methoxyethyl)aminosulphur trifluoride (1.48 g, 6.67 mmol) was added dropwise in an ice bath, and the mixture was slowly warmed to room temperature and stirred overnight. The reaction liquid was washed with a saturated sodium bicarbonate solution (20 mL), the aqueous phase was extracted with dichloromethane (2 × 20 mL), and the organic phases were combined, dried over anhydrous sodium sulphate, concentrated, and subjected to column chromatography (petroleum ether : ethyl acetate (v/v) = 10:1) to obtain the title compound ±**2C** (1.56 g, 82%).

LC-MS (ESI): m/z = 258.1 $[M-56+H]^+$.

Step 3: ±**2C** (1.56 g, 4.97 mmol) was added to a 100 mL single-mouth flask and dissolved with anhydrous methanol (10 mL), hydrogen chloride dioxane (10 mL) was then added, and the mixture was stirred at room temperature for 2 h and concentrated to obtain the title compound ±**2D** (1.24 g, 100%).

LC-MS (ESI): m/z = 214.1 $[M+H]^+$.

Step 4: **1E** (0.30 g, 1.04 mmol) was added to a 50 mL single-mouth flask and dissolved with dioxane (10 mL), and ±2D (0.32 g, 1.26 mmol) and diisopropylethylamine (0.4 g, 3.14 mmol) were added and reacted at 100 degrees Celsius overnight. The reaction liquid was concentrated and subjected to column chromatography (dichloromethane : methanol = 20 : 1-8 : 1) to obtain a mixture, which was subjected to chiral preparation to obtain the title compound **2** (0.11 g, 23%) and the title compound **3** (90 mg, 19%).

Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 2.9 min.

**Compound 2,** retention time: 1.94 min, $^1$H NMR (400 MHz, DMSO-*d6*) $\delta$ 7.43 (s, 1H), 7.40-7.33 (m, 4H), 5.04-5.01 (d, 1H), 4.82 (t, 1H), 4.71-4.63 (m, 1H), 4.57-4.51 (m, 1H), 3.77-3.66 (m, 2H), 3.47-3.39 (m, 1H), 3.27-3.19 (m, 1H), 3.09-2.81 (m, 5H), 2.46-2.25 (m, 2H), 2.20- 2.14 (m, 2H), 1.91-1.55 (m, 4H).

**Compound 3,** retention time: 2.23 min, $^1$H NMR (400 MHz, DMSO-*d6*) $\delta$ 7.43 (s, 1H), 7.39-7.34 (m, 4H), 5.05-5.02 (m, 1H), 4.82 (t, 1H), 4.69-4.52 (m, 2H), 3.71 (d, 2H), 3.48-3.39 (m, 1H), 3.27-3.19 (m, 1H), 3.08-2.81 (m, 5H), 2.41-2.29 (m, 2H), 2.21-2.10 (m, 2H), 1.89-1.55 (m, 4H).

LC-MS (ESI): m/z = 465.2 $[M+H]^+$.

**Example 3**

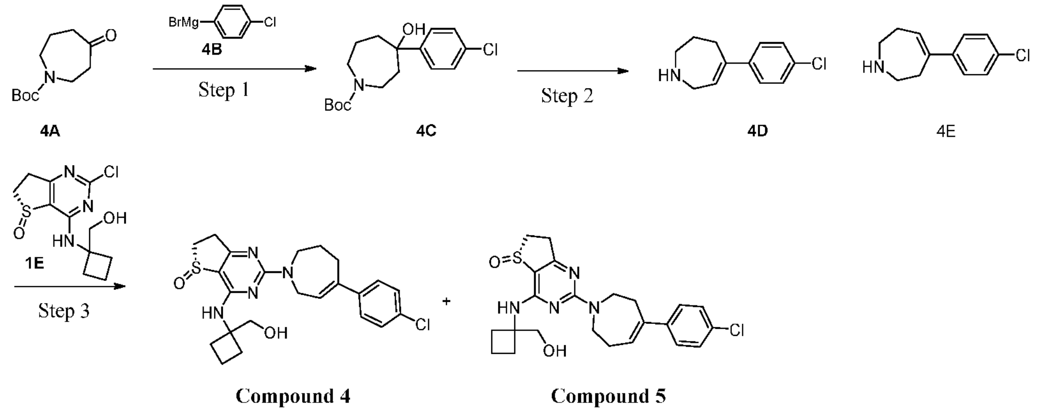


4A 4C 4D 4E

Compound 4 + Compound 5

Step 1: **4A** (2.0 g, 9.38 mmol) was added to a 100 mL single-mouth flask and dissolved with anhydrous tetrahydrofuran (10 mL), **4B** (10.3 mL, 1 M, 10.32 mmol) was added dropwise in an ice bath, the mixture was stirred for 3 h at room temperature, quenched with a saturated ammonium chloride solution (30 mL), and extracted with ethyl acetate (2 × 20 mL), and the organic phases were combined, dried over anhydrous sodium sulphate, concentrated, and subjected to column chromatography (petroleum ether : ethyl acetate (v/v) = 4 : 1) to obtain the title compound **4C** (2.6 g, 85%).

LC-MS (ESI): m/z = 252.1 $[M-56-18+H]^+$.

Step 2: Substrate **4C** (1.0 g, 3.07 mmol) was added to a 50 mL single-mouth flask and dissolved with dichloromethane (10 mL). Trifluoroacetic acid (5 mL) was added, the mixture was stirred overnight at room temperature, adjusted to pH 8-9 with an NaOH (2 M) solution, and extracted with dichloromethane (2 × 20 mL), and the organic phases were combined, dried over anhydrous sodium sulphate, and concentrated to obtain a mixture of the title compounds **4D** and **4E** (0.6 g, 94%).

LC-MS (ESI): m/z = 208.1 $[M+H]^+$.

Step 3: **1E** (0.20 g, 0.69 mmol) was added to a 50 mL single-mouth flask and dissolved with dioxane (5 mL), and a mixture of **4D** and **4E** (0.21 g, 1.01 mmol) and diisopropylethylamine (0.26 g, 2.05 mmol) were added and reacted at 100 degrees Celsius overnight. The reaction liquid was concentrated and separated by column chromatography (dichloromethane : methanol = 20 : 1-10 : 1) to obtain 0.5 g of a crude product, which was purified by HPLC to obtain the title compound **4** (0.13 g, 42%) and the title compound 5 (50 mg, 16%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution: mobile phase A: 35-70%; c. flow rate: 15 mL/min; d. elution time: 15 min; retention time: compound **4,** 4.0 min, and compound **5,** 7.0 min.

Compound **4,** $^1$H NMR (400 MHz, DMSO-d6) δ 7.33 (brs, 4H), 7.24 (s, 1H), 6.17 (t, 1H), 4.82 (t, 1H), 4.30-4.32 (m, 2H), 4.09-3.85 (m, 2H), 3.71 (d, 2H), 3.44-3.36 (m, 1H), 3.24-3.16 (m, 1H), 2.98-2.78 (m, 2H), 2.66-2.56 (m, 2H), 2.44-2.27 (m, 2H), 2.17 (brs, 2H), 1.93-1.69 (m, 4H).

Compound 5, $^1$H NMR (400 MHz, DMSO-*d*6) δ 7.34 (brs, 4H), 7.25 (s, 1H), 6.03 (t, 1H), 4.82 (t, 1H), 4.05-3.80 (m, 4H), 3.72 (d, 2H), 3.45-3.37 (m, 1H), 3.24-3.15 (m, 1H), 2.98-2.80 (m, 2H), 2.77-2.65 (m, 2H), 2.53-2.45 (m, 2H), 2.42-2.25 (m, 2H), 2.20-2.07 (m, 2H), 1.82-1.68 (m, 2H).

LC-MS (ESI): m/z = 459.1 $[M+H]^+$.

## Example 4

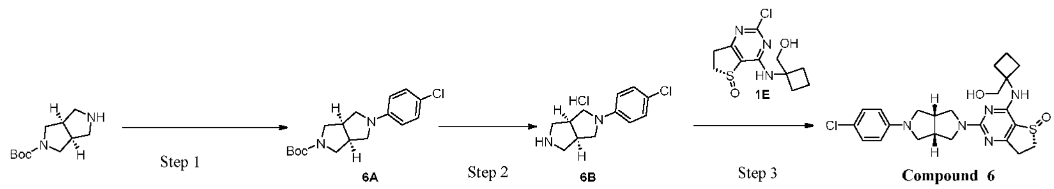


6A 6B Compound 6

Step 1: Tert-butyl (3aR,6aS)-hexahydropyrrolo[3,4-c]pyrrole-2(1H)-carboxylate (2.12 g, 10 mmol), 1-chloro-4-iodobenzene (2.61 g, 11 mmol), $Pd_2(dba)_3$ (915 mg, 1 mmol), Xphos (952 mg, 2 mmol), and sodium tert-butoxide (1.15 g, 12 mmol) were weighed into a 250 mL three-mouth flask, and toluene (50 mL) and tert-butyl alcohol (10 mL) were added.

After nitrogen displacement, a reaction was carried out at 100°C for 16 h. After the completion of the reaction was monitored by LCMS, the system was cooled to room temperature and filtered, and the filter cake was washed with ethyl acetate. The filtrate was concentrated and then separated by silica gel column chromatography (PE:EA = 20:1 to 5:1) to obtain the target compound **6A** (2.76 g, 85.2%).

LC-MS (ESI): m/z = 323.2 $[M+H]^+$.

Step 2: **6A** (2.76 g, 0.85 mmol) was dissolved in methanol (30 mL), and a solution of hydrogen chloride dioxane (5 mL, 4 M) was added. The mixture was reacted at room temperature for four hours and spin-dried to obtain title compound **6B** (2.56 mg, crude).

LC-MS (ESI): m/z = 223.2 $[M+H]^+$.

Step 3: **1E** (287 mg, 1 mmol) and **6B** (300 mg) were dissolved in 1,4-dioxane (20 mL). DIPEA (1 mL) was added, and the mixture was left at 90°C overnight. After the complete reaction of raw materials was detected by LCMS, the system was concentrated. Water (20 mL) was added, and the mixture was extracted with a mixed solution of DCM:MeOH = 20:1 (20 mL × 3). The organic phases were combined, dried over anhydrous sodium sulphate, concentrated, and then passed through a column (DCM:MeOH = 1:0 to 0:1) to obtain **compound 6** (289 mg, 60.9%).

$^1$H NMR (400 MHz, DMSO-$d_6$) $\delta$ 7.33-7.07 (m, 3H), 6.66-6.38 (m, 2H), 4.85-4.76 (m, 1H), 3.82-3.74 (m, 2H), 3.74-3.67 (m, 2H), 3.52-3.34 (m, 5H), 3.26-3.13 (m, 3H), 3.13-3.04 (m, 2H) 2.96-2.79 (m, 2H), 2.43-2.27 (m, 2H), 2.19-2.09 (m, 2H), 1.86-1.65 (m, 2H).

LC-MS (ESI): m/z = 474.2 $[M+H]^+$.

**Example 5**

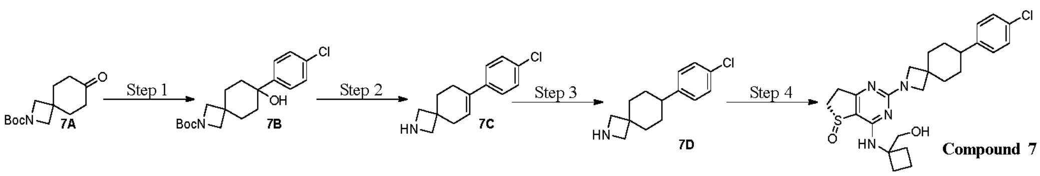


Step 1: Compound **7A** (1 g, 4.2 mmol) was dissolved in 50 mL of tetrahydrofuran in a nitrogen atmosphere, and the system was cooled to 0°C. 4.6 mL of 4-chlorophenylmagnesium bromide (1 M in THF) was slowly added dropwise, and the mixture was warmed to room temperature and reacted for 3 h. The reaction was quenched by adding a saturated ammonium chloride aqueous solution. The reaction system was concentrated and extracted with 50 mL of ethyl acetate three times, and the resulting product was dried over anhydrous sodium sulphate. The organic phase was concentrated to obtain crude compound **7B,** which was directly used in the next step.

LCMS m/z = 296.1 $[M-55]^+$.

Step 2: Crude compound **7B** was dissolved in a mixed solvent (DCM:TFA = 50 mL:4 mL) and reacted for 2 h at room temperature. After the reaction was complete, the system was concentrated to obtain crude compound **7C,** which was directly used in the next step.

LCMS m/z = 234.2 $[M+1]^+$.

Step 3: Crude compound **7C** was dissolved in 50 mL of methanol, and 300 mg palladium on carbon (10%) was added and reacted overnight in a hydrogen atmosphere. The solid residue in the system was removed by suction filtration, and the filtrate was concentrated to obtain crude compound **7D,** which was directly used in the next reaction.

LCMS m/z = 236.1 $[M+1]^+$.

Step 4: In a nitrogen atmosphere, crude compound **7D,** compound **1E** (150 mg, 0.52 mmol), and DIPEA (0.86 mL, 5.2 mmol) were dissolved in 30 mL of 1,4-dioxane, and the mixture was warmed to 100°C and reacted for 18 h. The reaction system was concentrated and then separated by thin-layer chromatography to obtain **compound 7** (98 mg, 38%).

LCMS m/z = 487.1 $[M+1]^+$.

$^1$H NMR (400 MHz, DMSO-$d6$) $\delta$ 7.34-7.26 (m, 5H), 4.87-4.84 (m, 1H), 3.85-3.59 (m, 6H), 3.44-3.34 (m, 1H), 3.26-3.15 (m, 1H), 2.95-2.81 (m, 2H), 2.54-2.46 (m, 1H), 2.41-2.27 (m, 2H), 2.15 (s, 2H), 1.99-1.93 (m, 2H), 1.86-1.68 (m, 4H), 1.64-1.57 (m, 2H), 1.50-1.41 (m, 2H).

**Example 6**

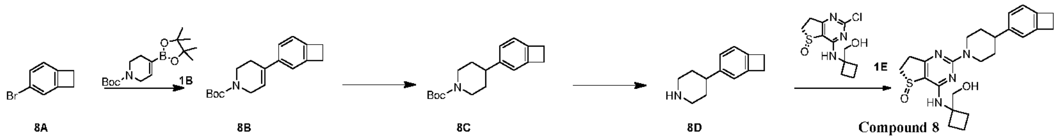


8A 8B 8C 8D Compound 8

Step 1: Compound **8A** (2.00 g, 10.91 mmol) and compound **1B** (3.31 g, 10.91 mmol) were weighed into a 100 mL single-mouth flask and then dissolved by adding 1,4-dioxane (15 mL), and a sodium carbonate solution (2N, 15 mL) and Pd(dppf)$Cl_2$ (0.66 g, 1.1 mmol) were added to the reaction liquid. After the addition was complete, the mixture was stirred at 100°C for 4 h. After the reaction was found complete by TLC plate spotting (petroleum ether : ethyl acetate = 5:1), water (20 mL) was added to the reaction liquid and stirred for 5 min. Ethyl acetate (20 mL) was added for extraction. An organic phase was separated. The organic phase was dried over anhydrous sodium sulphate, and the crude product was concentrated under reduced pressure, and purified by column chromatography (eluents: petroleum ether : ethyl acetate = 5:1) to obtain the target compound **8B** (2.50 g, 80%).

LCMS m/z = 286.1 $[M+1]^+$.

Step 2: Compound **8B** (1.00 g, 3.50 mmol) was weighed into a 100 mL single-mouth flask and then dissolved by adding ethyl acetate (20 mL), palladium on carbon (0.19 g, 1.74 mmol) was added to the reaction liquid, and after the addition was complete, the mixture was stirred for 1 h under hydrogen condition. After the reaction was complete, the reaction liquid was filtered and the filtrate was concentrated under reduced pressure to obtain the target compound **8C** (1.00 g, 99%).

LCMS m/z = 288.1 $[M +1]^+$.

Step 3: Compound **8C** (1.00 g, 3.48 mmol) was weighed into a 100 mL single-mouth flask and then dissolved by adding methanol (20 mL), a hydrogen chloride dioxane solution (4N, 20 mL) was added, and after the addition was complete, the mixture was stirred for 2 h. The organic phase was concentrated under reduced pressure to obtain the target compound **8D** (0.60 g, yield: 92%).

LCMS m/z = 188.2 $[M +1]^+$.

Step 4: Compound **8D** (0.20 g, 1.04 mmol) and compound **1E** (0.30 g, 1.04 mmol) were added to a 100 mL single-mouth flask and dissolved by adding 1,4-dioxane (10 mL), and finally, N,N-diisopropylethylamine (0.40 g, 3.12 mmol) was added. After the addition was complete, the system was placed under nitrogen protection and stirred at 100°C for 16 h, and the reaction liquid was concentrated under reduced pressure to obtain a crude product of the target compound, which was subjected to preparative HPLC to obtain the title compound **8** (190 mg, 62%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 40-80%; c. flow rate: 15 mL/min; d. elution time: 20 min; retention time: 10.38 min.

LCMS m/z = 439.2 $[M +1]^+$.

$^1$H NMR (400 MHz, DMSO-*d*6) $\delta$7.26(s, 1H), 7.02-7.04 (m, 1H), 6.94-6.97 (m, 1H), 4.75-4.82 (m, 3H), 3.71 (d, 3H), 3.36-3.45 (m, 1H), 3.17-3.25 (m, 1H), 3.07 (s, 4H),2.74-2.96 (m, 5H), 2.27-2.41(m, 2H), 2.12-2.19 (m, 2H), 1.70-1.80 (m, 4H), 1.44-1.54 (m, 2H).

## Example 7

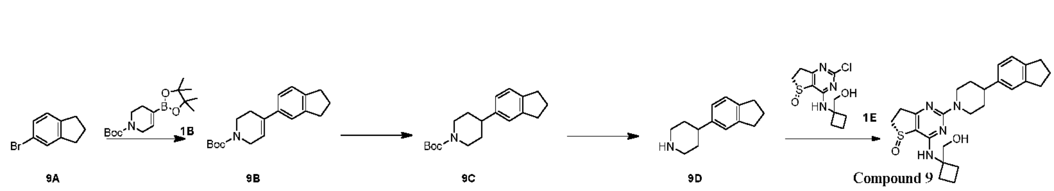


9A 9B 9C 9D Compound 9

Step 1: Compound **9A** (2.00 g, 10.05 mmol) and compound **1B** (3.11 g, 10.05 mmol) were weighed into a 100 mL single-mouth flask and then dissolved by adding 1,4-dioxane (15 mL), and a sodium carbonate solution (2N, 15 mL) and [1,1'-bis(diphenylphosphino)ferrocene]palladium dichloride (0.66 g, 1.01 mmol) were added to the reaction liquid. After the addition was complete, the mixture was stirred at 100°C for 4 h. After the completion of the reaction was monitored by TLC (petroleum ether : ethyl acetate = 5:1), water (20 mL) was added to the reaction liquid and stirred for 5 min. Ethyl acetate (20 mL) was added for extraction, and an organic phase was separated. The organic phase was dried over anhydrous sodium sulphate, and the crude product was concentrated under reduced pressure and purified by column chromatography (eluents: petroleum ether : ethyl acetate = 5:1) to obtain the target compound **9B** (2.50 g, 80%).

LCMS m/z = 300.1 [M +1]+.

Step 2: Compound **9B** (3.00 g, 9.95 mmol) was weighed into a 100 mL single-mouth flask and then dissolved by adding ethyl acetate (20 mL), palladium on carbon (0.50 g, 4.97 mmol) was added to the reaction liquid, and after the addition was complete, the mixture was stirred for 1 h under hydrogen condition. After the reaction was complete, the reaction liquid was filtered and the filtrate was concentrated under reduced pressure until no liquid dripped out to obtain the target compound **9C** (3.00 g, 99%).

LCMS m/z = 302.1 [M +1]+.

Step 3: Compound **9C** (3.00 g, 9.89 mmol) was weighed into a 100 mL single-mouth flask and dissolved by adding methanol (20 mL), a hydrogen chloride dioxane solution (4N, 20 mL) was added, and after the addition was complete, the mixture was stirred for 2 h. The organic phase was concentrated under reduced pressure to obtain the target compound **9D** (2.00 g, 99%).

LCMS m/z = 202.2 [M +1]+.

Step 4: Compound **9D** (0.20 g, 1.03 mmol) and compound **1E** (0.29 g, 1.03 mmol) were added to a 100 mL single-mouth flask and dissolved by adding 1,4-dioxane (10 mL), and finally, N,N-diisopropylethylamine (0.26 g, 2.05 mmol) was added. After the addition was complete, the system was placed under nitrogen protection and stirred at 100°C for 16 h, and the reaction liquid was concentrated under reduced pressure to obtain a crude product of the target compound, which was subjected to preparative HPLC to obtain the title compound **9** (190 mg, 64%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 45-80%; c. flow rate: 20 mL/min; d. elution time: 20 min; retention time: 8.11 min.

LCMS m/z = 453.2 [M +1]+.

$^1$H NMR (400 MHz, DMSO-*d6*) δ7.26(s, 1H), 7.07-7.12 (m, 2H), 6.96-6.97 (m, 1H), 4.75-4.82 (m, 3H), 3.72 (d, 2H), 3.36-3.45 (m, 1H), 3.18-3.25 (m, 1H), 2.75-2.97 (m, 9H), 2.28-2.41(m, 2H), 2.11-2.20 (m, 2H), 1.93-2.01 (m, 2H), 1.72-1.80 (m, 4H), 1.44-1.54 (m, 2H).

**Example 8**

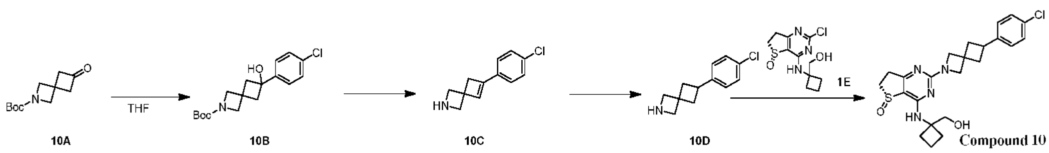


10A 10B 10C 10D Compound 10

Step 1: Compound **10A** (2.00 g, 9.47 mmol) was weighed into a 100 mL single-mouth flask and then dissolved by adding tetrahydrofuran (30 mL), 4-chlorophenylmagnesium chloride (18.94 mL, 18.94 mmol) was added to the reaction liquid. After the addition was complete, the mixture was stirred at room temperature for 4 h. After the reaction was found complete by TLC plate spotting (petroleum ether : ethyl acetate = 3:1), water (20 mL) was added to the reaction liquid and stirred for 5 min, ethyl acetate (20 mL) was added for extraction, and an organic phase was separated. The organic phase was dried over anhydrous sodium sulphate, and the crude product was concentrated under reduced pressure and purified by column chromatography (eluents: petroleum ether : ethyl acetate = 5:1) to obtain the target compound **10B** (1.50 g, 48%).

LCMS m/z = 324.1 [M +1]+.

Step 2: Compound **10B** (0.50 g, 1.54 mmol) was weighed into a 100 mL single-mouth flask and then dissolved by adding dichloromethane (15 mL), trifluoroacetic acid (5 mL) was added to the reaction liquid, and after the addition was complete, the mixture was stirred at room temperature for 16 h. After the reaction was complete, the filtrate was concentrated under reduced pressure to obtain the target compound **10C** (0.40 g, crude).

LCMS m/z = 206.1 [M +1]+.

Step 3: Compound **10C** (0.40 g, 1.94 mmol) was weighed into a 100 mL single-mouth flask and then dissolved by adding methanol (20 mL), palladium on carbon (0.10 g, 0.97 mmol) was added, and after the addition was complete, the mixture was stirred for 2 h. After the reaction was complete, the reaction liquid was filtered, and the organic phase was concentrated under reduced pressure to obtain the target compound **10D** (0.40 g, crude).

LCMS m/z = 208.2 [M +1]+.

Step 4: Compound **10D** (0.40 g, 1.91 mmol) and compound **1E** (0.55 g, 1.91 mmol) were added to a 100 mL single-mouth flask and dissolved by adding 1,4-dioxane (10 mL), and finally, N,N-diisopropylethylamine (0.74 g, 5.73

mmol) was added. After the addition was complete, the system was placed under nitrogen protection and stirred at 100°C for 16 h, and the reaction liquid was concentrated under reduced pressure to obtain a crude product of the target compound, which was subjected to preparative HPLC to obtain the title compound **10** (25 mg, 4%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 50-90%; c. flow rate: 15 mL/min; d. elution time: 20 min; retention time: 6.45 min.

LCMS m/z = 459.2 $[M+1]^+$.

$^1H$ NMR (400 MHz, DMSO-*d6*) δ7.34-7.36(m, 2H), 7.25-7.30 (m, 3H), 4.86 (t, 1H), 4.14 (s, 2H), 3.92 (s, 2H), 3.70 (d, 2H), 3.35-3.44(m, 2H), 3.17-3.24 (m, 1H), 2.82-2.95(m, 2H), 2.54-2.61 (m, 2H), 2.24-2.34 (m, 4H), 2.10-2.16 (m, 2H), 1.69-1.85 (m, 2H).

**Example 9**

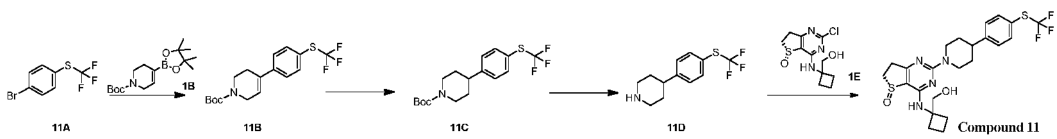

Step 1: Compound **11A** (2.00 g, 7.78 mmol) and compound **1B** (2.89 g, 9.34 mmol) were weighed into a 100 mL single-mouth flask and then dissolved by adding 1,4-dioxane (15 mL), and a sodium carbonate solution (2N, 15 mL) and [1,1'-bis(diphenylphosphino)ferrocene]palladium dichloride (0.57 g, 0.78 mmol) were added to the reaction liquid. After the addition was complete, the mixture was stirred at 100°C for 4 h. After the completion of the reaction was monitored by TLC plate spotting (petroleum ether : ethyl acetate = 5:1), water (20 mL) was added to the reaction liquid and stirred for 5 min, ethyl acetate (20 mL) was added for extraction, and an organic phase was separated. The organic phase was dried over anhydrous sodium sulphate, and the crude product was concentrated under reduced pressure and purified by column chromatography (eluents: petroleum ether : ethyl acetate = 5:1) to obtain the target compound **11B** (2.60 g, 93%).

LCMS m/z = 360.1 $[M+1]^+$.

Step 2: Compound **11B** (0.50 g, 1.39 mmol) was weighed into a 100 mL single-mouth flask and then dissolved by adding ethanol (20 mL), palladium on carbon (0.15 g, 1.39 mmol) was added to the reaction liquid, and after the addition was complete, the mixture was stirred for 16 h under hydrogen condition. After the reaction was complete, the reaction liquid was filtered and the filtrate was concentrated under reduced pressure to obtain the target compound **11C** (0.50 g, 99%).

LCMS m/z = 362.1 $[M+1]^+$.

Step 3: Compound **11C** (0.50 g, 1.38 mmol) was weighed into a 100 mL single-mouth flask and then dissolved by adding methanol (20 mL), a hydrogen chloride dioxane solution (4N, 20 mL) was added, and after the addition was complete, the mixture was stirred for 2 h. The organic phase was concentrated under reduced pressure to obtain the target compound **11D** (0.40 g, crude).

LCMS m/z = 262.2 $[M+1]^+$.

Step 4: Compound **11D** (0.20 g, 0.76 mmol) and compound **1E** (0.22 g, 0.76 mmol) were added to a 100 mL single-mouth flask and dissolved by adding 1,4-dioxane (10 mL), and finally, N,N-diisopropylethylamine (0.29 g, 2.28 mmol) was added. After the addition was complete, the system was placed under nitrogen protection and stirred at 100°C for 16 h, and the reaction liquid was concentrated under reduced pressure to obtain a crude product of the target compound, which was subjected to preparative HPLC to obtain the title compound **11** (240 mg, 61%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution: mobile phase A: 30-70%; c. flow rate: 15 mL/min; d. elution time: 20 min; retention time: 12.08 min.

LCMS m/z = 513.2 $[M+1]^+$.

$^1H$ NMR (400 MHz, DMSO-*d6*) δ7.62(d, 2H), 7.43 (d, 2H), 7.29 (s, 1H), 4.77-4.84(m, 3H), 3.71 (d, 2H), 3.37-3.45 (m, 1H), 3.18-3.26 (m, 1H), 2.82-2.98 (m, 5H), 2.28-2.41(m, 2H), 2.12-2.19 (m, 2H), 1.70-1.85 (m, 4H), 1.48-1.58 (m, 2H).

**Example 10**

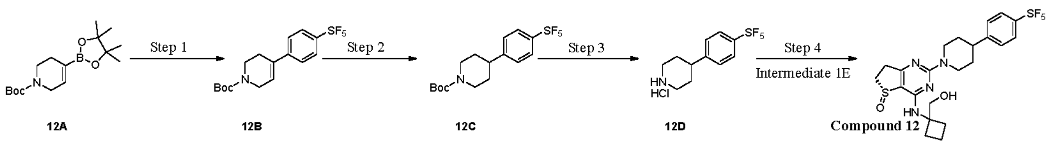

12A 12B 12C 12D Compound 12

Step 1: (4-Bromophenyl)sulphur pentafluoride (1.0 g, 3.55 mmol) and N-Boc-1,2,5,6-tetrahydropyridine-4-boronic acid pinacol ester (**12A**, 1.65 g, 5.33 mmol) were dissolved in 1,4-dioxane (10 mL). A sodium carbonate aqueous solution (2 mol/L, 10 mL) and 1,1'-bis(diphenylphosphino)ferrocene palladium dichloride (200 mg) were added, the mixture was refluxed for 4 hours under nitrogen protection and extracted with ethyl acetate (30 mL × 2), and the resulting product was dried over anhydrous sodium sulphate, concentrated, and then purified by silica gel column chromatography (petroleum ether : ethyl acetate (v/v) = 100:10) to obtain product **(12B)** (700 mg, yield 51%).
LCMS m/z = 330.1 $[M+1-56]^+$.
Step 2: Compound **12B** (700 mg, 1.8 mmol) was dissolved in methanol (10 mL), palladium on carbon (100 mg) was added, a hydrogenation reaction was carried out at room temperature for 3 hours, and after filtration, the filtrate was concentrated to obtain a crude product **(12C)** (700 mg, yield 100%).
LCMS m/z = 332.1 $[M+1-56]^+$.
Step 3: Hydrogen chloride-dioxane (10 mL) was added to compound **12C** (700 mg, 1.8 mmol), and the mixture was reacted at room temperature for 1 hour and concentrated to obtain a crude product **(12D)** (400 mg, yield 72%).
LCMS m/z = 288.1 $[M+1]^+$.
Step 4: Compound **12D** (0.1 g, 0.31 mmol) and intermediate **1E** (89 mg, 0.31 mmol) were dissolved in 1,4-dioxane (10 mL), N,N-diisopropylethylamine (120 mg, 0.93 mmol) was added, the mixture was refluxed and stirred overnight for a reaction. After concentration, the reaction product was directly separated and purified by a liquid phase preparative column (liquid phase preparation conditions: C18 reverse-phase preparative column, mobile phase: deionized water containing 0.1% aqueous ammonia (A) and acetonitrile (B), gradient elution, mobile phase B = 5-50%, elution time: 15 min, flow rate: 12 mL/min, and column temperature: 30°C) to obtain the title compound **12** (50 mg, yield 29%, retention time: about 9.0 min).
$^1$H NMR (400 MHz, DMSO-$d_6$) δ7.79 (d, 2H), 7.50 (d, 2H), 7.30 (s, 1H), 4.91-4.72 (m, 3H), 3.72 (d, 2H), 3.47-3.36 (m, 1H), 3.27-3.17 (m, 1H), 3.02-2.80 (m, 5H), 2.44-2.26 (m, 2H), 2.22-2.09 (m, 2H), 1.87-1.69 (m, 4H), 1.60-1.47 (m, 2H).
MS M/Z (ESI): m/z = 539.1 $[M+1]^+$.

**Example 11**

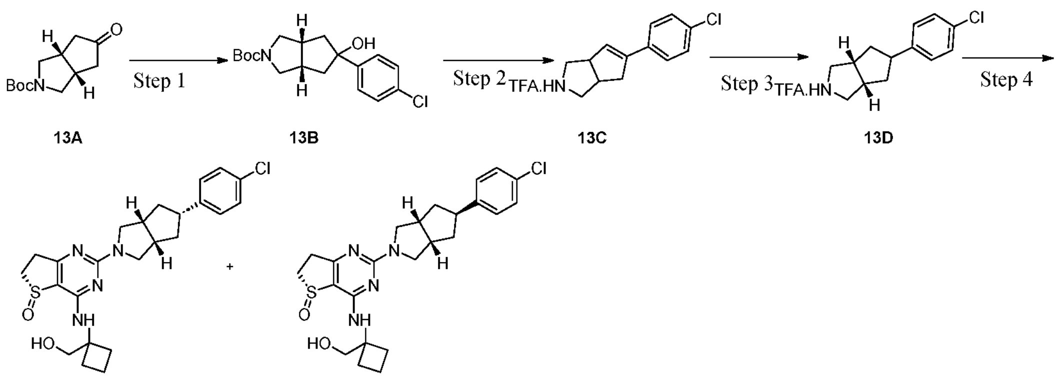

13A 13B 13C 13D
+
Compounds 13, 14

Step 1: p-Chloroiodobenzene (5.82 g, 24.41 mmol) and anhydrous tetrahydrofuran (50 mL) were added to a 250 mL three mouth flask, and after nitrogen displacement, n-butyl lithium (39 mmol, 11.5 mL) was dropwise added at -78°C and reacted at -78°C for 2 h. A solution of **12A** (5 g, 22.19 mmol) in tetrahydrofuran (20 mL) was dropwise added, and the mixture was maintained at - 78°C and reacted for 1 h. Water (100 mL) was added. The mixture was extracted with ethyl acetate (50 mL × 2), the organic phases were combined, washed with a saturated sodium chloride solution, dried over

anhydrous sodium sulphate, and filtered, and the filtrate was concentrated under reduced pressure and then separated and purified by silica gel column chromatography (eluent EA/PE = 0-35%) to obtain the target compound **13B** (4 g, 53.36%).

LC-MS (ESI): m/z = 338.1 $[M+H]^+$.

Step 2: **13B** (4 g, 11.84 mmol) was added to a mixed system of dichloromethane (45 mL) and trifluoroacetic acid (15 mL) and reacted at room temperature for 3 h. The reaction liquid was directly concentrated under reduced pressure and then brought to the next reaction.

LC-MS (ESI): m/z = 220.1 $[M+H]^+$.

Step 3: **13C** (4 g, 11.99 mmol), methanol (50 mL), and palladium on carbon 10% (0.8 g) were added to a 100 mL single-mouth flask and reacted at room temperature for 1 h under hydrogen displacement. After filtration, the filtrate was concentrated under reduced pressure and then separated and purified by HPLC to obtain the target compound **13D** (1.5 g, 37.26%).

LC-MS (ESI): m/z = 222.1 $[M+H]^+$.

Preparative HPLC separation method: 1. Instrument: waters 2767 preparative liquid chromatographic instrument; chromatographic column: SunFire@ Prep C18 (19 mm × 250 mm). 2. The sample was dissolved with DMF and filtered with a 0.45 μm filter to prepare a sample liquid. 3. Preparative chromatography conditions: a. composition of mobile phases A and B: mobile phase A: acetonitrile; mobile phase B: water (containing 0.1% acetic acid); b. gradient elution, mobile phase A: 5-40%; c. flow rate: 12 mL/min. d. elution time: 20 min.

Step 4: Compound **1E** (0.15 g, 0.52 mmol), **13D** (0.21 g, 0.63 mmol), dioxane (5 mL), and DIPEA (0.27 g, 2.05 mmol) were successively added to a 50 mL single-mouth flask and reacted at 100°C for 5 h. The reaction product was cooled to room temperature, and the reaction liquid was directly concentrated under reduced pressure and then separated and purified by silica gel column chromatography (eluent MeOH/DCM = 0-10%) to obtain 220 mg of a mixture.

After chiral preparation, the title compound **13** (0.14 g, 56%, retention time: 0.787 min) and the title compound **14** (20 mg, 8%, retention time: 0.995 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 120 mL/min; back pressure: 100 bar; column temperature: 25°C; wavelength: 220 nm; elution time: 5.5 min.

**Compound 13** $^1$H NMR (400 MHz, DMSO-*d6*) δ 7.34-7.25 (m, 4H), 7.22 (s, 1H), 4.83 (t, 1H), 3.73 (d, 2H), 3.66-3.62 (m, 2H), 3.52-3.50 (m, 2H), 3.45-3.33 (m, 1H), 3.25-3.08 (m, 2H), 2.97-2.74 (m, 4H), 2.44-2.23 (m, 4H), 2.17-2.13 (m, 2H), 1.84-1.67 (m, 2H), 1.45-1.38 (m, 2H).

LC-MS (ESI): m/z = 473.2 $[M+H]^+$.

**Compound 14** $^1$H NMR (400 MHz, DMSO-*d6*) δ 7.35-7.28 (m, 4H), 7.22 (s, 1H), 4.84 (t, 1H), 3.84-3.80 (m, 2H), 3.74 (d, 2H), 3.45-3.33 (m, 3H), 3.24-3.17 (m, 2H), 2.95-2.82 (m, 4H), 2.42-2.30 (m, 2H), 2.18-2.13(m, 2H), 1.94-1.72 (m, 6H).

LC-MS (ESI): m/z = 473.2 $[M+H]^+$.

**Example 12**

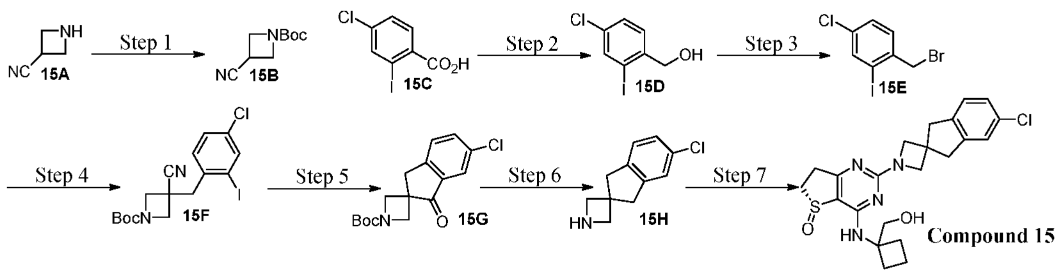


Step 1: Compound **15A** (5 g, 45 mmol) was dissolved in 200 mL of dichloromethane, triethylamine (17 mL, 122 mmol) and di-tert-butyl dicarbonate (11.5 mL, 50 mmol) were added and reacted at room temperature for 5 h. After the raw materials disappeared, a saturated ammonium chloride aqueous solution was added for quenching, followed by extraction with 200 mL of dichloromethane three times and then drying with anhydrous sodium sulphate, and the organic phase was concentrated to obtain compound **15B** (8 g, 95%).

LCMS m/z = 127.2 $[M-55]^+$.

Step 2: In a nitrogen atmosphere, compound **15C** (15 g, 53.2 mmol) was dissolved in 200 mL of tetrahydrofuran. The system was then cooled to 0°C, and a borane tetrahydrofuran solution (133 mL, 1 M in THF) was slowly added. The

mixture was warmed to room temperature, heated to reflux for 3 h, and then cooled to 0°C, and the reaction was quenched by adding 1N hydrochloric acid. The system was concentrated, extracted three times by adding 200 mL of ethyl acetate and dried over anhydrous sodium sulphate, and then the organic phase was concentrated to obtain 15 g of crude compound **15D.** The compound was directly used for the next reaction.

$^1H$ NMR (400 MHz, $CDCl_3$) δ 7.82-7.81 (m, 1H), 7.41-7.33 (m, 2H), 4.64 (s, 2H), 1.92 (s, 1H).

Step 3: Triphenylphosphine (22 g, 83.8 mmol) was dissolved in 250 mL of dichloromethane, and the system was cooled to 0°C. Bromine (4.5 mL, 83.5 mmol) was added slowly and stirred for 10 min, imidazole (7.6 g, 111 mmol) and 15 g of crude compound **15D** were then added, and the mixture was warmed to room temperature and reacted for 3 h. The reaction was quenched by adding sodium thiosulphate, and the reaction product was then extracted with 200 mL of dichloromethane. After drying with anhydrous sodium sulphate, the organic phase was concentrated and separated by column chromatography to obtain compound **15E** (12 g, 68%).

$^1H$ NMR (400 MHz, $CDCl_3$) δ 7.86-7.84 (m, 1H), 7.42-7.29 (m, 2H), 4.55 (s, 2H).

Step 4: In a nitrogen atmosphere, compound **15B** (2 g, 11 mmol) was dissolved in 100 mL of tetrahydrofuran and cooled to -40°C, and sodium bis(trimethylsilyl)amide (11 mL, 2 M in THF) was then added and stirred for 30 min. Compound **15E** (3.6 g, 10.8 mmol) was dissolved in a small amount of tetrahydrofuran and then added to the system. The system was maintained at -40°C and reacted for 2 h. After the reaction was complete, the reaction was quenched by adding a saturated ammonium chloride aqueous solution, and the reaction product was warmed to room temperature and extracted with 100 mL of ethyl acetate three times. After drying with anhydrous sodium sulphate, the organic phase was concentrated and separated by column chromatography to obtain compound **15F** (2 g, 41%).

LCMS m/z = 376.9 $[M-55]^+$.

Step 5: Under nitrogen, compound **15F** (1.9 g, 4.4 mmol) was dissolved in 20 mL of tetrahydrofuran, and the system was cooled to -78°C. n-Butyl lithium (9.5 mL, 2.5 M) was slowly added dropwise, and the mixture was maintained at -78°C and reacted for 15 min. After the raw materials disappeared, the reaction was quenched by adding a saturated ammonium chloride aqueous solution, and the reaction product was extracted with 50 mL of ethyl acetate three times. After drying with anhydrous sodium sulphate, the organic phase was concentrated and separated by column chromatography to obtain compound **15G** (800 mg, 59%).

LCMS m/z = 252.0 $[M-55]^+$.

Step 6: In a nitrogen atmosphere, compound **15G** (400 mg, 1.3 mmol) was dissolved in 20 mL of dichloromethane, boron trifluoride diethyl etherate (1.5 mL, 11.4 mmol) and triethylsilane (3 mL, 23 mmol) were added, and the mixture was warmed to 45°C and reacted for 10 h. After the raw materials disappeared, the reaction system was cooled to room temperature, poured into a saturated sodium bicarbonate aqueous solution, and extracted with 50 mL of dichloromethane, and after drying with anhydrous sodium sulphate, the organic phase was concentrated to obtain 280 mg of crude compound **15H,** which was directly brought to the next reaction.

LCMS m/z = 194.1 $[M+H]^+$.

Step 7: In a nitrogen atmosphere, 280 mg of crude compound **15H,** compound **1E** (150 mg, 0.52 mmol), and DIPEA (0.86 mL, 5.2 mmol) were dissolved in 30 mL of 1,4-dioxane, and the mixture was warmed to 100°C and reacted for 18 h. The reaction system was concentrated and then separated by thin-layer chromatography to obtain **compound 15** (71 mg, 31%).

LCMS m/z = 445.1 $[M+H]^+$.

$^1H$ NMR (400 MHz, DMSO-*d6*) δ 7.33-7.17 (m, 4H), 4.85-4.81 (m, 1H), 3.95 (s, 4H), 3.74-3.61 (m, 2H), 3.44-3.34 (m, 1H), 3.26-3.10 (m, 5H), 2.97-2.81 (m, 2H), 2.40-2.25 (m, 2H), 2.16-2.06 (m, 2H), 1.83-1.63 (m, 2H).

**Example 13**

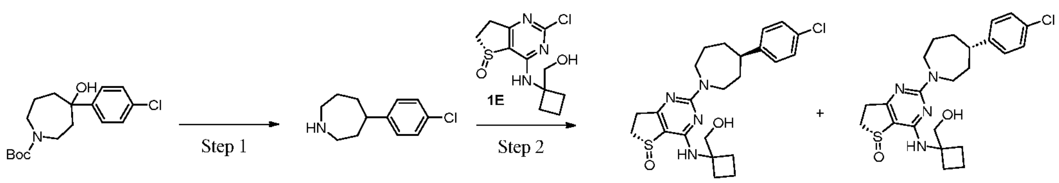


4C 16A Compounds 16, 17

Step 1: **4C** (0.5 g, 1.53 mmol) was added to a 50 mL single-mouth flask and dissolved with dichloromethane (10 mL), triethylsilane (0.53 g, 4.59 mmol) was added, boron trifluoride diethyl etherate (0.43 g, 3.06 mmol) was dropwise added in an ice bath, and the mixture was stirred at room temperature overnight. The reaction liquid was washed with a saturated sodium bicarbonate solution (20 mL) and extracted with dichloromethane (2 × 20 mL), and the organic phases

were combined, dried over anhydrous sodium sulphate, and concentrated to obtain the title compound **16A** (0.35 g, crude).

LC-MS (ESI): m/z = 210.1 [M+H]+.

Step 2: **1E** (0.40 g, 1.39 mmol) was added to a 50 mL single-mouth flask and dissolved with dioxane (10 mL), and **16A** (0.35 g, 1.67 mmol) and diisopropylethylamine (0.54 g, 4.18 mmol) were added and reacted at 100 degrees Celsius overnight. The reaction liquid was concentrated and separated by column chromatography (dichloromethane : methanol = 20 : 1-10 : 1) to obtain 0.2 g of a crude product, which was subjected to chiral preparation to obtain the title compound **16** (40 mg, 6%) and the title compound **17** (35 mg, 5%).

Chiral preparation method: instrument: Waters 150 MGM; chromatographic column: Chiralpak Column; mobile phase: A: carbon dioxide, and B: isopropanol (0.1% aqueous ammonia); isocratic elution: 25% mobile phase B; flow rate: 100 mL/min; back pressure: 100 bar; column temperature: 25°C; wavelength: 220 nm; elution time: 2.8 min.

Compound **16,** retention time: 2.16 min, $^1$H NMR (400 MHz, DMSO-*d6*) $\delta$ 7.29 (d, 2H), 7.22 (d, 2H), 7.16 (s, 1H), 4.85-4.74 (m, 1H), 4.09-3.87 (m, 2H), 3.73-3.61 (m, 3H), 3.45-3.36 (m, 2H), 3.26-3.19 (m, 1H), 3.00-2.77 (m, 2H), 2.71-2.62 (m, 1H), 2.42-2.34 (m, 2H), 2.15-2.10 (m, 2H), 1.98-1.91 (m, 2H), 1.75-1.60 (m, 6H).

Compound **17,** retention time: 2.47 min, $^1$H NMR (400 MHz, DMSO-*d6*) $\delta$ 7.32-7.27 (m, 2H), 7.25-7.13 (m, 3H), 4.86-4.76 (m, 1H), 4.06-3.96 (m, 1H), 3.83-3.64 (m, 4H), 3.58-3.55 (m, 1H), 3.50-3.36 (m, 1H), 3.26-3.18 (m, 1H), 2.98-2.80 (m, 2H), 2.69-2.66 (m, 1H), 2.42-2.32 (m, 2H), 2.21-2.06 (m, 2H), 1.98-1.95 (m, 2H), 1.89-1.53 (m, 6H).

LC-MS (ESI): m/z = 461.2 [M+H]+.

**Example 14**

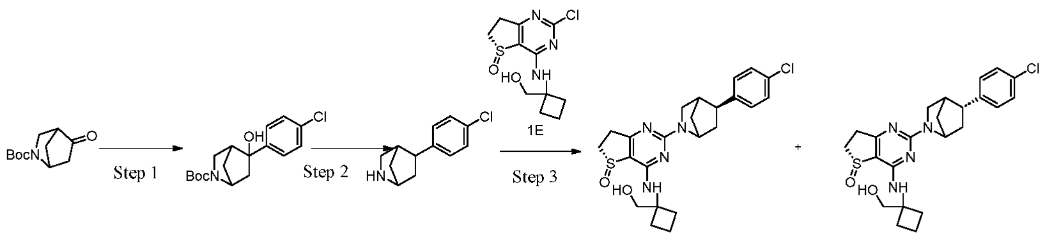


18A 18B 18C Compounds 18,19

Step 1: p-Chloroiodobenzene (2.5 g, 10.42 mmol) and anhydrous tetrahydrofuran (20 mL) were added to a 100 mL three mouth flask, and after nitrogen displacement, n-butyl lithium (12 mmol, 4.8 mL) was dropwise added at - 78°C and reacted at -78°C for 2 h. A solution of **18A** (2 g, 9.5 mmol) in tetrahydrofuran (10 mL) was then added dropwise, and the mixture was maintained at -78°C and reacted for 1 h. Water (100 mL) was added. The mixture was extracted with ethyl acetate (50 mL × 2), the organic phases were combined, washed with a saturated sodium chloride solution, dried over anhydrous sodium sulphate, and filtered, and the filtrate was concentrated under reduced pressure and then separated and purified by silica gel column chromatography (eluent EA/PE = 0-35%) to obtain the target compound **18B** (2.4 g, 78 %).

LC-MS (ESI): m/z = 324.1 [M+H]+.

Step 2: **18B** (2 g, 6.18 mmol), dichloroethane (30 mL), triethylsilane (3.59 g, 30.9 mmol), and boron trifluoride diethyl etherate (4.4 g, 3.81 mmol) were successively added to a 100 ml single-mouth flask. The mixture was reacted at 80°C for 3 h. The reaction product was cooled to room temperature, and the reaction liquid was directly concentrated under reduced pressure and then purified by C18 reverse-phase column, wherein the composition of mobile phases A and B were: mobile phase A: acetonitrile; mobile phase B: water (containing 0.1% TFA), (A/B = 30%/70%). After separation and purification, the title compound **18C** (450 mg, 22%) was obtained.

LC-MS (ESI): m/z = 208.1 [M+H]+.

Step 3: **1E** (0.15 g, 0.52 mmol), **18C** (0.14 g, 0.68 mmol), dioxane (5 mL), and DIPEA (0.27 g, 2.05 mmol) were successively added to a 50 mL single-mouth flask and reacted at 100°C for 5 h. The reaction product was cooled to room temperature, and the reaction liquid was directly concentrated under reduced pressure and then separated and purified by silica gel column chromatography (eluent MeOH/DCM = 0-10%) to obtain 220 mg of a mixture.

After chiral preparation, the title compound **18** (80 mg, 33%, retention time: 1.253 min) and the title compound **19** (78 mg, 32%, retention time: 1.392 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 2.7 min.

**Compound 18:**

$^1$H NMR (400 MHz, DMSO-*d6*) δ 7.37-7.31 (m, 2H), 7.21 (d, 1H), 7.15 (d, 1H), 7.09 (d, 1H), 4.89-4.78 (m, 1H), 4.65 (d, 1H), 3.79-3.69 (m, 1H), 3.65-3.57 (m, 1H), 3.52-3.44 (m, 1H), 3.43-3.33 (m, 1H), 3.27-3.03 (m, 3H), 3.01-2.68 (m, 3H), 2.46-2.22 (m, 2H), 2.21-2.09 (m, 2H), 2.04-1.93 (m, 1H), 1.91-1.58 (m, 5H).
LC-MS (ESI): m/z = 459.2 $[M+H]^+$.

**Compound 19:**

$^1$H NMR (400 MHz, DMSO-*d6*)δ 7.35-7.25(m, 3H), 7.15-7.11 (m, 2H), 4.84 (d, 1H), 4.66 (d, 1H), 3.78-3.71 (m, 1H), 3.66-3.57 (m, 1H), 3.48-3.42(m, 2H), 3.24-3.19 (m, 1H), 3.18-3.04 (m, 2H), 3.00-2.69 (m, 3H), 2.45-2.32 (m, 1H), 2.30-2.11 (m, 3H), 2.05-1.93 (m, 1H), 1.92-1.83 (m, 2H), 1.81-1.59 (m, 3H).
LC-MS (ESI): m/z = 459.2 $[M+H]^+$.

**Example 15**

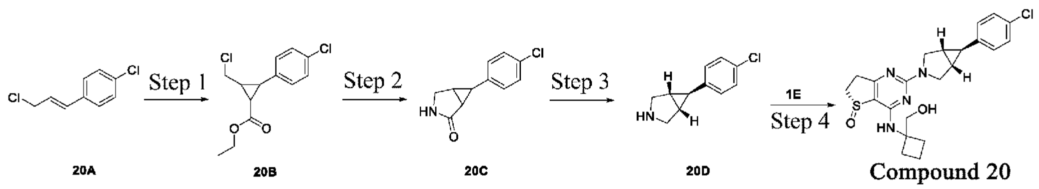


Step 1: Under nitrogen protection at room temperature, to a solution charged with **20A** (2.00 g, 10.70 mmol, synthesis reference: Chemistry-A European Journal, 2019, 25(14), 3521-3524) and rhodium acetate (190 mg, 0.4 mmol) in dichloromethane (200 mL), ethyl diazoacetate (2.50 g, 21.40 mmol) was slowly added dropwise, and after the addition was complete, the mixture was stirred at room temperature overnight. The reaction liquid was filtered by diatomite to obtain a filtrate, which was concentrated in vacuum to obtain a crude product. The crude product was purified by column chromatography (eluents: PE : EA = 100 : 1 to 20 : 1) to obtain the target compound **20B** (1.10 g, yield: 37.67%).
$^1$H NMR (400 MHz, $CDCl_3$) δ 7.19 (d, 2H), 6.97 (d, 2H), 4.20-4.13 (m, 2H), 3.96-3.91 (m, 1H), 3.81-3.76 (m, 1H), 2.60-2.57 (m, 1H), 2.14-2.10 (m, 1H), 2.02-1.96 (m, 1H), 1.27-1.22 (m, 3H).
Step 2: Compound **20B** (1.00 g, 3.66 mmol) was dissolved in a 7N ammonia methanol solution (40 mL), then heated to 80°C, and stirred for 16 h. The reaction liquid was cooled to room temperature and then concentrated under reduced pressure to obtain a crude product, which was purified by column chromatography (eluents: PE : EA = 100 : 1 to 1 : 1) to obtain the target compound **20C** (400 mg, yield: 52.62%).
LC-MS (ESI): m/z = 208.0 $[M+H]^+$ .
$^1$H NMR (400 MHz, $CDCl_3$) δ 7.27-7.23 (m, 2H), 6.99-6.96 (m, 2H), 5.58 (br s, 1H), 3.67-3.63 (m, 1H), 3.55-3.49 (m, 1H), 2.23-2.20 (m, 1H), 2.13-2.09 (m, 2H).
Step 3: Compound **20C** (400 mg, 1.93 mmol) was dissolved in 20 mL of tetrahydrofuran, lithium tetrahydroaluminate (220 mg, 5.79 mmol) was added in portions at 0°C, and the mixture was then warmed to room temperature and stirred for 16 h. 0.3 mL of water, 0.3 mL of 15% sodium hydroxide aqueous solution, and 0.9 mL of water were added, stirred for 1 h, and then suction-filtered to remove solids. The obtained filtrate was spin-dried to obtain compound **20D** (310 mg, 83.10%), which was directly used in the next reaction.
$^1$H NMR (400 MHz, $CDCl_3$) δ 7.22-7.19 (m, 2H), 6.99-6.95 (m, 2H), 3.12 (d, 2H), 2.99 (d, 2H), 1.69-1.64 (m, 3H).
Step 4: Compound **1E** (200 mg, 0.70 mmol) and compound **20D** (150 mg, 0.78 mmol) were dissolved in 1,4-dioxane (8 mL), and DIPEA (270 mg, 2.09 mmol) was then added. After the addition was complete, the mixture was warmed to 100°C under nitrogen protection and stirred for 16 h. After the reaction was complete, the reaction liquid was concentrated under reduced pressure to obtain a crude product, which was purified by column chromatography (eluents: DCM : MeOH = 100 : 1 to 9 : 1) and then further purified by prep.HPLC (preparation method: instrument: waters 2767 preparative liquid chromatographic instrument; chromatographic column: SunFire@ Prep C18 (19 mm × 250 mm; the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 20-85%; c. flow rate: 15 mL/min; d. elution time: 15 min; retention time: 6.0 min) to obtain the target compound 20 (120 mg, 38.80%).
LCMS m/z = 445.1 $[M+1]^+$.
$^1$H NMR (400 MHz, $CDCl_3$) δ 7.22 (d, 2H), 6.95 (d, 2H), 5.86 (s, 1H), 4.88 (s, 1H), 4.11-4.04 (m, 1H), 3.97-3.91 (m,

1H), 3.86 (s, 2H), 3.68-3.59 (m, 3H), 3.45-3.38 (m, 1H), 3.07-2.98 (m, 2H), 2.37-2.25 (m, 4H), 1.99-1.85 (m, 4H), 1.69-1.62 (m, 1H).

**Example 16**

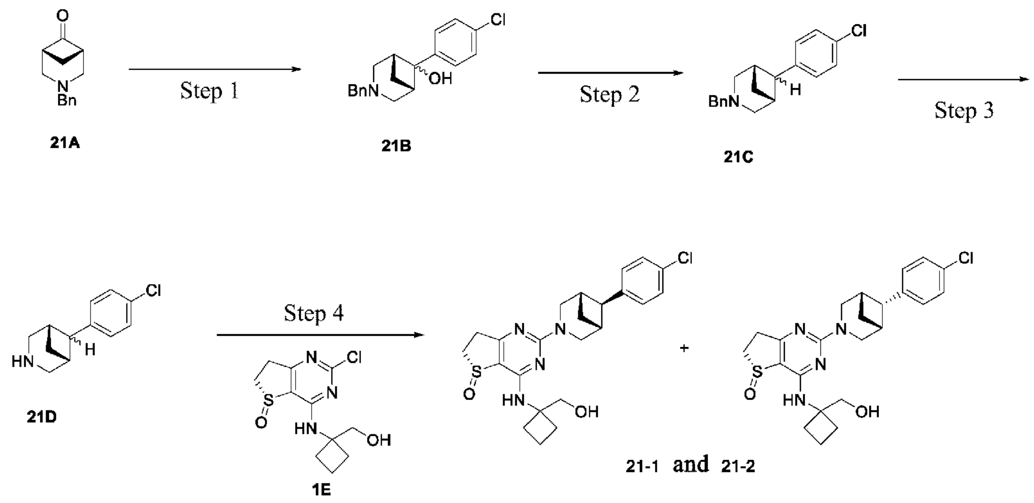


21A 21B 21C 21D 1E 21-1 and 21-2

Step 1: Under nitrogen protection, (1R,5S)-3-benzyl-3-azabicyclo[3.1.1]heptan-6-one (3.00 g, 14.9 mmol) was dissolved in dry tetrahydrofuran (80 mL), and the mixture was cooled to 0°C. A solution of 4-chlorophenylmagnesium bromide in tetrahydrofuran (22.4 mL, 22.4 mmol) was dropwise added, and after the addition was complete, the mixture was reacted at 0°C for about 1 hour. After the completion of the reaction was monitored by TLC, a saturated ammonium chloride aqueous solution (80 mL) was added. After the mixture was warmed to room temperature, the aqueous phase was separated from the organic phases. The aqueous phase was extracted with ethyl acetate (50 mL × 6). The organic phases were combined, dried over anhydrous sodium sulphate, filtered, and concentrated, and the obtained residue was then separated and purified by silica gel column chromatography (petroleum ether : ethyl acetate (v/v) = 5:1) to obtain compound **21B** (3.69 g, 78.8%).

LC-MS (ESI): m/z = 314.2 $[M+H]^+$.

Step 2: Compound **21B** (3.00 g, 9.58 mmol) was dissolved in 1,2-dichloroethane (80 mL), and boron trifluoride diethyl ether complex (4.08 g, 28.8 mmol) and triethylsilane (5.57 g, 47.9 mmol) were added at room temperature. After the addition was complete, the mixture was warmed to 80°C and reacted overnight. After the completion of the reaction was monitored by TLC, the reaction product was cooled to room temperature, a saturated sodium bicarbonate aqueous solution (80 mL) and water (80 mL) were added, the aqueous phase was separated from the organic phase, and the aqueous phase was extracted with ethyl acetate (50 mL × 4). The organic phases were combined, dried over anhydrous sodium sulphate, filtered, and concentrated, and the obtained residue was then separated and purified by silica gel column chromatography (petroleum ether : ethyl acetate (v/v) = 10:1) to obtain compound **21C** (1.38 g, 48.5%).

LC-MS (ESI): m/z = 298.1 $[M+H]^+$.

Step 3: Compound **21C** (700 mg, 2.35 mmol) was added to a microwave reaction tube and dissolved with dry 1,2-dichloroethane (12 mL), and 1-chloroethyl chloroformate (1.68 g, 11.7 mmol) was then added. After the addition was complete, the mixture was placed in a microwave reactor and warmed to 140°C for a reaction for 100 minutes. After the reaction was complete, the reaction liquid was transferred to a round-bottomed flask, suction-dried, re-dissolved by adding methanol (30 mL), and reacted at 70°C for about 3 hours. The reaction liquid was concentrated again and separated and purified by silica gel column chromatography (dichloromethane : methanol (v/v) = 10:1 to 5:1) to obtain compound **21D** (280 mg, 57.4%).

LC-MS (ESI): m/z = 208.0 $[M+H]^+$.

Step 4: **1E** (300 mg, 1.04 mmol), compound **21D** (238 mg, 1.15 mmol), and N,N-diisopropylethylamine (673 mg, 5.21 mmol) were dissolved in 1,4-dioxane (15 mL), and the mixture was warmed to 100°C and reacted for about 3 hours. After the completion of the reaction was monitored by TLC, the reaction product was cooled to room temperature, directly concentrated, and separated and purified by silica gel column chromatography (dichloromethane : methanol (v/v) = 10:1) to obtain compound **21.**

LC-MS (ESI): m/z = 459.2 $[M+H]^+$.

After further chiral preparation, compound **21-1** (80.5 mg, 16.8%) and compound **21-2** (87.4 mg, 18.3%) were

obtained. Chiral preparation method: instrument: Waters 150 MGM; chromatographic column: Chiralpak Column; mobile phase: A: carbon dioxide, and B: methanol (0.1% aqueous ammonia); isocratic elution: 35% mobile phase B; flow rate: 100 mL/min; back pressure: 100 bar; column temperature: 25°C; wavelength: 220 nm; elution time: 2.9 min.

Compound **21-1,** retention time: 1.78 min, $^1$H NMR (400 MHz, Methanol-d4) $\delta$ 7.26 (d, 2H), 7.06 (d, 2H), 3.93 (d, 2H), 3.81 (q, 1H), 3.70-3.49 (m, 3H), 3.42-3.29 (m, 2H), 3.22-3.10(m, 2H), 3.09-2.96 (m, 2H), 2.58 (q, 1H), 2.44-2.16 (m, 3H), 2.13-1.99 (m, 2H), 1.94-1.67 (m, 3H);

Compound **21-2,** retention time: 1.94 min, $^1$H NMR (400 MHz, Methanol-*d4*) $\delta$ 7.26 (d, 2H), 7.06 (d, 2H), 4.02-3.87 (m, 2H), 3.81 (q, 1H), 3.75-3.47 (m, 3H), 3.42-3.29 (m, 2H), 3.21-2.94(m, 4H), 2.58 (q, 1H), 2.44-2.16 (m, 3H), 2.13-1.98 (m, 2H), 1.95-1.68 (m, 3H).

**Example 17**

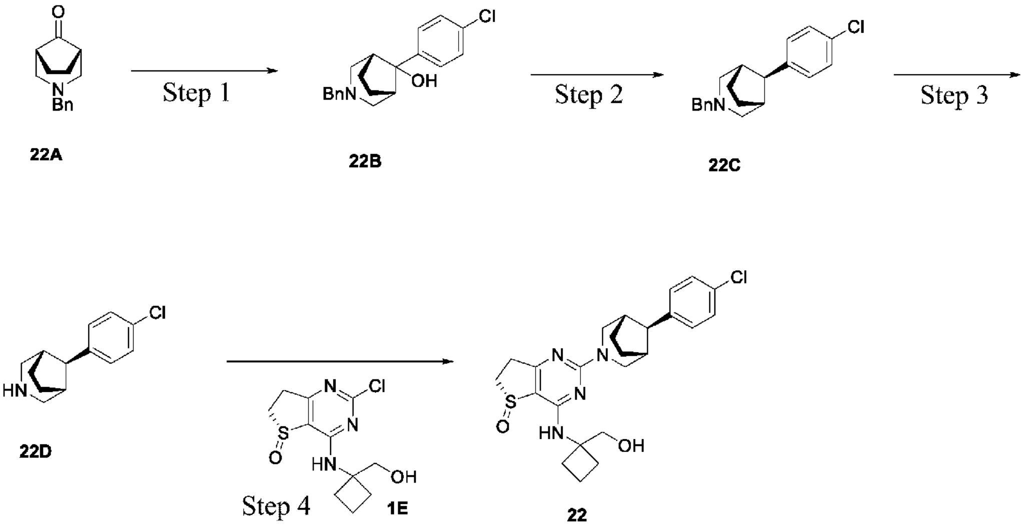


22A 22B 22C

22D 1E 22

Step 1: Under nitrogen protection, 22A (2.0 g, 9.29 mmol) was dissolved in dry tetrahydrofuran (80 mL), and the mixture was cooled to 0°C. A solution of 4-chlorophenylmagnesium bromide in tetrahydrofuran (18.6 mL, 18.6 mmol) was dropwise added, and after the addition was complete, the mixture was reacted at 0°C for about 1 hour. After the completion of the reaction was monitored by TLC, a saturated ammonium chloride aqueous solution (80 mL) was added, and after the mixture was warmed to room temperature, the aqueous phase was separated from the organic phase. The aqueous phase was extracted with ethyl acetate (50 mL × 6). The organic phases were combined, dried over anhydrous sodium sulphate, filtered, and concentrated, and the obtained residue was then separated and purified by silica gel column chromatography (petroleum ether : ethyl acetate (v/v) = 5:1) to obtain compound **22B** (2.89 g, 94.8%).

LC-MS (ESI): m/z = 328.1 $[M+H]^+$.

Step 2: Compound **22B** (710 mg, 2.16 mmol) was dissolved in 1,2-dichloroethane (30 mL), and boron trifluoride diethyl ether complex (920 mg, 6.48 mmol) and triethylsilane (1.26 g, 10.8 mmol) were added at room temperature. After the addition was complete, the mixture was warmed to 80°C and reacted for 3 hours. After the completion of the reaction was monitored by TLC, the reaction product was cooled to room temperature, and a saturated sodium bicarbonate aqueous solution (30 mL) and water (30 mL) were added. The aqueous phase was separated from the organic phase, and the aqueous phase was extracted with ethyl acetate (30 mL × 4). The organic phases were combined, dried over anhydrous sodium sulphate, filtered, and concentrated, and the obtained residue was then separated and purified by silica gel column chromatography (petroleum ether : ethyl acetate (v/v) = 10:1) to obtain compound **22C** (560 mg, 75.6%).

LC-MS (ESI): m/z = 312.1 $[M+H]^+$.

Step 3: Compound **22C** (560 mg, 1.80 mmol) was added to a microwave reaction tube and dissolved with dry 1,2-dichloroethane (12 mL). 1-Chloroethyl chloroformate (1.28 g, 8.98 mmol) was then added, and after the addition was complete, the mixture was placed in a microwave reactor and warmed to 140°C for a reaction for 100 minutes. After the reaction was complete, the reaction liquid was transferred to a round-bottomed flask, suction-dried, re-dissolved by adding methanol (30 mL), and reacted at 70°C for about 3 hours. The reaction liquid was concentrated again and separated and purified by silica gel column chromatography (dichloromethane : methanol (v/v) = 10:1 to 5:1) to obtain **22D** (340 mg, 85.4%).

LC-MS (ESI): m/z = 222.1 $[M+H]^+$.

Step 4: **1E** (300 mg, 1.04 mmol), compound **22D** (255 mg, 1.15 mmol), and N,N-diisopropylethylamine (673 mg, 5.21 mmol) were dissolved in 1,4-dioxane (15 mL), and the mixture was warmed to 100°C and reacted for about 3 hours. After the completion of the reaction was monitored by TLC, the reaction liquid was cooled to room temperature, directly concentrated, and preliminarily separated and purified by silica gel column chromatography (dichloromethane : methanol (v/v) = 10:1) to obtain **22,** and after further HPLC preparation and purification, compound **22** (450 mg, 91.1%) was obtained.

HPLC preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 2.9 min. Retention time: 1.81 min.

$^1$H NMR (400 MHz, Methanol-*d4*) $\delta$ 7.44-7.20 (m, 4H), 4.26 (d, 2H), 3.91 (dd, 2H), 3.52 (dt, 1H), 3.38-3.27 (m, 1H, overlapped), 3.20-3.07 (m, 3H), 3.07-2.97 (m, 2H), 2.85-2.76 (m, 2H), 2.38-2.21 (m, 4H), 2.03-1.83 (m, 4H), 1.74-1.61 (m, 2H).

LC-MS (ESI): m/z = 473.5 [M+H]$^+$.

**Example 18**

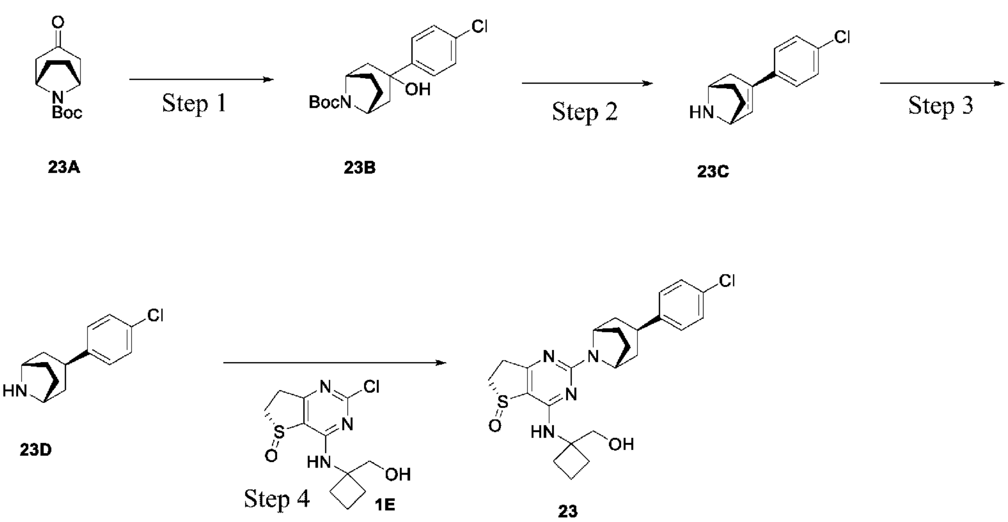


23A 23B 23C 23D 1E 23

Step 1: Under nitrogen protection, p-chloroiodobenzene (6.19 g, 26.0 mmol) was dissolved in dry tetrahydrofuran (150 mL), the mixture was cooled to -78°C, and n-butyl lithium in n-hexane solution (10.4 mL, 26.0 mmol) was then dropwise added. After the addition was complete, the reaction continued for about 1 hour. Furthermore, **23A** (4.5 g, 20.0 mmol) in dry tetrahydrofuran (80 mL) was dropwise added, and after the addition was complete, the mixture was warmed to -10°C and stirred for 2 hours. After the completion of the reaction was monitored by TLC, a saturated ammonium chloride aqueous solution (200 mL) was added, and the mixture was warmed to room temperature, after which the aqueous phase was separated from the organic phase. The aqueous phase was extracted with ethyl acetate (50 mL × 6). The organic phases were combined, dried over anhydrous sodium sulphate, filtered, and concentrated, and the obtained residue was then separated and purified by silica gel column chromatography (petroleum ether : ethyl acetate (v/v) = 5:1) to obtain compound **23B** (5.70 g, 84.4%).

LC-MS (ESI): m/z = 338.2 [M+H]$^+$.

Step 2: Compound **23B** (3.00 g, 15.1 mmol) was dissolved in dichloromethane (50 mL), and trifluoroacetic acid (10 mL) was added at room temperature. After the addition was complete, the mixture was reacted for another 1 hour. After the completion of the reaction was monitored by TLC, the reaction product was directly concentrated to obtain **23C** trifluoroacetate, and the crude product was directly brought to the next reaction without further purification.

LC-MS (ESI): m/z = 220.1 [M+H]$^+$.

Step 3: The crude compound **23C** trifluoroacetate (4.10 g) was added to a round-bottomed flask and dissolved by adding methanol (80 mL), and a palladium on carbon catalyst (615 mg, 0.580 mmol) was then added. A hydrogen balloon was inserted, and the mixture was maintained at room temperature and reacted for 20 minutes. After the disappearance of raw materials was monitored by TLC, the reaction liquid was directly filtered and washed with methanol. The obtained filtrate was suction-dried under reduced pressure and then directly sent to preparative liquid phase purification to obtain compound **23D** trifluoroacetate (312 mg, two-step yield 10.5%).

Preparative HPLC separation purification method: 1. Instrument: waters 2767 preparative liquid chromatographic instrument; chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.5% trifluoroacetic acid). b. gradient elution, mobile phase A: 5-50%; c. flow rate: 12 mL/min; d. elution time: 10 min,

LC-MS (ESI): m/z = 222.1 $[M+H]^+$.

Step 4: **1E** (266 mg, 0.923 mmol), compound **23D** trifluoroacetate (310 mg, 0.923 mmol), and *N,N*-diisopropylethylamine (595 mg, 4.62 mmol) were dissolved in 1,4-dioxane (15 mL), and the mixture was warmed to 100°C and reacted for about 3 hours. After the completion of the reaction was monitored by TLC, the reaction liquid was cooled to room temperature, directly concentrated, and preliminarily separated and purified by silica gel column chromatography (dichloromethane : methanol (v/v) = 10:1), and after concentration followed by HPLC preparation and purification, compound **23** (252 mg, 57.7%) was obtained.

HPLC preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 2.9 min. Retention time: 1.62 min.

$^1$H NMR (400 MHz, Methanol-*d4*) δ 7.23 (q, 4H), 4.81-4.76 (m, 1H), 4.75-4.68 (m, 1H), 4.01-3.87 (m, 2H), 3.66-3.53 (m, 1H), 3.44-3.33 (m, 1H), 3.16-3.02 (m, 2H), 2.57-2.43 (m, 3H), 2.43-2.23 (m, 4H), 2.13-2.00 (m, 2H), 2.01-1.84 (m, 2H), 1.79 (d, 2H), 1.75-1.61 (m, 2H).

LC-MS (ESI): m/z = 473.3 $[M+H]^+$.

**Example 19**

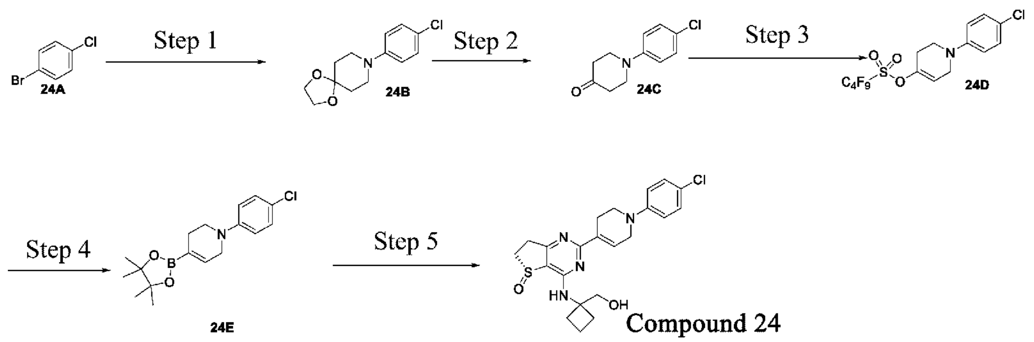


Step 1: 1-Bromo-4-chlorobenzene (15.0 g, 78.3 mmol), 1,4-dioxa-8-azaspiro[4.5]decane (11.8 g, 82.2 mmol), 1,1'-binaphthyl-2,2'-bisphenylphosphine (2.9 g, 4.7 mmol), and tris[dibenzylideneacetone]dipalladium (2.2 g, 2.4 mmol) were dissolved in 150 mL of dioxane, the mixture was then stirred at room temperature for 15 min, and sodium tert-butoxide (0.75 g, 7.8 mmol) was added. The system was warmed to 80°C and reacted for 2 h. The reaction system was poured into ice water and extracted twice with ethyl acetate, the organic phases were combined and dried, and the solvent was removed by rotary evaporation. A mobile phase of PE : EA = 20:1 was used for purification by silica gel column chromatography to obtain the target compound **24B** (3.5 g, yield 19%).

LCMS (ESI): m/z = 254.2 $[M+H]^+$.

Step 2: The raw material **24B** (3.5 g, 13.8 mmol) was dissolved in 30 mL of tetrahydrofuran, and 120 mL of 10% sulphuric acid was added, heated to 90°C, and reacted under reflux for 12 h. After the reaction was confirmed to be complete by TLC plate spotting, the mixture was cooled to room temperature and then adjusted to neutral pH with saturated sodium bicarbonate. After washing with 100 mL × 3 of ethyl acetate, the organic phases were washed with a saturated sodium chloride aqueous solution and dried over anhydrous sodium sulphate. The solvent was removed by rotary evaporation to obtain **24C** (2.2 g, yield 76%), which was directly used in the next reaction.

LCMS (ESI): m/z = 210.1 $[M+H]^+$.

Step 3: The raw material **24C** (2.2 g, 10.5 mmol) and 1,1,2,2,3,3,4,4,4-nonafluorobutane-1-sulfonyl fluoride (3.82 g, 12.6 mmol) were dissolved in 25 mL of tetrahydrofuran, and DBU (1.92 g, 12.6 mmol) was added and stirred for 4 h at room temperature. After the completion of the reaction was monitored by TLC, the reaction product was concentrated and then purified by silica gel column chromatography with the mobile phase system PE : EA = 50 : 1 to obtain **24D** (3.9 g, yield 75%).

LCMS (ESI): m/z = 492.1 $[M+H]^+$.

Step 4: The raw material **24D** (3.9 g, 8.0 mmol), pinacol diboronate (2.42 g, 9.6 mmol), [1,1'-bis(diphenylphosphino)ferrocene]palladium dichloride (173 mg, 0.24 mmol), and 1,1'-bis(diphenylphosphino)ferrocene (132 mg, 0.24 mmol) were suspended in dioxane (100 mL) and reacted at room temperature under stirring for 15 min. Potassium acetate (2.4 g, 24.5 mmol) was added and reacted at 90°C for 16 h. TLC, LCMS (ESI): After the completion of the reaction was monitored, sample mixing was directly carried out for silica gel column chromatography with PE:EA = 20:1 as the mobile phase to obtain the title compound **24E** (0.9 g, yield 38%).

LCMS (ESI): m/z = 320.1 $[M+H]^+$.

Step 5: Compound **24E** (900 mg, 2.8 mmol), XphosPd G2 (443 mg, 0.56 mmol), Xphos (538 mg, 1.12 mmol), and potassium phosphate (1.50 g, 7.0 mmol) were suspended in 10 mL of a mixed solvent of dioxane : water = 4:1 and reacted at 90°C for 4 h. After the reaction was complete, the reaction liquid was dropwise added to ice water and filtered. The filtrate was extracted with dichloromethane three times, the organic phases were combined with the filter residue, concentrated, and subjected to silica gel column chromatography with DCM : MeOH = 15 : 1 as the mobile phase to obtain the target compound **24** (280 mg, 22%).

$^1$H NMR (400 MHz, DMSO-*d6*) δ 7.84-7.82 (s, 1H), 7.28-7.19 (m, 3H), 7.03-6.94 (m, 2H), 4.87-4.83 (m, 1H), 3.94-3.90 (m, 2H), 3.77-3.73 (m, 2H), 3.56 (m, 1H), 3.46-3.40 (m, 2H), 3.35-3.30(m, 1H), 3.20-3.07 (m, 1H), 3.03-2.96 (m 1H), 2.68-2.64 (s, 2H), 2.44-2.18 (m, 4H), 1.88-1.72 (m, 2H).

LCMS (ESI): = 445.1 $[M+H]^+$.

**Example 20**

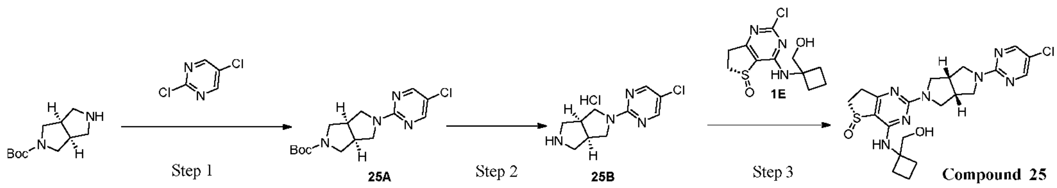


Step 1: Tert-butyl (3aR,6aS)-hexahydropyrrolo[3,4-c]pyrrole-2(1H)-carboxylate (2.12 g, 10 mmol), 2,5-dichloropyrimidine (1.64 g, 11 mmol), tris[dibenzylideneacetone]dipalladium (915 mg, 1 mmol), 2-dicyclohexylphosphine -2',4',6'-triisopropyl biphenyl (952 mg, 2 mmol), and sodium tert-butoxide (1.15 g, 12 mmol) were weighed into a 250 mL three-mouth flask, and toluene (50 mL) and tert-butyl alcohol (10 mL) were added. After nitrogen displacement, the reaction was carried out at 100°C for 2 h. After the completion of the reaction was monitored by LCMS, the system was cooled to room temperature and filtered, and the filter cake was washed with ethyl acetate (50 mL). The filtrate was concentrated and then separated by silica gel column chromatography (PE:EA = 10:1 to 5:1) to obtain the target compound **25A** (2.86 g, 88.2%).

LC-MS (ESI): m/z = 325.1 $[M+H]^+$.

Step 2: **25A** (2.86 g, 0.85 mmol) was dissolved in methanol (30 mL), and a solution of hydrogen chloride dioxane (5 mL, 4 M) was added. The mixture was reacted at room temperature for four hours and spin-dried to obtain title compound **25B** (2.01 g, crude).

LC-MS (ESI): m/z = 225.1 $[M+H]^+$.

Step 3: **1E** (287 mg, 1 mmol) and **25B** (300 mg) were dissolved in 1,4-dioxane (20 mL), DIPEA (1 mL) was added, and the mixture was left at 90°C overnight. After the complete reaction of raw materials was detected by LCMS, the system was concentrated, water (20 mL) was added, and the mixture was extracted with a mixed solution of DCM:MeOH = 20:1 (20 mL × 3). The organic phases were combined, dried over anhydrous sodium sulphate, concentrated, and then passed through a column (DCM:MeOH = 1:0 to 0:1) to obtain **compound 25** (262 mg, 55.1%).

$^1$H NMR (400 MHz, DMSO-$d_6$) δ 8.39 (s, 2H), 7.22 (s, 1H), 4.84-4.71 (m, 1H), 3.82-3.69 (m, 6H), 3.47-3.34 (m, 5H), 3.25-3.15 (m, 1H), 3.12-3.03 (m, 2H), 2.96-2.79 (m, 2H), 2.42-2.27(m, 2H), 2.19-2.10 (m, 2H), 1.85-1.67 (m, 2H).

LC-MS (ESI): m/z = 476.5 $[M+H]^+$.

**Example 21**

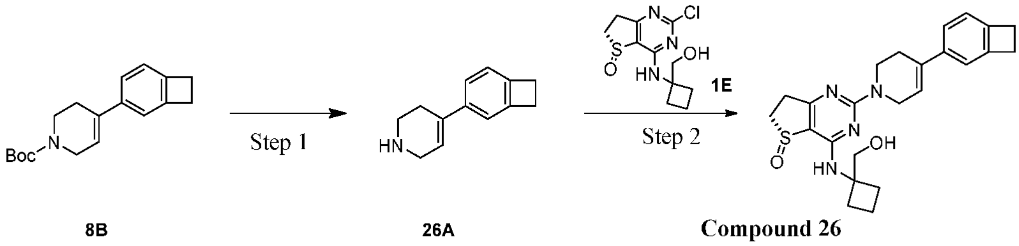


8B 26A Compound 26

Step 1: Compound **8B** (0.50 g, 1.75 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure until no liquid dripped out to obtain the target compound **26A** (0.40 g, yield: 100%).

LCMS m/z = 186.1 [M +1]$^+$.

Step 2: Compound **1E** (0.20 g, 0.69 mmol), compound **26A** (0.15 g, 0.83 mmol), and N,N-dimethylethylamine (0.27 g, 2.07 mmol) were added to a 100 mL single-mouth flask. After the addition was complete, the system was placed under nitrogen protection and stirred at 90°C for 16 h, and the reaction liquid was concentrated under reduced pressure to obtain a crude product of the target compound, which was subjected to preparative HPLC to obtain the title compound **26** (170 mg, 56%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 40-80%; c. flow rate: 15 mL/min; d. elution time: 20 min; retention time: 11.25 min.

LCMS m/z = 437.2 [M +1]$^+$.

$^1$H NMR (400 MHz, DMSO-*d6*) δ7.31 (s, 1H), 7.25 (d, 1H), 7.16 (s, 1H), 7.04 (s, 1H), 6.13 (s, 1H), 4.82 (t, 1H), 4.33 (s, 2H), 3.96 (t, 2H), 3.74 (d, 2H), 3.46-3.38(m, 1H), 3.30 (s, 2H), 3.18-3.25 (m, 1H), 3.12 (s, 4H), 2.83-2.98 (m, 2H), 2.30-2.42(m, 2H), 2.15-2.23 (m, 2H), 1.75-1.85 (m, 2H).

## Example 22

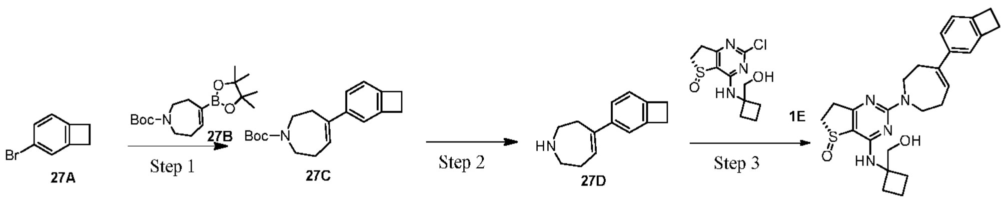


27A 27C 27D Compound 27

Step 1: Compound **27A** (2.00 g, 10.91 mmol) and compound **27B** (3.52 g, 10.91 mmol, referring to Patent WO 2021158829 for the synthesis method) were weighed into a 100 mL single-mouth flask and then dissolved by adding 1,4-dioxane (15 mL), and a sodium carbonate solution (2N, 15 mL) and [1,1'-bis(diphenylphosphino)ferrocene]palladium dichloride (0.71 g, 1.09 mmol) were added to the reaction liquid. After the addition was complete, the mixture was stirred at 100°C for 4 h. After the completion of the reaction was monitored by TLC plate spotting (petroleum ether : ethyl acetate = 5:1), water (20 mL) was added to the reaction liquid and stirred for 5 min, ethyl acetate (20 mL) was added for extraction, and an organic phase was separated. The organic phase was dried over anhydrous sodium sulphate, and the crude product was concentrated under reduced pressure and purified by column chromatography (eluents: petroleum ether : ethyl acetate = 5:1) to obtain the target compound **27C** (0.50 g, yield 15%).

LCMS m/z = 300.1 [M +1]$^+$.

Step 2: Compound **27C** (0.50 g, 1.67 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure until no liquid dripped out to obtain the target compound **27D** (0.40 g, yield: 100%).

LCMS m/z = 200.2 [M +1]$^+$.

Step 3: Compound **1E** (0.20 g, 0.69 mmol), compound **27D** (0.17 g, 0.83 mmol), and N,N-dimethylethylamine (0.27 g, 2.07 mmol) were added to a 100 mL single-mouth flask. After the addition was complete, the system was placed

under nitrogen protection and stirred at 100°C for 4 h, and the reaction liquid was concentrated under reduced pressure to obtain a crude product of the target compound, which was subjected to preparative HPLC to obtain the title compound **27** (12 mg, 3.9%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 40-80%; c. flow rate: 15 mL/min; d. elution time: 15 min; retention time: 9.0 min.

LCMS m/z = 451.6 $[M +1]^+$.

$^1H$ NMR (400 MHz, DMSO-*d6*) δ7.24(s, 1H), 7.11 (d, 1H), 6.97-7.00 (m, 2H), 5.84 (t, 1H), 5.82 (t, 1H), 3.77-4.03 (m, 4H), 3.71 (d, 2H), 3.37-3.45 (m, 1H), 3.17-3.25 (m, 1H), 3.09 (s, 4H), 2.83-2.96 (m, 2H), 3.72 (m, 2H), 2.29-2.47 (m, 4H), 2.12-2.18 (m, 2H), 1.69-1.81 (m, 2H).

**Example 23**

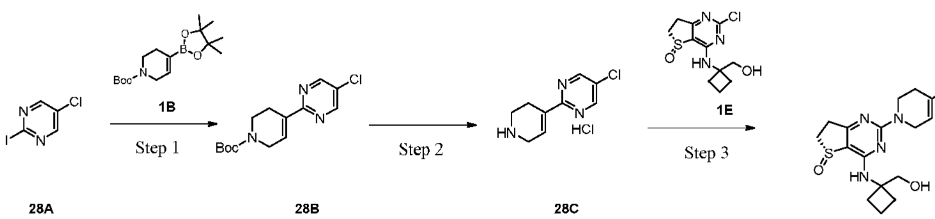


28A 28B 28C Compound 28

Step 1: Substrate **28A** (5.0 g, 20.77 mmol) was added to a 250 mL single-mouth flask and dissolved with dioxane (100 mL) and water (20 mL), and sodium carbonate (6.6 g, 62.31 mmol), **1B** (7.1 g, 22.85 mmol), and [1,1'-bis(diphenylphosphino)ferrocene]palladium dichloride (1.5 g, 2.08 mmol) were added and stirred at 100 degrees Celsius overnight under nitrogen protection. The reaction liquid was diluted by adding water (50 mL) and suction-filtered with diatomite, the filtrate was extracted with ethyl acetate (2 × 100 mL), and the organic phases were combined, dried over anhydrous sodium sulphate, concentrated, and subjected to column chromatography (petroleum ether : ethyl acetate (v/v) = 10 : 1) to obtain the title compound **28B** (5.0 g, 81%).

LC-MS (ESI): m/z = 240.1 $[M-56+H]^+$.

Step 2: Substrate **28B** (1.0 g, 3.38 mmol) was added to a 50 mL single-mouth flask, dissolved with methanol (5 mL) and hydrogen chloride dioxane (4 M, 5 mL), and stirred at room temperature for 3 h, and the reaction liquid was concentrated to obtain the title compound **28C** (0.79 g, 100%).

LC-MS (ESI): m/z = 196.2 $[M+H]^+$.

Step 3: Substrate **1E** (0.20 g, 0.69 mmol) was added to a 50 mL single-mouth flask and dissolved with dioxane (10 mL), **28C** (0.21 g, 0.92 mmol) and diisopropylethylamine (0.26 g, 2.05 mmol) were added and reacted at 90 degrees Celsius overnight, and the reaction liquid was concentrated, purified by HPLC, and freeze-dried to obtain the title compound **28** (130 mg, 42%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution: mobile phase A: 30-70%; c. flow rate: 15 mL/min; d. elution time: 15 min; retention time: 8.0 min.

$^1H$ NMR (400 MHz, DMSO-*d6*) δ 8.89 (s, 2H), 7.34 (s, 1H), 7.31-7.26 (m, 1H), 4.83 (t, 1H), 4.46 (s, 2H), 3.97 (t, 2H), 3.75 (d, 2H), 3.45-3.41 (m, 1H), 3.24-3.21 (m, 1H), 2.98-2.83 (m, 2H), 2.65 (s, 2H), 2.43-2.28 (m, 2H), 2.25-2.14 (m, 2H), 1.90-1.73 (m, 2H).

LC-MS (ESI): m/z = 447.1 $[M+H]^+$.

**Example 24**

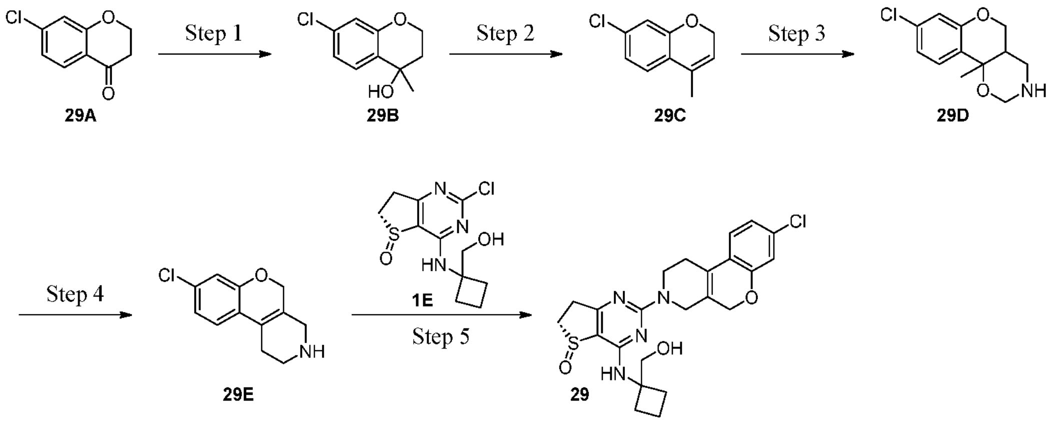


29A 29B 29C 29D 29E 29

Step 1: Compound **29A** (7.3 g, 40.0 mmol) was dissolved in tetrahydrofuran (100 mL), and a methyl magnesium chloride solution (20.0 mL, 3.0 M in THF, 60.0 mmol) was slowly added in ice bath. After the addition was complete, the mixture was slowly warmed to room temperature, reacted for 2 h, and quenched by adding a saturated ammonium chloride solution, and the reaction product was extracted with ethyl acetate (50.0 mL × 3), dried, filtered, and concentrated to obtain compound **29B** (8.0 g, 100%), which was directly used for the next reaction.
LC-MS (ESI): m/z = 181.0 $[M-OH]^+$.
Step 2: Compound **29B** (8.0 g, 40.0 mmol) was dissolved in methanol (100 mL), p-toluenesulfonic acid (350.0 mg, 2.0 mmol) was then added, the mixture was heated to 70°C and reacted for 2 h, and the reaction product was then cooled to room temperature, concentrated to remove the solvent, and subjected to column chromatography (petroleum ether/ethyl acetate = 20/1) to obtain compound **29C** (2.6 g, 36%).
LC-MS (ESI): m/z = 181.0 $[M+H]^+$.
Step 3: Compound **29C** (1.8 g, 10.0 mmol) was dissolved in glacial acetic acid (3.5 mL), a formaldehyde aqueous solution (37% in $H_2O$, 43.0 mmol) and ammonium chloride (1.1 g, 20.0 mmol) were then added, and the mixture was heated to 100°C and reacted for 18 h, then adjusted to alkalinity by adding a sodium hydroxide solution, and extracted with dichloromethane (50 mL × 3). After drying and concentration, compound **29D** (1.9 g, 80%) was obtained, which was used for the next step.
Step 4: Compound **29D** (1.9 g, 8.0 mmol) was dissolved in concentrated hydrochloric acid (30.0 mL), and the mixture was heated to reflux and reacted for 3 h, then cooled to room temperature, adjusted to alkalinity by adding a sodium hydroxide solution, and extracted with ethyl acetate (50 mL × 3). After drying, concentration, and column chromatography (dichloromethane/methanol = 15/1), compound **29E** (0.6 g, 27%) was obtained.
LC-MS (ESI): m/z = 222.0 $[M+H]^+$.
Step 5: Compound **29E** (110.0 mg, 0.5 mmol) was dissolved in 1,4-dioxane (10.0 mL), compound **1E** (140.0 mg, 0.5 mmol) was then added, and the mixture was heated back to 90°C and reacted for 12 h, after which the reaction product was cooled to room temperature, concentrated, and subjected to column chromatography (dichloromethane/methanol = 15/1) to obtain compound **29** (0.05 g, 21%).
LC-MS (ESI): m/z = 473.1 $[M+H]^+$.
$^1H$ NMR (400 MHz, DMSO-*d6*) δ 7.39 (s, 1H), 7.14 (d, 1H), 6.98-6.95 (m, 1H), 6.88 (d, 1H), 4.85-4.82 (m, 1H), 4.74 (s, 2H), 4.20 (s, 2H), 4.01-3.98 (m, 2H), 3.74 (d, 2H), 3.50-3.35 (m, 1H), 3.26-3.18 (m, 1H), 2.97-2.84 (m, 2H), 2.46-2.26 (m, 4H), 2.19 (s, 2H), 1.91-1.70 (m, 2H).

**Example 25**

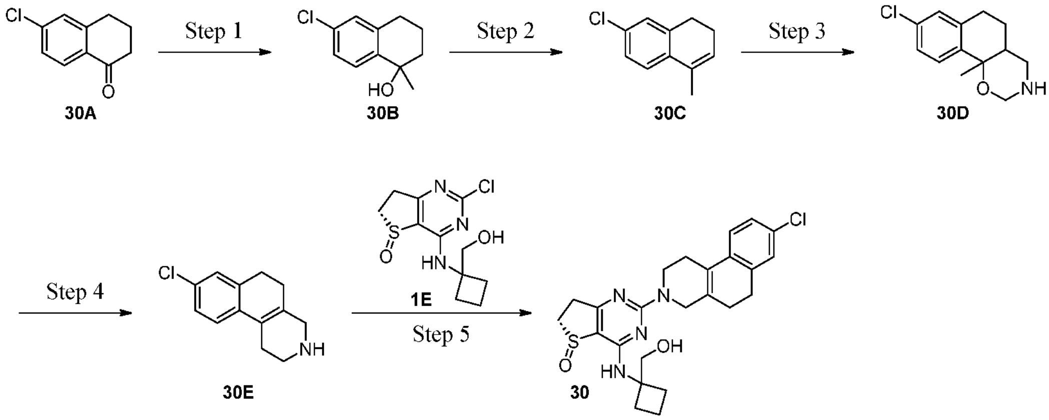


30A 30B 30C 30D 1E 30E 30

Step 1: Compound **30A** (7.2 g, 40.0 mmol) was dissolved in tetrahydrofuran (100 mL), a methyl magnesium chloride solution (20.0 mL, 3.0 M in THF, 60.0 mmol) was slowly added in ice bath. After the addition was complete, the mixture was slowly warmed to room temperature, reacted for 2 h, and quenched by adding a saturated ammonium chloride solution, and the reaction product was extracted with ethyl acetate (50.0 mL × 3), dried, filtered, and concentrated to obtain compound **30B** (8.0 g, 99%), which was directly used for the next reaction.

LC-MS (ESI): m/z = 179.0 [M-OH]+.

Step 2: Compound **30B** (8.0 g, 40.0 mmol) was dissolved in methanol (100 mL), p-toluenesulfonic acid (350.0 mg, 2.0 mmol) was then added, and the mixture was heated to 70°C and reacted for 2 h, after which the reaction product was then cooled to room temperature, concentrated to remove the solvent, and subjected to column chromatography (petroleum ether/ethyl acetate = 20/1) to obtain compound **30C** (5.6 g, 80%).

LC-MS (ESI): m/z = 179.0 [M+H]+.

Step 3: Compound **30C** (3.6 g, 20.0 mmol) was dissolved in glacial acetic acid (7.0 mL), a formaldehyde aqueous solution (37% in $H_2O$, 86.0 mmol) and ammonium chloride (2.2 g, 40.0 mmol) were then added, and the mixture was heated to 100°C and reacted for 18 h, then adjusted to alkalinity by adding a sodium hydroxide solution, and extracted with dichloromethane (50 mL × 3). After drying and concentration, compound **30D** (5.0 g, 89%) was obtained, which was used for the next step.

Step 4: Compound **30D** (5.0 g, 21.0 mmol) was dissolved in concentrated hydrochloric acid (50.0 mL), and the mixture was heated to reflux and reacted for 3 h, then cooled to room temperature, adjusted to alkalinity by adding a sodium hydroxide solution, and extracted with ethyl acetate (50 mL × 3). After drying, concentration, and column chromatography (dichloromethane/methanol = 15/1), compound **30E** (1.0 g, 22%) was obtained.

LC-MS (ESI): m/z = 220.0 [M+H]+.

Step 5: Compound **30E** (110.0 mg, 0.5 mmol) was dissolved in 1,4-dioxane (10.0 mL), compound **1E** (140.0 mg, 0.5 mmol) was then added, and the mixture was heated back to 90°C and reacted for 12 h, after which the reaction product was cooled to room temperature, concentrated, and subjected to column chromatography (dichloromethane/methanol = 15/1) to obtain compound **30** (0.04 g, 19%).

LC-MS (ESI): m/z = 471.1 [M+H]+.

$^1H$ NMR (400 MHz, DMSO-*d6*) δ 7.33 (s, 1H), 7.22 (s, 2H), 7.20-7.13 (m, 1H), 4.85-4.83 (m, 1H), 4.27 (s, 2H), 3.99 (s, 2H), 3.75 (s, 2H), 3.45-3.38 (m, 1H), 3.27-3.16 (m, 1H), 2.97-2.83 (m, 2H), 2.79-2.75 (t, 2H), 2.45-2.34 (m, 4H), 2.20 (d, 4H), 1.79 (d, 2H).

**Example 26**

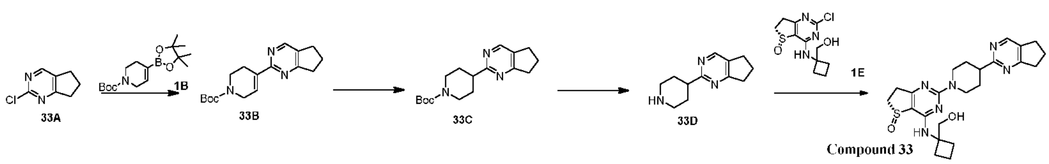

33A 1B 33B 33C 33D 1E Compound 33

Step 1: Compound **33A** (3.00 g, 19.40 mmol) and compound **1B** (7.20 g, 23.28 mmol) were weighed into a 100 mL single-mouth flask and then dissolved by adding 1,4-dioxane (15 mL). A sodium carbonate solution (2N, 15 mL) and [1,1'-bis(diphenylphosphino)ferrocene]palladium dichloride (1.42 g, 1.94 mmol) were added to the reaction liquid. After the

addition was complete, the mixture was stirred at 100°C for 4 h. After the completion of the reaction was monitored by TLC plate spotting (petroleum ether : ethyl acetate = 5:1), water (20 mL) was added to the reaction liquid and stirred for 5 min, ethyl acetate (20 mL) was added for extraction, and an organic phase was separated. The organic phase was dried over anhydrous sodium sulphate, and the crude product was concentrated under reduced pressure and purified by column chromatography (eluents: petroleum ether : ethyl acetate = 5:1) to obtain the target compound **33B** (4.0 g, yield 68%).

LCMS m/z = 302.1 $[M +1]^+$.

$^1H$ NMR (400 MHz, $CDCl_3$) δ 8.46 (s, 1H), 7.10(s, 1H), 4.16(s, 2H), 3.62(t, 2H), 2.92-3.00 (m, 4H), 2.73(s, 2H), 2.11-2.19 (m, 2H), 1.49(s, 9H).

Step 2: Compound **33B** (1.00 g, 3.32 mmol) was weighed into a 100 mL single-mouth flask and then dissolved by adding ethyl acetate (20 mL), and palladium on carbon (0.18 g, 1.66 mmol) was added to the reaction liquid. After the addition was complete, the mixture was stirred for 2 h under hydrogen and atmospheric pressure conditions. After the reaction was complete, the reaction liquid was filtered with diatomite and the filtrate was concentrated under reduced pressure to obtain the target compound **33C** (0.90 g, yield: 89%).

LCMS m/z = 304.2 $[M +1]^+$ .

Step 3: Compound **33C** (0.90 g, 2.97 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure to obtain the target compound **33D** (0.70 g, crude).

LCMS m/z = 204.1 $[M +1]^+$.

Step 4: Compound **1E** (0.20 g, 0.69 mmol), compound **33D** (0.17 g, 0.83 mmol), and N,N-diisopropylethylamine (0.26 g, 2.05 mmol) were added to a 100 mL single-mouth flask. After the addition was complete, the system was placed under nitrogen protection and stirred at 90°C for 16 h, and the reaction liquid was concentrated under reduced pressure to obtain a crude product of the target compound, which was subjected to preparative HPLC to obtain the title compound **33** (120 mg, 38%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 40-80%; c. flow rate: 15 mL/min; d. elution time: 20 min; retention time: 10.38 min.

LCMS m/z = 455.2 $[M +1]^+$.

$^1H$ NMR (400 MHz, DMSO-*d6*) δ 8.50 (s, 1H), 7.29 (s, 1H), 4.84 (s, 1H), 4.71 (d, 2H), 3.72 (t, 2H), 3.37-3.45 (m, 1H), 3.18-3.25 (m, 1H), 2.94-3.15 (m, 4H), 2.82-2.89 (m, 5H), 2.27-2.38(m, 2H), 2.12-2.19 (m, 2H), 2.00-2.11 (m, 2H), 1.86-1.93 (m, 2H), 1.61-1.80 (m, 4H).

**Example 27**

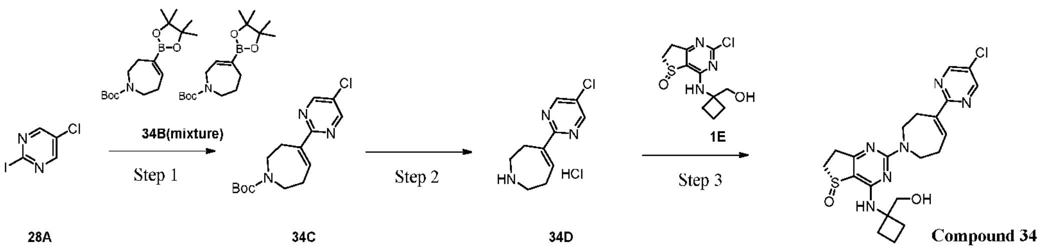


28A 34C 34D Compound 34

Step 1: Substrate **28A** (1.1 g, 4.55 mmol) was added to a 100 mL single-mouth flask and dissolved with dioxane (20 mL) and water (5 mL). Sodium carbonate (1.5 g, 13.70 mmol), **34B** (2.1 g, 4.55 mmol, containing 50% regioisomer, the synthesis method was according to Patent WO 2021158829), and [1,1'-bis(diphenylphosphino)ferrocene]palladium dichloride (0.33 g, 0.45 mmol) were added and stirred at 100 degrees Celsius overnight under nitrogen protection. The reaction liquid was diluted by adding water (50 mL) and suction-filtered with diatomite, and the filtrate was extracted with ethyl acetate (2 × 50 mL). The organic phases were combined, dried over anhydrous sodium sulphate, concentrated, and subjected to column chromatography (petroleum ether : ethyl acetate (v/v) = 10 : 1) to obtain the title compound **34C** (1.0 g, 71%, containing 50% regioisomer).

LC-MS (ESI): m/z = 254.3 $[M-56+H]^+$.

Step 2: Substrate **34C** (1.0 g, 3.23 mmol) was added to a 50 mL single-mouth flask, dissolved with methanol (10 mL) and hydrogen chloride dioxane (4 M, 10 mL), and stirred at room temperature for 3 h, and the reaction liquid was

concentrated to obtain the title compound **34D** (0.80 g, 100%, containing 50% regioisomer).

LC-MS (ESI): m/z = 210.2 $[M+H]^+$.

Step 3: Substrate **1E** (0.10 g, 0.35 mmol) was added to a 50 mL single-mouth flask and dissolved with dioxane (10 mL). **34D** (0.11 g, 0.52 mmol) and diisopropylethylamine (0.14 g, 1.05 mmol) were added and reacted at 90 degrees Celsius overnight, and the reaction liquid was concentrated, purified by HPLC, and freeze-dried to obtain the title compound **34** (16 mg, 10%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 30-80%; c. flow rate: 15 mL/min; d. elution time: 15 min; retention time: 7.5 min.

$^1$H NMR (400 MHz, DMSO-*d6*) δ 8.84 (s, 2H), 7.39 (s, 1H), 7.24 (s, 1H), 4.83 (t, 1H), 3.93-3.88 (m, 4H), 3.73-3.72 (m, 2H), 3.23-3.20 (m, 1H), 3.06-2.79 (m, 5H), 2.60 (s, 2H), 2.42-2.25 (m, 2H), 2.17 (s, 2H), 1.79 (s, 2H).

LC-MS (ESI): m/z = 461.2 $[M+H]^+$.

**Example 28**

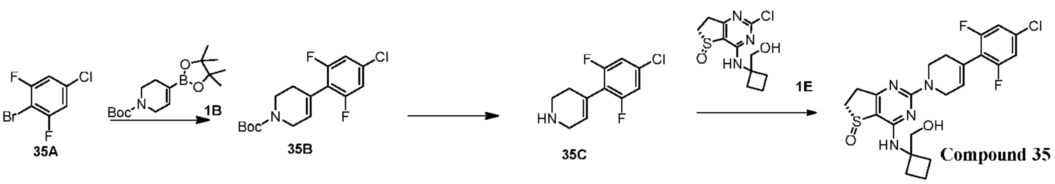

35A 35B 35C Compound 35

Step 1: Compound **35A** (2.00 g, 8.79 mmol) and compound **1B** (3.26 g, 10.55 mmol) were weighed into a 100 mL single-mouth flask and then dissolved by adding 1,4-dioxane (20 mL), and a sodium carbonate solution (2N, 20 mL) and [1,1'-bis(diphenylphosphino)ferrocene]palladium dichloride (0.64 g, 0.88 mmol) were added to the reaction liquid. After the addition was complete, the mixture was stirred at 100°C for 4 h. After the completion of the reaction was monitored by TLC plate spotting (petroleum ether : ethyl acetate = 5:1), water (20 mL) was added to the reaction liquid and stirred for 5 min, ethyl acetate (20 mL) was added for extraction, and an organic phase was separated. The organic phase was dried over anhydrous sodium sulphate, and the crude product was concentrated under reduced pressure and purified by column chromatography (eluents: petroleum ether : ethyl acetate = 5:1) to obtain the target compound **35B** (2.0 g, yield 69%).

LCMS m/z = 330.1 $[M +1]^+$.

$^1$H NMR (400 MHz, $CDCl_3$) δ6.93 (s, 1H), 6.91(s, 1H),5.81(s, 1H), 4.06(d, 2H), 3.62 (t, 2H), 2.38(s, 2H), 1.49(s, 9H).

Step 2: Compound **35B** (0.30 g, 1.02 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 hours. The organic phase was concentrated under reduced pressure to obtain the target compound **35C** (0.20 g, crude).

LCMS m/z = 230.1 $[M +1]^+$.

Step 3: Compound **1E** (0.20 g, 0.69 mmol), compound **35C** (0.19 g, 0.83 mmol), and N,N-diisopropylethylamine (0.26 g, 2.05 mmol) were added to a 100 mL single-mouth flask. After the addition was complete, the system was placed under nitrogen protection and stirred at 90°C for 16 h, and the reaction liquid was concentrated under reduced pressure to obtain a crude product of the target compound, which was subjected to preparative HPLC to obtain the title compound **35** (220 mg, 66%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 40-80%; c. flow rate: 15 mL/min; d. elution time: 20 min; retention time: 11.20 min.

LCMS m/z = 481.1 $[M +1]^+$.

$^1$H NMR (400 MHz, DMSO-*d6*) δ 7.35-7.40 (m, 3H), 6.00 (s, 1H), 4.83 (t, 1H), 4.34 (s, 2H), 3.96 (t, 2H), 3.73 (d, 2H), 3.39-3.47(m, 1H), 3.19-3.26 (m, 1H), 2.84-2.98 (m, 2H), 2.27-2.40 (m, 4H), 2.15-2.21 (m, 2H), 1.69-1.82 (m, 2H).

**Example 29**

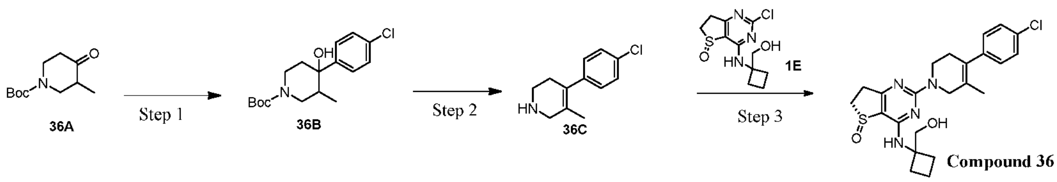


36A 36B 36C 1E Compound 36

Step 1: Compound **36A** (2.00 g, 9.39 mmol) was weighed into a 100 mL single-mouth flask and then dissolved by adding tetrahydrofuran (40 mL). 4-Chlorophenylmagnesium chloride (11.27 mL, 11.27 mmol) was added to the reaction liquid, and after the addition was complete, the mixture was stirred at room temperature for 4 h. After the reaction was found complete by TLC plate spotting (petroleum ether : ethyl acetate = 3:1), water (40 mL) was added to the reaction liquid, and the mixture was stirred for 5 min and extracted by adding ethyl acetate (20 mL). The organic phase was separated and dried over anhydrous sodium sulphate, and the crude product was concentrated under reduced pressure and purified by column chromatography (eluents: petroleum ether : ethyl acetate = 5:1) to obtain the target compound **36B** (2.0 g, yield 65%).

LCMS m/z = 326.1 $[M+1]^+$.

Step 2: Compound **36B** (0.30 g, 0.97 mmol) was weighed into a 100 mL single-mouth flask and then dissolved by adding methanol (10 mL). A hydrogen chloride dioxane solution (4N, 10 mL) was added, and after the addition was complete, the mixture was stirred for 2 h. The organic phase was concentrated under reduced pressure until no liquid dripped out to obtain the target compound **36C** (0.20 g, crude).

LCMS m/z = 208.1 $[M+1]^+$.

Step 3: Compound **1E** (0.20 g, 0.69 mmol), compound **36C** (0.17 g, 0.83 mmol), and N,N-diisopropylethylamine (0.26 g, 2.05 mmol) were added to a 100 mL single-mouth flask. After the addition was complete, the system was placed under nitrogen protection and stirred at 90°C for 16 h, and the reaction liquid was concentrated under reduced pressure and purified by preparative HPLC to obtain the title compound **36** (88 mg, 27%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM ammonium acetate); b. gradient elution, mobile phase A: 30-90%; c. flow rate: 15 mL/min; d. elution time: 20 min; retention time: 12.82 min.

LCMS m/z = 459.2 $[M+1]^+$.

$^1H$ NMR (400 MHz, DMSO-*d6*) $\delta$7.40(d, 2H), 7.32 (s, 1H), 7.24 (d, 2H), 4.84 (t, 1H), 4.20 (s, 2H), 3.94 (t, 2H), 3.73 (d, 2H), 3.39-3.47 (m, 1H), 3.19-3.27 (m, 1H), 2.84-2.99 (m, 2H), 2.29-2.42(m, 4H), 2.14-2.19 (m, 2H), 1.73 -1.84 (m, 2H), 1.63(s, 3H).

**Example 30**

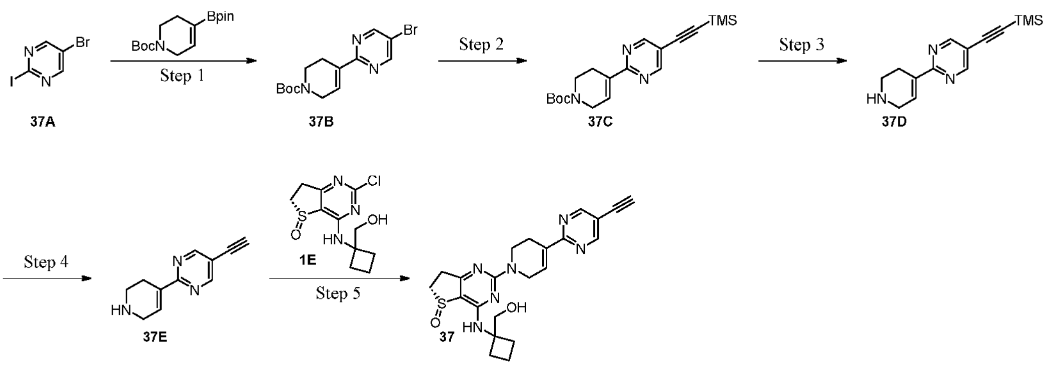


37A 37B 37C 37D 37E 1E 37

Step 1: Compound **37A** (1.4 g, 5.0 mmol) was dissolved in 1,4-dioxane (30 mL), 1,1-bis(diphenylphosphino) ferrocene palladium dichloride (360.0 mg, 0.5 mmol), potassium carbonate (1.4 g, 10.0 mmol), N-Boc-1,2,5,6-tetrahydropyridine-4-boronic acid pinacol ester (1.8 g, 6.0 mmol), and water (6.0 mL) were then added. After nitrogen

displacement three times, the mixture was heated to 45°C and reacted for 14 h, after which the reaction product was passed through a short silica gel column, rinsed with ethyl acetate, concentrated, and subjected to column chromatography (dichloromethane/methanol = 20/1) to obtain compound **37B** (1.7 g, 99%).

LC-MS (ESI): m/z = 284.1 [M-56]+.

Step 2: Compound **37B** (340.0 mg, 1.0 mmol) was dissolved in tetrahydrofuran (10 mL). Bis(triphenylphosphino) palladium dichloride (70.0 mg, 0.1 mmol), triethylamine (202.0 mg, 2.0 mmol), trimethylsilylacetylene (130.0 mg, 1.3 mmol), and cuprous iodide (8.0 mg, 0.05 mmol) were then added. After nitrogen displacement three times, the mixture was heated to 50°C and reacted for 14 h, after which the reaction product was passed through a short silica gel column, rinsed with ethyl acetate, concentrated, and subjected to column chromatography (dichloromethane/methanol = 20/1) to obtain compound **37C** (240.0 mg, 72%).

LC-MS (ESI): m/z = 302.1 [M-56]+.

Step 3: Compound **37C** (240.0 mg, 0.67 mmol) was dissolved in dichloromethane (10 mL), trifluoroacetic acid (1.0 mL) was then added, and the mixture was reacted at room temperature for half an hour and then concentrated to obtain compound **37D** (150.0 mg, 87%), which was directly used in the next step.

LC-MS (ESI): m/z = 258.1 [M+H]+.

Step 4: Compound **37D** (150.0 mg, 0.58 mmol) was dissolved in methanol (10.0 mL), potassium carbonate (150.0 mg, 1.1 mmol) was then added, and the mixture was then reacted at room temperature for 2 h, concentrated, and then subjected to column chromatography (dichloromethane/methanol = 15/1) to obtain compound **37E** (70.0 mg, 65%).

LC-MS (ESI): m/z = 186.1 [M+H]+.

Step 5: Compound **37E** (70.0 mg, 0.38 mmol) was dissolved in 1,4-dioxane (10.0 mL), compound **1E** (115.0 mg, 0.4 mmol) was then added, and the mixture was heated back to 90°C and reacted for 12 h, after which the reaction product was cooled to room temperature, concentrated, and subjected to column chromatography (dichloromethane/methanol = 10/1) to obtain compound **37** (20.0 mg, 12%).

LC-MS (ESI): m/z = 437.1 [M+H]+.

$^1$H NMR (400 MHz, DMSO-*d6*) δ 8.90 (s, 2H), 7.36 (s, 1H), 7.34 (s, 1H), 4.84 (t, 1H), 4.68 (s, 1H), 4.48 (s, 2H), 3.97 (t, 2H), 3.75 (d, 2H), 3.51-3.36 (m, 1H), 3.28-3.18 (m, 1H), 2.98-2.84 (m, 2H), 2.66 (s, 2H), 2.43-2.30 (m, 2H), 2.24-2.17 (m, 2H), 1.86-1.71 (m, 2H).

**Example 31**

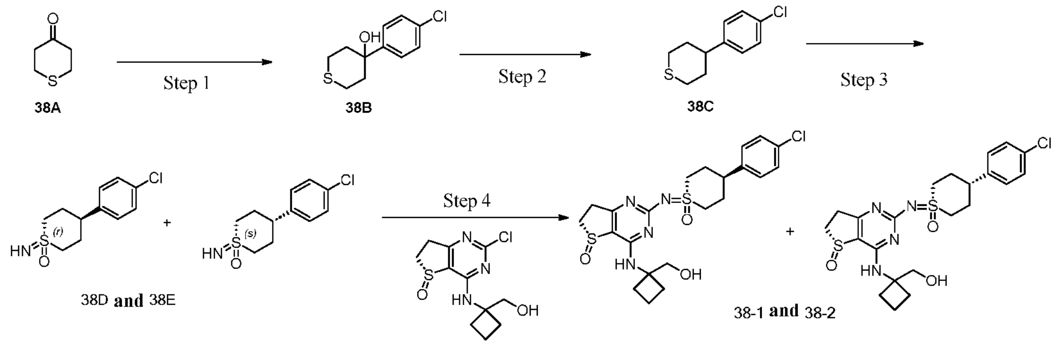


Step 1: Under nitrogen protection, **38 A** (2.50 g, 21.5 mmol) was dissolved in dry tetrahydrofuran (80 mL), and the mixture was cooled to 0°C. A solution of 4-chlorophenylmagnesium bromide in tetrahydrofuran (32.3 mL, 32.3 mmol) was dropwise added, and after the addition was complete, the mixture was reacted at 0°C for about 1 hour. After the completion of the reaction was monitored by TLC, a saturated ammonium chloride aqueous solution (100 mL) was added. After the mixture was warmed to room temperature, the aqueous phase was separated from the organic phase. The aqueous phase was extracted with ethyl acetate (50 mL × 5). The organic phases were combined, dried over anhydrous sodium sulphate, filtered, and concentrated, and the obtained residue was then separated and purified by silica gel column chromatography (petroleum ether : ethyl acetate (v/v) = 5:1) to obtain compound **38B** (4.51 g, 91.7%).

LC-MS (ESI): m/z = 229.1 [M+H]+.

Step 2: Compound **38B** (4.00 g, 17.5 mmol) was dissolved in 1,2-dichloroethane (200 mL), and triethylsilane (10.2 g, 87.4 mmol) and trifluoroacetic acid (5.98 g, 52.5 mmol) were successively added at room temperature. After the addition was complete, the reaction continued overnight at room temperature. After the completion of the reaction was monitored by TLC, a saturated sodium bicarbonate aqueous solution (80 mL) and water (80 mL) were added, and the aqueous phase was separated from the organic phase. The aqueous phase was extracted with ethyl acetate (50 mL × 4).

The organic phases were combined, dried over anhydrous sodium sulphate, filtered, and concentrated, and the obtained residue was then preliminarily separated and purified by silica gel column chromatography (petroleum ether : ethyl acetate (v/v) = 10:1) to obtain crude compound **38C.** After further purification by preparative HPLC, compound **38C** (950 mg, 25.5%) was obtained.

Preparative HPLC separation purification method: 1. Instrument: waters 2767 preparative liquid chromatographic instrument; chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 5-50%; c. flow rate: 12 mL/min; d. elution time: 30 min. Compound **38C,** retention time: 17.2 min.

$^1H$ NMR (400 MHz, Chloroform-*d*) δ 7.27 (d, 2H), 7.12 (d, 2H), 2.83 (ddd, 2H), 2.75-2.65 (m, 2H), 2.50 (tt, 1H), 2.11 (dq, 2H), 1.82 (qd, 2H).

Step 3: Compound **38C** (600 mg, 2.82 mmol) and ammonium carbamate (418 mg, 5.36 mmol) were added to methanol (15 mL) and stirred until uniform, and iodobenzene diacetic acid (2.27 g, 7.05 mmol) was then added. After the addition was complete, the mixture was reacted overnight at room temperature in an air environment. After the reaction was complete, a saturated ammonium chloride aqueous solution (50 mL) was added, and the aqueous phase was extracted with ethyl acetate (30 mL × 6). The organic phases were combined, dried over anhydrous sodium sulphate, filtered, and concentrated, and the obtained residue was then separated and purified by silica gel column chromatography (petroleum ether : ethyl acetate (v/v) = 1:2) to obtain compound **38D** (250 mg, 36.4%) and **38E** (240 mg, 34.9%).

LC-MS (ESI): m/z = 244.1 $[M+H]^+$ .

Step 4: Under nitrogen protection, compound **38D** (150 mg, 0.615 mmol), (R)-2-chloro-4-(1-(hydroxymethyl) cyclobutyl)amino)-6,7-dihydrothieno[3,2-d]pyrimidine 5-oxide (230 mg, 0.800 mmol), palladium acetate (27.6 mg, 0.123 mmol), 2,2'-bis(diphenylphosphino)-1,1'-binaphthyl (153 mg, 0.246 mmol), and potassium phosphate (327 mg, 1.54 mmol) were dissolved in toluene (10 mL), warmed to 110°C, and reacted for about 5 hours. After the completion of the reaction was monitored by TLC, the reaction product was cooled to room temperature, directly concentrated, and preliminarily separated and purified by silica gel column chromatography (dichloromethane : methanol (v/v) = 10:1), and after preparative HPLC purification, compound **38-1** (95 mg, 31.2%) was obtained.

Preparative HPLC separation purification method: 1. Instrument: waters 2767 preparative liquid chromatographic instrument; chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: methanol; mobile phase B: water (containing 0.1% ammonium acetate); b. gradient elution, mobile phase A: 5-50%; c. flow rate: 12 mL/min; d. elution time: 30 min.

Compound **38-1,** retention time: 20.2 min, $^1H$ NMR (400 MHz, DMSO-*d*6) δ 7.42-7.32 (m, 3H), 7.29-7.21 (m, 2H), 4.88 (t, 1H), 4.00-3.87 (m, 2H), 3.77-3.63 (m, 2H), 3.58-3.39 (m, 3H), 3.30-3.22 (m, 1H), 3.12-2.83 (m, 3H), 2.40-2.17 (m, 4H), 2.16-2.00 (m, 4H), 1.87-1.67 (m, 2H).

LC-MS (ESI): m/z = 495.1 $[M+H]^+$;
referring to the above synthesis method, compound **38-2** (107 mg, 35.1%) was synthesized from compound **38E** (150 mg, 0.615 mmol) as a raw material.

Compound **38-2,** retention time: 16.3 min, $^1H$ NMR (400 MHz, DMSO-*d*6) δ 7.45-7.36 (m, 2H), 7.36-7.26 (m, 3H), 4.90 (t, 1H), 3.89-3.75 (m, 2H), 3.75-3.63 (m, 2H), 3.58-3.37 (m, 3H), 3.29-3.22 (m, 1H), 3.10-2.83 (m, 3H), 2.39-2.24 (m, 2H), 2.24-2.09 (m, 6H), 1.86-1.65 (m, 2H).

LC-MS (ESI): m/z = 495.1 $[M+H]^+$.

**Example 32**

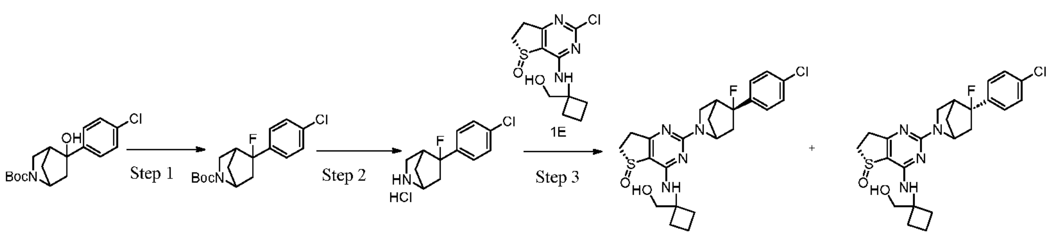


18B 39A 39B Compounds 39-1,39-2

Step 1: **18B** (500 mg, 1.55 mmol) and dichloromethane (20 mL) were added to a 100 mL three mouth flask. After nitrogen displacement, bis(2-methoxyethyl)aminosulphur trifluoride (685 mg, 3.1 mmoL) was dropwise added at -78°C, and the mixture was naturally warmed to room temperature and reacted for 1 h. A saturated sodium bicarbonate aqueous

solution (50 mL) and dichloromethane (50 mL × 2) were added. The organic phases were combined, washed with a saturated sodium chloride solution, dried over anhydrous sodium sulphate, and filtered, and the filtrate was concentrated under reduced pressure and then separated and purified by silica gel column chromatography (eluent EA/PE = 0-20%) to obtain the target compound (0.5 g, 99 %).

LC-MS (ESI): m/z = 270.1 $[M-56+H]^+$.

Step 2: **39A** (0.5 g, 1.54 mmol) and hydrogen chloride-dioxane (4 M, 20 mL) were successively added to a 50 mL single-mouth flask. The mixture was reacted at room temperature for 1 h. The reaction liquid was directly concentrated under reduced pressure to obtain the title compound (400 mg, 100%).

LC-MS (ESI): m/z = 226.1 $[M+H]^+$.

Step 3: **1E** (287 mg, 1 mmol), **39B** (261 mg, 1 mmol), dioxane (5 mL), and DIPEA (645 mg, 5 mmol) were successively added to a 50 mL single-mouth flask and reacted at 100°C for 8 h. The reaction product was cooled to room temperature, and the reaction liquid was directly concentrated under reduced pressure and then separated and purified by silica gel column chromatography (eluent MeOH/DCM = 0-10%) to obtain 400 mg of a mixture.

After chiral preparation, the title compound **39-1** (160 mg, 33%, retention time: 1.311 min) and the title compound **39-2** (101 mg, 21%, retention time: 1.501 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 2.0 min.

**Compound 39-1:**

$^1$H NMR (400 MHz, DMSO-*d6*) δ7.49-7.41 (m, 2H), 7.38-7.25 (m, 2H), 4.91-4.79 (m, 2H), 3.80-3.69 (m, 1H), 3.66-3.53 (m, 1H), 3.47-3.31 (m, 2H), 3.30-3.05 (m, 3H), 3.02-2.93 (m, 1H), 2.88-2.77 (m, 2H), 2.49-2.14 (m, 6H), 2.08-1.89 (m, 2H), 1.86-1.52 (m, 2H).

LC-MS (ESI): m/z = 477.1 $[M+H]^+$.

**Compound 39-2:**

$^1$H NMR (400 MHz, DMSO-*d6*)δ 7.48-7.46 (m, 1H), 7.38-7.32 (m, 2H), 7.20-7.17 (m, 1H), 7.07-7.04 (m, 1H), 4.84-4.71 (m, 1H), 3.81-3.71 (m, 1H), 3.61-3.09 (m, 5H), 3.08-2.65 (m, 4H), 2.49-2.11 (m, 6H), 2.05-1.90 (m, 2H), 1.88-1.70 (m, 1H), 1.71-1.45 (m, 1H).

LC-MS (ESI): m/z = 477.1 $[M+H]^+$.

**Example 33**

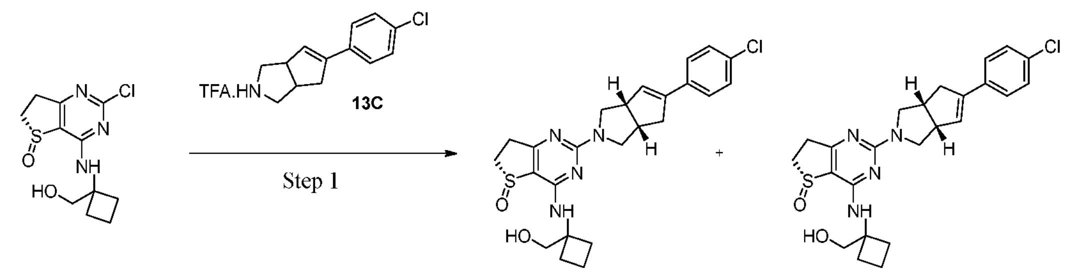


40A

Compounds 40-1, 40-2

Step 1: 40A (0.15 g, 0.52 mmol), 13C (0.21 g, 0.62 mmol), dioxane (5 mL), and DIPEA (0.34 g, 2.59 mmol) were successively added to a 50 mL single-mouth flask and reacted at 100°C for 5 h. The reaction product was cooled to room temperature, and the reaction liquid was directly concentrated under reduced pressure and then separated and purified by silica gel column chromatography (eluent MeOH/DCM = 0-10%) to obtain 220 mg of a mixture.

After chiral preparation, the title compound **40-1** (42 mg, 17%, retention time: 0.786 min) and the title compound **40-2** (82 mg, 33%, retention time: 0.993 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 120 mL/min; back pressure: 100 bar; column temperature: 25°C; wavelength: 220 nm; elution time: 5.5 min.

**Compound 40-1:**

$^1$H NMR (400 MHz, DMSO-*d6*) δ 7.50-7.48 (m, 2H), 7.39-7.37 (m, 2H), 7.18 (s, 1H), 6.26 (s, 1H), 4.82 (s, 1H), 3.87 (s, 1H), 3.79-3.54 (m, 5H), 3.44-3.34 (m, 1H), 3.22-3.12 (m, 2H), 3.12-3.05 (m, 1H), 2.98-2.77 (m, 3H), 2.62 (d, 1H), 2.40-2.29 (s, 2H), 2.15 (s, 2H), 1.85-1.70(m, 2H).
LC-MS (ESI): m/z = 471.2 $[M+H]^+$.

**Compound 40-2:**

$^1$H NMR (400 MHz, DMSO-*d6*) δ 7.50-7.48 (m, 2H), 7.39-7.37(m, 2H), 7.18 (s, 1H), 6.26 (s, 1H), 4.82 (s, 1H), 3.90-3.83 (m, 1H), 3.79-3.54 (m, 5H), 3.46-3.32 (m, 1H), 3.24-3.13 (m, 2H), 3.12-3.05 (m, 1H), 2.97-2.78 (m, 3H), 2.65-2.58 (m, 1H), 2.43-2.28 (m, 2H), 2.14 (s, 2H), 1.85-1.68 (m, 2H).
LC-MS (ESI): m/z = 471.2 $[M+H]^+$.

**Example 34**

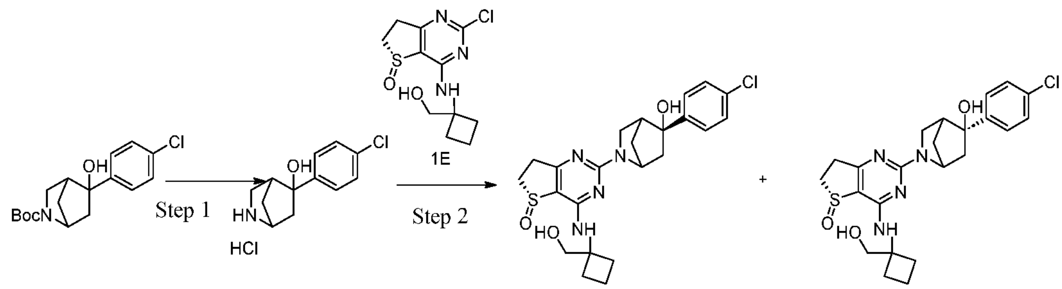


18B 41A Compounds 41-1,41-2

Step 1: **18B** (0.5 g, 1.55 mmol) and hydrogen chloride-dioxane (4 M, 20 mL) were successively added to a 50 mL single-mouth flask. The mixture was reacted at room temperature for 1 h. The reaction liquid was directly concentrated under reduced pressure to obtain the title compound (400 mg, 100%).
LC-MS (ESI): m/z = 224.1 $[M+H]^+$.
Step 2: **1E** (287 mg, 1.0 mmol), **41A** (233 mg, 1.0 mmol), dioxane (5 mL), and DIPEA (645 mg, 5.0 mmol) were successively added to a 50 mL single-mouth flask and reacted at 100°C for 8 h. The reaction product was cooled to room temperature, and the reaction liquid was directly concentrated under reduced pressure and then separated and purified by silica gel column chromatography (eluent MeOH/DCM = 0-10%) to obtain 410 mg of a mixture.
After chiral preparation, the title compound **41-1** (150 mg, 32%, retention time: 1.302 min) and the title compound **41-2** (100 mg, 21%, retention time: 1.488 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 2.0 min.

**Compound 41-1:**

$^1$H NMR (400 MHz, DMSO-*d6*) δ 7.53 (d, 2H), 7.42-7.33 (m, 2H), 7.20 (s, 1H), 5.43 (d, 1H), 4.87 (s, 1H), 4.55 (d, 1H), 4.12-3.97 (m, 1H), 3.90-3.62 (m, 2H), 3.46-3.38 (m, 1H), 3.30-3.16 (m, 2H), 3.01-2.81 (m, 2H), 2.79-2.67 (m, 1H), 2.46-2.07 (m, 5H), 1.87-1.54 (m, 5H).
LC-MS (ESI): m/z = 475.2 $[M+H]^+$.

**Compound 41-2:**

$^1$H NMR (400 MHz, DMSO-*d6*) δ 7.55 (d, 2H), 7.43-7.38 (m, 2H), 5.60 (d, 2H), 4.71-4.48 (m, 1H), 4.05 (d, 1H), 3.99-2.93 (m, 8H), 2.84 (d, 1H), 2.44-2.13 (m, 5H), 1.91-1.67 (m, 5H).
LC-MS (ESI): m/z = 475.2 $[M+H]^+$.

**Example 35**

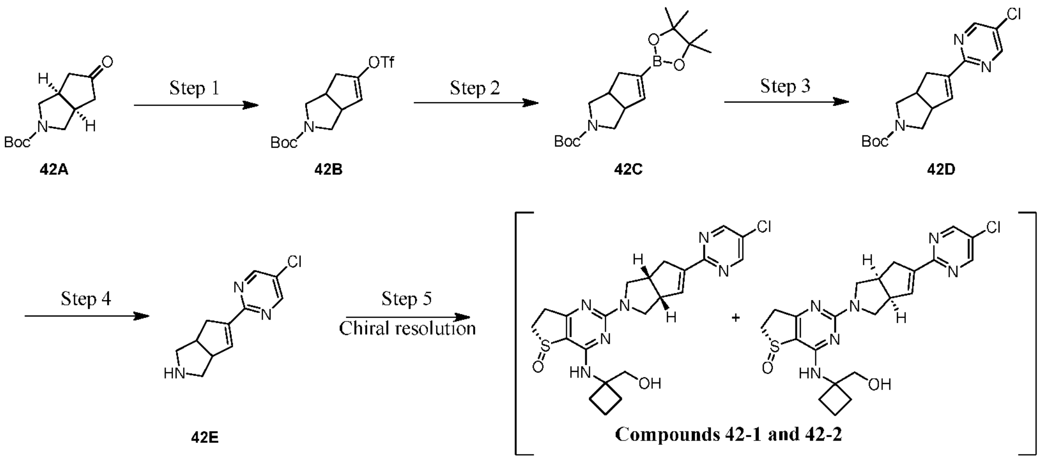


42A 42B 42C 42D 42E

Compounds 42-1 and 42-2

Step 1: Compound **42A** (1 g, 4.44 mmol) was dissolved in anhydrous tetrahydrofuran (10 ml). After nitrogen displacement three times, the reaction system was cooled to -78°C. Lithium bis(trimethylsilyl)amide (8.88 mmol) was dropwise added, and the mixture was maintained at -78°C for 30 min. N-phenyl bis(trifluoromethyl)sulfonimide (2.59 g, 6.66 mmol) dissolved in anhydrous tetrahydrofuran (5 ml) was added dropwise. The mixture was maintained at -78°C for another 30 min, and then reacted at room temperature for 2 h. The reaction was complete and then quenched by adding a saturated ammonium chloride solution (30 ml). The organic phase was extracted with ethyl acetate (3 × 40 ml). The organic phases were combined, dried over anhydrous sodium sulphate, filtered and concentrated, and the residue was quickly separated and purified by column chromatography (eluent ratio: EA/PE = 0-10%) to obtain compound **42B** (1.3 g, 82%).

LCMS (ESI): m/z = 302.2 [M+H-56].

Step 2: Under nitrogen, bis(triphenylphosphino)palladium dichloride (260 mg, 0.37 mmol), pinacol diboronate (1.11 g, 4.37 mmol), and potassium acetate (1.07 g, 10.92 mmol) were added to compound **42B** (1.3 g, 3.64 mmol) dissolved in 1,4-dioxane (20 ml), and the mixture was reacted for 18 h in an oil bath at 90°C. After the reaction was complete, the reaction product was cooled to room temperature, filtered with diatomite, and washed with ethyl acetate, and the filtrate was concentrated and directly quickly separated and purified by direct column chromatography (eluent ratio: EA/PE = 0-10%) to obtain the target compound **42C** (0.89 g, 73%).

LCMS (ESI): m/z = 280.2 [M+H-56].

Step 3: Under nitrogen, 5-chloro-2-iodopyrimidine (578 mg, 2.41 mmol), 1,1-bis(diphenylphosphino)ferrocene palladium dichloride (176 mg, 0.24 mmol), and sodium carbonate (766 mg, 7.32 mmol) were added to compound **42C** (0.89 g, 2.65 mmol) dissolved in 1,4-dioxane/water (10 ml/2 ml), and the mixture was reacted for 18 h in an oil bath at 90°C. After the reaction was complete, the reaction product was cooled to room temperature, filtered with diatomite, and washed with ethyl acetate, and the filtrate was concentrated and directly quickly separated and purified by direct column chromatography (eluent ratio: EA/PE = 0-10%) to obtain the target compound **42D** (0.44 g, 52%).

LCMS (ESI): m/z = 266.1 [M+H-56].

Step 4: Compound **42D** (220 mg, 0.69 mmol) was dissolved in hydrogen chloride 1,4-dioxane (2 ml), stirred at room temperature for 1 h, and directly spin-dried to obtain compound **42E** hydrochloride (170 mg).

LCMS (ESI): m/z = 222.1 $[M+H]^+$.

Step 5: Intermediate **1E** (198 mg, 0.69 mmol) was dissolved in 1,4-dioxane (5 ml), **42E** (170 mg, 0.69 mmol) and diisopropylethylamine (267 mg, 2.07 mmol) were added, and the reaction system was placed in an oil bath at 100°C and reacted for 15 h. After the reaction was complete, the reaction product was cooled to room temperature. The reaction was monitored by LCMS. The reaction product was spin-dried and then subjected to preparation. Preparative HPLC separation method: 1. Instrument: waters 2767 preparative liquid chromatographic instrument; chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10-55%; c. flow rate: 12 mL/min. Retention time: 7.0 min. Thus, **compound 42** (240 mg, 74%) was obtained.

LCMS (ESI): m/z = 473.2 $[M+H]^+$.

Resolution of compound **42:**

**Compound 42** (240 mg) was taken for resolution, and after separation, **compound 42-1** (retention time: 1.257 min, 74 mg, ee% = 100%) and **compound 42-2** (retention time: 1.553 min, 77 mg, ee% = 100%) were obtained.
Resolution conditions: instrument: Waters 150 MGM; column: DAICEL CHIRALPAK AD; mobile phases: A for CO2 and B for EtOH (0.1% NH3•H2O)); gradient: B 40%; flow rate: 120 mL /min; back pressure: 100 bar; column temperature: 35°C; wavelength: 220 nm; period: 13 min;

**Compound 42-1:**

$^1$H NMR (400 MHz, $CDCl_3$)$\delta$ = 8.61 (s, 2H), 6.85 (s, 1H), 5.91 (s, 1H), 5.11 (s, 1H), 3.99 (d, 1H), 3.84 (s, 2H), 3.78 (s, 1H), 3.70 (d, 1H), 3.64-3.51 (m, 1H), 3.39 (d, 1H), 3.30 (d, 1H), 3.24-3.12 (m, 1H), 3.11-2.91 (m, 3H), 2.85 (d, 1H), 2.45-2.12 (m, 4H), 1.91 (d, 2H), 1.67 (s, 1H).

**Compound 42-2:**

$^1$H NMR (400 MHz, $CDCl_3$) $\delta$ = 8.62 (s, 2H), 6.85 (s, 1H), 5.88 (s, 1H), 5.10 (d, 1H), 4.10-3.92 (m, 1H), 3.81 (d, 3H), 3.68 (s, 1H), 3.58 (d, 1H), 3.43-3.28 (m, 2H), 3.17 (s, 1H), 3.12-2.92 (m, 3H), 2.85 (d, 1H), 2.29 (t, 4H), 1.91 (d, 2H), 1.69 (d, 1H).

**Example 36**

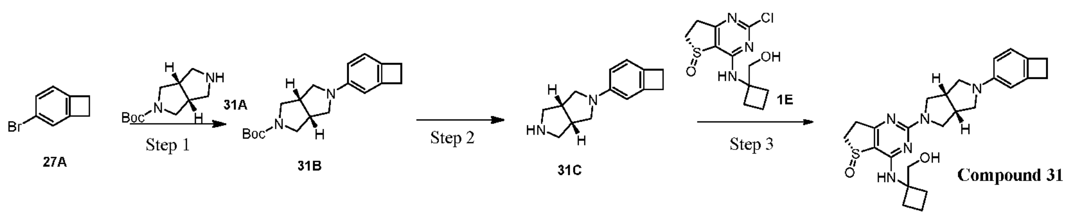

27A 31A 31B 31C 1E Compound 31

Step 1: Compound **27A** (1.00 g, 5.45 mmol) and compound **31A** (1.16 g, 5.45 mmol) were weighed into a 100 mL single-mouth flask and then dissolved by adding 1,4-dioxane (30 mL). Sodium tert-butoxide (1.57 g, 16.34 mmol), tris(dibenzylideneacetone)dipalladium (0.15 g, 0.27 mmol), and 2,2'-bis(diphenylphosphino)-1,1'-binaphthyl (0.34 g, 0.55 mmol) were added to the reaction liquid. After the addition was complete, the mixture was stirred at 90°C for 16 h. After the completion of the reaction was monitored by TLC plate spotting (petroleum ether : ethyl acetate = 5:1), water (20 mL) was added to the reaction liquid and stirred for 5 min, ethyl acetate (20 mL) was added for extraction, and an organic phase was separated. The organic phase was dried over anhydrous sodium sulphate, and the crude product was concentrated under reduced pressure and purified by column chromatography (eluents: petroleum ether : ethyl acetate = 5:1) to obtain the target compound **31B** (1.0 g, yield 58%).
LCMS m/z = 315.1 [M +1]$^+$.
Step 2: Compound **31B** (0.30 g, 0.95 mmol) was weighed into a 100 mL single-mouth flask and then dissolved by adding methanol (10 mL), and a hydrogen chloride dioxane solution (4N, 10 mL) was added. After the addition was complete, the mixture was stirred for 2 h. The organic phase was concentrated under reduced pressure until no liquid dripped out to obtain the target compound **31C** (0.20 g, crude).
LCMS m/z = 215.1 [M +1]$^+$.
Step 3: Compound **1E** (0.20 g, 0.69 mmol), compound **31C** (0.15 g, 0.69 mmol), and N,N-diisopropylethylamine (0.26 g, 2.05 mmol) were added to a 100 mL single-mouth flask. After the addition was complete, the system was placed under nitrogen protection and stirred at 90°C for 16 h, and the reaction liquid was concentrated under reduced pressure to obtain a crude product of the target compound, which was subjected to preparative HPLC to obtain the title compound **31** (120 mg, 37%).
Preparation method: instrument: waters 2767 preparative liquid chromatographic instrument; chromatographic column: SunFire@ Prep C18 (19 mm × 250 mm); the sample was dissolved with DMF and filtered with a 0.45 $\mu$m filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 40-80%; c. flow rate: 15 mL/min; d. elution time: 20 min; retention time: 10.20 min.
LCMS m/z = 466.2 [M +1]$^+$.

$^1$H NMR (400 MHz, DMSO-*d6*) $\delta$ 7.21 (s, 1H), 6.85 (d, 1H), 6.34-6.37(m, 2H), 4.81 (t, 1H), 3.75-3.80(m, 2H), 3.72 (d, 2H), 3.35-3.45 (m, 5H), 2.97-3.24 (m, 9H), 2.81-2.94 (m, 2H), 2.29-2.42 (m, 2H), 2.11 -2.17 (m, 2H), 1.69-1.81 (m, 2H).

**Example 37**

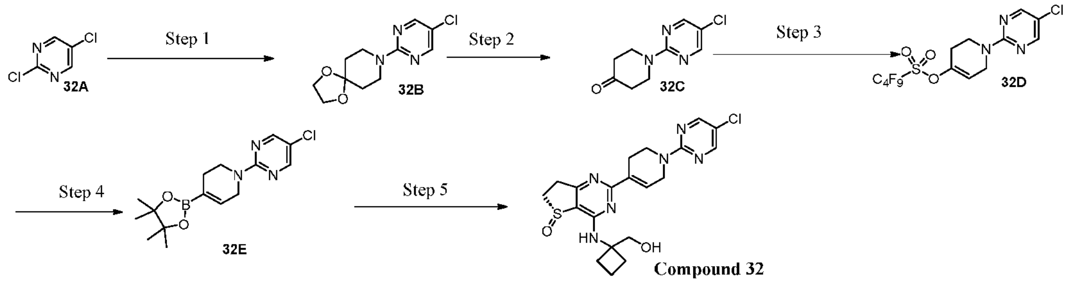


32A, 32B, 32C, 32D, 32E, Compound 32

Step 1: 2,5-Dichloropyrimidine (5.0 g, 33.6 mmol), 1,4-dioxa-8-azaspiro[4.5]decane (5.3 g, 36.9 mmol), and DIPEA(13.0 g, 100.8 mmol) were dissolved in 100 mL of dioxane, and the system was warmed to 80°C and reacted for 2 h. The reaction system was poured into ice water and extracted twice with ethyl acetate, the organic phases were combined and dried, and the solvent was removed by rotary evaporation. A mobile phase of PE : EA = 20:1 was used for silica gel column chromatography purification to obtain the target compound **32B.** (8.5 g, yield 99%).

LCMS (ESI): m/z = 256.2 $[M+H]^+$.

Step 2: The raw material **32B** (8.5 g, 33.2 mmol) was dissolved in 30 mL of tetrahydrofuran, and 120 mL of 10% sulphuric acid was added, heated to 90°C, and reacted under reflux for 12 h. After the reaction was confirmed to be complete by TLC plate spotting, the mixture was cooled to room temperature and then adjusted to neutral pH with saturated sodium bicarbonate. After washing with ethyl acetate (100 mL $\times$ 3), the organic phases were washed with a saturated sodium chloride aqueous solution and dried over anhydrous sodium sulphate, and the solvent was removed by rotary evaporation to obtain **32C** as a solid (6.2 g, yield 88 %), which was directly used in the next reaction.

LCMS (ESI): m/z = 212.3 $[M+H]^+$.

Step 3: The raw material **32C** (6.2 g, 29.2 mmol) and 1,1,2,2,3,3,4,4,4-nonafluorobutane-1-sulfonyl fluoride (10.6 g, 35.0 mmol) were dissolved in 62 mL of tetrahydrofuran, and DBU (5.3 g, 35.0 mmol) was added and stirred for 4 h at room temperature. After the completion of the reaction was monitored by TLC, the reaction product was concentrated and then purified by silica gel column chromatography with the mobile phase system PE : EA = 50:1 to obtain **32D** as an oil (10.6 g, yield 73%).

LCMS (ESI): m/z = 494.0 $[M+H]^+$.

Step 4: The raw material **32D** (6.50 g, 13.2 mmol), pinacol diboronate (4.02 g, 15.8 mmol), [1,1'-bis(diphenylphosphine)ferrocene]palladium dichloride (190 mg, 0.26 mmol), and 1,1'-bis(diphenylphosphine)ferrocene (144 mg, 0.26 mmol) were suspended in dioxane (65 mL) and reacted at room temperature under stirring for 15 min. Potassium acetate (2.58 g, 26.4 mmol) was added and reacted at 90°C for 16 h. After the completion of the reaction was monitored by TLC and LCMS, silica gel column chromatography was carried out with PE:EA = 20:1 as the mobile phase to obtain a white title compound **32E** (3.37 g, yield 79 %).

LCMS (ESI): m/z = 322.4 $[M+H]^+$.

Step 5: Compounds **32E** (500 mg, 1.55 mmol) and **1E** (405 mg, 1.40 mmol), [1,1'-bis(diphenylphosphino) ferrocene]palladium dichloride (22 mg, 0.03 mmol), 1,1'-bis(diphenylphosphino)ferrocene (17 mg, 0.03 mmol), and sodium carbonate (493 mg, 4.65 mmol) were suspended in 10 mL of a mixed solvent of dioxane : water = 4:1 and reacted at 90°C for 4 h. After the reaction was complete, the reaction liquid was dropwise added to ice water and extracted with 20 mL of dichloromethane three times, and the organic phases were combined, concentrated, and subjected to silica gel column chromatography with DCM : MeOH = 15 : 1 as the mobile phase to obtain the target compound **32.** (142 mg, 23%).

$^1$H NMR (400 MHz, DMSO-*d6*) $\delta$ 8.45 (s, 2H), 7.84 (s, 1H), 7.32-7.16 (m, 1H), 4.87 (t, 1H), 4.39 (q, 2H), 3.94 (t, 2H), 3.80-3.70 (m, 2H), 3.61-3.55(m, 1H), 3.35-3.29(m, 1H), 3.18-3.09 (m, 1H), 3.03-2.95 (m, 1H), 2.65-2.57 (m, 2H), 2.49-2.12 (m, 4H), 1.86-1.72 (m, 2H).

LCMS (ESI): m/z = 447.6 [M+H]-.

**Example 38**

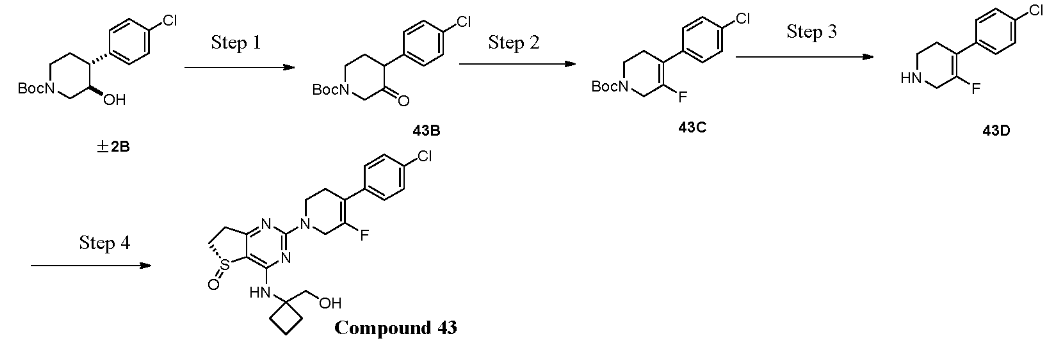


Step 1: The raw material ±**2B** (1.5 g, 4.81 mmol) and Dess-Martin periodinane (5.1 g, 12.0 mmol) were dissolved in 15 mL of dichloromethane and reacted for 4 h. After the completion of the reaction was confirmed by TLC monitoring, the reaction system was filtered, the filter cake was soaked in 15 mL of dichloromethane for ultrasonic treatment. After filtration, the filtrates were combined, the solvent was removed by rotary evaporator, and after purification by silica gel column chromatography (PE : EA = 5 : 1), the target compound **43B** (970 mg, yield 66%) was obtained.

LCMS (ESI): m/z = 254.2 [M+H-tBu]$^+$.

Step 2: The raw material **43B** (970 mg, 3.13 mmol) was dissolved in 10 mL of tetrahydrofuran, and diethylaminosulphur trifluoride (2.53 g, 15.69 mmol) was slowly dropwise added at -20 degrees Celsius and reacted for 4 h. After the completion of the reaction was confirmed by TLC monitoring, the reaction was quenched by adding 20 ml of saturated sodium bicarbonate in an ice bath. After extraction with dichloromethane (20 mL × 2), the organic phase was washed with a saturated sodium chloride aqueous solution and dried over anhydrous sodium sulphate. The solvent was removed by rotary evaporator, and after silica gel column chromatography (PE : EA = 40 : 1), the target compound **43C** (126 mg, yield 13%) was obtained.

LCMS (ESI): m/z = 256.3 [M+H-tBu]$^+$.

Step 3: The raw material **43C** (126 mg, 0.41 mmol) was dissolved in a mixed solution of 0.5 mL 4N hydrogen chloride dioxane and 0.5 mL methanol and stirred at room temperature for 4 h. After the completion of the reaction was monitored by LCMS, the reaction product was concentrated to obtain the target compound **43D** (84 mg, yield 98%).

LCMS (ESI): m/z = 212.1 [M+H]$^+$.

Step 4: Compounds **43D** (84 mg, 0.40 mmol) and **1E** (104 mg, 0.37 mmol) and DIPEA (258 mg, 2.00 mmol) were dissolved in 2 mL of dioxane and reacted at 80°C for 4 h. After the reaction was complete, the reaction liquid was dropwise added to 10 mL of ice water and extracted with dichloromethane (10 mL × 3) three times, and the organic phases were combined, dried over anhydrous sodium sulphate, concentrated, and subjected to HPLC preparation to obtain the target **compound 43** (39 mg, 23%).

$^1$H NMR (400 MHz, DMSO-*d6*) δ 7.50 (s, 1H), 7.49-7.40 (m, 4H), 4.86 (s, 1H), 4.42 (s, 2H), 3.99-3.93 (m, 2H), 3.76-3.70 (m, 2H), 3.51-3.40 (m, 1H), 3.27-3.19 (m, 1H), 3.02-2.85 (m, 2H), 2.61-2.51 (m, 2H), 2.43-2.27 (m, 2H), 2.24-2.15 (m, 2H), 1.78-1.73 (m, 2H).

LCMS (ESI): = 463.1 [M+H]$^+$.

$^{19}$F NMR (376 MHz, DMSO) δ -116.81.

**Example 39**

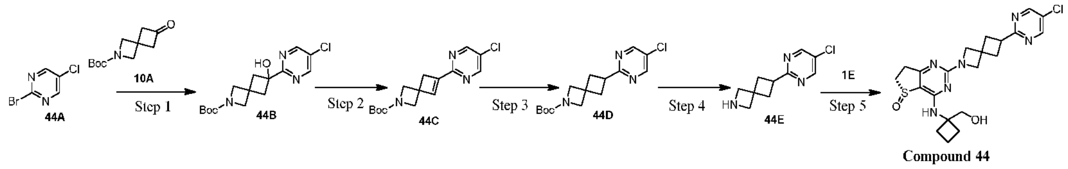


Step 1: Compound **44A** (4.00 g, 20.67 mmol) was weighed into a 100 mL single-mouth flask and then dissolved by adding anhydrous toluene (50 mL). At - 78°C, n-butyl lithium (1.32 g, 20.67 mmol) was added to the reaction liquid. After the addition was complete, the mixture was stirred for 0.5 h, compound **10A** (5.24 g, 24.80 mmol) was then added. After the addition was complete, the mixture was warmed to 0°C and further stirred for 2 hours. After the reaction was found complete by TLC plate spotting (petroleum ether : ethyl acetate = 3:1), a saturated ammonium chloride solution (50 mL) was added to the reaction liquid and stirred for 5 min, ethyl acetate (30 mL) was added for extraction, and an organic phase

was separated. The organic phase was dried over anhydrous sodium sulphate, and the crude product was concentrated under reduced pressure and purified by column chromatography (eluents: petroleum ether : ethyl acetate = 5:1) to obtain the target compound **44B** (1.0 g, 15%).

LCMS m/z = 326.1 $[M+1]^+$.

Step 2: Compound **44B** (1.00 g, 3.01 mmol) was weighed into a 100 mL single-mouth flask and then dissolved by adding pyridine (15 mL). Thionyl chloride (0.22 mL) was added to the reaction liquid, and after the addition was complete, the mixture was stirred at room temperature for 16 h. After the reaction was complete, the filtrate was concentrated under reduced pressure until no liquid dripped out, and the crude product was purified by column chromatography (eluents: petroleum ether : ethyl acetate = 10:1) to obtain the target compound **44C** (0.26 g, 27%).

LCMS m/z = 308.1 $[M+1]^+$.

Step 3: Compound **44C** (0.10 g, 0.32 mmol) was weighed into a 100 mL single-mouth flask and then dissolved by adding ethyl acetate (5 mL), palladium on carbon (0.10 g, 0.97 mmol) was added, and after the addition was complete, the mixture was stirred for 2 h. After the reaction was complete, the reaction liquid was filtered, and the organic phase was concentrated under reduced pressure until no liquid dripped out to obtain the target compound **44D** (0.09 g, crude).

LCMS m/z = 310.1 $[M+1]^+$.

Step 4: Compound **44D** (0.09 g, 0.29 mmol) was weighed into a 50 mL single-mouth flask and then dissolved by adding methanol (5 mL). A hydrogen chloride dioxane solution (4N, 5 mL) was added, and after the addition was complete, the mixture was stirred for 2 h. The organic phase was concentrated under reduced pressure until no liquid dripped out to obtain the target compound **44E** (0.07 g, crude).

LCMS m/z = 210.2 $[M+1]^+$.

Step 5: Compound **1E** (0.10 g, 0.35 mmol) and compound **44E** (0.07 g, 0.35 mmol) were added to a 100 mL single-mouth flask and dissolved by adding 1,4-dioxane (10 mL). Finally, N,N-diisopropylethylamine (0.13 g, 1.03 mmol) was added. After the addition was complete, the system was placed under nitrogen protection and stirred at 100°C for 16 h, and the reaction liquid was concentrated under reduced pressure to obtain a crude product of the target compound, which was subjected to preparative HPLC to obtain the title compound **44** (3.3 mg, 2%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 30-80%; c. flow rate: 15 mL/min; d. elution time: 20 min; retention time: 10.20 min.

LCMS m/z = 461.2 $[M+1]^+$.

$^1H$ NMR (400 MHz, DMSO-d6) δ 8.86(s, 2H), 7.29 (s, 1H), 4.87 (t, 1H), 4.13 (s, 2H), 3.95 (s, 2H), 3.62-3.72 (m, 3H), 3.36-3.43(m, 2H), 3.14-3.25 (m, 1H), 2.82-2.95(m, 2H), 2.59-2.65 (m, 2H), 2.51-2.54 (m, 1H), 2.23-2.41 (m, 2H), 2.13 (t, 2H), 1.69-1.85 (m, 2H).

**Example 40**

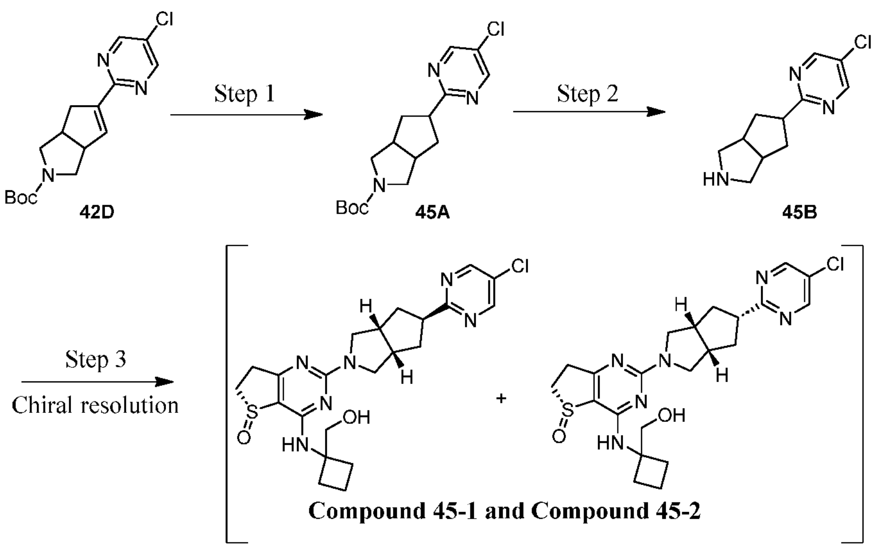


42D 45A 45B

Compound 45-1 and Compound 45-2

Step 1: Compound **42D** (1 g, 3.11 mmol) was dissolved in 150 ml of ethyl acetate, palladium on carbon (100 mg)

was added. After hydrogen displacement three times, the mixture was stirred overnight in a hydrogen atmosphere at room temperature. After the reaction was complete, the reaction product was filtered, spin-dried, and subjected to preparation. Preparative HPLC separation method: 1. Instrument: waters 2767 preparative liquid chromatographic instrument; chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate). b. gradient elution, mobile phase A: 10-55%. c. flow rate: 12 mL/min. Retention time: 10.0 min. Thus, compound **45** (300 mg, 30%) was obtained.

LCMS (ESI): m/z = 268.3 $[M+H-56]^+$.

Step 2: Compound **45A** (300 mg, 0.93 mmol) was dissolved in hydrogen chloride 1,4-dioxane (2 ml), stirred at room temperature for 1 h, and directly spin-dried to obtain compound **45B** hydrochloride (242 mg).

LCMS (ESI): m/z = 224.1 $[M+H]^+$.

Step 3: Intermediate **1E** (267 mg, 0.93 mmol) was dissolved in 1,4-dioxane (5 ml). Compound **45B** hydrochloride (242 mg, 0.93 mmol) and diisopropylethylamine (360 mg, 2.79 mmol) were added, and the reaction system was placed in an oil bath at 100°C and reacted for 15 h. After the reaction was complete, the reaction product was cooled to room temperature. The reaction was monitored by LCMS. The reaction product was spin-dried and then subjected to preparation. Preparative HPLC separation method: 1. Instrument: waters 2767 preparative liquid chromatographic instrument; chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate). b. gradient elution, mobile phase A: 10-55%. c. flow rate: 12 mL/min. Retention time: 7.0 min. Thus, **compound 45** (300 mg, 68%) was obtained.

LCMS (ESI): m/z = 475.2 $[M+H]^+$.

Resolution of compound **45:**

**Compound 45** (130 mg) was taken for resolution, and after separation, **compound 45-1** (retention time: 1.344 min, 254 mg, ee% = 100%) and **compound 45-2** (retention time: 1.621 min, 20 mg, ee% = 100%) were obtained.

Resolution conditions:

instrument: Waters 150 MGM; column: DAICEL CHIRALPAK AD;
mobile phase: A for $CO_2$ and B for EtOH (0.1% $NH_3 \bullet H_2O$)); gradient: B 40%; flow rate: 120 mL /min; back pressure: 100 bar;
column temperature: 35°C; wavelength: 220 nm; period: 13 min;

**Compound 45-1:**

$^1$H NMR (400 MHz, $CDCl_3$) δ = 8.58 (s, 2H), 5.94 (s, 1H), 5.17 (s, 1H), 3.85 (s, 2H), 3.75-3.70 (m, 2H), 3.68-3.48 (m, 4H), 3.43-3.36 (m, 1H), 3.09-2.94 (m, 2H), 2.88 (s, 2H), 2.44-2.37 (m, 2H), 2.36-2.17 (m, 4H), 2.02-1.81 (m, 4H).

**Compound 45-2:**

$^1$H NMR (400 MHz, $CDCl_3$) δ = 8.60 (s, 2H), 5.93 (s, 1H), 3.92-3.85 (m, 4H), 3.64-3.58 (m, 2H), 3.53-3.39 (m, 3H), 3.15-2.96 (m, 4H), 2.33 (s, 2H), 2.33-2.23 (m, 5H), 2.09-2.01 (m, 2H), 2.00-1.94 (m, 1H), 1.94-1.87 (m, 1H).

**Example 41**

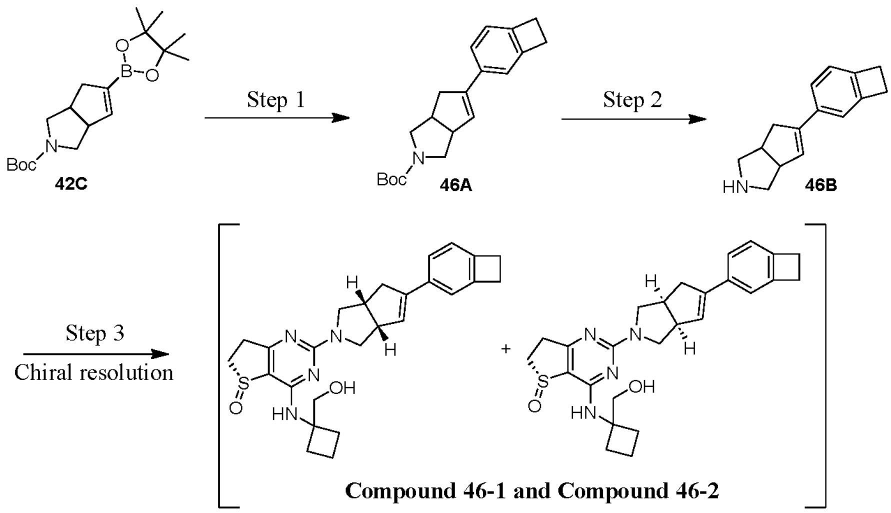


42C 46A 46B

Compound 46-1 and Compound 46-2

Step 1: Under nitrogen, 4-bromobenzocyclobutane (497 mg, 2.71 mmol), 1,1-bis(diphenylphosphino)ferrocene palladium dichloride (220 mg, 0.3 mmol), and sodium carbonate (951 mg, 8.97 mmol) were added to compound **42C** (1 g, 2.99 mmol) dissolved in 1,4-dioxane/water (10 ml/2 ml), and the mixture was reacted for 18 h in an oil bath at 90°C. After the reaction was complete, the reaction product was cooled to room temperature, filtered with diatomite, and washed with ethyl acetate, and the filtrate was concentrated and directly quickly separated and purified by direct column chromatography (eluent ratio: EA/PE = 0-10%) to obtain the target compound **46A** (0.44 g, 52%).

LCMS (ESI): m/z = 256.2 $[M+H-56]^+$.

Step 2: Compound **46A** (220 mg, 0.69 mmol) was dissolved in hydrogen chloride 1,4-dioxane (2 ml), stirred at room temperature for 1 h, and directly spin-dried to obtain compound **46B** hydrochloride (170 mg).

LCMS (ESI): m/z = 212.1 $[M+H]^+$.

Step 3: Intermediate **1E** (200 mg, 0.69 mmol) was dissolved in 1,4-dioxane (5 ml). Compound **46B** hydrochloride (170 mg) and diisopropylethylamine (267 mg, 2.07 mmol) were added, and the reaction system was placed in an oil bath at 100°C and reacted for 15 h. After the reaction was complete, the reaction product was cooled to room temperature. The reaction was monitored by LCMS. The reaction product was spin-dried and then subjected to preparation. Preparative HPLC separation method: 1. Instrument: waters 2767 preparative liquid chromatographic instrument; chromatographic column: SunFire@ Prep C18 (19 mm × 250 mm). 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate). b. gradient elution, mobile phase A: 10-55%. c. flow rate: 12 mL/min. Retention time: 7.0 min. Thus, **compound 46** (200 mg, 63%) was obtained.

LCMS (ESI): m/z = 463.3 $[M+H]^+$.

Resolution of compound **46:**

**Compound 46** (200 mg) was taken for resolution, and after separation, **compound 46-1** (retention time: 1.214 min, 73 mg, ee% = 100%) and **compound 46-2** (retention time: 1.541 min, 68 mg, ee% = 100%) were obtained.

Resolution conditions:

instrument: Waters 150 MGM; column: DAICEL CHIRALPAK AD;
mobile phases: A for CO2 and B for EtOH (0.1% NH3•H2O)); gradient: B 40%; flow rate: 120 mL /min; back pressure: 100 bar;
column temperature: 35°C; wavelength: 220 nm; period: 13 min;

**Compound 46-1:**

$^1H$ NMR (400 MHz, $CDCl_3$) δ = 7.24 (s, 1H), 7.11 (s, 1H), 6.99 (d, 1H), 5.95 (s, 1H), 5.89 (s, 1H), 4.08-3.87 (m, 1H), 3.88-3.78 (m, 3H), 3.80-3.65 (m, 2H), 3.64-3.52 (m, 2H), 3.44-3.33 (m, 1H), 3.32-3.19 (m, 1H), 3.15 (s, 4H), 3.13-3.05 (m, 1H), 3.06-2.92 (m, 3H), 2.72-2.58 (m, 1H), 2.36-2.21 (m, 4H), 1.98-1.85 (m, 2H).

**Compound 46-2:**

$^1H$ NMR (400 MHz, $CDCl_3$) δ = 7.24 (s, 1H), 7.12 (s, 1H), 6.99 (d, 1H), 5.95 (s, 1H), 5.88 (s, 1H), 4.04-3.91 (m, 1H),

3.84 (d, J=14.1, 2H), 3.80-3.66 (m, 2H), 3.60 (s, 2H), 3.44-3.20 (m, 2H), 3.15 (s, 4H), 3.13-3.06 (m, 1H), 3.00 (t, 3H), 2.64 (d, 1H), 2.43-2.14 (m, 4H), 1.98-1.80 (m, 2H).

**Example 42**

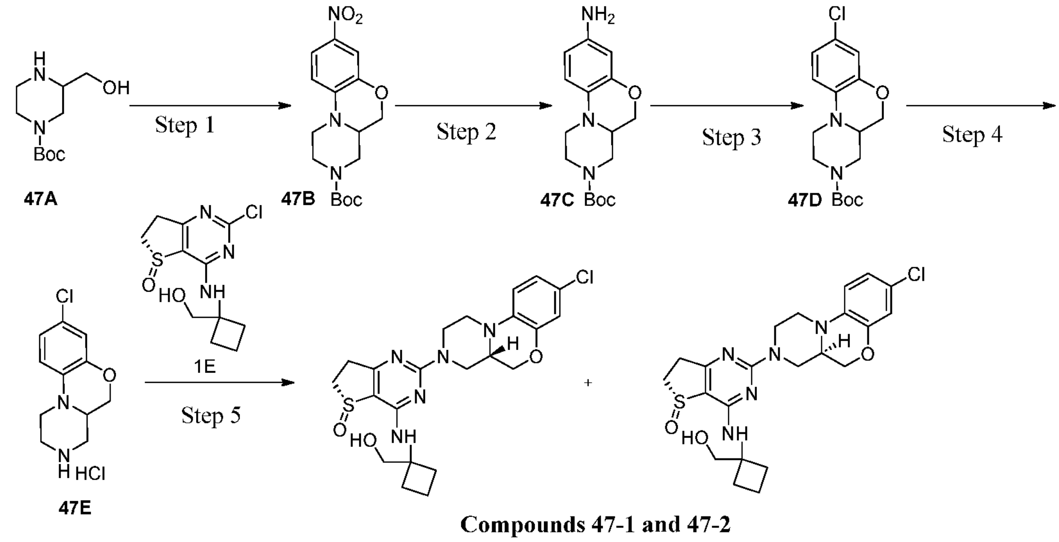


47A 47B 47C 47D 47E

**Compounds 47-1 and 47-2**

Step 1: **47A** (10.0 g, 46.3 mmol), 1,2-difluoro-4-nitrobenzene (7.3 g, 46.3 mmol), potassium hydroxide (7.8 g, 138.9 mmol), and N,N-dimethylformamide (100 mL) were successively added to a 250 mL single-mouth flask. After reaction at room temperature for 6 hours, The reaction product was further warmed to 60°C and reacted for 24 hours. After filtration, ethyl acetate (500 mL) was added to the filtrate, which was washed with water (100 mL × 3). The organic layer was dried over anhydrous sodium sulphate and filtered, and the filtrate was concentrated under reduced pressure and then separated and purified by silica gel column chromatography (eluents: ethyl acetate/petroleum ether (v/v) = 20/100) to obtain **47B** (10.0 g, yield 64.5%).

LC-MS (ESI): m/z = 336.2 $[M+H]^+$.

Step 2: **47B** (10.0 g, 29.9 mmol), ethyl acetate (100 mL), and palladium on carbon (2 g) were successively added to a 250 mL single-mouth flask, and the mixture was subjected to a hydrogenation reaction at room temperature for 12 hours. After filtration, the filtrate was concentrated under reduced pressure to obtain **47C** (7.0 g, yield 76.9%).

LC-MS (ESI): m/z = 306.2 $[M+H]^+$.

Step 3: Acetonitrile (30 mL), tert-butyl nitrite (1.5 g, 14.7 mmol), and cuprous chloride (1.2 g, 11.8 mmol) were successively added to a 100 mL single-mouth flask and heated to 65°C, and a solution of **47C** (3.0 g, 9.8 mmol) in acetonitrile (10 mL) was dropwise added. After the dropwise addition was complete, the mixture was reacted at 65°C for 4 hours. After heating was stopped, the reaction was continued for 12 hours. The reaction product was concentrated under reduced pressure and then separated and purified by silica gel column chromatography (eluents: ethyl acetate/petroleum ether (v/v) = 20/100) to obtain **47D** (0.4 g, yield 12.6%).

LC-MS (ESI): m/z = 269.2 $[M-56+H]^+$.

Step 4: **47D** (0.4 g, 1.2 mmol) and a hydrogen chloride-dioxane solution (4N, 10 mL) were successively added to a 50 mL single-mouth flask and reacted at room temperature for 1 hour, and the reaction product was then concentrated under reduced pressure to obtain **47E** (0.4 g, yield 100.0%).

LC-MS (ESI): m/z = 225.1 $[M+H]^+$.

Step 5: **1E** (344 mg, 1.2 mmol), **47E** (400 mg, 1.2 mmol), dioxane (5 mL), and DIPEA (774 mg, 6.0 mmol) were successively added to a 50 mL single-mouth flask and reacted at 100°C for 12 hours. The reaction product was cooled to room temperature, and the reaction liquid was directly concentrated under reduced pressure and then separated and purified by silica gel column chromatography (eluent dichloromethane/methanol (v/v) = 100/8) to obtain 400 mg of a mixture.

After chiral preparation, the title compound **47-1** (90 mg, 31.6%, retention time: 1.897 min) and the title compound **47-2** (80 mg, 28.0%, retention time: 2.355 min) were obtained. Chiral preparation method: instrument: Waters 150 MGM; chromatographic column: Chiralpak Column; mobile phase: A: carbon dioxide, and B: isopropanol (0.1% aqueous ammonia); isocratic elution: 30% mobile phase B; flow rate: 100 mL/min; back pressure: 100 bar; column temperature: 25°C; wavelength: 220 nm; elution time: 20 min.

**(Compound 47-1)**

$^1H$ NMR (400 MHz, DMSO-$d_6$) $\delta$ 7.45 (s, 1H), 6.92 (d, 1H), 6.86-6.81 (m, 1H), 6.77 (d, 1H), 4.93-4.80 (m, 1H), 4.77-4.58 (m, 2H), 4.45-4.30 (m, 1H), 4.05-3.92 (m, 1H), 3.88-3.78 (m, 1H), 3.77-3.65 (m, 2H), 3.52-3.39 (m, 1H), 3.26-3.17 (m, 1H), 3.14-2.84 (m, 4H), 2.77-2.57 (m, 2H), 2.40-2.25 (m, 2H), 2.23-2.14 (m, 2H), 1.89-1.66 (m, 2H).
LC-MS (ESI): m/z = 476.2 $[M+H]^+$.

**(Compound 47-2)**

$^1H$ NMR (400 MHz, DMSO-$d_6$) $\delta$ 7.45 (s, 1H), 6.92 (d, 1H), 6.86-6.80 (m, 1H), 6.77 (d, 1H), 4.90-4.78 (m, 1H), 4.80-4.58 (m, 2H), 4.43-4.31 (m, 1H), 4.07-3.93 (m, 1H), 3.89-3.80 (m, 1H), 3.76-3.68 (m, 2H), 3.52-3.38 (m, 1H), 3.27-3.17 (m, 1H), 3.13-2.83 (m, 4H), 2.76-2.57 (m, 2H), 2.44-2.25 (m, 2H), 2.24-2.12 (m, 2H), 1.86-1.66 (m, 2H).

LC-MS (ESI): m/z = 476.2 $[M+H]^+$.

**Example 43**

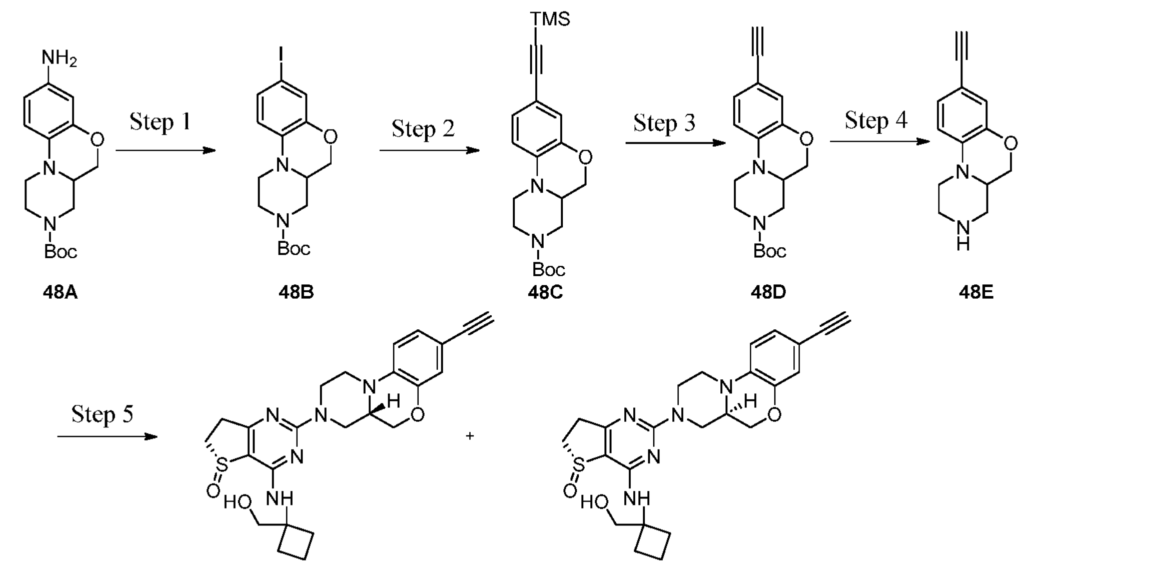


48A 48B 48C 48D 48E

+

**Compounds 48-1 and 48-2**

Step 1: Tert-butyl nitrite (3.55 g, 34.38 mmol) and cuprous iodide (5.24 g, 27.50 mmol) were added to acetonitrile (70 mL), the mixture was warmed to 65°C, and **48A** (7 g, 22.92 mmol) in acetonitrile solution (20 ml) was then dropwise added. The system was maintained at 65°C and reacted for 4 h. After cooling to room temperature, the reaction liquid was concentrated under reduced pressure. Water (50 ml) was then added, the liquid was washed with ethyl acetate, dried over anhydrous sodium sulphate, and filtered, and the filtrate was concentrated under reduced pressure and then separated and purified by silica gel column chromatography (eluent EA/PE = 0-25%) to obtain crude product **48B** (5 g). The compound was directly used for the next reaction.
LC-MS (ESI): m/z = 361.3 $[M+H-56]^+$.
Step 2: **48B** (5 g), trimethylethynylsilane (1.42 g, 14.41 mmol), bis(triphenylphosphino)palladium dichloride (0.84 g, 1.20 mmol), cuprous iodide (0.46 g, 2.40 mmol), and triethylamine (3.65 g, 36.03 mmol) were added to tetrahydrofuran (40 mL). After nitrogen displacement, the mixture was reacted overnight at room temperature, and the reaction liquid was directly concentrated under reduced pressure and then separated and purified by silica gel column chromatography (eluent EA/PE = 0-25%) to obtain 3.5 g of a crude product. Purification was further carried out with a C18 reverse-phase column, wherein the composition of mobile phases was: mobile phase A: acetonitrile; mobile phase B: water (containing 0.1% TFA), so that **48C** (2 g, 43%) was obtained by separation and purification (A/B = 25%/75%).
LC-MS (ESI): m/z = 387.2 $[M+H]^+$.
Step 3: **48C** (1.5 g, 3.88 mmol) and potassium carbonate (1.07 g, 7.76 mmol) were added to methanol (15 mL) and reacted at room temperature for 3 h. The completion of the reaction was monitored by LC-MS. The reaction product

was concentrated under reduced pressure to remove most methanol. Water (20 ml) was added and the mixture was extracted with ethyl acetate (20 ml × 2). The organic phases were combined, washed with a saturated sodium chloride solution, dried over anhydrous sodium sulphate, and filtered. The filtrate was concentrated under reduced pressure to obtain **48D** (1.1 g, 90.18%).

LC-MS (ESI): m/z = 315.5 $[M+H]^+$.

Step 4: **48D** (0.35 g, 1.11 mmol) and triethylamine (1.12 g, 11.11 mmol) were added to dichloromethane (15 mL), and iodotrimethylsilane (1.34 g, 6.69 mmol) was then added dropwise and reacted at room temperature for 2 h. After the reaction was complete, the reaction liquid was directly concentrated under reduced pressure to obtain crude product **48E** (1.7 g). The product was directly brought to the next reaction.

LC-MS (ESI): m/z = 215.5 $[M+H]^+$.

Step 5: Crude product **48E** (1.7 g), **1E** (0.32 g, 1.1 mmol), and N,N-diisopropylethylamine (0.57 g, 4.41 mmol) were added to 1,4-dioxane (15 mL). The mixture was warmed to 100°C and reacted for 5 h. The reaction product was cooled to room temperature, and the reaction liquid was directly concentrated under reduced pressure and then separated and purified by silica gel column chromatography (eluent MeOH/DCM = 0-10%) to obtain 0.4 g of a mixture. After chiral preparation, **48-1** (120 mg, 23.4%, retention time: 1.249 min) and **48-2** (130 mg, 25.4%, retention time: 1.586 min) were obtained. Chiral preparation method: instrument: Waters 150 preparative SFC (SFC-26); chromatographic column: ChiralPak AD, 250 × 30 mm I.D., 10 μm; mobile phase: A: carbon dioxide, and B: isopropanol (0.1% aqueous ammonia); isocratic elution: 40% mobile phase B; flow rate: 100 mL/min; back pressure: 100 bar; column temperature: 38°C; wavelength: 220 nm; elution time: 3.4 min.

**(Compound 48-1)**

$^1H$ NMR (400 MHz, $CDCl_3$) δ 7.02-7.00 (m, 1H), 6.92 (d, 1H), 6.72 (d, 1H), 6.13 (s, 1H), 4.86-4.65(m, 2H), 4.31-4.25 (m, 1H), 4.14-3.93 (m, 2H), 3.90-3.80(m, 2H), 3.79-3.72 (m, 1H), 3.65-3.53 (m, 1H), 3.45-3.36(m, 1H), 3.24-3.12 (m, 2H), 3.10-2.97 (m, 2H), 2.96 (s, 1H), 2.85-2.72 (m, 2H), 2.39-2.29 (m, 2H), 2.28-2.19 (m, 2H), 2.01-1.83 (m, 2H).

LC-MS (ESI): m/z = 466.6 $[M+H]^+$.

**(Compound 48-2)**

$^1H$ NMR (400 MHz, $CDCl_3$) δ 7.04-6.99 (m, 1H), 6.92 (d, 1H), 6.72 (d, 1H), 6.12 (s, 1H), 4.88-4.62 (m, 2H), 4.33-4.25 (m, 1H), 4.15-3.95 (m, 2H), 3.90-3.79 (m, 2H), 3.78-3.72 (m, 1H), 3.65-3.54 (m, 1H), 3.45-3.35 (m, 1H), 3.23-3.13 (m, 2H), 3.10-2.96 (m, 2H), 2.96 (s, 1H), 2.85-2.73 (m, 2H), 2.39-3.00 (m, 2H), 2.29-2.18 (m, 2H), 2.01-1.84 (m, 2H).

LC-MS (ESI): m/z = 466.6 $[M+H]^+$.

## Example 44

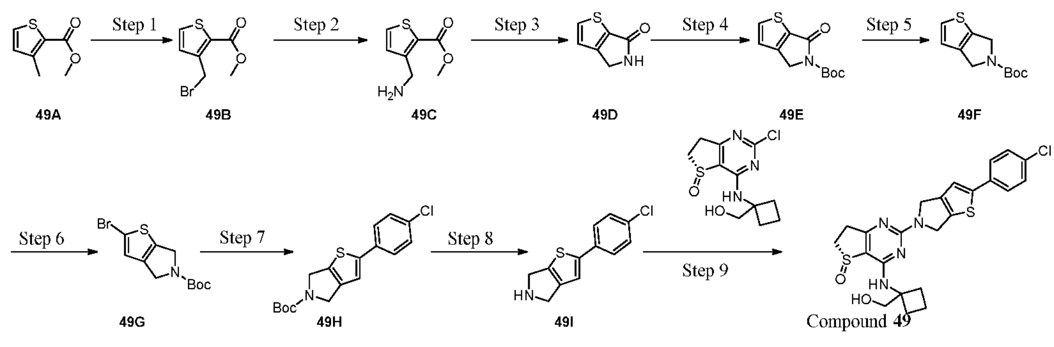


Step 1: Compound **49A** (15 g, 96.03 mmol) was dissolved in carbon tetrachloride (600 mL). N-bromosuccinimide (17.09 g, 96.03 mmol) and azodiisobutyronitrile (1.58 g, 9.60 mmol) were added thereto. Under nitrogen protection, the mixture was heated to 70°C and reacted under stirring overnight. The reaction product was cooled to room temperature, washed with water and a saturated sodium chloride aqueous solution, and dried by anhydrous sodium sulphate, and the filtrate was then spin-dried to obtain compound **49B** (24 g, crude), which was directly used in the next reaction without separation and purification.

LCMS (ESI): = 235.1, 237.1 $[M+H]^+$.

Step 2: The crude product **49B** (17 g, 63.8 mmol) was dissolved in an ammonia methanol solution (7.0 M, 20 mL) and methanol (20 mL), stirred at room temperature for 2 hours, evaporated under reduced pressure to remove the solvent, and then separated by a silica gel chromatographic column (DCM : MeOH, v/v = 10 : 1) to obtain compound **49C** (8 g, two-step yield 48.7%).

LCMS (ESI): = 172.1 [M+H] $^+$.

Step 3: Compound **49C** (8 g, 46.73 mmol) was dissolved in a mixed solvent of methanol/ethanol (120 mL, v/v = 1 : 1). Potassium carbonate (12.92 g, 93.45 mmol) was added thereto, and the mixture was stirred, heated to 90°C, and reacted for 3 hours. The reaction product was cooled to room temperature, evaporated under reduced pressure to remove the solvent, and 150 ml of ethyl acetate was then added. After dissolution, the solid was filtered off, and after the solvent was removed by spin drying, compound **49D** (3.25 g, 36%) was obtained.

LCMS (ESI): = 140.0 [M+H] $^+$.

Step 4: **49D** (3.25 g, 23.35 mmol) was dissolved in dichloromethane (50 mL), triethylamine (7.09 g, 70.06 mmol) and a catalytic amount of 4-dimethylaminopyridine (285 mg, 2.34 mmol) were added thereto. The mixture was cooled in ice bath, di-tert-butyl dicarbonate (10.2 g, 46.71 mmol) was added dropwise and stirred at room temperature for 3 hours, and the mixture was directly spin-dried and separated by a silica gel chromatographic column (EA:PE = 1:3) to obtain compound **49E** (4.45 g, 79.6%).

LCMS (ESI): = 184.1 [M+H] $^+$.

Step 5: **49E** (4.45 g, 18.60 mmol) was dissolved in tetrahydrofuran (50 mL). Under nitrogen protection, the mixture was cooled by ice water, and a borane tetrahydrofuran complex (55.8 mL, 1.0 mol/L) was added dropwise thereto. After the dropwise addition was complete, the mixture was reacted at room temperature for 3 hours. After cooling in ice water, the reaction was quenched with a small amount of methanol, and the reaction product was evaporated under reduced pressure to remove the solvent to obtain a crude product, which was separated by a silica gel chromatographic column (EA:PE = 1:3) to obtain **49F** (2.26 g, 53.9%).

LCMS (ESI): = 170.1 [M+H] $^+$.

Step 6: **49F** (2.26 g, 10.03 mmol) was dissolved in chloroform (25 mL). A catalytic amount of glacial acetic acid was added. Liquid bromine (1.6 g, 10.03 mmol) was added dropwise under ice water cooling. The mixture was stirred at room temperature overnight, and triethylamine (3.04 g, 30.09 mmol) and a catalytic amount of 4-dimethylaminopyridine (123 mg, 1.00 mmol) were added thereto. The mixture was cooled in ice bath, di-tert-butyl dicarbonate (4.38 g, 20.06 mmol) was added dropwise and stirred at room temperature for 3 hours, and the mixture was directly spin-dried and separated by a silica gel chromatographic column (EA:PE = 1:3) to obtain compound **49G** (2.26 g, 74.1%).

LCMS (ESI): = 248.1, 250.1 [M+H] $^+$.

Step 7: **49G** (1 g, 3.29 mmol), 4-chlorophenylboronic acid (616.8 mg, 3.94 mmol), sodium carbonate (697 mg, 6.57 mmol), and tetrakis(triphenylphosphino)palladium (380 mg, 0.33 mmol) were added to a mixed solution of 1,4-dioxane and water (27.5 mL, v/v = 10:1). After nitrogen displacement, the mixture was reacted at 80°C for 3 hours, and the reaction was quenched by adding water. The reaction product was extracted with ethyl acetate, washed with a saturated sodium chloride aqueous solution, dried over anhydrous sodium sulphate, and separated by a silica gel chromatographic column to obtain **49H** (640 mg, 58.0%).

LCMS (ESI): = 280.1, 282.1 [M+H] $^+$.

Step 8: **49H** (300 mg) was added to hydrogen chloride dioxane (5 mL, 4N) and reacted at room temperature for 2 hours. Then, the system was spin-dried to obtain compound **49I** (300 mg, crude), which was directly used in the next reaction.

LCMS (ESI): = 236.1, 238.1 [M+H] $^+$.

Step 9: Compounds **49I** (0.28 g, 1.18 mmol) and **1E** (0.34 g, 1.18 mmol) were dissolved in 1,4-dioxane (10 ml). N,N-diisopropylethylamine (0.46 g, 3.54 mmol) was added. After the addition was complete, the mixture was warmed to 100°C and reacted for 5 hours. After the reaction was complete, the reaction product was cooled to room temperature and then filtered. The filter cake was warmed to 70°C and slurried for 1 hour with acetonitrile/water (v/v = 9/1, 5 mL), followed by hot filtration. The filter cake was rinsed with a small amount of acetonitrile and then dried to obtain the title **compound 49** (350.0 mg, 60.9%).

$^1$H NMR (400 MHz, DMSO-$d_6$) δ 7.69-7.64 (m, 2H), 7.51-7.47 (m, 3H), 4.92-4.70 (m, 4H), 3.81(s, 2H), 3.67-3.62 (m, 1H), 3.39-3.32 (m, 2H) , 3.07-3.03 (m, 1H), 2.39-2.30 (m, 4H), 1.87-1.84 (m, 2H).

MS M/Z (ESI): m/z = 487.1 [M+1]$^+$.

**Example 45**

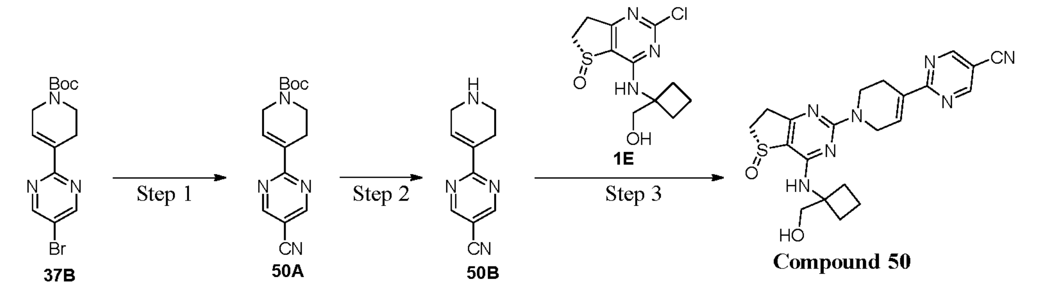


37B 50A 50B Compound 50

Step 1: Compound **37B** (2.0 g, 5.9 mmol) was dissolved in N,N-dimethylformamide (50 ml). Zinc cyanide (0.7 g, 5.9 mmol) and tetrakis(triphenylphosphino)palladium (0.7 g, 5.9 mmol) were then added. After nitrogen displacement three times, the mixture was heated to 90°C and reacted for 3 h, after which the reaction product was passed through a short silica gel column, rinsed with ethyl acetate, concentrated, and subjected to column chromatography (ethyl acetate/petroleum ether = 1/9) to obtain compound **50A** (1.5 g, 89%).

LC-MS (ESI): m/z = 287.4 $[M+H]^+$.

Step 2: Compound **50A** (1.5 g, 5.2 mmol) was dissolved in dichloromethane (50 mL). Trifluoroacetic acid (6.0 mL) was then added, and the mixture was reacted at room temperature for half an hour and then concentrated to obtain compound **50B** (0.9 g, 92%), which was directly used in the next step.

LC-MS (ESI): m/z = 187.2 $[M+H]^+$.

Step 3: Compound **50B** (0.9 mg, 4.8 mmol) was dissolved in 1,4-dioxane (50.0 mL). Compound **1E** (1.4 g, 4.8 mmol) and N,N-diisopropylethylamine (0.7 g, 5.3 mmol) were then added, and the mixture was heated back to 90°C and reacted for 12 h, after which the reaction product was cooled to room temperature, concentrated, and subjected to column chromatography (dichloromethane/methanol = 10/1) to obtain compound **50** (0.3 g, 14%).

LC-MS (ESI): m/z = 438.5 $[M+H]^+$.

$^1$H NMR (400 MHz, $CDCl_3$) δ 8.92 (s, 1H), 7.52 (s, 1H), 6.21 (s, 1H), 4.55 (s, 1H), 4.05 (t, 2H), 3.93-3.82 (m, 2H), 3.65-3.57 (m, 1H), 3.43-3.35 (m, 1H), 3.08-2.99 (m, 2H), 2.78 (s, 2H), 2.39-2.31 (m, 2H), 2.31-2.25 (m, 2H), 1.97-1.84 (m, 2H).

## Example 46

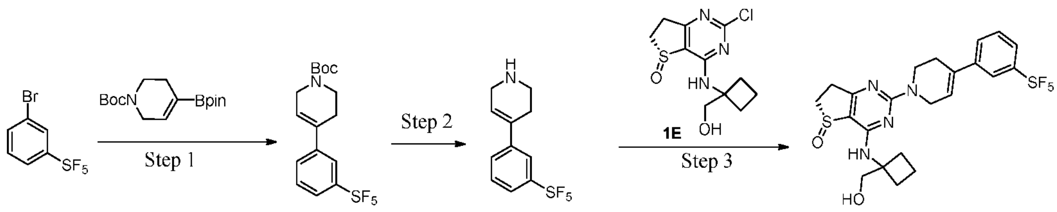


51A 51B 51C 51

Step 1: Compound **51A** (0.9 g, 3.2 mmol) was dissolved in 1,4-dioxane (40 mL). 1,1-Bis(diphenylphosphino) ferrocene palladium dichloride (0.2 mg, 0.3 mmol), caesium carbonate (2.1 g, 6.3 mmol), N-Boc-1,2,5,6-tetrahydropyridine-4-boronic acid pinacol ester (1.1 g, 3.5 mmol), and water (10 mL) were then added. After nitrogen displacement three times, the mixture was heated to 90°C and reacted for 4 h, after which the reaction product was passed through a short silica gel column, rinsed with ethyl acetate, concentrated, and subjected to column chromatography (petroleum ether/ethyl acetate = 9/1) to obtain compound **51B** (0.7 g, 57%).

LC-MS (ESI): m/z = 386.4 $[M+H]^+$.

Step 2: Compound **51B** (0.7 g, 1.8 mmol) was dissolved in dichloromethane (20 mL). Trifluoroacetic acid (2.8 mL) was then added, the mixture was reacted at room temperature for half an hour and then concentrated to obtain compound **51C** (0.5 g, 96%), which was directly used in the next step.

Step 3: Compound **50C** (0.5 mg, 1.8 mmol) was dissolved in 1,4-dioxane (30.0 mL). Compound **1E** (0.5 g, 1.8 mmol) and N,N-diisopropylethylamine (1.8 g, 14.5 mmol) were then added, and the mixture was heated back to 90°C and reacted for 12 h, after which the reaction product was cooled to room temperature, concentrated, and subjected to column chromatography (dichloromethane/methanol = 10/1) to obtain compound 51 (0.5 g, 53%).

LC-MS (ESI): m/z = 537.1 $[M+H]^+$.

$^1$H NMR (400 MHz, $CDCl_3$) δ 7.74 (s, 1H), 7.64 (d, 1H), 7.51 (d, 1H), 7.43 (t, 1H), 6.34 (s, 1H), 6.18 (s, 1H), 4.43 (s,

2H), 4.07 (s, 2H), 3.90 -3.81 (m, 2H), 3.67 -3.59 (m, 1H), 3.42-3.34 (m, 1H), 3.08-3.04 (m, 2H), 2.61 (s, 2H), 2.40-2.33 (m, 2H), 2.27-2.22 (m, 2H), 1.96-1.83 (m, 2H).

$^{19}F$ NMR (377 MHz, $CDCl_3$) δ 64.91 (s), 64.62 (s).

### Example 47

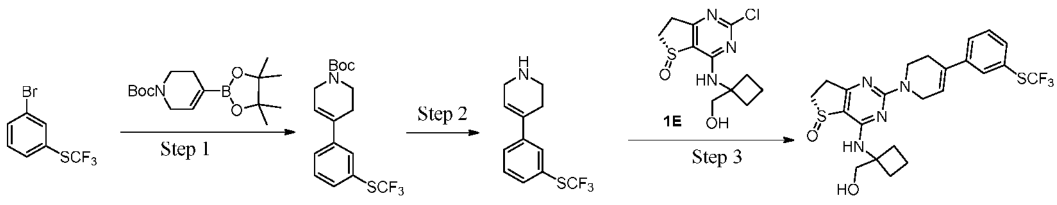


52A 52B 52C Compound 52

Step 1: Compound **52A** (1.0 g, 3.8 mmol) was dissolved in 1,4-dioxane (40 mL). 1,1-Bis(diphenylphosphino) ferrocene palladium dichloride (0.3 mg, 0.4 mmol), caesium carbonate (2.5 g, 7.7 mmol), N-Boc-1,2,5,6-tetrahydropyridine-4-boronic acid pinacol ester (1.3 g, 4.3 mmol), and water (10 mL) were then added. After nitrogen displacement three times, the mixture was heated to 90°C and reacted for 3 h, after which the reaction product was passed through a short silica gel column, rinsed with ethyl acetate, concentrated, and subjected to column chromatography (petroleum ether/ethyl acetate = 9/1) to obtain compound **52B** (1.3 g, 94%).

LC-MS (ESI): m/z = 360.4 $[M+H]^+$.

Step 2: Compound **52B** (1.3 g, 3.6 mmol) was dissolved in dichloromethane (20 mL). Trifluoroacetic acid (2.8 mL) was then added, and the mixture was reacted at room temperature for half an hour and then concentrated to obtain compound **52C** (0.8 g, 85%), which was directly used in the next step.

Step 3: Compound **52C** (0.7 mg, 2.7 mmol) was dissolved in 1,4-dioxane (30.0 mL). Compound **1E** (0.78 g, 2.7 mmol) and N,N-diisopropylethylamine (1.1 g, 8.1 mmol) were then added, and the mixture was heated back to 90°C and reacted for 12 h, after which the reaction product was cooled to room temperature, concentrated, and subjected to column chromatography (dichloromethane/methanol = 10/1) to obtain compound 52 (0.55 g, 40%).

LC-MS (ESI): m/z = 511.1 $[M+H]^+$.

$^1H$ NMR (400 MHz, $CDCl_3$) δ 7.66 (s, 1H), 7.55 (d, 1H), 7.50 (d, 1H), 7.39 (t, 1H), 6.18 (s, 1H), 6.13 (s, 1H), 4.41 (s, 2H), 4.35 (s, 1H), 4.05 (s, 2H), 3.87 (s, 2H), 3.65-3.57 (m, 1H), 3.43-3.35 (m, 1H), 3.08-2.96 (m, 2H), 2.60 (s, 2H), 3.35-3.25 (m, 4H), 2.00-1.85 (m, 2H).

$^{19}F$ NMR (377 MHz, $CDCl_3$) δ -40.50 (s).

### Example 48

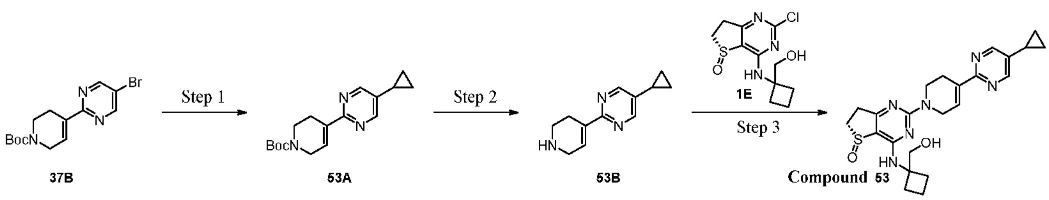


37B 53A 53B Compound 53

Step 1: Compound **37B** (1.0 g, 3.0 mmol), cyclopropylboronic acid (356.0 mg, 4.0 mmol), palladium acetate (25.0 mg, 0.15 mmol), tricyclohexylphosphine (84.2 mg, 0.3 mmol), potassium phosphate (2.3 g, 10.5 mmol), and finally, toluene (14 mL) and water (0.7 mL) were added. After nitrogen displacement three times, the mixture was heated to 100°C and reacted for 3 h, the reaction product was then passed through a short silica gel column, rinsed with ethyl acetate, concentrated, and subjected to column chromatography (petroleum ether/ethyl acetate = 10/1) to obtain compound **53A** (820.0 mg, 91%).

LC-MS (ESI): m/z = 302.1 $[M+H]^+$.

Step 2: Compound **53A** (820.0 mg, 2.7 mmol) was dissolved in dichloromethane (20 mL). Trifluoroacetic acid (5.0 mL) was then added, the mixture was reacted at room temperature for half an hour and then concentrated to obtain compound **53B** (1.0 g, 99%), which was directly used in the next step.

LC-MS (ESI): m/z = 202.1 [M+H]+.

Step 3: Compound **53B** (400.0 mg, 2.0 mmol) was dissolved in 1,4-dioxane (10.0 mL). Compound **1E** (540.0 mg, 2.0 mmol) and diisopropylethylamine (1.0 g, 8.0 mmol) were then added, and the mixture was heated back to 90°C and reacted for 12 h, after which the reaction product was cooled to room temperature, concentrated, and subjected to column chromatography (dichloromethane/methanol = 10/1) to obtain compound **53** (180.0 mg, 20%).

LC-MS (ESI): m/z = 453.1 [M+H]+.

$^1$H NMR (400 MHz, DMSO-$d_6$) δ 8.54 (s, 2H), 7.35 (s, 1H), 7.18 (s, 1H), 4.86-4.83 (m, 1H), 4.43 (s, 2H), 3.97-3.94 (m, 2H), 3.75 (d, 2H), 3.45-3.38 (m, 1H), 3.23-3.18 (m, 1H), 3.00-2.78 (m, 2H), 2.64 (s, 2H), 2.43-2.29 (m, 2H), 2.22-2.18 (m, 2H), 2.02-1.89 (m, 1H), 1.89-1.69 (m, 2H), 1.13-0.96 (m, 2H), 0.89-0.77 (m, 2H).

**Example 49**

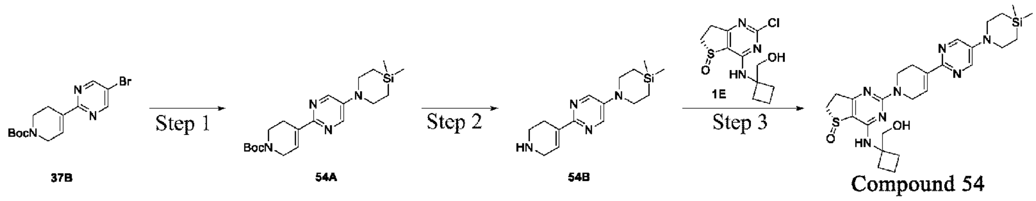


37B 54A 54B Compound 54

Step 1: Compound **37B** (1.0 g, 2.9 mmol) was dissolved in toluene (50 mL), and tris(dibenzylideneacetone) dipalladium (266.1 mg, 0.29 mmol), 2-(di-tert-butylphosphino)biphenyl (179.0 mg, 0.6 mmol) and sodium tert-butoxide (576.0 mg, 6.0 mmol) were then added. After nitrogen displacement three times, the mixture was heated to 100°C and reacted for 3 h. The reaction product was passed through a short silica gel column, rinsed with ethyl acetate, concentrated, and subjected to column chromatography (ethyl acetate/petroleum ether = 1/9) to obtain compound **54A** (258.0 mg, 23%).

LC-MS (ESI): m/z = 389.2 [M+H]+.

Step 2: Compound **54A** (258.0 mg, 0.66 mmol) was dissolved in dichloromethane (50 mL), and trifluoroacetic acid (3.0 mL) was then added. The mixture was reacted at room temperature for half an hour and then concentrated to obtain compound **54B** (218.0 mg, 99%), which was directly used in the next step.

LC-MS (ESI): m/z = 289.2 [M+H]+.

Step 3: Compound **54B** (218.0 mg, 0.66 mmol) was dissolved in 1,4-dioxane (30.0 mL), compound **1E** (190.0 mg, 0.66 mmol) and N,N-diisopropylethylamine (0.35 g, 2.6 mmol) were then added, and the mixture was heated back to 90°C and reacted for 3 h, after which the reaction product was cooled to room temperature, concentrated, and subjected to column chromatography (dichloromethane/methanol = 10/1) to obtain compound **54** (0.06 g, 17%).

LC-MS (ESI): m/z = 540.2 [M+H]+.

$^1$H NMR (400 MHz, DMSO-$d_6$) δ 8.30 (s, 2H), 7.30 (s, 1H), 6.86 (s, 1H), 4.80-4.78 (m, 1H), 4.31 (s, 2H), 3.87 (s, 2H), 3.76-3.47 (m, 6H), 3.37-3.31 (m, 1H), 3.17-3.09 (m, 1H), 2.90-2.76 (m, 2H), 2.55 (s, 2H), 2.34-2.21 (m, 2H), 2.15-2.10 (m, 2H), 1.83-1.60 (m, 2H), 0.76-0.53 (m, 4H), 0.00 (s, 6H).

**Example 50**

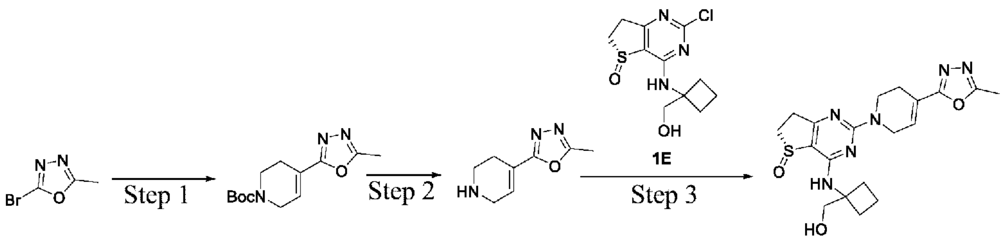


55A 55B 55C Compound 55

Step 1: Compound **55A** (750 mg, 4.6 mmol) was dissolved in 1,4-dioxane (12 mL), 1,1-bis(diphenylphosphino) ferrocene palladium dichloride (340 mg, 0.46 mmol), caesium carbonate (4.5 g, 13.8 mmol), N-Boc-1,2,5,6-tetrahydropyridine-4-boronic acid pinacol ester (2.1 g, 6.89 mmol), and water (3 mL) were then added. After nitrogen displacement

three times, the mixture was heated to 90°C and reacted for 16 h, after which the reaction product was passed through a short silica gel column, rinsed with ethyl acetate, concentrated, and subjected to column chromatography (petroleum ether/ethyl acetate = 1/1) to obtain compound **55B** (0.8 g, 66%).

LC-MS (ESI): m/z = 266.1 $[M+H]^+$.

Step 2: Compound **55B** (400 mg, 1.51 mmol) was dissolved in dichloromethane (10 mL), trifluoroacetic acid (5.0 mL) was then added, and the mixture was reacted at room temperature for half an hour and then concentrated. The residue was dissolved with dichloromethane and washed with a saturated sodium bicarbonate aqueous solution, and the organic phase was dried and concentrated to obtain compound **55C** (240 mg, 96%), which was directly used in the next step.

LC-MS (ESI): m/z = 166.1 $[M+H]^+$.

Step 3: Compound **55C** (390 mg, 1.36 mmol) was dissolved in 1,4-dioxane (10.0 mL), compound **1E** (220 mg, 1.33 mmol) and N,N-diisopropylethylamine (530 mg, 4.08 mmol) were then added, and the mixture was heated back to 90°C and reacted for 12 h, after which the reaction product was cooled to room temperature, concentrated, and subjected to column chromatography (dichloromethane/methanol = 10/1) to obtain compound **55** (440 mg, 78%).

LC-MS (ESI): m/z = 417.2 $[M+H]^+$.

$^1H$ NMR (400 MHz, $CDCl_3$) δ 6.75-6.70 (m, 1H), 6.24 (s, 1H), 4.46 (s, 2H), 4.03 (s, 2H), 3.95-3.79 (m, 2H), 3.67-3.53 (m, 1H), 3.44-3.34 (m, 1H), 3.10-2.96 (m, 2H), 2.76-2.68(m, 2H), 2.54 (s, 3H), 2.42-2.20 (m, 4H), 2.00-1.78 (m, 2H).

**Example 51**

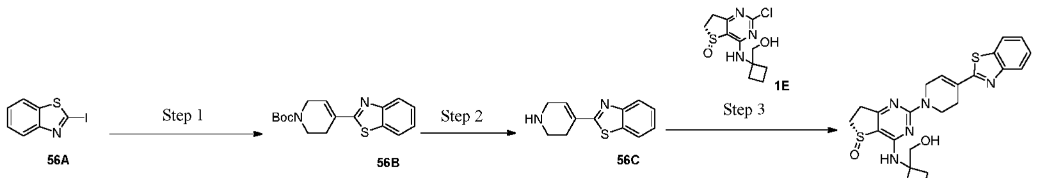

56A 56B 56C 1E Compound 56

Step 1: Compound **56A** (2.00 g, 7.67 mmol), N-Boc-1,2,5,6-tetrahydropyridine-4-boronic acid pinacol ester (2.84 mg, 9.19 mmol), tetrakis(triphenylphosphino)palladium (886 mg, 0.767 mmol), and sodium carbonate (2.43 mg, 23.0 mmol) were suspended in 50 mL of a mixed solvent of dioxane : water = 4:1 and reacted at 80°C for 16 h. After the reaction was complete, the reaction liquid was dropwise added to 100 mL of ice water and filtered. The filtrate was extracted with dichloromethane (100 mL × 3), and the organic phases were combined, concentrated, and purified by silica gel column chromatography (PE:EA = 40:1) to obtain the target compound **56B** (420 mg, 17%).

LCMS (ESI): m/z = 317.1 $[M+H]^+$.

Step 2: Compound **56 B** (420 mg, 1.33 mmol) was dissolved in 2 mL of methanol, 2 mL of hydrogen chloride/dioxane was added and stirred at room temperature for 1.5 h, and the mixture was filtered. The filter cake was namely the target compound **56C** (260 mg, 77%), which was directly used in the next reaction without purification.

LCMS (ESI): m/z = 217.1 $[M+H]^+$.

Step 3: Compound **56C** (200 mg, 1.02 mmol), intermediate **1E** (355 mg, 1.23 mmol), and DIPEA (33 mg, 0.03 mmol) were suspended in 2 mL of dioxane and reacted at 80°C for 16 h. After the reaction was complete, the reaction product was filtered. The filter cake was slurried with 5 mL of methanol, stirred at room temperature for 0.5 h, and filtered. The filter cake was namely the target compound **56** (190 mg, 51%).

LCMS (ESI): m/z = 468.1 $[M+H]^+$.

$^1H$ NMR (400 MHz, DMSO-$d_6$) δ 8.07 (d, 1H), 7.97 (d, 1H), 7.53-7.47 (m, 1H), 7.46-7.37 (m, 2H), 6.92-6.89(m, 1H), 4.84 (t, 1H), 4.47 (s, 2H), 4.01 (t, 2H), 3.76 (d, 2H), 3.50-3.39(m, 1H), 3.29-3.17 (m, 1H), 3.02-2.83 (m, 2H), 2.76 (s, 2H), 2.44-2.29 (m, 2H), 2.29-2.17 (m, 2H), 1.91-1.61 (m, 2H).

**Example 52**

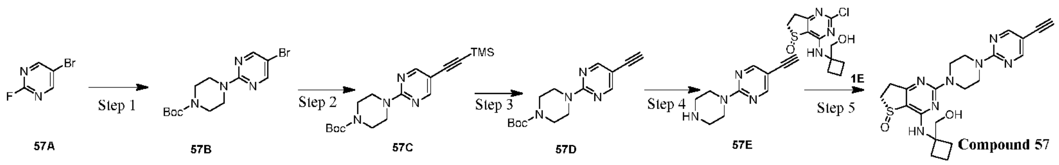


Step 1: Compound **57A** (3.00 g, 16.92 mmol) and the compound tert-butyl piperazine-1-carboxylate (3.78 g, 20.30 mmol) were weighed into a 100 mL single-mouth flask and dissolved by adding 1,4-dioxane (30 mL), and N,N-diisopropylethylamine (6.56 g, 50.76 mmol) was added. After the addition was complete, the mixture was stirred at 80°C for 16 h. After the completion of the reaction was detected by TLC plate spotting (petroleum ether : ethyl acetate = 5:1), water (20 mL) was added and stirred for 5 min, and ethyl acetate (20 mL) was added for extraction. The organic phase was separated, which was dried over anhydrous sodium sulphate, concentrated under reduced pressure, and purified by column chromatography (eluents: petroleum ether : ethyl acetate = 5:1) to obtain the target compound **57B** (2.8 g, yield: 48%).

LCMS m/z = 343.1 $[M+1]^+$.

Step 2: Compound **57B** (1.0 g, 2.92 mmol) and trimethylethynylsilane (1.43 g, 14.61 mmol) were weighed into a 100 mL stewing pot and dissolved with tetrahydrofuran (20 mL), and triethylamine (1.21 mL), [1,1'-bis(diphenylphosphino) ferrocene]palladium dichloride (0.24 g, 0.29 mmol), and cuprous iodide (56 mg, 0.29 mmol) were added. After the addition was complete, the mixture was stirred at 80°C for 12 h. After the completion of the reaction was monitored by TLC plate spotting (petroleum ether : ethyl acetate = 5:1), water (20 mL) was added and stirred for 5 min, ethyl acetate (20 mL) was added for extraction, and an organic phase was separated, which was dried over anhydrous sodium sulphate, concentrated under reduced pressure, and purified by column chromatography (eluents: petroleum ether : ethyl acetate = 5:1) to obtain the target compound **57C** (0.50 g, 47%).

LCMS m/z = 361.1 $[M+1]^+$.

Step 3: Compound **57C** (0.5 g, 1.39 mmol) was weighed into a 100 mL single-mouth flask and dissolved with methanol (10 mL), and potassium carbonate (0.58 g, 4.2 mmol) was added. After the addition was complete, the mixture was stirred at room temperature for 2 h. After the reaction was found complete by TLC plate spotting (petroleum ether : ethyl acetate = 3:1), the reaction liquid was filtered, and the filtrate was concentrated under reduced pressure and purified by column chromatography (eluents: petroleum ether : ethyl acetate = 3:1) to obtain the target compound **57D** (0.40 g, 100%).

LCMS m/z = 289.2 $[M+1]^+$.

Step 4: Compound **57D** (0.2 g, 0.69 mmol) was weighed into a 100 mL single-mouth flask and dissolved with methanol (5 mL), and a hydrogen chloride dioxane solution (4N, 5 mL) was added. After the addition was complete, the mixture was stirred at room temperature for 2 h and concentrated under reduced pressure to obtain the target compound **57E** (0.15 g, crude).

LCMS m/z = 189.1 $[M+1]^+$.

Step 5: Compound **1E** (200 mg, 0.66 mmol) was added to a 100 mL single-mouth flask and dissolved in 1,4-dioxane (8 mL), and compound **57E** (131 mg, 0.66 mmol) and N,N-diisopropylethylamine (0.26 g, 1.98 mmol) were added. After the addition was complete, the system was placed under nitrogen protection and stirred at 90°C for 16 h, and the reaction liquid was concentrated under reduced pressure to obtain a crude product of the target compound, which was separated by preparative HPLC to obtain the title compound **57** (100 mg, 33%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 40-80%; c. flow rate: 15 mL/min; d. elution time: 20 min; retention time: 10.50 min.

LCMS m/z = 440.2 $[M+1]^+$.

$^1H$ NMR (400 MHz, DMSO-$d_6$) δ 8.51 (s, 2H), 7.46 (s, 1H), 4.85 (t, 1H), 4.31 (s, 1H), 3.83 (s, 8H), 3.72 (d, 2H), 3.33-3.45 (m, 1H), 3.15-3.25 (m, 1H), 2.84-2.98 (m, 2H), 2.26 -2.40 (m, 2H), 2.13-2.17 (m, 2H), 1.71-1.82 (m, 2H).

**Example 53**

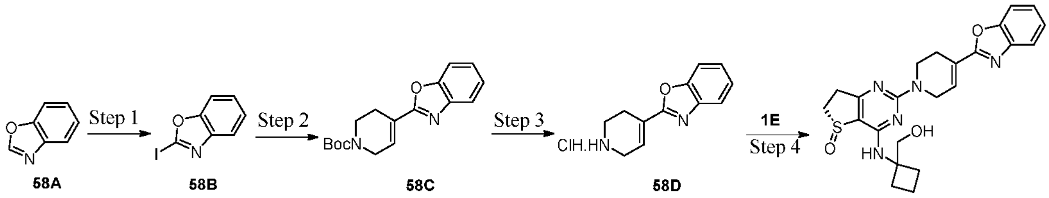


58A 58B 58C 58D

Compound 58

Step 1: Under nitrogen protection, **58A** (1.00 g, 8.39 mmol) was dissolved in tetrahydrofuran (30 mL). After the solution was cooled to -78°C, n-butyl lithium (810 mg, 12.59 mmol) was slowly added. After the addition was complete, the reaction liquid was warmed to -40°C, stirred for 1 h, and then cooled to -78°C. A solution of iodine (2.56 g, 10.07 mmol) in tetrahydrofuran (10 mL) was added. The system naturally returned to room temperature and was stirred for 1 h. The reaction was quenched by adding water (100 mL) and extracted with ethyl acetate (100 mL) three times. The organic phases were combined, dried over anhydrous sodium sulphate, and then concentrated in vacuum to obtain a crude product. The crude product was purified by column chromatography (eluents: PE : EA = 100 : 1 to 20 : 1) to obtain the target compound **58B** (700 mg, yield: 34.03%).

$^1$H NMR (400 MHz, DMSO-*d6*) δ 7.76-7.73 (m, 2H), 7.38-7.36 (m, 2H).

Step 2: Compound **58B** (700 mg, 2.86 mmol), [1-(tert-butoxycarbonyl)-1,2,3,6-tetrahydropyridin-4-yl]boronic acid (910 mg, 4.00 mmol), potassium carbonate (990 mg, 7.14 mmol), and [1,1'-bis(diphenylphosphino)ferrocene] palladium dichloride (110 mg, 0.14 mmol) were added to a reaction flask. 20 mL of tetrahydrofuran and water (5 mL) were added, and after nitrogen displacement, the mixture was warmed to 85°C and reacted for 16 h. After the disappearance of raw materials was detected by plate spotting, the reaction product was diluted by adding water (100 mL) and extracted with ethyl acetate (100 mL) three times. The organic phases were combined, dried over anhydrous sodium sulphate, and then concentrated in vacuum to obtain a crude product. The crude product was purified by column chromatography (eluents: PE : EA = 100 : 1 to 20 : 1) to obtain the target compound **58C** (850 mg, yield: 99.06%).

LC-MS (ESI): m/z = 301.2 $[M+H]^+$.

$^1$H NMR (400 MHz, $CDCl_3$) δ 7.72-7.70 (m, 1H), 7.51-7.48 (m, 1H), 7.33-7.31 (m, 2H), 6.97 (s, 1H), 4.21-4.20 (m, 2H), 3.68-3.65 (m, 2H), 2.76 (s, 2H),1.50 (s, 9H).

Step 3: Compound **58C** (850 mg, 2.83 mmol) was dissolved in 20 mL of dichloromethane, and hydrogen chloride dioxane (4 mL) was added and then stirred at 0°C for 30 min. After the disappearance of raw materials was detected by plate spotting, the reaction liquid was concentrated at room temperature to obtain crude **58D** (850 mg), which was directly used in the next reaction.

Step 4: Compound **1E** (300 mg, 1.04 mmol) and compound **58D** (320 mg, 1.36 mmol) were dissolved in 1,4-dioxane (8 mL), and DIPEA (405 mg, 3.13 mmol) was then added. After the addition was complete, the mixture was warmed to 85°C under nitrogen protection and stirred for 16 h. After the reaction was complete, the reaction liquid was concentrated under reduced pressure to obtain a crude product, which was purified by column chromatography (eluents: DCM : MeOH= 100 : 1 to 9 : 1) and then further purified by preparative HPLC (preparation method: instrument: waters 2767 preparative liquid chromatographic instrument; chromatographic column: SunFire@PrepC18 (19 mm × 250 mm; the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution, mobile phase A: 25-90%; c. flow rate: 15 mL/min; d. elution time: 15 min; retention time: 6.0 min) to obtain the target compound **58** (105 mg, 22.30%).

LCMS m/z = 452.2 $[M+H]^+$.

$^1$H NMR (400 MHz, $CDCl_3$) δ 7.73-7.70 (m, 1H), 7.52-7.50 (m, 1H), 7.34-7.31 (m, 2H), 7.05 (s, 1H), 6.24 (s, 1H), 4.52 (s, 2H), 4.08 (s, 2H), 3.92-3.84 (m, 2H), 3.67-3.58 (m, 1H), 3.43-3.35 (m, 1H), 3.09-2.99 (m, 2H), 2.83 (s, 2H), 2.42-2.23 (m, 5H), 1.99-1.84 (m, 2H).

**Example 54**

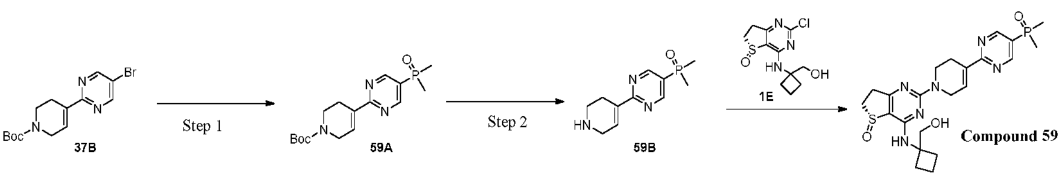


37B 59A 59B Compound 59

Step 1: **37B** (1.1 g, 3.23 mmol) was added to a 100 mL single-mouth flask and dissolved with dioxane (20 mL). Dimethylphosphine oxide (0.38 g, 4.88 mmol), anhydrous potassium phosphate (0.75 g, 3.53 mmol), 4,5-bis(diphenylphosphino)-9,9-dimethylxanthene (0.37 g, 0.65 mmol), and tris(dibenzylideneacetone)dipalladium (0.30 g, 0.32 mmol) were successively added. Under nitrogen protection, the mixture was reacted at 100 degrees Celsius for 7 h, and the product was concentrated and purified by column chromatography (DCM : MeOH = 10 : 1) to obtain the title compound 59A (0.8 g, 73%).

LC-MS (ESI): m/z = 282.4 $[M-56+H]^+$.

Step 2: **59A** (0.5 g, 1.48 mmol) was added to a 50 mL single-mouth flask, dissolved with methanol (10 mL) and hydrogen chloride dioxane (4 M, 10 mL), and stirred at room temperature for 3 h, and the reaction liquid was concentrated to obtain the title compound **59B** (0.40 g, 100%).

LC-MS (ESI): m/z = 238.1 $[M+H]^+$.

Step 3: **1E** (0.15 g, 0.52 mmol) was added to a 50 mL single-mouth flask and dissolved with dioxane (10 mL), **59B** (0.25 g, 1.06 mmol) and diisopropylethylamine (0.27 g, 2.05 mmol) were added and reacted at 90 degrees Celsius overnight, and the reaction liquid was concentrated, purified by HPLC, and freeze-dried to obtain the title compound **59** (23 mg, 7%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 0.1% TFA); b. gradient elution, mobile phase A: 5-60%; c. flow rate: 15 mL/min; d. elution time: 15 min; retention time: 8.0 min.

$^1$H NMR (400 MHz, DMSO-d6) δ 9.08 (d, 2H), 8.29-8.14 (m, 1H), 7.46-7.38 (m, 1H), 4.50 (s, 2H), 3.99 (t, 2H), 3.80-3.74 (m, 2H), 3.61-3.49 (m, 1H), 3.34-3.24 (m, 1H), 3.15-3.09 (m, 1H), 3.04-2.92 (m, 1H), 2.74 (s, 2H), 2.42-2.19 (m, 4H), 1.85-1.76 (m, 8H).

LC-MS (ESI): m/z = 489.5 $[M+H]^+$.

### Example 55

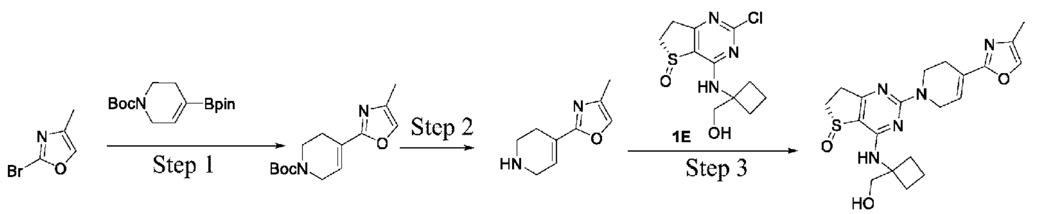


60A 60B 60C Compound 60

Step 1: Compound **60A** (800 mg, 4.94 mmol) was dissolved in 1,4-dioxane (20 mL), and 1,1-bis(diphenylphosphino)ferrocene palladium dichloride (360 mg, 0.49 mmol), caesium carbonate (4.83 g, 14.82 mmol), N-Boc-1,2,5,6-tetrahydropyridine-4-boronic acid pinacol ester (2.29 g, 7.41 mmol), and water (4 mL) were then added. After nitrogen displacement three times, the mixture was heated to 90°C and reacted for 16 h, after which the reaction product was passed through a short silica gel column, rinsed with ethyl acetate, concentrated, and subjected to column chromatography (petroleum ether/ethyl acetate = 1/1) to obtain compound **60B** (0.9 g, 69%).

LC-MS (ESI): m/z = 265.1 $[M+H]^+$.

Step 2: Compound **60B** (5800 mg, 1.89 mmol) was dissolved in dichloromethane (10 mL), trifluoroacetic acid (5.0 mL) was then added, and the mixture was reacted at room temperature for half an hour and then concentrated. The residue was dissolved with dichloromethane and washed with a saturated sodium bicarbonate aqueous solution, and the organic phase was dried and concentrated to obtain compound **60C** (300 mg, 97%), which was directly used in the next step.

LC-MS (ESI): m/z = 165.1 $[M+H]^+$.

Step 3: Compound **60C** (300 mg, 1.04 mmol) was dissolved in 1,4-dioxane (10.0 mL), and compound **1E** (170 mg, 1.04 mmol) and N,N-diisopropylethylamine (400 mg, 3.12 mmol) were then added. The mixture was heated back to 90°C and reacted for 12 h, after which the reaction product was cooled to room temperature, concentrated, and subjected to column chromatography (dichloromethane/methanol = 10/1) to obtain compound 60 (340 mg, 79%).

LC-MS (ESI): m/z = 416.2 $[M+H]^+$.

$^1$H NMR (400 MHz, $CDCl_3$) δ 7.30 (d, 1H), 6.71-6.67 (m, 1H), 6.30 (s, 1H), 4.42 (s, 2H), 4.01 (s, 2H), 3.93-3.77 (m, 2H), 3.67-3.54 (m, 1H), 3.42-3.32 (m, 1H), 3.09-2.96 m, 2H), 2.72-2.64 (m, 2H), 2.45-2.20 (m, 4H), 2.18 (d, 3H), 1.97-1.79 (m, 2H).

**Example 56**

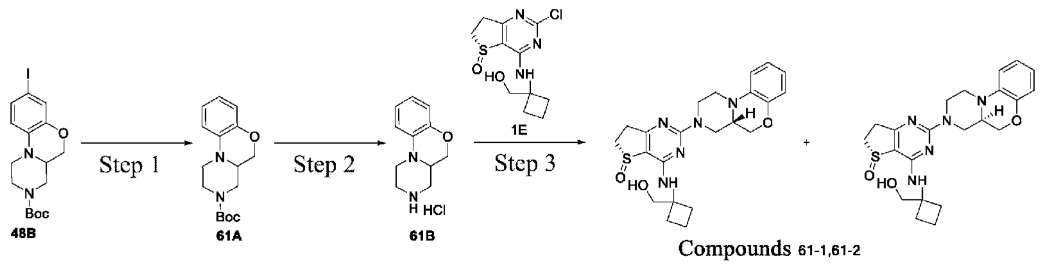

Compounds 61-1,61-2

Step 1: Methanol (10 mL), **48B** (0.5 g, 1.20 mmol), and palladium on carbon (100 mg) were successively added to a 50 mL single-mouth flask, the mixture was subjected to a hydrogenation reaction at room temperature for 16 hours, followed by filtration, and the filtrate was concentrated under reduced pressure to obtain **61A** (0.3 g, yield 86.1%).
LC-MS (ESI): m/z = 235.2 $[M-56+H]^+$.
Step 2: **61A** (0.3 g, 1.03 mmol) and a hydrogen chloride-dioxane solution (4N, 15 mL) were successively added to a 50 mL single-mouth flask and reacted at room temperature for 1 hour, and the reaction product was then concentrated under reduced pressure to obtain **61B** (0.27 g, yield 99.6%).
LC-MS (ESI): m/z = 191.2 $[M+H]^+$.
Step 3: **1E** (287 mg, 1.0 mmol), **61B** (270 mg, 1.03 mmol), dioxane (10 mL), and DIPEA (774 mg, 6.0 mmol) were successively added to a 50 mL single-mouth flask and reacted at 100°C for 12 hours. The reaction product was cooled to room temperature, and the reaction liquid was directly concentrated under reduced pressure and then separated and purified by silica gel column chromatography (eluent dichloromethane/methanol (v/v) = 100/8) to obtain 400 mg of a mixture.
After chiral preparation, the title compound **61-1** (62 mg, 14.1%, retention time: 1.733 min) and the title compound **61-2** (51 mg, 11.5%, retention time: 1.884 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 3.0 min.

**(Compound 61-1)**

$^1H$ NMR (400 MHz, DMSO-$d_6$) δ 7.43 (s, 1H), 6.98-6.86 (m, 1H), 6.86-6.75 (m, 1H), 6.76-6.59 (m, 2H), 4.90-4.79 (m, 1H), 4.78-4.59 (m, 2H), 4.37-4.28 (m, 1H), 4.00-3.92 (m, 1H), 3.90-3.78 (m, 1H), 3.78-3.68 (m, 2H), 3.49-3.36 (m, 1H), 3.27-2.81 (m, 5H), 2.76-2.55 (m, 2H), 2.41-2.12 (m, 4H), 1.87-1.70 (m, 2H).
LC-MS (ESI): m/z = 442.2 $[M+H]^+$.

**(Compound 61-2)**

$^1H$ NMR (400 MHz, DMSO-$d_6$) δ 7.44 (s, 1H), 6.95-6.89 (m, 1H), 6.84-6.77 (m, 1H), 6.75-6.62 (m, 2H), 4.89-4.78 (m, 1H), 4.79-4.59 (m, 2H), 4.39-4.28 (m, 1H), 4.02-3.81 (m, 2H), 3.79-3.68 (m, 2H), 3.51-3.39 (m, 1H), 3.26-3.16 (m, 1H), 3.14-2.83 (m, 4H), 2.76-2.55 (m, 2H), 2.43-2.25 (m, 2H), 2.26-2.13 (m, 2H), 1.89-1.69 (m, 2H).
LC-MS (ESI): m/z = 442.2 $[M+H]^+$.

**Example 57**

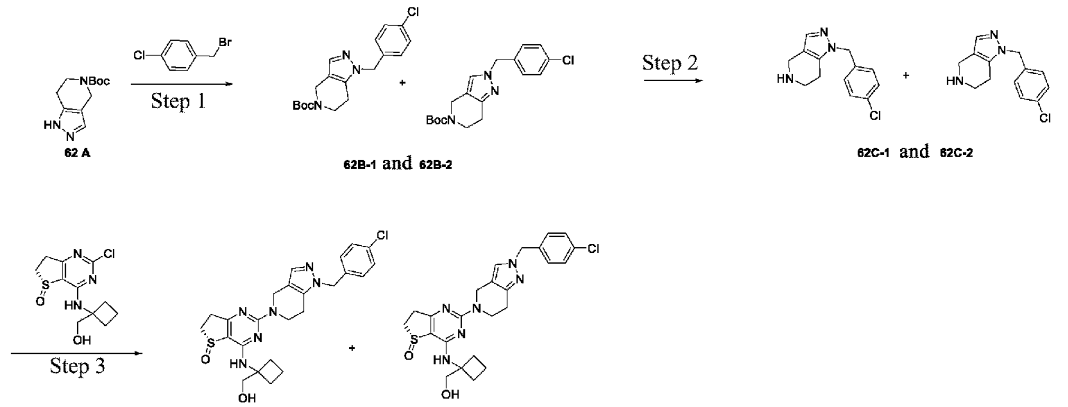


62 A

62B-1 and 62B-2

62C-1 and 62C-2

Compounds 62-1 and 62-2

Step 1: Compound **62A** (1.0 g, 4.5 mmol) was dissolved in DMF (30 mL), and caesium carbonate (2.2 g, 6.7 mmol) and 4-chlorobenzyl bromide (1.4 g, 6.7 mmol) were then added. After nitrogen displacement three times, the mixture was heated to 50°C and reacted for 5 h. The reaction product was passed through a short silica gel column, rinsed with ethyl acetate, concentrated, and subjected to column chromatography (dichloromethane/methanol = 9/1) to obtain a mixture of **62 B-1** and **62 B-2** (0.5 g, 32%).

LC-MS (ESI): m/z = 348.8 $[M+H]^+$.

Step 2: The mixture of compounds **62 B-1** and **62 B-2** (0.5 g, 1.4 mmol) was dissolved in dichloromethane (20 mL), and trifluoroacetic acid (2.5 mL) was then added. After reacting at room temperature for half an hour, the reaction product was concentrated to obtain a mixture of compounds **62 C-1** and **62 C-2** (0.3 g, 85%), which was directly used in the next step.

Step 3: The mixture of compounds **62 C-1** and **62 C-2** (0.3 g, 1.2 mmol) was dissolved in 1,4-dioxane (30.0 mL), and compound **1E** (0.35 g, 1.2 mmol) and N,N-diisopropylethylamine (0.47 g, 3.7 mmol) were then added. The mixture was heated back to 90°C and reacted for 12 h, after which the reaction product was cooled to room temperature, concentrated, and subjected to column chromatography (dichloromethane/methanol = 10/1) to obtain a crude mixture of compounds **62-1 and 62-2.**

The crude mixture of compounds **62-1 and 62-2** (380 mg) was subjected to chiral resolution to obtain compound **62-1** (175 mg) and compound **62-2** (45 mg).

Preparation method: Instrument: Waters 150 SFC, column: Chiralpak AS, mobile phase: (A for $CO_2$ and B for MeOH (0.1% $NH_3$•$H_2O$)); gradient: 35% phase B elution; flow rate: 100 mL/min, back pressure: 100 bar; column temperature: 25°C; wavelength: 220 nm; cycle time: 2.2 min; sample preparation: sample concentration 10 mg/mL, acetonitrile solution; injection: 3.5 mL/injection. After separation, the fractions were dried on a rotary evaporator at a bath temperature of 35°C to obtain P1 (retention time: 1.905 minutes, assigned to compound **62-1)** and P2 (retention time: 2.072 minutes, assigned to compound **62-2).**

LC-MS (ESI): m/z = 499.6 $[M+H]^+$.

$^1$H NMR (400 MHz, DMSO-$d_6$) δ 7.64 (s, 1H), 7.4-7.38 (m, 2H), 7.34 (s, 1H), 7.25-7.23 (m, 2H), 5.22 (s, 1H), 4.82 (t, 2H), 4.76 (s, 2H), 4.03-3.98 (m, 2H), 3.73 (d, 2H), 3.45-3.36 (m, 1H), 3.22-2.18 (m, 1H), 2.95-2.82 (m, 2H), 2.67 (t, 2H), 2.37-2.27 (m, 2H), 2.22-2.14 (m, 2H), 1.82-1.73 (m, 2H).

LC-MS (ESI): m/z = 499.7 $[M+H]^+$.

$^1$H NMR (400 MHz, $CDCl_3$) δ 7.38 (s, 1H), 7.29 -7.27 (m, 2H), 7.07 (s, 1H), 7.05 (s, 1H), 5.98 (s, 1H), 5.19 (s, 2H), 4.79 (s, 2H), 4.07 (s, 2H), 3.91-3.83 (m, 2H), 3.62 -3.54 (m, 2H), 3.41 -3.32 (m, 2H), 3.07-2.94 (m, 2H), 2.62 (t, 2H), 2.34-2.23 (m, 2H), 1.97-1.86 (m, 2H).

**Example 58**

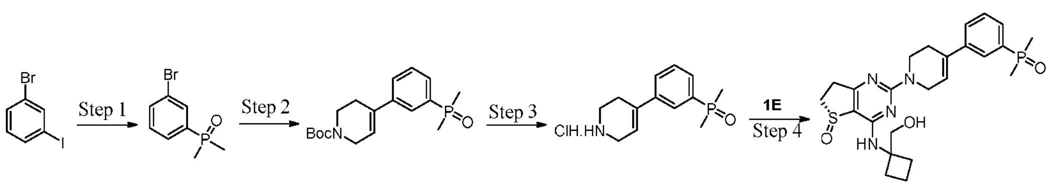


63A 63B 63C 63D Compound 63

Step 1: Under nitrogen protection, **63A** (5.00 g, 17.67 mmol) was dissolved in N,N-dimethylformamide (60 mL). Dimethylphosphine oxide (1.70 g, 21.20 mmol), palladium acetate (0.43 g, 1.77 mmol), 4,5-bis(diphenylphosphino)-9,9-dimethylxanthene (2.00 g, 3.53 mmol), and potassium phosphate (10.00 g, 47.11 mmol) were added successively and separately with stirring at room temperature. After the addition was complete, the reaction liquid was warmed to 100°C, stirred for 16 h, and then cooled to room temperature. The liquid was concentrated under reduced pressure using an oil pump, diluted by adding water (100 mL), and extracted with ethyl acetate (100 mL) three times. The organic phases were combined, dried over anhydrous sodium sulphate, and then concentrated in vacuum to obtain a crude product. The crude product was purified by column chromatography (eluents: DCM : MeOH = 100 : 1 to 20 : 1) to obtain the target compound **63B** (2.00 g, yield: 48.56%).

$^1$H NMR (400 MHz, $CDCl_3$) δ 7.89-7.85 (m, 1H), 7.69-7.64 (m, 2H), 7.40-7.29 (m, 1H), 1.76 (s, 3H), 1.73 (s, 3H).

Step 2: Under nitrogen protection, compound **63B** (2.00 g, 8.58 mmol) was added to a mixed solution of 1,4-dioxane (40 mL) and water (8 mL). [1-(tert-butoxycarbonyl)-1,2,3,6-tetrahydropyridin-4-yl]boronic acid (2.34 g, 10.30 mmol), [1,1'-bis(diphenylphosphino)ferrocene]palladium dichloride (630 mg, 0.86 mmol), and potassium carbonate (3.56 g, 25.75 mmol) were added successively and separately with stirring at room temperature. After the addition was complete, the reaction liquid was warmed to 85°C and stirred under nitrogen protection for 16 h. After the disappearance of raw materials was detected by plate spotting, the reaction product was cooled to room temperature, concentrated under reduced pressure, then diluted by adding water (100 mL), and extracted with ethyl acetate (50 mL) three times. The organic phases were combined, dried over anhydrous sodium sulphate, and then concentrated in vacuum to obtain a crude product. The crude product was purified by column chromatography (eluents: DCM : MeOH = 100 : 1 to 20 : 1) to obtain the target compound **63C** (2.50 g, yield: 86.86%).

LC-MS (ESI): m/z = 280.60 $[M+H-56]^+$.

$^1$H NMR (400 MHz, $CDCl_3$) δ 7.80-7.77 (m, 1H), 7.53-7.51 (m, 1H), 7.48-7.44 (m, 2H), 6.11 (s, 1H), 4.09-4.08 (m, 2H), 3.66-3.60 (m, 2H), 2.55 (s, 2H),1.76 (s, 3H), 1.73 (s, 3H), 1.48 (s, 9H).

Step 3: Compound **63C** (1.10 g, 3.28 mmol) was dissolved in 20 mL of dichloromethane, and hydrogen chloride dioxane (4 mL) was added and then stirred at 0°C for 30 min. After the disappearance of raw materials was detected by TLC, the reaction liquid was concentrated at room temperature to obtain crude **63D** (800 mg), which was directly used in the next reaction.

Step 4: Compound **1E** (500 mg, 1.74 mmol) and compound **63D** (800 mg, 2.94 mmol) were dissolved in 1,4-dioxane (8 mL), and DIPEA (675 mg, 5.21 mmol) was then added. After the addition was complete, the mixture was warmed to 85°C under nitrogen protection and stirred for 16 h. After the reaction was complete, the reaction liquid was concentrated under reduced pressure to obtain a crude product, which was purified by column chromatography (eluents: DCM : MeOH = 100 : 1 to 9 : 1) and then further purified by a reverse-phase column (eluents: water : acetonitrile = 100:1 to 1:9) to obtain the target compound 63 (320 mg, 37.85%).

LCMS m/z = 487.3 $[M+H]^+$.

$^1$H NMR (400 MHz, DMSO-*d6*) δ 7.82-7.79 (m, 1H), 7.68-7.61 (m, 2H), 7.51-7.47 (m, 1H), 7.39 (s, 1H), 6.35 (s, 1H), 4.87-4.85 (m, 1H), 4.38 (s, 2H), 4.00-3.98 (m, 2H), 3.76-3.75 (m, 2H), 3.48-2.39 (m, 1H), 3.26-3.18 (m, 1H), 2.98-2.84 (m, 2H), 2.57 (s, 2H), 2.43-2.30 (m, 2H), 2.23-2.17 (m, 2H), 1.86-1.73 (m, 2H), 1.67 (s, 3H), 1.64 (s, 3H).

**Example 59**

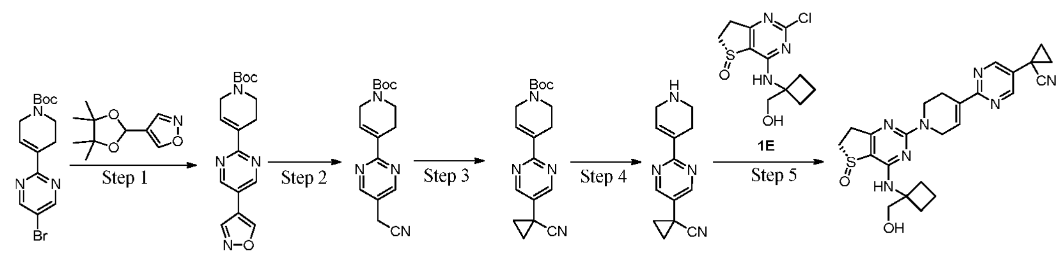


37B 64A 64B 64C 64D 64

Step 1: Compound **37B** (3.4 g, 10 mmol) was dissolved in N,N-dimethylformamide (100 mL), and 1,1-bis(diphenylphosphino)ferrocene palladium dichloride (725 mg, 1.0 mmol), potassium fluoride (1.7 g, 30 mmol), 4-isoxazoleboronic acid pinacol ester (2.4 g, 12 mmol), and water (30 mL) were then added. After nitrogen displacement three times, the mixture was heated to 90°C and reacted for 4 h, after which the reaction product was passed through a short silica gel column, rinsed with ethyl acetate, concentrated, and subjected to column chromatography (petroleum ether/ethyl acetate = 5/1) to obtain compound **64A** (1.3 g, 39%).

LC-MS (ESI): m/z = 329.1 $[M+H]^+$.

Step 2: Compound **64A** (1.3 g, 4.0 mmol) was dissolved in methanol (40 mL), and potassium fluoride (0.7 g, 12 mmol) and water (12 mL) were then added. The mixture was heated to 90°C and reacted for 4 h, after which the reaction product was passed through a short silica gel column, rinsed with ethyl acetate, concentrated, and subjected to column chromatography (petroleum ether/ethyl acetate = 4/1) to obtain compound **64B** (1.0 g, 84%).

LC-MS (ESI): m/z = 301.2 $[M+H]^+$.

Step 3: Compound **64B** (1.0 g, 3.3 mmol) was dissolved in water (5 mL), and tetrabutylammonium bromide (96 mg, 0.3 mmol), 1,2-dibromoethane (751 mg, 4 mmol), and potassium hydroxide (560 mg, 10 mmol) were then added. The mixture was heated to 50°C and reacted for 4 h, after which the mixture was passed through a short silica gel column, rinsed with ethyl acetate, concentrated, and subjected to column chromatography (petroleum ether/ethyl acetate = 9/1) to obtain compound **64C** (0.7 g, 65%).

LC-MS (ESI): m/z = 327.2 $[M+H]^+$.

Step 4: Compound **64C** (0.7 g, 2.1 mmol) was dissolved in dichloromethane (20 mL), trifluoroacetic acid (2.8 mL) was then added, and the mixture was reacted at room temperature for half an hour and then concentrated to obtain compound **64D** (0.4 g, 90%), which was directly used in the next step.

Step 5: Compound **64D** (0.4 mg, 1.7 mmol) was dissolved in 1,4-dioxane (30.0 mL), compound **1E** (0.5 g, 1.8 mmol) and N,N-diisopropylethylamine (1.8 g, 14.5 mmol) were then added, and the mixture was heated back to 90°C and reacted for 12 h, after which the reaction product was cooled to room temperature, concentrated, and subjected to column chromatography (dichloromethane/methanol = 10/1) to obtain compound **64** (0.4 g, 49%).

LC-MS (ESI): m/z = 478.2 $[M+H]^+$.

$^1H$ NMR (400 MHz, $CDCl_3$) δ 8.77 (s, 2H), 7.36 (s, 1H), 7.29 (s, 1H), 4.84 (t, 1H), 4.46 (s, 2H), 3.97 (t, 2H), 3.75 (d, 2H), 3.47 -3.39 (m, 1H), 3.26 -3.18 (m, 1H), 2.98 -2.92 (m, 1H), 2.89 -2.84 (m, 1H), 2.66 (s, 2H), 2.43 -2.30 (m, 2H), 2.23 -2.18 (m, 2H), 1.84 -1.75 (m, 4H), 1.71 -1.67 (m, 2H).

## Example 60

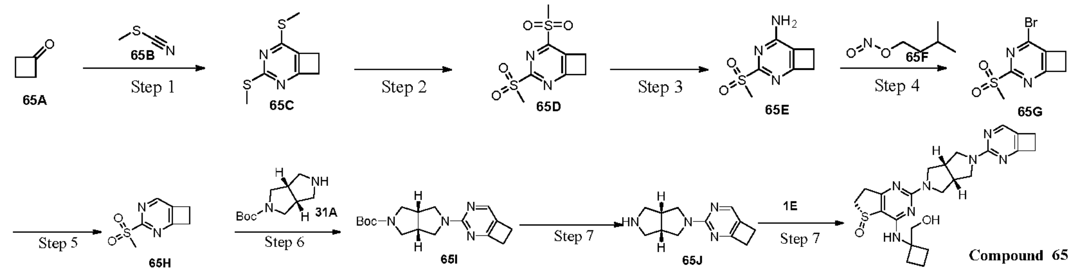


Step 1: Methyl thiocyanate (131.45 g, 1.80 mol) was added to a 3 L three-mouth flask and dissolved by adding 1,2-dichloroethane (1.2 L), and trifluoromethanesulfonic anhydride (265.7 g, 0.94 mol) was then added. Cyclobutanone (60.0 g, 0.86 mol) was slowly dropwise added at room temperature, and after the addition was complete, the mixture was stirred overnight at room temperature. The reaction liquid was washed by adding a saturated sodium bicarbonate solution and adjusted to pH 8-9. After leaving to stand for layering, the organic phase was dried over anhydrous sodium sulphate, concentrated, and purified by silica gel column chromatography (DCM : EA = 100 : 1-20 : 1) and recrystallized (EA : PE = 1 : 2) to obtain the target compound **65C** (15.0 g, yield: 9%).

LCMS m/z = 199.1 $[M +1]^+$.

Step 2: **65C** (15.0 g, 75.6 mmol) was added to a 500 mL single-mouth flask and dissolved with dichloromethane (300 mL), m-chloroperoxybenzoic acid (78.3 g, 453.8 mmol) was added, and the mixture was stirred at room temperature overnight. The reaction liquid was washed with 10% sodium hydroxide (100 mL), the organic phase was dried over anhydrous sodium sulphate, concentrated, and purified by column chromatography (PE : EA = 1:1-EA) to obtain the target compound **65D** (3.8 g, yield: 19%).

LCMS m/z = 263.1 $[M +1]^+$.

Step 3: **65D** (3.8 g, 14.5 mmol) was added to a 100 mL single-mouth flask and dissolved with dichloromethane (40 mL), and aqueous ammonia (20 mL) was then added and stirred at room temperature for 4 h. The reaction liquid was directly separated into phases, the aqueous phase was extracted once with dichloromethane (20 mL), and the organic phases were combined, dried over anhydrous sodium sulphate, concentrated and purified by column chromatography (DCM : EA = 2 : 1-EA) to obtain the target compound **65E** (2.2 g, yield: 76%).

LCMS m/z = 200.1 $[M +1]^+$.

Step 4: **65E** (2.2 g, 11.04 mmol) was added to a 100 mL single-mouth flask and dissolved with acetonitrile (40 ml), and isoamyl nitrite (3.9 g, 33.12 mmol) and copper bromide (4.9 g, 22.07 mmol) were then added and stirred for 6 h at 75 degrees Celsius under nitrogen protection. The reaction liquid was concentrated. Water (100 mL) was added, and the mixture was extracted with dichloromethane (2 × 100 mL). The organic phases were combined, dried over anhydrous sodium sulphate, concentrated, and purified by column chromatography (PE : EA = 3 : 1-1 : 1) to obtain the target compound **65G** (2.0 g, yield: 69%).

LCMS m/z = 263.0 $[M +1]^+$.

Step 5: **65G** (2.0 g, 7.6 mmol) was added to a 100 mL single-mouth flask and dissolved with ethyl acetate (40 mL), and palladium on carbon (10%, 6.0 g) was added and stirred at 60 degrees Celsius for 72 h. The reaction liquid was filtered by diatomite, and the filtrate was concentrated and purified by column chromatography (DCM : EA = 2 : 1-EA) to obtain the target compound **65H** (0.48 g, yield: 34%).

LCMS m/z = 185.1 $[M +1]^+$.

Step 6: **65H** (0.18 g, 0.98 mmol) was added to a 50 mL single-mouth flask and dissolved with dioxane (8 mL), and **31A** (0.42 g, 1.96 mmol) and N,N-diisopropylethylamine (0.38 g, 2.94 mmol) were added and stirred at 95 degrees Celsius overnight. The reaction liquid was concentrated and purified by column chromatography (DCM : EA = 5 : 1-1 : 1) to obtain the target compound **65I** (60 mg, yield: 19%).

LCMS m/z = 317.2 $[M +1]^+$.

Step 7: **65I** (60 mg, 0.19 mmol) was added to a 50 mL single-mouth flask and dissolved with dioxane (3 mL), and hydrogen chloride dioxane (4 M, 3 mL) was added and stirred at room temperature for 4 h. The reaction liquid was concentrated to obtain the target compound **65J** (48 mg, yield: 100%), which was directly used in the next reaction.

LCMS m/z = 217.2 $[M +1]^+$.

Step 8: **1E** (42 mg, 0.15 mmol) was added to a 50 mL single-mouth flask and dissolved with dioxane (6 mL), and **65J** (48 mg, 0.18 mmol) and N,N-diisopropylethylamine (59 mg, 0.45 mmol) were successively added and stirred at 95 degrees Celsius overnight. The reaction liquid was concentrated and purified by HPLC to obtain the target compound **65** (30 mg, yield: 43%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM ammonium acetate); b. gradient elution: mobile phase A: 20-70%; c. flow rate: 15 mL/min; d. elution time: 20 min; retention time: 9.5 min.

LCMS m/z = 468.1 $[M +1]^+$.

$^1H$ NMR (400 MHz, DMSO-d6) δ 7.90 (s, 1H), 7.23 (s, 1H), 4.81 (s, 1H), 3.83-3.66 (m, 6H), 3.43-3.35 (m, 6H), 3.23-3.18 (m, 2H), 3.07-3.00 (m, 4H), 2.94-2.76 (m, 2H), 2.42-2.26 (m, 2H), 2.17-2.12 (m, 2H), 1.84-1.68 (m, 2H).

## Example 61

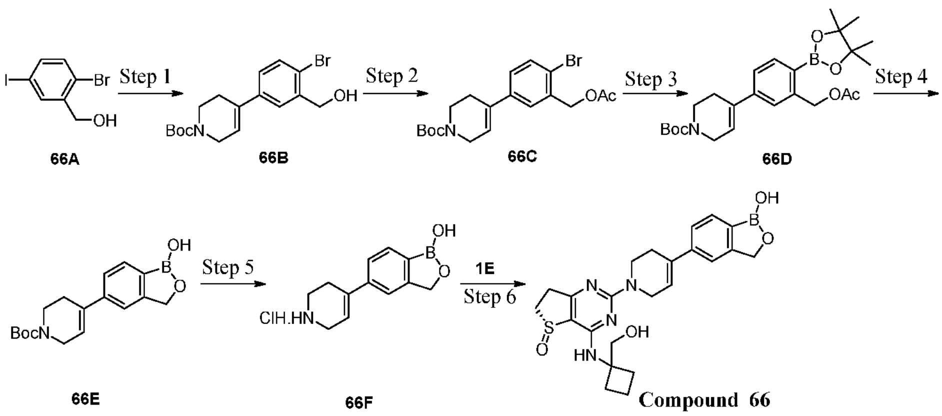


66A 66B 66C 66D 66E 66F Compound 66

Step 1: Under nitrogen protection, compound **66A** (10.00 g, 31.96 mmol) was added to a mixed solution of 1,4-dioxane (100 mL) and water (10 mL). [1-(tert-butoxycarbonyl)-1,2,3,6-tetrahydropyridin-4-yl]boronic acid (7.98 g, 35.15 mmol), [1,1'-bis(diphenylphosphino)ferrocene]palladium dichloride (2.30 g, 3.20 mmol), and potassium carbonate (11.04 g, 79.89 mmol) were added successively and separately with stirring at room temperature. After the addition was complete, the reaction liquid was warmed to 85°C and stirred for 16 h. After the disappearance of raw materials was detected by TLC, the reaction product was cooled to room temperature, concentrated under reduced pressure into a small

volume, then diluted by adding water (150 mL), and extracted with ethyl acetate (100 mL) three times. The organic phases were combined, dried over anhydrous sodium sulphate, and then concentrated in vacuum to obtain a crude product. The crude product was purified by column chromatography (eluents: DCM : MeOH = 100 : 1 to 20 : 1) to obtain the target compound **66B** (8.00 g, yield: 67.98%).

LC-MS (ESI): m/z = 312.20 & 314.20 $[M+H-56]^+$.

$^1$H NMR (400 MHz, $CDCl_3$) δ 7.51-7.46 (m, 2H), 7.15-7.12 (m, 1H), 6.04 (s, 1H), 4.73 (s, 2H), 4.05-4.03 (m, 2H), 3.62-3.60 (m, 2H), 2.49 (s, 2H), 1.48 (s, 3H).

Step 2: Under nitrogen protection, compound **66B** (2.00 g, 5.43 mmol) was dissolved in dichloromethane (40 mL), triethylamine (1.37 g, 13.58 mmol) was added with stirring in ice bath, and acetyl chloride (0.49 g, 6.25 mmol) was then slowly added dropwise. After the addition was complete, the reaction liquid was stirred at 0°C for 1 h. After the disappearance of raw materials was detected by TLC, the reaction was quenched by adding a saturated ammonium chloride aqueous solution (100 mL), and the reaction product was extracted with dichloromethane (50 mL) three times. The organic phases were combined, dried over anhydrous sodium sulphate, and then concentrated in vacuum to obtain a crude product. The crude product was purified by column chromatography (eluents: PE : EA = 100 : 1 to 4 : 1) to obtain the target compound **66C** (2.00 g, yield: 95.96%).

$^1$H NMR (400 MHz, $CDCl_3$) δ 7.54-7.52 (m, 1H), 7.40-7.39 (m, 1H), 7.20-7.17 (m, 1H), 6.05 (s, 1H), 5.19 (s, 2H), 4.07-4.06 (m, 2H), 3.65-3.62 (m, 2H), 2.50 (s, 2H), 2.14 (s, 3H), 1.49 (s, 9H).

Step 3: Under nitrogen protection, compound **66C** (2.00 g, 4.87 mmol) was dissolved in 1,4-dioxane (80 mL). Pinacol diboronate (1.49 g, 5.85 mmol), [1,1'-bis(diphenylphosphino)ferrocene]palladium dichloride (178 mg, 0.24 mmol), and potassium acetate (1.44 g, 14.62 mmol) were added successively and separately with stirring at room temperature. After the addition was complete, the reaction liquid was warmed to 80°C and stirred for 5 h. After the disappearance of raw materials was detected by TLC, the reaction product was cooled to room temperature, concentrated under reduced pressure into a small volume, then diluted by adding water (100 mL), and extracted with ethyl acetate (100 mL) three times. The organic phases were combined, dried by anhydrous sodium sulphate, and then concentrated in vacuum to obtain crude product **66D** (2.5 g), which was directly used in the next reaction.

Step 4: Crude compound **66D** (2.5 g, 5.47 mmol) was dissolved in methanol (60 mL), and a sodium hydroxide aqueous solution (655 mg, 16.40 mmol, 1 M) was then added. After the addition was complete, the mixture was stirred at room temperature for 16 h under nitrogen protection. The reaction liquid was concentrated under reduced pressure into a small volume, diluted by adding water (50 mL), and extracted twice with ethyl acetate (100 mL). The aqueous phase was further concentrated and then purified by reverse-phase column (eluents: water : acetonitrile = 100 : 1 to 1 : 9) to obtain compound **66E** (1.20 g, 69.66%).

LCMS m/z = 260.30 $[M-56+H]^+$.

$^1$H NMR (400 MHz, $D_2O$) δ 7.34-7.32 (m, 1H), 7.18-7.16 (m, 1H), 7.11 (s, 1H), 5.96 (s, 1H), 4.63 (s, 2H), 3.93 (s, 2H), 3.50 (s, 2H), 2.41 (s, 2H), 1.34 (s, 9H).

Step 5: Compound **66E** (380 mg, 1.21 mmol) was dissolved in dichloromethane (6 mL), a hydrogen chloride dioxane solution (176 mg, 4.82 mmol, 4 M) was then added in ice bath, and after the addition was complete, the mixture was stirred for 1.5 h under nitrogen protection. The reaction liquid was concentrated under reduced pressure to obtain crude compound **66F** (310 mg), which was directly used in the next reaction.

LCMS m/z = 216.30 $[M+H]^+$.

Step 6: Compound **1E** (300 mg, 1.04 mmol) and compound **66F** (310 mg, 1.23 mmol) were dissolved in 1,4-dioxane (8 mL), and DIPEA (405 mg, 3.13 mmol) was then added. After the addition was complete, the mixture was warmed to 85°C under nitrogen protection and stirred for 16 h. After the reaction was complete, the reaction liquid was concentrated under reduced pressure to obtain a crude product, which was purified by column chromatography (eluents: DCM : MeOH = 100 : 1 to 9 : 1) and then further purified by a reverse-phase column (eluents: water : acetonitrile = 100:1 to 1:9) to obtain the target compound **66** (150 mg, 30.85%).

LCMS m/z = 467.20 $[M+H]^+$.

$^1$H NMR (400 MHz, DMSO-d6) δ 9.13 (s, 1H), 7.70-7.68 (m, 1H), 7.46-7.44 (m, 2H), 7.38 (s, 1H), 6.34 (s, 1H), 4.98 (s, 2H), 4.87-4.84 (m, 1H), 4.37 (s, 2H), 4.00-3.97 (m, 2H), 3.76-3.75 (m, 2H), 3.47-3.39 (m, 1H), 3.26-3.18 (m, 1H), 2.98-2.84 (m, 2H), 2.56 (s, 2H), 2.43-2.30 (m, 2H), 2.22-2.18 (m, 2H), 1.82-1.76 (m, 2H).

**Example 62**

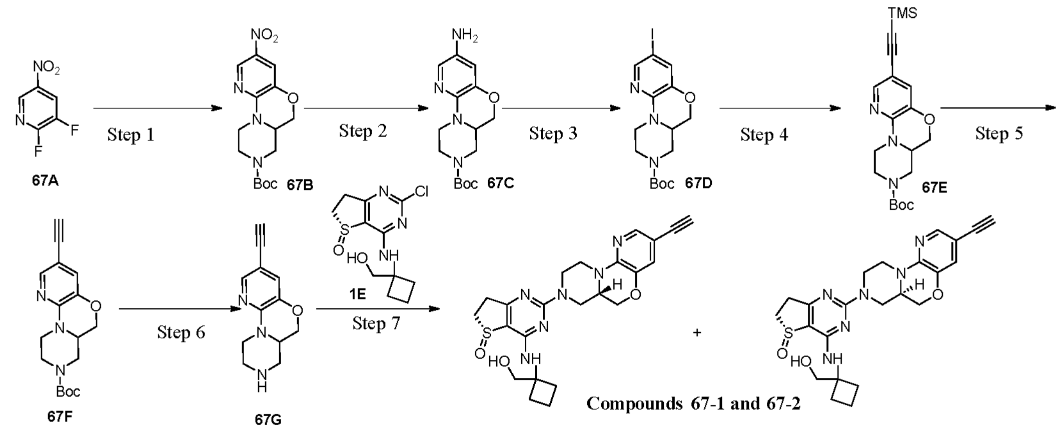

67A 67B 67C 67D 67E 67F 67G
Compounds 67-1 and 67-2

Step 1: **67A** (5.0 g, 31.3 mmol), tert-butyl 3-(hydroxymethyl)piperazine-1-carboxylate (6.8 g, 31.3 mmol), potassium hydroxide (5.3 g, 93.9 mmol), and N,N-dimethylformamide (100 mL) were successively added to a 250 mL single-mouth flask. After reaction at room temperature for 6 hours, The reaction product was further warmed to 80°C and reacted for 24 hours. After filtration, ethyl acetate (500 mL) was added to the filtrate, which was washed with water (100 mL × 3) and with a saturated sodium chloride aqueous solution (100 mL × 1), the organic phase was dried over anhydrous sodium sulphate and filtered, and the filtrate was concentrated under reduced pressure to obtain **67B** (10.0 g, yield 95.2%).

LC-MS (ESI): m/z = 281.1 $[M-56+H]^+$.

Step 2: **67B** (10.0 g, 29.8 mmol), ethyl acetate (300 mL), and palladium on carbon (2 g) were successively added to a 1000 mL single-mouth flask, and the mixture was subjected to a hydrogenation reaction at room temperature for 12 hours. After filtration, the filtrate was concentrated under reduced pressure to obtain **67C** (8.0 g, yield 87.9%).

LC-MS (ESI): m/z = 307.1 $[M+H]^+$.

Step 3: Acetonitrile (30 mL), tert-butyl nitrite (3.5 g, 33.83 mmol), and cuprous iodide (5.1 g, 27.06 mmol) were successively added to a 100 mL single-mouth flask and heated to 65°C, and a solution of **67C** (6.9 g, 22.55 mmol) in acetonitrile (40 mL) was dropwise added. After the dropwise addition was complete, the mixture was reacted at 65°C for 4 hours. After heating was turned off, the reaction was continued for 12 hours. The reaction product was concentrated under reduced pressure and then separated and purified by silica gel column chromatography (eluents: ethyl acetate/petroleum ether (v/v) = 20/100) to obtain **67D** (1.5 g, yield 15.6%).

LC-MS (ESI): m/z = 418.4 $[M+H]^+$.

Step 4: **67D** (1.4 g, 3.36 mmol), tetrahydrofuran (30 mL), cuprous iodide (127 mg, 0.67 mmol), triethylamine (1.0 g, 10.08 mmol), bis(triphenylphosphino)palladium dichloride (238 mg, 0.34 mmol), and trimethylsilylacetylene (1.01 g, 5.05 mmol) were successively added to a 100 mL single-mouth flask and reacted for 6 hours at room temperature under nitrogen protection, and the reaction product was concentrated under reduced pressure and then separated and purified by silica gel column chromatography (eluents: ethyl acetate/petroleum ether (v/v) = 20/100) to obtain **67E** (1.2 g, yield 92.3%).

LC-MS (ESI): m/z = 388.2 $[M+H]^+$.

Step 5: **67E** (1.2 g, 3.1 mmol), methanol (30 mL), and potassium carbonate (1.3 g, 9.3 mmol) were successively added to a 50 mL single-mouth flask and reacted at room temperature for 2 hours. The reaction product was filtered, and the filtrate was concentrated under reduced pressure and then separated and purified by silica gel column chromatography (eluents: ethyl acetate/petroleum ether (v/v) = 20/100) to obtain **67F** (0.8 g, yield 81.9%).

LC-MS (ESI): m/z = 316.1 $[M+H]^+$.

Step 6: **67F** (0.3 g, 0.95 mmol), triethylamine (1.9 g, 19 mmol), and iodotrimethylsilane (1.9 g, 9.5 mmol) were successively added to a 50 mL single-mouth flask and reacted at room temperature for 2 hours. The reaction product was concentrated under reduced pressure to obtain crude **67G,** which was directly used in the next step.

LC-MS (ESI): m/z = 216.1 $[M+H]^+$.

Step 7: **1E** (273 mg, 0.95 mmol), **67G** (crude, 0.8 g, 0.95 mmol), dioxane (5 mL), and DIPEA (774 mg, 6.0 mmol) were successively added to a 50 mL single-mouth flask and reacted at 100°C for 12 hours. The reaction product was cooled to room temperature, and the reaction liquid was directly concentrated under reduced pressure and then separated and purified by silica gel column chromatography (eluent dichloromethane/methanol (v/v) = 100/8) to obtain 400 mg of a mixture.

After chiral preparation, the title compound **67-1** (80 mg, 18.06%, retention time: 1.855 min) and the title compound **67-2** (50 mg, 11.31%, retention time: 2.423 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 150 mL/min; back pressure: 100 bar; column temperature:

38°C; wavelength: 220 nm; elution time: 3.9 min.

**(Compound 67-1)**

$^1$H NMR (400 MHz, DMSO-$d_6$) δ7.90-7.83 (m, 1H), 7.46 (s, 1H), 7.09-7.04 (m, 1H), 4.87-4.79 (m, 1H), 4.81-4.60 (m, 2H), 4.52-4.35 (m, 2H), 4.08 (s, 1H), 4.04-3.95 (m, 1H), 3.78-3.65 (m, 2H), 3.51-3.37 (m, 2H), 3.28-3.15 (m, 1H), 3.07-2.74 (m, 5H), 2.43-2.16 (m, 4H), 1.86-1.70 (m, 2H).
LC-MS (ESI): m/z = 467.1 [M+H]$^+$.

**(Compound 67-2)**

$^1$H NMR (400 MHz, DMSO-$d_6$) δ7.96-7.71 (m, 1H), 7.47 (s, 1H), 7.25-6.78 (m, 1H), 4.88-4.64 (m, 3H), 4.53-4.36 (m, 2H), 4.08 (s, 1H), 4.04-3.90 (m, 1H), 3.72 (s, 2H), 3.48-3.39 (m, 2H), 3.27-3.21 (m, 1H), 3.06-2.75 (m, 5H), 2.40-2.17 (m, 4H), 1.87-1.72 (m, 2H).
LC-MS (ESI): m/z = 467.1 [M+H]$^+$.

**Example 63**

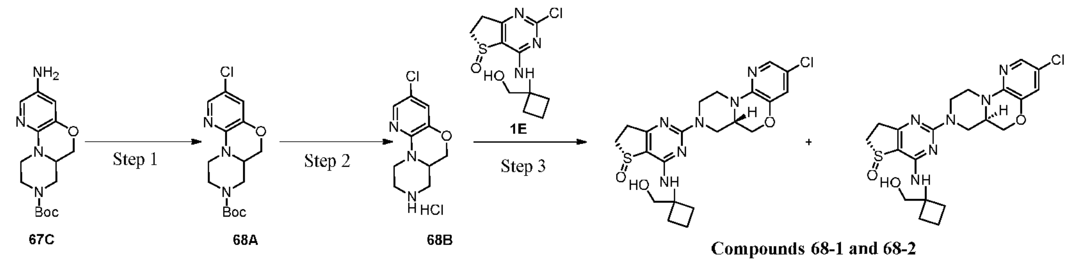


67C 68A 68B Compounds 68-1 and 68-2

Step 1: Acetonitrile (30 mL), tert-butyl nitrite (1.5 g, 14.7 mmol), and cuprous chloride (1.2 g, 11.8 mmol) were successively added to a 100 mL single-mouth flask and heated to 65°C, and a solution of **67C** (3.0 g, 9.8 mmol) in acetonitrile (10 mL) was dropwise added. After the dropwise addition was complete, the mixture was reacted at 65°C for 4 hours. After heating was turned off, the reaction was continued for 12 hours. The reaction product was concentrated under reduced pressure and then separated and purified by silica gel column chromatography (eluents: ethyl acetate/petroleum ether (v/v) = 20/100) to obtain **68A** (0.5 g, yield 13.7%).
LC-MS (ESI): m/z = 270.1 [M-56+H]$^+$.
Step 2: **68A** (0.5 g, 1.5 mmol) and a hydrogen chloride-dioxane solution (4N, 10 mL) were successively added to a 50 mL single-mouth flask and reacted at room temperature for 1 hour, and the reaction product was then concentrated under reduced pressure to obtain **68B** (0.5 g, yield 100.0%).
LC-MS (ESI): m/z = 226.1 [M+H]$^+$.
Step 3: **1E** (430 mg, 1.5 mmol), **68B** (500 mg, 1.5 mmol), dioxane (5 mL), and DIPEA (774 mg, 6.0 mmol) were successively added to a 50 mL single-mouth flask and reacted at 100°C for 12 hours. The reaction product was cooled to room temperature, and the reaction liquid was directly concentrated under reduced pressure and then separated and purified by silica gel column chromatography (eluent dichloromethane/methanol (v/v) = 100/8) to obtain 500 mg of a mixture.
After chiral preparation, the title compound **68-1** (50 mg, 7.0%, retention time: 1.704 min) and the title compound **68-2** (110 mg, 15.4%, retention time: 2.183 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 120 mL/min; back pressure: 100 bar; column temperature: 25°C; wavelength: 220 nm; elution time: 4 min.

**(Compound 68-1)**

$^1$H NMR (400 MHz, DMSO-$d_6$) δ 7.77-7.73 (m, 1H), 7.48 (s, 1H), 7.21-7.15 (m, 1H), 4.88-4.81 (m, 1H), 4.80-4.59 (m, 2H), 4.48-4.33 (m, 2H), 4.09-3.95 (m, 1H), 3.79-3.68 (m, 2H), 3.47-3.34 (m, 2H), 3.28-3.17 (m, 1H), 3.07-2.84 (m, 3H), 2.80-2.67 (m, 2H), 2.42-2.15 (m, 4H), 1.87-1.67 (m, 2H).
LC-MS (ESI): m/z = 477.1 [M+H]$^+$.

**(Compound 68-2)**

$^1$H NMR (400 MHz, DMSO-$d_6$) δ7.77-7.73 (m, 1H), 7.48 (s, 1H), 7.19-7.15 (m, 1H), 4.90-4.82 (m, 1H), 4.82-4.63 (m, 2H), 4.47-4.31 (m, 2H), 4.10-3.94 (m, 1H), 3.76-3.67 (m, 2H), 3.48-3.33 (m, 2H), 3.27-3.17 (m, 1H), 3.06-2.84 (m, 3H), 2.80-2.69 (m, 2H), 2.38-2.15 (m, 4H), 1.87-1.68 (m, 2H).

LC-MS (ESI): m/z = 477.1 [M+H]$^+$.

**Example 64**

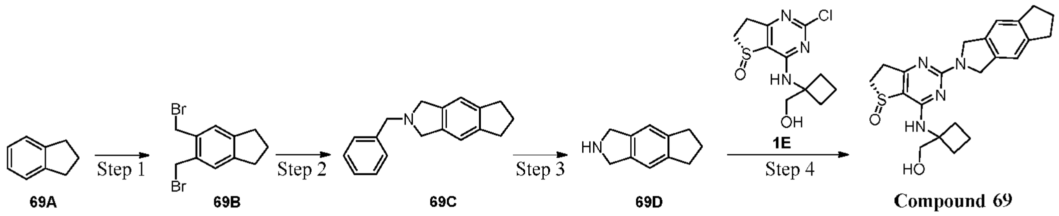


69A 69B 69C 69D Compound 69

Step 1: Compound **69A** (10 g, 84.6 mmol) and paraformaldehyde (25.4 g, 0.85 mol) were dissolved in acetic acid (30 mL), and a 40% hydrobromic acid solution (49.2 mL, 0.85 mol) was then slowly added at 0°C and stirred for 30 min. The mixture was heated to 80°C, reacted for 12 h, then concentrated, and subjected to column chromatography (pure petroleum ether) to obtain compound **69B** (2 g, 8%).

Step 2: Compound **69B** (1.0 g, 3.3 mmol) was dissolved in ethanol (30 mL), and benzylamine (0.35 g, 3.3 mmol) was added. The mixture was heated to reflux for 4 h, then passed through a short silica gel column, rinsed with ethyl acetate, concentrated, and subjected to column chromatography (petroleum ether/ethyl acetate = 4/1) to obtain compound **69C** (0.6 g, 73%).

LC-MS (ESI): m/z = 250.4 [M+H]$^+$.

Step 3: Compound **69C** (0.5 g, 2.0 mmol) was dissolved in methanol (30 mL), and palladium on carbon (50 mg) was then added. After displacement with a hydrogen balloon three times, the mixture was reacted at room temperature for 12 h, filtered and concentrated, and the residue was subjected to column chromatography (methanol/triethylamine = 100/1) to obtain compound **69D** (0.2 g, 62%).

LC-MS (ESI): m/z = 160.2 [M+H]$^+$.

Step 4: Compound **69D** (0.2 mg, 1.3 mmol) was dissolved in 1,4-dioxane (30.0 mL), compound **1E** (0.36 g, 1.3 mmol) and N,N-diisopropylethylamine (0.32 g, 2.6 mmol) were then added, and the mixture was heated back to 90°C and reacted for 4 h, after which the reaction product was cooled to room temperature, concentrated, and subjected to column chromatography (dichloromethane/methanol = 10/1) to obtain compound **69** (0.1 g, 19%).

LC-MS (ESI): m/z = 411.2 [M+H]$^+$.

$^1$H NMR (400 MHz, $CDCl_3$) δ 7.38 -7.32 (m, 1H), 7.23 -7.12 (m, 2H), 4.91 - 4.85 (m, 1H), 4.78 -4.71 (m, 3H), 3.79 (d, 2H), 3.49 -3.41 (m, 1H), 3.27 -3.15 (m, 1H), 3.00 -2.94 (m, 1H), 2.94 -2.83 (m, 5H), 2.44 -2.33 (m, 2H), 2.25 -2.20 (m, 2H), 2.08 -1.99 (m, 2H), 1.87 -1.74 (m, 2H).

**Example 65**

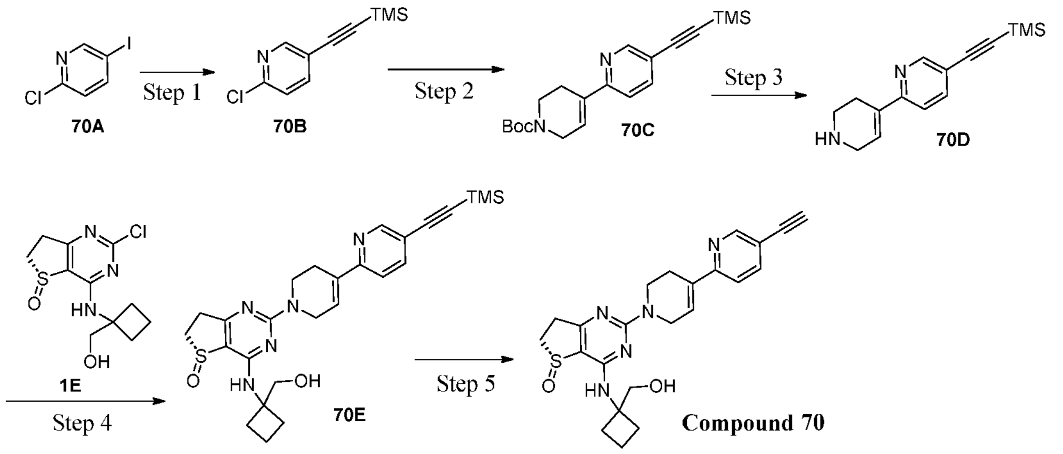


Step 1: Compound 70A (1.2 g, 5.0 mmol) was dissolved in tetrahydrofuran (50 mL), and bis(triphenylphosphino) palladium dichloride (350.0 mg, 0.5 mmol), triethylamine (1.1 g, 10.0 mmol), trimethylsilylacetylene (650.0 mg, 6.5 mmol), and cuprous iodide (40.0 mg, 0.25 mmol) were then added. After nitrogen displacement three times, the mixture was heated to 50°C and reacted for 14 h, after which the reaction product was passed through a short silica gel column, rinsed with ethyl acetate, concentrated, and subjected to column chromatography (petroleum ether/ethyl acetate = 5/1) to obtain compound **70B** (800 mg, 76%).

Step 2: Compound **70B** (420.0 mg, 2.0 mmol) was dissolved in 1,4-dioxane (30 mL), 1,1-bis(diphenylphosphino) ferrocene palladium dichloride (144.0 mg, 0.2 mmol), potassium carbonate (560.0 mg, 4.0 mmol), N-Boc-1,2,5,6-tetrahydropyridine-4-boronic acid pinacol ester (720.0 mg, 2.4 mmol), and water (6.0 mL) were then added. After nitrogen displacement three times, the mixture was heated to 45°C and reacted for 14 h, after which the reaction product was passed through a short silica gel column, rinsed with ethyl acetate, concentrated, and subjected to column chromatography (dichloromethane/methanol = 20/1) to obtain compound **70C** (400 mg, 56%).

LC-MS (ESI): m/z = 357.2 $[M+H]^+$.

Step 3: Compound **70C** (356.0 mg, 1.0 mmol) was dissolved in dichloromethane (10 mL), trifluoroacetic acid (1.2 mL) was then added, and the mixture was reacted at room temperature for half an hour and then concentrated to obtain compound **70D** (150.0 mg, 78%), which was directly used in the next step.

LC-MS (ESI): m/z = 257.1 $[M+H]^+$.

Step 4: Compound **70D** (128.1 mg, 0.5 mmol) was dissolved in 1,4-dioxane (10.0 mL), compound **1E** (172.0 mg, 0.6 mmol) was then added, and the mixture was heated back to 90°C and reacted for 4 h, after which the reaction product was cooled to room temperature, concentrated, and subjected to column chromatography (dichloromethane/methanol = 10/1) to obtain compound **70E** (200 mg, 79%).

LC-MS (ESI): m/z = 508.2 $[M+H]^+$.

Step 5: Compound **70E** (200.0 mg, 0.4 mmol) was dissolved in methanol (10.0 mL), potassium carbonate (150.0 mg, 1.1 mmol) was then added, and the mixture was then reacted at room temperature for 2 h, concentrated, and then subjected to column chromatography (dichloromethane/methanol = 15/1) to obtain compound **70** (110.0 mg, 62%).

LC-MS (ESI): m/z = 436.2 $[M+H]^+$.

$^1H$ NMR (400 MHz, $CDCl_3$) δ 8.66 (d, 1H), 7.74 -7.72 (m, 1H), 7.35 (d, 1H), 6.75 (t, 1H), 6.57 (s, 1H), 4.47 (s, 2H), 4.06 (s, 2H), 3.87 -3.79 (m, 2H), 3.65 -3.57 (m, 1H), 3.40 -3.32 (m, 1H), 3.23 (s,1H), 3.08 -2.98 (m, 2H), 2.71 (s,2H), 2.43 - 2.35 (m, 2H), 2.26 -2.18 (m, 2H), 1.96 -1.78 (m, 2H).

## Example 66

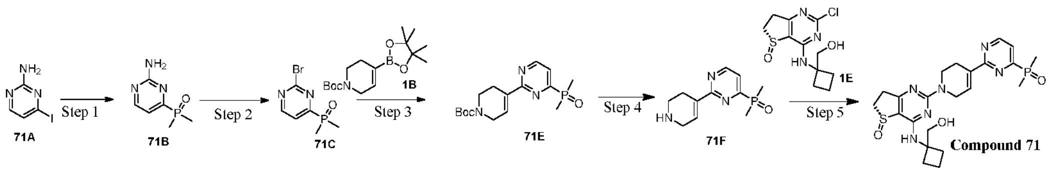


Step 1: Compound **71A** (5.00 g, 22.60 mmol) and dimethylphosphine oxide (2.65 g, 33.90 mmol) were weighed into a 250 mL single-mouth flask and dissolved with 1,4-dioxane (50 mL), potassium phosphate (14.39 g, 67.80 mmol),

tris(dibenzylideneacetone)dipalladium (1.03 g, 1.13 mmol), and 5-bis(diphenylphosphino)-9,9-dimethylxanthene (1.31 g, 2.26 mmol) were added, and after the addition was complete, the mixture was stirred at 90°C for 16 h. After the completion of the reaction was detected by TLC plate spotting (petroleum ether : ethyl acetate = 5:1), water (50 mL) was added and stirred for 5 min, and the mixture was extracted with ethyl acetate (50 mL). The organic phase was separated, dried over anhydrous sodium sulphate, and concentrated under reduced pressure, and the crude product was purified by column chromatography (eluents: petroleum ether : ethyl acetate = 1:1) to obtain the target compound **71B** (2.2 g, yield: 57%).

LCMS m/z = 172.1 $[M +1]^+$.

Step 2: Compound **71B** (2.2 g, 12.85 mmol) was weighed into a 100 mL single-mouth flask and dissolved with acetonitrile (30 mL). Isoamyl nitrite (3.01 g, 25.70 mmol) and cuprous bromide (4.37 g, 19.22 mmol) were added, and after the addition was complete, the mixture was stirred at 70°C for 12 h. After the completion of the reaction was detected by TLC plate spotting (petroleum ether : ethyl acetate = 5:1), water (50 mL) was added and stirred for 5 min, and ethyl acetate (50 mL) was added for extraction. An organic phase was separated, which was dried over anhydrous sodium sulphate and concentrated under reduced pressure, and the crude product was purified by column chromatography (eluents: petroleum ether : ethyl acetate = 3:1) to obtain the target compound **71C** (0.43 g, yield 14%).

LCMS m/z = 235.0 $[M +1]^+$.

Step 3: Compound **71C** (0.20 g, 0.85 mmol) and compound **1B** (0.32 g, 1.02 mmol) were weighed into a 100 mL single-mouth flask and dissolved with 1,4-dioxane (8 mL), and a 2N sodium carbonate solution (2 mL) and tetrakis(triphenylphosphino)palladium (0.1 g, 0.09 mmol) were added to the reaction liquid. After the addition was complete, the mixture was stirred at 90°C for 16 h. After the completion of the reaction was detected by TLC plate spotting (petroleum ether : ethyl acetate = 5:1), water (20 mL) was added and stirred for 5 min, ethyl acetate (20 mL) was added for extraction, an organic phase was separated, which was dried over anhydrous sodium sulphate and concentrated under reduced pressure, and the crude product was purified by column chromatography (eluents: petroleum ether : ethyl acetate = 5:1) to obtain the target compound **71E** (1.0 g, yield 58%).

LCMS m/z = 338.1 $[M +1]^+$.

Step 4: Compound **71E** (0.20 g, 0.59 mmol) was weighed into a 100 mL single-mouth flask and dissolved with dichloromethane (6 mL), trifluoroacetic acid (2 mL) was added, and after the addition was complete, the mixture was stirred for 2 h. The organic phase was concentrated under reduced pressure until no liquid dripped out to obtain the target compound **71F** (0.20 g, crude).

LCMS m/z = 238.1 $[M +1]^+$.

Step 5: Compound **1E** (150 mg, 0.52 mmol) and compound **71F** (150 mg, 0.62 mmol) were added to a 100 mL single-mouth flask and dissolved with 1,4-dioxane (10 mL), and N,N-diisopropylethylamine (0.16 g, 1.56 mmol) was added. After the addition was complete, the system was placed under nitrogen protection and stirred at 90°C for 16 h, and the reaction liquid was concentrated under reduced pressure to obtain a crude product, which was separated by preparative HPLC to obtain the title compound **71** (110 mg, 43%).

Preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution: mobile phase A: 40-70%; c. flow rate: 15 mL/min; d. elution time: 20 min; retention time: 11.20 min.

LCMS m/z = 489.2 $[M +1]^+$.

$^1H$ NMR (400 MHZ, DMSO-$d_6$) δ 8.99 (t, 1H), 7.79 (t, 1H), 7.38 (s, 2H), 4.84 (t, 1H), 4.48 (s, 2H), 3.99 (t, 2H), 3.75 (d, 2H), 3.48-3.39 (m, 1H), 3.26-3.18 (m, 1H), 2.98-2.84 (m, 2H), 2.70 (s, 2H), 2.42-2.30 (m, 2H), 2.24-2.18 (m, 2H), 1.86-1.76 (m, 2H), 1.73 (s, 3H), 1.70 (s, 3H).

## Example 67

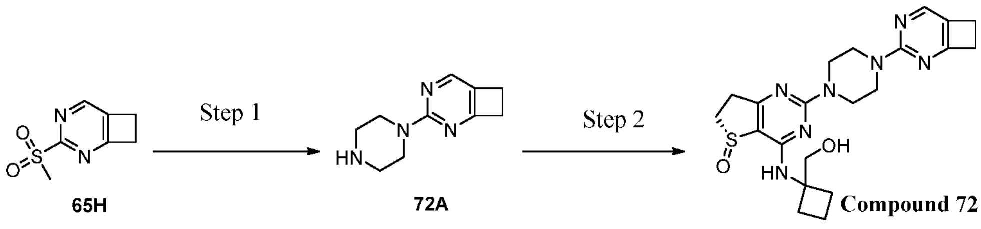

65H 72A Compound 72

Step 1: **65H** (90 mg, 0.49 mmol) was added to a 50 mL single-mouth flask and dissolved with dioxane (6 mL), and piperazine (84 mg, 0.98 mmol) was added and stirred at 90 degrees Celsius for 3 h. Water (5 mL) was added to the reaction

liquid, and ethyl acetate (3 × 10 mL) was added for extraction. The organic phases were combined, dried over anhydrous sodium sulphate, and concentrated to obtain the target compound **72A** (60 mg, crude), which was directly used in the next reaction.

LCMS m/z = 191.2 [M +1]$^+$.

Step 2: **1E** (60 mg, 0.21 mmol) was added to a 50 mL single-mouth flask and dissolved with dioxane (6 mL), and **72A** (60 mg, 0.32 mmol) and N,N-diisopropylethylamine (68 mg, 0.53 mmol) were successively added and stirred at 95 degrees Celsius overnight. The reaction liquid was concentrated and purified by SFC to obtain the target compound **72** (9 mg, yield: 10%).

Preparation method: instrument: SFC Prep 150; chromatographic column: SunFire silica (19 mm × 250 mm; the sample was dissolved with MeOH and filtered with a 0.45 μm filter to prepare a sample liquid; preparative chromatography conditions: a. composition of mobile phases A and B: mobile phase A: CO2, mobile phase B: methanol/acetonitrile (7:3); b. isocratic elution, mobile phase B: 35%; c. flow rate: 40 mL/min; d. elution time: 15 min.

LCMS m/z = 442.31 [M +1]$^+$.

$^1$H NMR (400 MHz, DMSO-*d6*) δ 7.95 (s, 1H), 7.36 (s, 1H), 4.81 (s, 1H), 3.84-3.69 (m, 10H), 3.26-3.20 (m, 4H), 3.04-3.02 (m, 2H), 2.98-2.83 (m, 2H), 2.40-2.26 (m, 2H), 2.22-2.16 (m, 2H), 1.83-1.71 (m, 2H).

**Example 68**

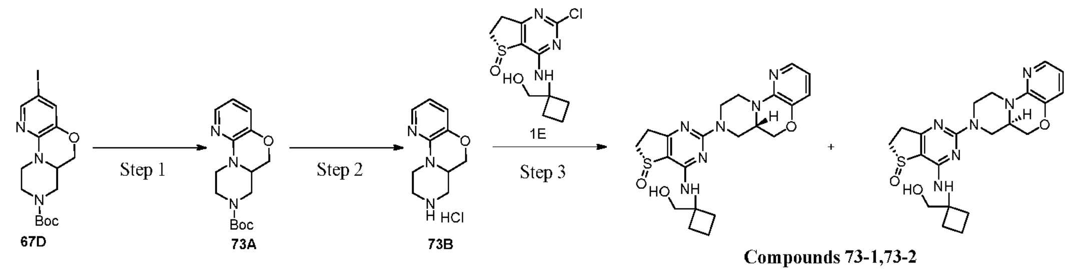


67D 73A 73B Compounds 73-1,73-2

Step 1: Methanol (10 mL), **67D** (0.5 g, 1.20 mmol), and palladium on carbon (100 mg) were successively added to a 50 mL single-mouth flask, and the mixture was subjected to a hydrogenation reaction at room temperature for 16 hours, followed by filtration. The filtrate was concentrated under reduced pressure to obtain **73A** (0.31 g, yield 88.9%).

LC-MS (ESI): m/z = 292.1 [M+H]$^+$.

Step 2: **73A** (0.31 g, 1.07 mmol) and a hydrogen chloride-dioxane solution (4N, 15 mL) were successively added to a 50 mL single-mouth flask and reacted at room temperature for 1 hour, and the reaction product was then concentrated under reduced pressure to obtain **73B** (0.25 g, yield 100.0%).

LC-MS (ESI): m/z = 192.1 [M+H]$^+$.

Step 3: **1E** (287 mg, 1.0 mmol), **73B** (250 mg, 1.07 mmol), dioxane (10 mL), and DIPEA (774 mg, 6.0 mmol) were successively added to a 50 mL single-mouth flask and reacted at 100°C for 12 hours. The reaction product was cooled to room temperature, and the reaction liquid was directly concentrated under reduced pressure and then separated and purified by silica gel column chromatography (eluent dichloromethane/methanol (v/v) = 100/8) to obtain 350 mg of a mixture.

After chiral preparation, the title compound **73-1** (91 mg, 20.6%, retention time: 1.472 min) and the title compound **73-2** (63 mg, 14.3%, retention time: 1.770 min) were obtained. Chiral preparation method: instrument: Waters 150 MGM; chromatographic column: Chiralpak Column; mobile phase: A: carbon dioxide, and B: methanol (0.1% aqueous ammonia); isocratic elution: 30% mobile phase B; flow rate: 100 mL/min; back pressure: 100 bar; column temperature: 25°C; wavelength: 220 nm; elution time: 4.2 min.

**(Compound 73-1)**

$^1$H NMR (400 MHz, DMSO-$d_6$) δ 7.79-7.67 (m, 1H), 7.44 (s, 1H), 7.06-6.94 (m, 1H), 6.68-6.55 (m, 1H), 4.89-4.80 (m, 1H), 4.79-4.62 (m, 2H), 4.50-4.34 (m, 2H), 4.06-3.92 (m, 1H), 3.79-3.65 (m, 2H), 3.50-3.37 (m, 1H), 3.36-3.32 (m, 1H), 3.28-3.16 (m, 1H), 3.06-2.81 (m, 3H), 2.79-2.63 (m, 2H), 2.41-2.25 (m, 2H), 2.25-2.14 (m, 2H), 1.89-1.71 (m, 2H).

LC-MS (ESI): m/z = 443.2 [M+H]$^+$.

**(Compound 73-2)**

$^1$H NMR (400 MHz, DMSO-$d_6$) δ 7.84-7.64 (m, 1H), 7.44 (s, 1H), 7.15-6.83 (m, 1H), 6.80-6.46 (m, 1H), 4.87-4.81 (m, 1H), 4.80-4.62 (m, 2H), 4.50-4.31 (m, 2H), 4.04-3.94 (m, 1H), 3.76-3.68 (m, 2H), 3.50-3.38 (m, 1H), 3.36-3.31 (m, 1H), 3.28-3.16 (m, 1H), 3.05-2.82 (m, 3H), 2.81-2.64 (m, 2H), 2.43-2.25 (m, 2H), 2.25-2.14 (m, 2H), 1.85-1.71 (m, 2H).
LC-MS (ESI): m/z = 443.2 [M+H]$^+$.

**Example 69**

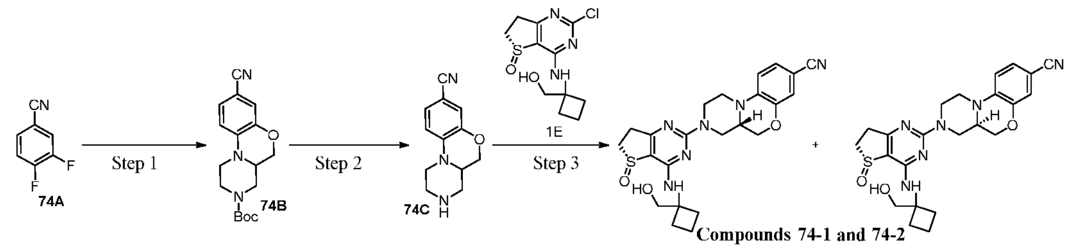


Step 1: N,N-dimethylformamide (50 mL), **74A** (5.0 g, 35.97 mmol), tert-butyl 3-(hydroxymethyl)piperazine-1-carboxylate (7.77 g, 35.97 mmol), and potassium hydroxide (6.05 g, 107.85 mmol) were successively added to a 250 mL single-mouth flask and reacted at 100°C for 16 hours, followed by filtration, after which ethyl acetate (100 mL) was added to the filtrate, which was washed with water (50 mL × 3) and with a saturated sodium chloride aqueous solution (100 mL × 1). The organic phase was dried over anhydrous sodium sulphate and filtered, and the filtrate was concentrated under reduced pressure and then separated and purified by silica gel column chromatography (eluents: ethyl acetate/petroleum ether (v/v) = 20/100) to obtain **74B** (0.5 g, yield 4.4%).
LC-MS (ESI): m/z = 316.2 [M+H]$^+$.
Step 2: **74B** (0.5 g, 1.59 mmol), dichloromethane (15 mL), and triethylamine (3.21 g, 31.8 mmol) were successively added to a 50 mL single-mouth flask, and iodotrimethylsilane (3.18 g, 15.9 mmol) was dropwise added at 0°C. The mixture was reacted at room temperature for 1 hour, and the reaction product was concentrated under reduced pressure to obtain crude product **74C** (4.5 g, yield 100.0%).
LC-MS (ESI): m/z = 216.2 [M+H]$^+$.
Step 3: **1E** (456 mg, 1.59 mmol), crude product 74C (4.5 g, 1.59 mmol), dioxane (10 mL), and DIPEA (1.03 g, 8.0 mmol) were successively added to a 50 mL single-mouth flask and reacted at 100°C for 12 hours. The reaction product was cooled to room temperature, and the reaction liquid was directly concentrated under reduced pressure and then separated and purified by silica gel column chromatography (eluent dichloromethane/methanol (v/v) = 100/10) to obtain 450 mg of a mixture.
After chiral preparation, the title compound **74-1** (112 mg, 15.1%, retention time: 0.925 min) and the title compound **74-2** (86 mg, 11.6%, retention time: 1.896 min) were obtained. Chiral preparation method: instrument: Waters 150 MGM; chromatographic column: Chiralpak Column; mobile phase: A: carbon dioxide, and B: isopropanol (0.1% aqueous ammonia); isocratic elution: 70% mobile phase B; flow rate: 100 mL/min; back pressure: 100 bar; column temperature: 25°C; wavelength: 220 nm; elution time: 5.1 min.

**(Compound 74-1)**

$^1$H NMR (400 MHz, DMSO-$d_6$) δ 7.46 (s, 1H), 7.35-7.31 (m, 1H), 7.14-7.09 (m, 1H), 6.89-6.84 (m, 1H), 4.88-4.79 (m, 1H), 4.78-4.60 (m, 2H), 4.51-4.39 (m, 1H), 4.10-4.00 (m, 1H), 3.97-3.88 (m, 1H), 3.77-3.66 (m, 2H), 3.52-3.36 (m, 1H), 3.29-3.16 (m, 1H), 3.16-3.02 (m, 2H), 3.00-2.81 (m, 2H), 2.79-2.62 (m, 2H), 2.44-2.25 (m, 2H), 2.25-2.14 (m, 2H), 1.88-1.69 (m, 2H).
LC-MS (ESI): m/z = 467.1 [M+H]$^+$.

**(Compound 74-2)**

$^1$H NMR (400 MHz, DMSO-$d_6$) δ 7.46 (s, 1H), 7.40-7.30 (m, 1H), 7.14-7.10 (m, 1H), 6.88-6.84 (m, 1H), 4.88-4.79 (m, 1H), 4.79-4.60 (m, 2H), 4.51-4.38 (m, 1H), 4.12-3.98 (m, 1H), 3.95-3.87 (m, 1H), 3.79-3.65 (m, 2H), 3.52-3.38 (m, 1H), 3.28-3.17 (m, 1H), 3.15-3.02 (m, 2H), 3.01-2.83 (m, 2H), 2.79-2.61 (m, 2H), 2.45-2.25 (m, 2H), 2.24-2.13 (m, 2H), 1.87-1.67 (m, 2H).

LC-MS (ESI): m/z = 467.1 $[M+H]^+$.

**Example 70**

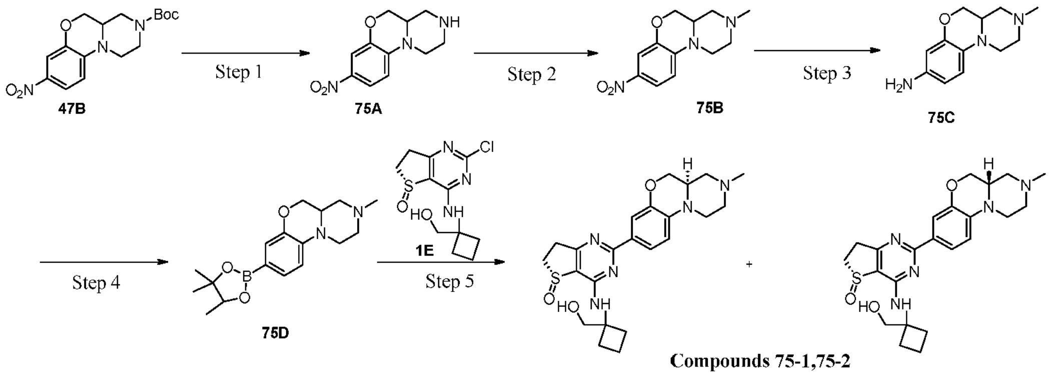


47B 75A 75B 75C 75D 1E

Compounds 75-1,75-2

Step 1: **47B** (10.0 g, 29.8 mmol) and hydrogen chloride-1,4-dioxane (100 mL) were successively added to a 250 mL single-mouth flask and reacted at room temperature for 1 hour. After concentration, a saturated sodium bicarbonate aqueous solution (100 mL) was added to adjust the pH to > 7, and dichloromethane (100 mL × 2) was used for extraction. The organic phase was dried over anhydrous sodium sulphate and filtered, and the filtrate was concentrated under reduced pressure to obtain **75A** (7.0 g, 99.8%).
LC-MS (ESI): m/z = 236.1 $[M+H]^+$.
Step 2: **75A** (3.5 g, 14.9 mmol), anhydrous ethanol (40 mL), paraformaldehyde (3.5 g), and sodium cyanoborohydride (1.88 g, 29.8 mmol) were successively added to a 50 mL single-mouth flask and reacted at room temperature for 12 hours. The reaction product was concentrated under reduced pressure and then separated and purified by silica gel column chromatography (dichloromethane/methanol (v/v) = 90/10) to obtain **75B** (2.5 g, 67.6%).
LC-MS (ESI): m/z = 250.2 $[M+H]^+$.
Step 3: **75B** (2.5 g, 10.0 mmol), ethyl acetate (50 mL), and palladium on carbon (10%, 0.5 g) were successively added to a 100 mL single-mouth flask, and the mixture was subjected to a hydrogenation reduction reaction at room temperature for 12 hours. After filtration, the filtrate was concentrated under reduced pressure to obtain **75C** (2.2 g, 100%).
LC-MS (ESI): m/z = 220.2 $[M+H]^+$.
Step 4: **75C** (2.2 g, 10.0 mmol), methanol (20 mL), and a hydrogen chloride aqueous solution (3 M, 10 mL) were successively added to a 100 mL single-mouth flask, and sodium nitrite (693 mg, 10.0 mmol) was added at -5°C. Pinacol diborate (5.1 g, 20.1 mmol) was immediately added. The mixture was reacted at room temperature for 1 hour, the reaction product was adjusted to pH > 7 by adding saturated sodium bicarbonate and extracted with dichloromethane (100 mL × 2), and the organic phase was dried over anhydrous sodium sulphate and filtered. The filtrate was concentrated under reduced pressure and then separated and purified by silica gel column chromatography (dichloromethane/methanol (v/v) = 90/10) to obtain **75D** (2.0 g, 63.1%).
LC-MS (ESI): m/z = 317.2 $[M+H]^+$.
Step 5: Under nitrogen protection, **1E** (907 mg, 3.16 mmol), **75D** (1.0 g, 3.16 mmol), water (4 mL), 1,1'-bis(diphenylphosphino)ferrocene palladium dichloride (224 mg, 0.32 mmol), and potassium carbonate (1.3 g, 9.48 mmol) were successively added to a 100 mL single-mouth flask, dissolved by adding 1,4-dioxane (20 mL), warmed to 100°C, and reacted for 5 hours. The reaction product was cooled to room temperature, and the reaction liquid was directly concentrated under reduced pressure and then separated and purified by silica gel column chromatography (dichloromethane/methanol (v/v) = 90/10) to obtain a mixture of compounds **75-1** and **75-2** (200 mg, 13.8%).
After chiral preparation, the title compound **75-1** (81 mg, 5.6%, retention time: 0.885 min) and the title compound **75-2** (74 mg, 5.1%, retention time: 1.795 min) were obtained.
Chiral preparation method: instrument: Waters 150 MGM; chromatographic column: Chiralpak Column; mobile phase: A: carbon dioxide, and B: isopropanol (0.1% aqueous ammonia); isocratic elution: 70% mobile phase B; flow rate: 100 mL/min; back pressure: 100 bar; column temperature: 25°C; wavelength: 220 nm; elution time: 5.1 min.

**(Compound 75-1)**

$^1H$ NMR (400 MHz, DMSO-$d_6$) δ 7.84-7.79 (m, 1H), 7.75 (s, 1H), 7.64-7.61 (m, 1H), 6.98-6.89 (m, 1H), 4.93-4.83 (m, 1H), 4.31-4.23 (m, 1H), 3.96-3.89 (m, 1H), 3.84-3.73 (m, 3H), 3.66-3.51 (m, 1H), 3.40-3.31 (m, 1H), 3.22-3.09 (m, 2H),

3.02-2.94 (m, 1H), 2.91-2.71 (m, 3H), 2.47-2.32 (m, 2H), 2.31-2.24 (m, 2H), 2.23 (s, 3H), 2.11-2.02 (m, 1H), 1.90-1.77 (m, 2H), 1.75-1.66 (m, 1H).

LC-MS (ESI): m/z = 456.2 $[M+H]^+$.

**(Compound 75-2)**

$^1H$ NMR (400 MHz, DMSO-$d_6$) δ7.83-7.80 (m, 1H), 7.75 (s, 1H), 7.64-7.61 (m, 1H), 6.98-6.91 (m, 1H), 4.93-4.86 (m, 1H), 4.30-4.23 (m, 1H), 3.99-3.87 (m, 1H), 3.85-3.73 (m, 3H), 3.63-3.52 (m, 1H), 3.38-3.31 (m, 1H), 3.21-3.11 (m, 2H), 3.02-2.93 (m, 1H), 2.91-2.71 (m, 3H), 2.46-2.32 (m, 2H), 2.31-2.24 (m, 2H), 2.23 (s, 3H), 2.14-1.99 (m, 1H), 1.89-1.78 (m, 2H), 1.75-1.66 (m, 1H).

LC-MS (ESI): m/z = 456.2 $[M+H]^+$.

**Example 71**

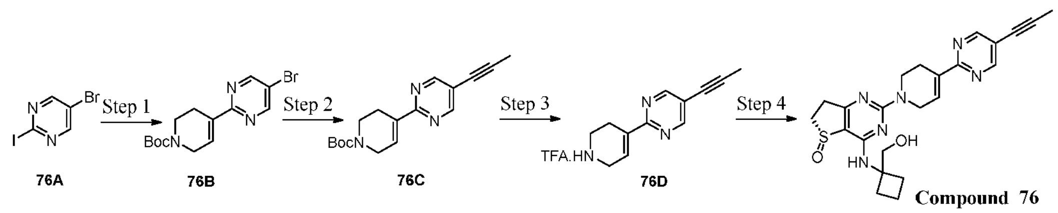


76A 76B 76C 76D Compound 76

Step 1: Under nitrogen protection, compound **76A** (2.00 g, 7.02 mmol), [1-(tert-butoxycarbonyl)-1,2,3,6-tetrahydropyridin-4-yl]boronic acid (2.28 g, 7.37 mmol), potassium carbonate (1.94 g, 14.04 mmol), and [1,1'-bis(diphenylphosphino)ferrocene]palladium dichloride (770 mg, 1.05 mmol) were added to a reaction flask. Tetrahydrofuran (40 mL) and water (8 mL) were added, and after nitrogen displacement, the mixture was warmed to 75°C and reacted for 16 h. After the disappearance of raw materials was detected by TLC, the reaction product was diluted by adding water (100 mL) and extracted with ethyl acetate (100 mL) three times. The organic phases were combined, dried over anhydrous sodium sulphate, and then concentrated in vacuum to obtain a crude product. The crude product was purified by column chromatography (petroleum ether/ethyl acetate (v/v) = 100/0 to 95/5) to obtain the target compound **76B** (1.00 g, 41.9%).

LC-MS (ESI): m/z = 254.60 $[M-56+H]^+$.

$^1H$ NMR (400 MHz, $CDCl_3$) δ 8.71 (s, 2H), 7.21-7.20 (m, 1H), 4.16-4.15 (m, 2H), 3.64-3.61 (m, 2H), 2.69-2.68 (m, 2H), 1.49 (s, 9H).

Step 2: Compound **76B** (1.00 g, 2.94 mmol), 1-(trimethylsilyl)propyne (660 mg, 5.88 mmol), potassium carbonate (1.02 g, 7.35 mmol), cuprous iodide (112 mg, 0.59 mmol), and [1,1'-bis(diphenylphosphino)ferrocene]palladium dichloride (160 mg, 0.44 mmol) were added to a reaction flask, and a solution of 1,4-dioxane (20 mL) was added. After nitrogen displacement, the mixture was warmed to 85°C and reacted for 16 h. After the disappearance of raw materials was detected by TLC, the reaction product was diluted by adding water (100 mL) and extracted with ethyl acetate (100 mL) three times. The organic phases were combined, dried over anhydrous sodium sulphate, and then concentrated in vacuum to obtain a crude product. The crude product was purified by column chromatography (petroleum ether/ethyl acetate (v/v) = 100/0 to 95/5) three times to obtain the target compound 76C (200 mg, 22.7%).

LC-MS (ESI): m/z = 244.10 $[M-56+H]^+$.

Step 3: Compound **76C** (200 mg, 0.67 mmol) was dissolved in dichloromethane (8 mL), and trifluoroacetic acid (2 mL) was added and then stirred at 0°C for 30 min. After the disappearance of raw materials was detected by TLC, the reaction liquid was concentrated at room temperature to obtain crude **76D** (230 mg), which was directly used in the next reaction.

LC-MS (ESI): m/z = 200.20 $[M+H]^+$.

Step 4: Compound **1E** (130 mg, 0.45 mmol) and compound **76D** (230 mg, 0.63 mmol) were dissolved in 1,4-dioxane (6 mL), and N,N-diisopropylethylamine (350 mg, 2.71 mmol) was then added. After the addition was complete, the mixture was warmed to 85°C under nitrogen protection and stirred for 16 h. After the reaction was complete, the reaction liquid was cooled to room temperature and concentrated under reduced pressure to obtain a crude product, which was purified by column chromatography (dichloromethane/methanol (v/v) = 100/0 to 90/10) and then further by prep.HPLC (preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b. gradient elution: mobile phase A: 30-95%;

c. flow rate: 15 mL/min; d. elution time: 15 min; retention time: 6.0 min) to obtain the target compound **76** (48 mg, 23.6%).

$^1$H NMR (400 MHz, DMSO-d6) δ 8.80 (s, 2H), 7.34 (s, 1H), 7.29 (s, 1H), 4.85-4.82 (m, 1H), 4.46 (s, 2H), 3.98-3.95 (m, 2H), 3.75-3.74 (m, 2H), 3.47-3.39 (m, 1H), 3.26-3.19 (m, 1H), 2.98-2.92 (m, 1H), 2.89-2.84 (m, 1H), 2.65 (s, 2H), 2.43-2.30 (m ,2H), 2.22-2.18 (m ,2H), 2.12 (s, 3H), 1.83-1.73 (m, 2H).

LCMS m/z = 451.2 [M+H]$^+$.

**Example 72**

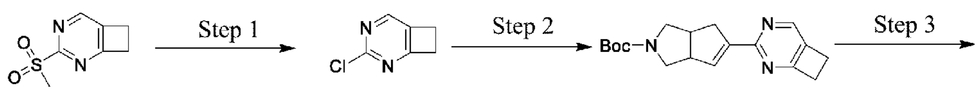


65H 77A 77B

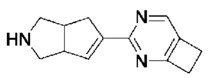

77C

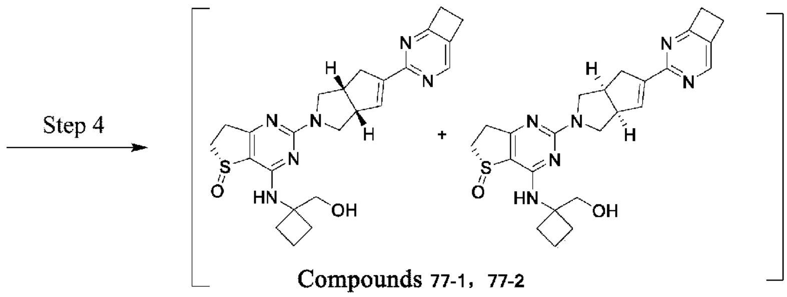


Compounds 77-1, 77-2

Step 1: Compound **65H** (0.5 g, 2.72 mmol) was dissolved in glacial acetic acid (20 mL), and thionyl dichloride (10 mL) was added and stirred at 60°C overnight. After the reaction was complete, the reaction product was cooled to room temperature, and the reaction system was diluted by adding ethyl acetate (50 mL). The pH of the reaction system was adjusted to neutrality with a saturated sodium bicarbonate aqueous solution and extracted with ethyl acetate (50 mL x 3). The organic phase was dried over anhydrous sodium sulphate, filtered, and concentrated, and the residue was subjected to silica gel column chromatography (petroleum ether/ethyl acetate (v/v) = 100/0 to 80/20) for fast separation and purification to obtain compound **77A** (100 mg, 26%).

LCMS (ESI): m/z = 141.1 [M+H]$^+$.

Step 2: Under nitrogen protection, compound **42C** (238 mg, 0.71 mmol), **77A** (100 mg, 0.71 mmol), 1,1-bis(diphenylphosphino)ferrocene palladium dichloride (51 mg, 0.07 mmol), and sodium carbonate (226 mg, 2.13 mmol) were all dissolved in a mixed solvent of 1,4-dioxane/water (10 mL/2 mL), warmed to 90°C, and reacted for 18 h. After the reaction was complete, the reaction product was cooled to room temperature, filtered with diatomite, and washed with ethyl acetate. After the filtrate was concentrated, the obtained residue was separated and purified by column chromatography (petroleum ether/ethyl acetate (v/v) = 100/0 to 90/10) to obtain the target compound **77B** (102 mg, 45%).

LCMS (ESI): m/z = 258.1 [M+H-56]$^+$.

Step 3: Compound **77B** (102 mg, 0.33 mmol) was dissolved in a mixed solvent of dichloromethane/trifluoroacetic acid (6 mL/2 mL), stirred at room temperature for 1 h, and directly spin-dried to obtain compound **77C** trifluoroacetate, which was directly used in the next reaction (111 mg).

LCMS (ESI): m/z = 214.1 [M+H]$^+$.

Step 4: Compound **77C** trifluoroacetate compound (111 mg) was dissolved in 1,4-dioxane (5 ml), **1E** (95 mg, 0.33 mmol) and diisopropylethylamine (128 mg, 0.99 mmol) were added, and the reaction system was placed in an oil bath at 100°C and reacted for 15 h. After the reaction was complete, the reaction product was cooled to room temperature, and the reaction was monitored by LCMS. After the reaction was complete, the reaction product was directly spin-dried. The crude product was separated and purified by silica gel column chromatography (dichloromethane/methanol (v/v) = 100/0 to 80/20) to obtain a mixture of compounds **77-1** and **77-2** (50 mg, 33%). After further chiral resolution, **compound 77-1** (26 mg, retention time: 1.891 s) and **compound 77-2** (21.6 mg, retention time: 2.901 s) were obtained.

Chiral preparation method: instrument: Waters 150 Prep-SFC E; chromatographic column: Chiralcel Cellulose-2 column; mobile phase: A: carbon dioxide, and B: methanol (0.1% aqueous ammonia); gradient elution: B 40%; flow rate: 100 mL/min; back pressure: 100 bar; column temperature: 25°C; wavelength: 220 nm; elution time: 7.0 min.

**(Compound 77-1)**

$^1$H NMR (400 MHz, $CDCl_3$) δ 8.23 (s, 1H), 6.78 (s, 1H), 5.67 (s, 1H), 5.09 (d, 1H), 4.07-3.92 (m, 1H), 3.89-3.73 (m, 4H), 3.67 (s, 1H), 3.61-3.53 (m, 1H), 3.49-3.44 (m, 2H), 3.40 (d, 1H), 3.31 (d, 1H), 3.23 (t, 2H), 3.16 (s, 1H), 3.11-2.92 (m, 3H), 2.85 (d, 1H), 2.36 (d, 2H), 2.23-2.15 (m, 2H), 1.97-1.89 (m, 2H).
LCMS (ESI): m/z = 465.2 $[M+H]^+$.

**(Compound 77-2)**

$^1$H NMR (400 MHz, $CDCl_3$) δ 8.23 (s, 1H), 6.78 (s, 1H), 5.83 (s, 1H), 5.09 (d, 1H), 4.06-3.90 (m, 1H), 3.89-3.73 (m, 4H), 3.67 (s, 1H), 3.57 (d, 1H), 3.46 (s, 2H), 3.43-3.28 (m, 2H), 3.23 (s, 2H), 3.18-2.96 (m, 4H), 2.87 (d, 1H), 2.41-2.31 (m, 1H), 2.28-2.15 (m, 3H), 1.99-1.83 (m, 2H).
LCMS (ESI): m/z = 465.2 $[M+H]^+$.

**Example 73**

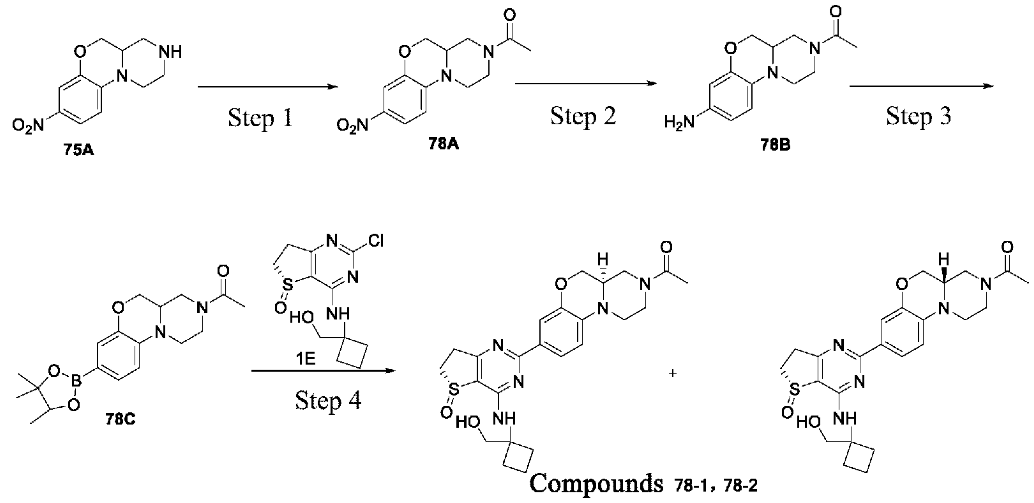


Compounds 78-1, 78-2

Step 1: **75A** (3.5 g, 14.89 mmol) was dissolved in dichloromethane (35 mL), and triethylamine (4.5 g, 44.67 mmol) and acetic anhydride (1.5 g, 14.89 mmol) were added and reacted at room temperature for 1 hour, after which water (50 mL) was added, and the mixture was extracted with dichloromethane (50 mL × 2). The organic phase was dried over anhydrous sodium sulphate and filtered, and the filtrate was concentrated under reduced pressure and then separated and purified by silica gel column chromatography (dichloromethane/methanol (v/v) = 91/9) to obtain **78A** (3.2 g, 77.6%).
LC-MS (ESI): m/z = 278.1 $[M+H]^+$.
Step 2: Compound **78A** (3.2 g, 11.55 mmol) was dissolved in ethyl acetate (50 mL), palladium on carbon (10%, 0.5 g) was added, and the mixture was warmed to 50°C in a hydrogen atmosphere and reacted for 12 hours. After the reaction was complete, the reaction product was directly filtered and the filtrate was concentrated under reduced pressure to obtain **78B** (2.8 g, 98.2%).
LC-MS (ESI): m/z = 248.2 $[M+H]^+$.
Step 3: **78B** (2.5 g, 10.12 mmol), methanol (20 mL), and a hydrogen chloride aqueous solution (3 M, 10 mL) were successively added to a 100 mL single-mouth flask, and sodium nitrite (698 mg, 10.12 mmol) was added at -5°C. Pinacol diboronate (5.1 g, 20.24 mmol) was immediately added, and the mixture was reacted at room temperature for 1 hour. After the completion of the reaction was monitored by TLC, the reaction product was adjusted to pH > 7 by adding saturated sodium bicarbonate and extracted with dichloromethane (100 mL × 2), and the organic phase was dried over anhydrous sodium sulphate and filtered. The filtrate was concentrated under reduced pressure and then separated and purified by silica gel column chromatography (dichloromethane/methanol (v/v) = 91/9) to obtain **78C** (2.5 g, 71.8%).
LC-MS (ESI): m/z = 345.2 $[M+H]^+$.
Step 4: **1E** (907 mg, 3.16 mmol), **78C** (1.1 g, 3.16 mmol), 1,1'-bis(diphenylphosphino)ferrocene palladium dichloride (224 mg, 0.32 mmol), and potassium carbonate (1.3 g, 9.48 mmol) were successively added to a 100 mL single-mouth flask, dissolved by adding a mixed solvent of 1,4-dioxane (20 mL) and water (4 mL), warmed to 100°C under nitrogen protection, and reacted for 5 hours. The reaction product was cooled to room temperature, and the reaction liquid

was directly concentrated under reduced pressure and then separated and purified by silica gel column chromatography (dichloromethane/methanol (v/v) = 91/9) to obtain a mixture of **78-1** and **78-2** (250 mg).

After chiral preparation, the title compound **78-1** (78 mg, 5.1%, retention time: 0.960 min) and the title compound **78-2** (69 mg, 4.5%, retention time: 1.256 min) were obtained. Chiral preparation method: instrument: Waters 150 MGM; chromatographic column: Chiralpak Column; mobile phase: A: carbon dioxide, and B: ethanol (0.1% aqueous ammonia); isocratic elution: 70% mobile phase B; flow rate: 100 mL/min; back pressure: 100 bar; column temperature: 25°C; wavelength: 220 nm; elution time: 4.0 min.

**(Compound 78-1)**

$^1$H NMR (400 MHz, DMSO-$d_6$) δ7.92-7.75 (m, 2H), 7.69-7.55 (m, 1H), 7.05-6.93 (m, 1H), 4.97-4.82 (m, 1H), 4.50-4.31 (m, 2H), 4.06-3.85 (m, 3H), 3.85-3.73 (m, 2H), 3.64-3.52 (m, 1H), 3.38-3.31 (m, 1H), 3.30-2.60 (m, 6H), 2.46-2.34 (m, 2H), 2.32-2.21 (m, 2H), 2.12-1.98 (m, 3H), 1.94-1.71 (m, 2H).

LC-MS (ESI): m/z = 484.4 $[M+H]^+$.

**(Compound 78-2)**

$^1$H NMR (400 MHz, DMSO-$d_6$) δ7.94-7.74 (m, 2H), 7.70-7.56 (m, 1H), 7.05-6.82 (m, 1H), 5.00-4.84 (m, 1H), 4.51-4.29 (m, 2H), 4.02-3.87 (m, 3H), 3.84-3.75 (m, 2H), 3.64-3.52 (m, 1H), 3.39-3.32 (m, 1H), 3.29-2.62 (m, 6H), 2.46-2.35 (m, 2H), 2.32-2.20 (m, 2H), 2.09-2.02 (m, 3H), 1.91-1.77 (m, 2H).

LC-MS (ESI): m/z = 484.4 $[M+H]^+$.

**Example 74**

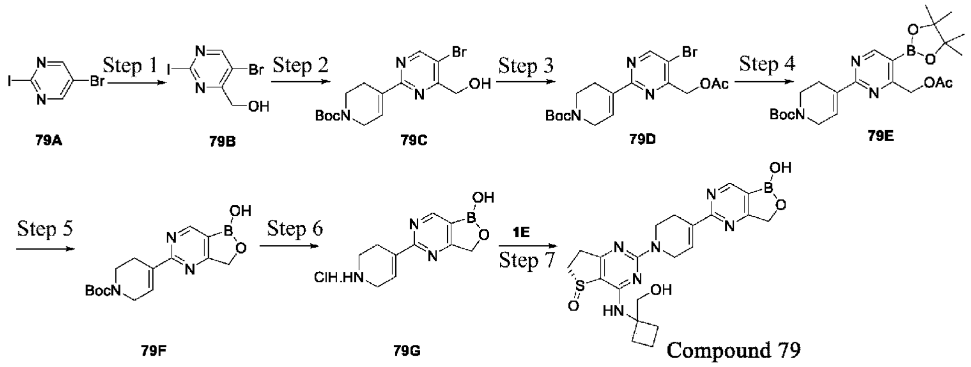


79A 79B 79C 79D 79E 79F 79G Compound 79

Step 1: Under nitrogen protection, compound **79A** (20.00 g, 70.20 mmol) was dissolved in methanol (200 mL). Trifluoroacetic acid (8.17 g, 71.61 mmol) and benzoyl peroxide (19.05 g, 78.63 mmol) were successively and separately added while stirring at room temperature. After the addition was complete, the reaction liquid was warmed to 65°C and stirred for 16 h. After the disappearance of raw materials was detected by TLC, the reaction product was cooled to room temperature, concentrated under reduced pressure into a small volume, then diluted by adding a saturated sodium carbonate aqueous solution (400 mL), and extracted with ethyl acetate (100 mL) three times. The organic phases were combined, dried over anhydrous sodium sulphate, and then concentrated in vacuum to obtain a crude product. The crude product was purified by column chromatography (petroleum ether/ethyl acetate (v/v) = 100/0 to 80/20) to obtain the target compound **79B** (2.00 g, 9.05%).

$^1$H NMR (400 MHz, DMSO-*d6*) δ 8.67 (s, 1H), 5.58-5.55 (m, 1H), 4.56-4.54 (m, 2H).

LC-MS (ESI): m/z = 314.9 $[M+H]^+$.

Step 2: Under nitrogen protection, compound **79B** (2.00 g, 6.35 mmol) was added to a mixed solution of 1,4-dioxane (20 mL) and water (4 mL). [1-(tert-butoxycarbonyl)-1,2,3,6-tetrahydropyridin-4-yl]boronic acid (2.00 g, 6.48 mmol), [1,1'-bis(diphenylphosphino)ferrocene]palladium dichloride (0.68 g, 0.93 mmol), and potassium carbonate (1.76 g, 12.70 mmol) were added successively and separately with stirring at room temperature. After the addition was complete, the reaction liquid was warmed to 85°C and stirred for 16 h. After the disappearance of raw materials was detected by TLC, the reaction product was cooled to room temperature, concentrated under reduced pressure into a small volume, then diluted by adding water (100 mL), and extracted with ethyl acetate (50 mL) three times. The organic phases were combined, dried over anhydrous sodium sulphate, and then concentrated in vacuum to obtain a crude product. The

crude product was purified by column chromatography (petroleum ether/ethyl acetate (v/v) = 100/0 to 80/20) to obtain the target compound **79C** (950 mg, 40.40%).

$^1$H NMR (400 MHz, $CDCl_3$) δ 8.67 (s, 1H), 7.27-7.26 (m, 1H), 4.74 (s, 2H), 4.19-4.18 (m, 2H), 3.66-3.63 (m, 2H), 2.73 (s, 2H), 1.49 (s, 9H).

LC-MS (ESI): m/z = 314.9 $[M+H-56]^+$.

Step 3: Under nitrogen protection, compound **79C** (950 mg, 2.57 mmol) was dissolved in dichloromethane (20 mL), triethylamine (650 mg, 6.41 mmol) was added with stirring in ice bath, and acetyl chloride (300 mg, 3.85 mmol) was slowly added dropwise. After the addition was complete, the reaction liquid was stirred at 0°C for 1 h. After the disappearance of raw materials was detected by TLC, the reaction was quenched by adding a saturated ammonium chloride aqueous solution (50 mL), and the reaction product was extracted with dichloromethane (40 mL) three times. The organic phases were combined, dried over anhydrous sodium sulphate, and then concentrated in vacuum to obtain a crude product. The crude product was further purified by column chromatography (petroleum ether/ethyl acetate (v/v) = 100/0 to 80/20) to obtain the target compound **79D** (750 mg, 70.90%).

$^1$H NMR (400 MHz, $CDCl_3$) δ 8.67 (s, 1H), 7.19 (br s, 1H), 5.25 (s, 2H), 4.16-4.15 (m, 2H), 3.63-3.60 (m, 2H), 2.66 (s, 2H), 2.22 (s, 3H), 1.49 (s, 9H).

LC-MS (ESI): m/z = 356.3 $[M+H-56]^+$.

Step 4: Under nitrogen protection, compound **79D** (750 mg, 1.82 mmol) was dissolved in 1,4-dioxane (20 mL). Pinacol diboronate (530 mg, 2.08 mmol), [1,1'-bis(diphenylphosphino)ferrocene]palladium dichloride (110 mg, 0.20 mmol), and potassium acetate (540 mg, 5.48 mmol) were successively added with stirring at room temperature. After the addition was complete, the reaction liquid was warmed to 80°C and stirred for 5 h. After the disappearance of raw materials was detected by TLC, the reaction product was cooled to room temperature, concentrated under reduced pressure into a small volume, then diluted by adding water (50 mL), and extracted with ethyl acetate (50 mL) three times. The organic phases were combined, dried by anhydrous sodium sulphate, and then concentrated in vacuum to obtain crude product **79E** (1.2 g), which was directly used in the next reaction.

Step 5: Crude compound **79E** (1.2 g) was dissolved in methanol (20 mL), and a sodium hydroxide aqueous solution (240 mg, 6.01 mmol, 1 M) was added, and after the addition was complete, the mixture was stirred at room temperature for 16 h under nitrogen protection. The reaction liquid was concentrated under reduced pressure into a small volume, diluted by adding water (20 mL), and extracted twice with ethyl acetate (50 mL). The aqueous phase was further concentrated and then purified by a reverse-phase column (water/acetonitrile (v/v) = 100/0 to 10/90) to obtain compound **79F** (270 mg, 42.51%).

LCMS m/z = 262.2 $[M-56+H]^+$.

Step 6: Compound **79F** (270 mg, 0.85 mmol) was dissolved in dichloromethane (4 mL), and a hydrogen chloride dioxane solution (310 mg, 8.51 mmol, 4 M) was added in ice bath. After the addition was complete, the mixture was stirred for 1.5 h under nitrogen protection. The reaction liquid was concentrated under reduced pressure to obtain crude compound **79G** (200 mg), which was directly used in the next reaction.

LCMS m/z = 218.2 $[M+H]^+$.

Step 7: Compound **1E** (100 mg, 0.35 mmol) and crude compound **79G** (200 mg) were dissolved in 1,4-dioxane (6 mL), and N,N-diisopropylethylamine (135 mg, 1.04 mmol) was then added. After the addition was complete, the mixture was warmed to 85°C under nitrogen protection and stirred for 16 h. After the reaction was complete, the reaction liquid was concentrated under reduced pressure to obtain a crude product, which was purified by column chromatography (dichloromethane/methanol (v/v) = 100/0 to 91/9) and then further purified by a reverse-phase column (water/acetonitrile (v/v) = 100/1 to 1/85) to obtain the target compound **79** (21 mg, 12.90%).

$^1$H NMR (400 MHz, DMSO-*d6*) δ 8.92 (s, 1H), 7.33-7.27 (m, 1H), 4.84 (s, 1H), 4.67-4.55 (m, 1H), 4.43 (br s, 1H), 3.97 (s, 1H), 3.76-3.75 (m, 2H), 3.51-3.40 (m, 1H), 3.24-3.19 (m, 3H), 2.99-2.83 (m, 2H), 2.67 (s, 2H), 2.42-2.34 (m, 2H), 2.22-2.21 (m, 2H), 1.81-1.76 (m, 2H), 1.53-1.47 (m, 1H), 1.27-1.21 (m, 1H), 1.10-0.95 (m, 1H).

LCMS m/z = 469.40 $[M+H]^+$.

**Example 75**

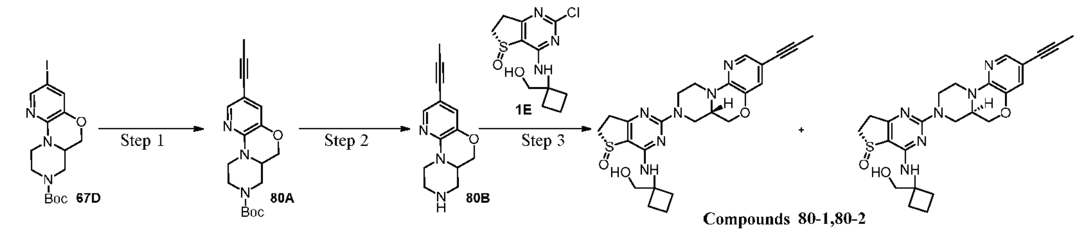


67D 80A 80B 1E Compounds 80-1,80-2

Step 1: **67D** (0.8 g, 1.92 mmol), tetrahydrofuran (15 mL), cuprous iodide (72 mg, 0.38 mmol), triethylamine (582 mg, 5.76 mmol), bis(triphenylphosphino)palladium dichloride (139 mg, 0.19 mmol), and a propyne-tetrahydrofuran solution (1 M, 19.2 mL, 19.2 mmol) were successively added to a 100 mL single-mouth flask and reacted for 6 hours at room temperature under nitrogen protection, and the reaction product was concentrated under reduced pressure and then separated and purified by silica gel column chromatography (eluents: ethyl acetate/petroleum ether (v/v) = 20/100) to obtain **80A** (500 mg, 79.2%).

LC-MS (ESI): m/z = 330.2 $[M+H]^+$.

Step 2: **80A** (0.5 g, 1.5 mmol), triethylamine (3.0 g, 30 mmol), and iodotrimethylsilane (3.0 g, 15 mmol) were successively added to a 50 mL single-mouth flask and reacted at room temperature for 2 hours. The reaction product was concentrated under reduced pressure to obtain crude **80B**, which was directly used in the next reaction.

LC-MS (ESI): m/z = 230.1 $[M+H]^+$.

Step 3: **1E** (431 mg, 1.5 mmol), **80B** (crude, 0.8 g, 1.5 mmol), dioxane (5 mL), and N,N-diisopropylethylamine (1.16 g, 9.0 mmol) were successively added to a 50 mL single-mouth flask and reacted at 100°C for 6 hours. The reaction product was cooled to room temperature, and the reaction liquid was directly concentrated under reduced pressure and then separated and purified by silica gel column chromatography (dichloromethane/methanol (v/v) = 100/8) to obtain 450 mg of a mixture.

After chiral preparation, the title compound **80-1** (110 mg, 15.3%, retention time: 0.983 min) and the title compound **80-2** (106 mg, 14.7%, retention time: 1.247 min) were obtained. Chiral preparation method: instrument: Waters 150 MGM; chromatographic column: Chiralpak Column; mobile phase: A: carbon dioxide, and B: ethanol (0.1% aqueous ammonia); isocratic elution: 70% mobile phase B; flow rate: 100 mL/min; back pressure: 100 bar; column temperature: 25°C; wavelength: 220 nm; elution time: 4.0 min.

**(Compound 80-1)**

$^1$H NMR (400 MHz, DMSO-$d_6$) δ7.80-7.75 (m, 1H), 7.43 (s, 1H), 7.03-6.94 (m, 1H), 4.89-4.79 (m, 1H), 4.79-4.60 (m, 2H), 4.52-4.31 (m, 2H), 4.01-3.92 (m, 1H), 3.78-3.65 (m, 2H), 3.49-3.35 (m, 2H), 3.27-3.16 (m, 1H), 3.05-2.83 (m, 3H), 2.81-2.69 (m, 2H), 2.42-2.26 (m, 2H), 2.24-2.14 (m, 2H), 2.00 (s, 3H), 1.87-1.71 (m, 2H).

LC-MS (ESI): m/z = 481.2 $[M+H]^+$.

**(Compound 80-2)**

$^1$H NMR (400 MHz, DMSO-$d_6$) δ7.85-7.70 (m, 1H), 7.43 (s, 1H), 7.12-6.77 (m, 1H), 4.93-4.80 (m, 1H), 4.79-4.59 (m, 2H), 4.50-4.32 (m, 2H), 4.10-3.89 (m, 1H), 3.78-3.62 (m, 2H), 3.49-3.34 (m, 2H), 3.28-3.16 (m, 1H), 3.06-2.83 (m, 3H), 2.81-2.71 (m, 2H), 2.40-2.25 (m, 2H), 2.24-2.14 (m, 2H), 2.00 (s, 3H), 1.86-1.72 (m, 2H).

LC-MS (ESI): m/z = 481.2 $[M+H]^+$.

## Example 76

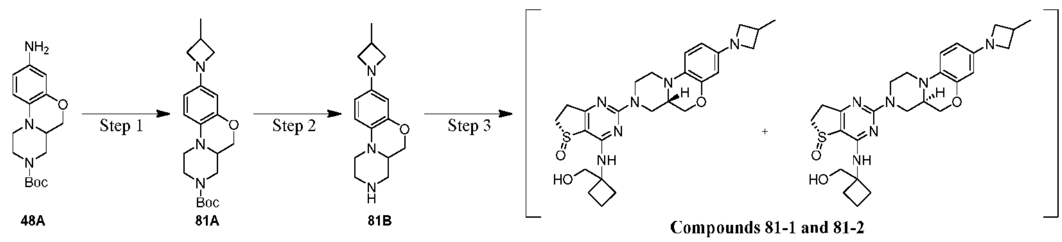


48A 81A 81B Compounds 81-1 and 81-2

Step 1: At room temperature, compound **48A** (1.0 g, 3.27 mmol), 1,3-dibromo-2-methylpropane (0.78 g, 3.60

mmol), and N,N-dimethylformamide (15 mL) were added to a 50 mL round-bottomed flask, and caesium carbonate (3.20 g, 9.82 mmol) was then added. The mixture was heated to 70°C and reacted for 14 h. After the reaction was complete, the reaction liquid was cooled to room temperature, concentrated under reduced pressure, and then separated and purified by silica gel column chromatography (eluents: EA/PE = 0-35%) to obtain the target compound **81A** (0.3 g, 25%).

LC-MS (ESI): m/z = 360.5 $[M+H]^+$.

Step 2: At room temperature, **81A** (0.30 g, 0.83 mmol), dichloromethane (3 mL), and trifluoroacetic acid (1 mL) were added to a 25 mL round-bottomed flask and continuously stirred for 2 h at this temperature. After the reaction was complete, the reaction liquid was concentrated under reduced pressure to obtain a crude product of target compound **81B** trifluoroacetate (0.20 g, 92%).

LC-MS (ESI): m/z = 260.3 $[M+H]^+$.

Step 3: At room temperature, the crude product of compound **81B** trifluoroacetate (0.20 g, 0.77 mmol), compound **1E** (0.27 g, 0.92 mmol), and 1,4-dioxane (5 mL) were added to a 25 mL round-bottomed flask, and N,N-diisopropylethylamine (0.30 g, 2.31 mmol) was added at room temperature. The mixture was then heated to 100°C and reacted for 12 h. After the reaction was complete, the reaction liquid was cooled to room temperature, concentrated under reduced pressure, and then separated and purified by silica gel column chromatography (eluents: MeOH/DCM = 0-10%) to obtain 0.26 g of a racemate. The racemate was separated by SFC to obtain two isomers, compound **81-1** (50 mg, retention time 1.94 min, 13%) and compound **81-2** (50 mg, retention time 2.18 min, 13%).

Separation conditions of preparative chromatography: 1. Instrument: SHIMADZU LC-20AP; 2. chromatographic column: C18; 3. mobile phase system: A for 0.1% TFA in $H_2O$; B for ACN; 4. gradient: B 3-33%; 5. flow rate: 75 mL/min.

Compound **81-1**:

$^1H$ NMR (400 MHz, Chloroform-*d*) δ 6.76-6.66 (m, 1H), 6.09-5.95 (m, 3H), 4.83-4.57 (m, 2H), 4.27-4.21 (m, 1H), 4.07-4.01 (m, 1H), 3.98-3.90 (m, 2H), 3.85 (s, 2H), 3.70-3.62 (m, 1H), 3.62-3.54 (m, 1H), 3.44-3.32 (m, 3H), 3.25-3.17 (m, 1H), 3.08-2.96 (m, 3H), 2.84-2.74 (m, 2H), 2.70-2.61 (m, 1H), 2.36-2.20 (m, 4H), 1.98-1.84 (m, 2H), 1.24 (d, 3H).

LC-MS (ESI): m/z = 511.3 $[M+H]^+$.

Compound 81-2: $^1H$ NMR (400 MHz, Chloroform-d) δ 6.76-6.68 (m, 1H), 6.19-6.08 (m, 2H), 6.04 (s, 1H), 4.80-4.58 (m, 2H), 4.31-4.22 (m, 1H), 4.09-3.98 (m, 3H), 3.92-3.81 (m, 2H), 3.70-3.54 (m, 2H), 3.47-3.36 (m, 3H), 3.26-3.16 (m, 1H), 3.09-2.95 (m, 3H), 2.89-2.75 (m, 2H), 2.72-2.60 (m, 1H), 2.32-2.26 (m, 4H), 1.97-1.84 (m, 2H), 1.25 (d, 3H).

LC-MS (ESI): m/z = 511.3 $[M+H]^+$.

**Example 77**

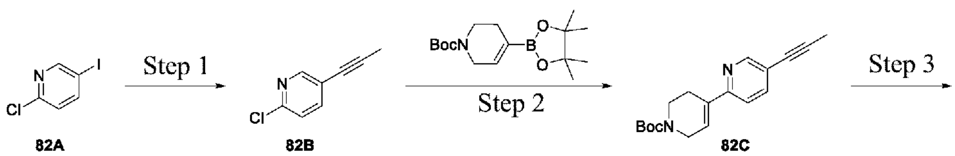


82A 82B 82C

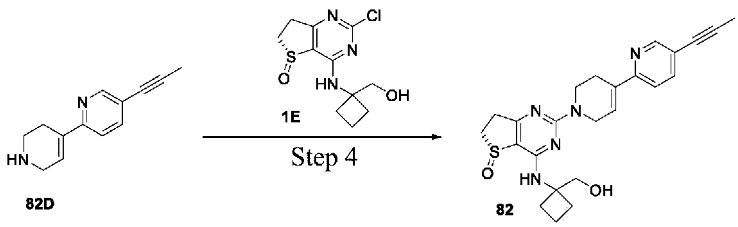


82D 82

Step 1: Compound **82A** (2.0 g, 8.4 mmol) was dissolved in toluene (50 mL), tributylpropynylstannane (3.1 g, 9.2 mmol) and tetrakis(triphenylphosphino)palladium (0.1 g, 0.8 mmol) were successively added, and after nitrogen displacement three times, the mixture was heated to 100°C and reacted for 14 h. After the completion of the reaction was monitored by a TLC plate, the reaction product was cooled to room temperature, concentrated, and purified by a silica gel column (PE/EA = 10/1) to obtain product **82B** (1.2 g, 94.4%).

$^1H$ NMR (400 MHz, $CDCl_3$) δ 8.37 (s, 1H), 7.62 (d, 1H), 7.25 (d, 1H), 2.07 (s, 3H).

Step 2: Compound **82B** (1.2 g, 7.9 mmol) was dissolved in 1,4-dioxane (20 mL), and 1,1-bis(diphenylphosphino) ferrocene palladium dichloride (0.6 g, 0.8 mmol), caesium carbonate (5.2 g, 15.8 mmol), N-Boc-1,2,5,6-tetrahydropyridine-4-boronic acid pinacol ester (2.9 g, 9.5 mmol), and water (4 mL) were then added. After nitrogen displacement three times, the mixture was heated to 90°C and reacted for 4 h, after which the reaction product was passed through a short silica gel column, rinsed with ethyl acetate, concentrated, and subjected to column chromatography (petroleum ether/ethyl acetate = 9/1) to obtain compound **82C** (2.0 g, 84.6%).

LC-MS (ESI): m/z = 299.4 $[M+H]^+$.

Step 3: Compound **82C** (1.5 g, 5.0 mmol) was dissolved in dichloromethane (10 mL), and trifluoroacetic acid (3.8 mL) was then added. The mixture was reacted at room temperature for half an hour and then concentrated to obtain compound **82D** trifluoroacetate (0.9 g, 90.3%), which was directly used in the next step.

LC-MS (ESI): m/z = 199.4 $[M+H]^+$.

Step 4: Compound **1E** (1.68 g, 5.8 mmol) was dissolved in 1,4-dioxane (20.0 mL), and compound **82D** trifluoroacetate (1.4 g, 7.0 mmol) and diisopropylethylamine (3.0 g, 23.3 mmol) were then added. The mixture was heated to 90°C and reacted for 12 h, after which the reaction product was cooled to room temperature, concentrated, and subjected to column chromatography (dichloromethane/methanol = 10/1) to obtain the target product **82** (2.5 g, 95%).

$^1$H NMR (400 MHz, DMSO-*d6*) δ 8.54 (d, 1H), 7.78 7.76 (m, 1H), 7.57 (d, 1H), 7.42 (s, 1H), 6.84 (s, 1H), 4.87 (s, 1H), 4.41 (s, 2H), 3.96 (t, 2H), 3.75 (s, 2H), 3.51-3.38 (m, 1H), 3.25-2.17 (m, 1H), 2.98-2.84 (m, 2H), 2.61 (s, 2H), 2.39-2.28 (m, 2H), 2.22-2.18 (m, 2H), 2.08 (s, 3H), 1.87-1.72 (m, 2H).

LC-MS (ESI): m/z = 450.1 $[M+H]^+$.

**Example 78**

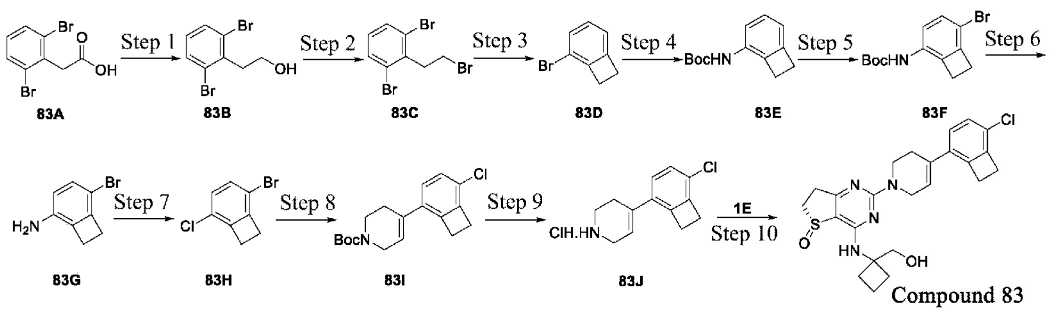


Step 1: Under nitrogen protection, tetrahydrofuran (50 mL) was added to a reaction flask containing compound **83A** (5.00 g, 17.01 mmol), and the mixture was stirred at 0°C for 15 min. A borane tetrahydrofuran complex (4.39 g, 51.03 mmol, 1 M) was slowly added dropwise, and after the addition was complete, the reaction liquid was warmed to 80°C and stirred for 16 h. After the disappearance of raw materials was detected by TLC, the reaction product was cooled to room temperature, and the reaction was quenched by adding water (20 mL), after which the reaction liquid was concentrated under reduced pressure into a small volume, then diluted by adding water (100 mL), and extracted with ethyl acetate (100 mL) three times. The organic phases were combined, dried by anhydrous sodium sulphate, and then concentrated in vacuum to obtain crude product **83B** (4.50 g, 94.50%), which was directly used in the next reaction.

$^1$H NMR (400 MHz, DMSO-*d6*) δ 7.65-7.62 (m, 2H), 7.11-7.07 (m, 1H), 3.57-3.53 (m, 2H), 3.16-3.12 (m, 2H).

Step 2: Under nitrogen protection, compound **83B** (4.50 g, 16.07 mmol) was dissolved in dichloromethane (45 mL). N-bromosuccinimide (3.43 g, 19.29 mmol) and triphenylphosphine (5.86 g, 22.34 mmol) were added while stirring in ice bath. After the addition was complete, the reaction liquid was warmed to room temperature and stirred for 16 h. After the disappearance of raw materials was monitored by TLC, tert-butyl hydroperoxide (2 mL) was added and stirred for 10 min, the reaction was quenched by adding a saturated sodium bisulphite aqueous solution (100 mL), and the reaction product was extracted with dichloromethane (50 mL) three times. The organic phases were combined, dried over anhydrous sodium sulphate, and then concentrated in vacuum to obtain a crude product. The crude product was purified by column chromatography (eluents: PE) to obtain the target compound **83C** (3.20 g, 58.07%).

$^1$H NMR (400 MHz, $CDCl_3$) δ 7.53-7.51 (m, 2H), 6.98-6.94 (m, 1H), 3.58-3.47 (m, 4H).

Step 3: Under nitrogen protection, compound **83C** (3.20 g, 9.33 mmol) was dissolved in anhydrous tetrahydrofuran (100 mL), and n-butyl lithium (3.6 mL, 2.5 M) was slowly added dropwise with stirring at -70°C. After the addition was complete, the reaction liquid was stirred at -70°C for 5 h. The reaction was quenched by slowly dropwise adding water (20 mL) and the reaction liquid was extracted with ethyl acetate (100 mL) three times. The organic phases were combined, dried by anhydrous sodium sulphate, and then concentrated in vacuum to obtain **83D** (2.10 g, 86.04%).

$^1$H NMR (400 MHz, $CDCl_3$) δ 7.29-7.25 (m, 1H), 7.08-7.04 (m, 1H), 6.99-6.98 (m, 1H), 3.13 (s, 4H).

Step 4: Compound **83D** (1.00 g, 5.46 mmol) was dissolved in 1,4-dioxane (20 mL), and tert-butyl carbamate (960 mg, 8.19 mmol), 2-dicyclohexylphosphine-2',4',6'-triisopropyl biphenyl (260 mg, 0.55 mmol), caesium carbonate (3.56 g, 10.93 mmol), and palladium acetate (62 mg, 0.27 mmol) were successively added. After the addition was complete, the mixture was stirred at 100°C for 4 h under nitrogen protection. After the disappearance of raw materials was monitored by TLC, the reaction liquid was cooled to room temperature, the reaction was quenched by adding a saturated sodium bicarbonate solution (30 mL), and the reaction product was extracted with ethyl acetate (50 mL) three times. The organic phases were combined, dried over anhydrous sodium sulphate, and then concentrated in vacuum to obtain a crude product. The crude product was purified by column chromatography (eluents: PE : EA = 100:1 to 50:1) to obtain the target compound **83E** (1.00 g, 93.27%).

$^1$H NMR (400 MHz, $CDCl_3$) δ 7.28-7.26 (m, 1H), 7.15-7.11 (m, 1H), 6.77-6.75 (m, 1H), 6.30 (s, 1H), 3.20-3.18 (m, 2H), 3.12-3.10 (m, 2H), 1.52 (s, 9H).

LCMS m/z = 164.1 $[M-56+H]^+$.

Step 5: Compound **83E** (880 mg, 3.65 mmol) was dissolved in acetic acid (6 mL), N-bromosuccinimide (715 mg, 4.01 mmol) was then added, and after the addition was complete, the mixture was stirred at room temperature for 16 h under nitrogen protection. The reaction was diluted by adding water (30 mL) and extracted with ethyl acetate (50 mL) three times. The organic phases were combined, dried over anhydrous sodium sulphate, and then concentrated in vacuum to obtain a crude product. The crude product was purified by column chromatography (eluents: PE : EA = 100:1 to 50:1) to obtain the target compound **83F** (700 mg, 64.35%).

$^1$H NMR (400 MHz, $CDCl_3$) δ 7.48-7.47 (m, 1H), 7.27-7.26 (m, 1H), 6.77 (s, 1H), 3.36-3.33 (m, 2H), 2.98-2.96 (m, 2H), 1.51 (s, 9H).

Step 6: Compound **83F** (700 mg, 2.35 mmol) was dissolved in 6 mL of dichloromethane, hydrogen chloride dioxane (6 mL) was added and stirred at 0°C for 30 min. After the disappearance of raw materials was detected by TLC, the reaction liquid was concentrated at room temperature to obtain crude **83G** (750 mg), which was directly used in the next reaction.

Step 7: Crude compound **83G** (750 mg, 2.34 mmol) was dissolved in a 15% wt hydrogen chloride aqueous solution (5 mL) in ice bath, and sodium nitrite (265 mg, 3.84 mmol) was added. After stirring for 20 min, it was slowly dropwise added to a reaction flask containing cuprous chloride (1.14 g, 11.51 mmol) and concentrated hydrochloric acid (10 mL), and after the addition was complete, the reaction liquid was stirred for 16 h at room temperature. The mixture was diluted by adding water (20 mL) and extracted with ethyl acetate (50 mL) three times. The organic phases were combined, dried over anhydrous sodium sulphate, and then concentrated in vacuum to obtain a crude product. The crude product was purified by column chromatography (eluent: PE) to obtain the target compound **83H** (300 mg, 43.13%).

$^1$H NMR (400 MHz, $CDCl_3$) δ 7.60-7.59 (m, 1H), 7.48-7.47 (m, 1H), 3.16-3.15 (m, 2H), 3.04-3.02 (m, 2H).

Step 8: Under nitrogen protection, compound **83H** (300 mg, 1.38 mmol) was dissolved in tetrahydrofuran (12 mL) and water (2 mL), and [1-(tert-butoxycarbonyl)-1,2,3,6-tetrahydropyridin-4-yl]boronic acid (435 mg, 1.41 mmol), potassium carbonate (480 mg, 3.45 mmol), and [1,1'-bis(diphenylphosphino)ferrocene]palladium dichloride (150 mg, 0.21 mmol) were successively added. After the addition was complete, the reaction liquid was reacted at 75°C for 16 h under nitrogen protection. After the disappearance of raw materials was monitored by TLC, the reaction product was diluted by adding water (50 mL) and extracted with ethyl acetate (50 mL) three times. The organic phases were combined, dried over anhydrous sodium sulphate, and then concentrated in vacuum to obtain a crude product. The crude product was purified by column chromatography (eluents: PE : EA = 100 : 1 to 10 : 1) to obtain the target compound **83I** (140 mg, 31.37%).

$^1$H NMR (400 MHz, $CDCl_3$) δ 7.43-7.42 (m, 2H), 6.07-6.05 (m, 1H), 4.08-4.07 (m, 2H), 3.62-3.59 (m, 2H), 3.20-3.16 (m, 4H), 2.48-2.42 (m, 2H), 1.49 (s, 9H).

LC-MS (ESI): m/z = 282.0 $[M-56+H+18]^+$.

Step 9: Compound **83I** (130 mg, 0.41 mmol) was dissolved in 2 mL of dichloromethane, and hydrogen chloride dioxane (2 mL) was added and stirred at 0°C for 30 min. After the disappearance of raw materials was monitored by TLC, the reaction liquid was concentrated at room temperature to obtain crude **83J** (110 mg), which was directly used in the next reaction.

Step 10: **1E** (80 mg, 0.28 mmol) was dissolved in 1,4-dioxane (4 mL), and **83J** (110 mg, 0.42 mmol) and N,N-diisopropylethylamine (110 mg, 0.84 mmol) were successively added. After the addition was complete, the reaction liquid was warmed to 85°C under nitrogen protection and stirred for 16 h. After the reaction liquid naturally returned to room temperature, the reaction was diluted by adding water (50 mL) and extracted with ethyl acetate (50 mL) three times. The organic phases were combined, dried over anhydrous sodium sulphate, and then concentrated in vacuum to obtain a crude product. The crude product was purified by column chromatography (eluents: DCM : MeOH = 100 : 1 to 10 : 1) twice and then further by prep.HPLC (preparation method: instrument: waters 2767 preparative liquid chromatographic instrument; chromatographic column: SunFire@ Prep C18 (19 mm × 250 mm); the sample was dissolved with DMF and filtered with a 0.45 μm filter to prepare a sample liquid; preparative chromatography conditions: a. composition of mobile phases A and B: mobile phase A: acetonitrile, and mobile phase B: water (containing 5 mM aqueous ammonia); b.

gradient elution, mobile phase A: 35-90%; c. flow rate: 15 mL/min; d. elution time: 15 min; retention time: 6.0 min) to obtain the target compound **83** (2.5 mg, 1.91%).

$^1$H NMR (400 MHz, $CDCl_3$) δ 7.16-7.15 (m, 1H), 7.12-7.11 (m, 1H), 6.98-6.97 (m, 1H), 4.36 (s, 2H), 4.02 (s, 2H), 3.91-3.90 (m, 2H), 3.65-3.56 (m, 1H), 3.45-3.38 (m, 1H), 3.19-3.16 (m, 2H), 3.15-3.13 (m, 2H), 2.57-2.53 (m, 3H), 2.34-2.27 (m, 6H), 2.00-1.89 (m, 3H).

LCMS m/z = 471.4 $[M+H]^+$.

**Biological test:**

**1. Effect of compounds on PDE4B2 activity**

The effect of compounds on PDE4B2 activity was detected by a fluorescence polarization kit (BPS Bioscience, Catalogue #60343). According to the instructions of the kit, FAM-Cyclic-3',5'-AMP at a final concentration of 0.1 μM, 1 ng/well of PDE4B2 (PDE buffer was added to the negative control well), and a compound diluted in a gradient manner (PDE buffer containing 10% DMSO was added to the positive control well) were added to each well, fully mixed, and then reacted at room temperature for 1 h. The Binding Agent was diluted with the Binding Agent Diluent (cAMP) at a ratio of 1 : 100 for later use, the Binding Agent dilution was taken and added to the test plate at 50 μl/well and incubated at room temperature with slow shaking for 20 minutes. After the incubation was complete, Envision was used for FP detection, with Excitation at 480 nm and Emission at 535 nm. FP was usually represented by mP value.

$$mP = \left(\frac{I_{II} - G(I_\perp)}{I_{II} + G(I_\perp)}\right) x\ 1000$$

$I_{//}$ (S535): fluorescence intensity in parallel direction
$I\perp$ (P535): fluorescence intensity in perpendicular direction
G: G factor = 1

Calculation of inhibition rate (% Inhibition):

% Inhibition = [1 - ($mP_{(sample)}$ - $mP_{(negative\ control)}$)/($mP_{(positive}$ control) - $mP_{(negative}$ control))] × 100%

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the $IC_{50}$ value of each compound was calculated by GraphPad Prism 8 software.

The $IC_{50}$ values of the compounds of the present invention against PDE4B2 are less than 300 nM, and the $IC_{50}$ values of the compounds are preferably less than 100 nM, more preferably less than 50 nM, and further preferably less than 10 nM. The $IC_{50}$ values of some specific compounds are as shown in Table 3 below, wherein A < 10 nM, 10 nM ≤ B < 50 nM, and 50 nM ≤ C < 100 nM.

Table 3. PDE4B2 activity

| Compound | PDE4B2 $IC_{50}$ (nM) | Compound | PDE4B2 $IC_{50}$ (nM) |
|---|---|---|---|
| Compound 1 | A | Compound 62-1 | A |
| Compound 4 | B | Compound 62-2 | B |
| Compound 5 | A | Compound 63 | A |
| Compound 6 | B | Compound 64 | A |
| Compound 8 | B | Compound 65 | A |
| Compound 10 | B | Compound 66 | A |
| Compound 13 | B | Compound 67-1 | A |

(continued)

| Compound | PDE4B2 $IC_{50}$ (nM) | Compound | PDE4B2 $IC_{50}$ (nM) |
|---|---|---|---|
| Compound 14 | B | Compound 67-2 | B |
| Compound 19 | B | Compound 68-1 | A |
| Compound 20 | B | Compound 68-2 | A |
| Compound 21-1 | B | Compound 69 | A |
| Compound 22 | B | Compound 70 | A |
| Compound 25 | B | Compound 71 | B |
| Compound 43 | A | Compound 74-1 | A |
| Compound 46-1 | A | Compound 75-1 | A |
| Compound 46-2 | A | Compound 75-2 | B |
| Compound 47-1 | B | Compound 76 | A |
| Compound 47-2 | A | Compound 77-1 | A |
| Compound 48-1 | A | Compound 77-2 | B |
| Compound 48-2 | A | Compound 78-1 | B |
| Compound 50 | B | Compound 78-2 | B |
| Compound 51 | A | Compound 79 | A |
| Compound 53 | A | Compound 80-1 | A |
| Compound 54 | A | Compound 80-2 | A |
| Compound 55 | A | Compound 81-1 | A |
| Compound 56 | A | Compound 81-2 | A |
| Compound 57 | B | Compound 82 | A |
| Compound 58 | A | Compound 83 | A |
| Compound 59 | B | | |
| Compound 60 | B | | |
| Compound 61-1 | A | | |
| Compound 61-2 | B | | |

From the above, it can be seen that the compounds of the present invention, such as the examples, have good inhibitory activities, for example, the $IC_{50}$ values of compound 1 and compound 5 are 2.45 nM and 8.10 nM, respectively.

**2. Effect of compounds on PDE4D3 activity**

The effect of compounds on PDE4D3 activity was detected by a fluorescence polarization kit (BPS Bioscience, Catalogue #60346). According to the instructions of the kit, FAM-Cyclic-3',5'-AMP at a final concentration of 0.1 μM, 0.05 ng/well of PDE4D3 (PDE buffer was added to the negative control well), and a compound diluted in a gradient manner (PDE buffer containing 10% DMSO was added to the positive control well) were added to each well, fully mixed, and then reacted at room temperature for 1 h. The Binding Agent was diluted with the Binding Agent Diluent (cAMP) at a ratio of 1 : 100 for later use, the Binding Agent dilution was taken and added to the test plate at 100 μl/well and incubated at room temperature with slow shaking for 1 h. After the incubation was complete, Envision was used for FP detection, with Excitation at 480 nm and Emission at 535 nm. FP was usually represented by mP value.

$$mP = \left(\frac{I_{II} - G(I_{\perp})}{I_{II} + G(I_{\perp})}\right) x\ 1000$$

$I_{//}$ (S535): fluorescence intensity in parallel direction
I⊥ (P535): fluorescence intensity in perpendicular direction
G: G factor = 1

Calculation of inhibition rate (% Inhibition):

% Inhibition = [1 - ($mP_{(sample)}$ - $mP_{(negative\ control)}$)/($mP_{(positive}$ control) - $mP_{(negative}$ control))] × 100%

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the $IC_{50}$ value of each compound was calculated by GraphPad Prism 8 software.
Conclusion: The compounds of the present invention, such as the examples, have good selectivity.

**3. Effect of compounds on PDE4B1 activity**

The effect of compounds on PDE4B1 activity was detected by a fluorescence polarization kit (IMAP FP IPP Explorer KIT, Molecular Device, Cat# R8124). The IMAP Reaction Buffer containing 0.1% BSA (5×) in the kit was diluted into a 1-fold reaction buffer containing 1 mM DTT. 0.12 nM PDE4B1 (BPS, Cat#60041) was added to the 1-fold reaction buffer to form a 2-fold enzyme solution. 10 μL of the 2-fold enzyme solution was added to wells of a 384-well reaction plate. For the negative control well, 10 μL of the 1-fold reaction buffer was used instead of the enzyme solution. Centrifugation at 1000 rpm for 1 minute and incubation at room temperature for 15 minutes were carried out. 0.2 uM FAM-labelled cAMP (FAM-cAMP, Molecular Device, Cat# R7506) was added to the 1-fold reaction buffer to form a 2-fold substrate solution. 10 μL of the 2-fold substrate solution was added to each well of the 384-well reaction plate. Centrifugation at 1000 rpm for 1 minute was carried out. A reaction was carried out at room temperature for 20 minutes. IMAP Progressive Binding Buffer A (5×), IMAP Progressive Binding Buffer B (5×), and IMAP Progressive Binding Reagent (provided by IMAP FP IPP Explorer Kit) were prepared into a reaction stop solution according to the instructions for use. 60 μL of an 80/60-fold reaction stop solution was added to each well of the 384-well reaction plate to stop the reaction, and the reaction product was centrifuged at 1000 rpm for 1 minute. Incubation at room temperature in the dark for 60 minutes was carried out. The mP values (Ex480/Em535 (s), Em535 (p)) were read by a microplate reader (Envision).

FP was usually represented by mP value. mP value for FP measurement = 1000 * (S - G * P)/(S + G * P)

S: fluorescence intensity in parallel direction
P: fluorescence intensity in perpendicular direction
G: G factor = 1

Calculation of inhibition rate (% Inhibition):

% Inhibition = ($mP_{(positive}$ control) - $mP_{(sample)}$)/($mP_{(positive}$ control) - $mP_{(negative}$ control)) x 100%

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the $IC_{50}$ value of each compound was calculated by XLFit excel add-in version 5.4.0.8 software.
Fitting formula:

$$Y = Bottom + (Top - Bottom)/(1 + (IC_{50}/X)^{\wedge}HillSlope)$$

The $IC_{50}$ values of the compounds of the present invention against PDE4B 1 are less than 300 nM, and the $IC_{50}$ values of the compounds are preferably less than 100 nM, more preferably less than 50 nM, and further preferably less than 10 nM. The $IC_{50}$ values of some specific compounds are as shown in Table 4 below, wherein A < 10 nM, 10 nM ≤ B < 50 nM, and 50 nM ≤ C < 100 nM.

Table 4. PDE4B 1 activity

| Compound | PDE4B 1 $IC_{50}$ (nM) | Compound | PDE4B 1 $IC_{50}$ (nM) |
|---|---|---|---|
| Compound 20 | A | Compound 34 | A |
| Compound 22 | B | Compound 35 | A |
| Compound 25 | A | Compound 36 | A |
| Compound 26 | A | Compound 37 | A |
| Compound 27 | A | Compound 40-1 | A |
| Compound 28 | A | Compound 40-2 | A |
| Compound 29 | A | Compound 42-1 | A |
| Compound 30 | A | Compound 42-2 | A |
| Compound 31 | A | | |

**4. Effect of compounds on PDE4D2 activity**

The effect of compounds on PDE4D2 activity was detected by a fluorescence polarization kit (BPS Bioscience, Catalogue #60345). During the reaction, 12.5 μL of 0.272 ng/well of an enzyme (final concentration: 0.068 ng/well) and 12.5 μL of the compound diluted in a gradient manner (DMSO concentration: 4%) were first pre-incubated at room temperature for 15 minutes, the same volume of the enzyme at the same concentration and 12.5 μL of PDE buffer containing 4% DMSO were added to the positive control well, and 25 μL of PDE buffer containing 2% DMSO was added to the negative control well. After completion, 25 μL of 0.2 μM FAM-Cyclic-3',5'-AMP (final concentration: 0.1 μM) was added to each well, fully mixed, and then incubated at room temperature with slow shaking for 30 minutes. The Binding Agent was diluted with the Binding Agent Diluent (cAMP) at a ratio of 1 : 100 for later use, 100 μl/well of the Binding Agent dilution was taken and added to all wells of the test plate and incubated at room temperature with slow shaking for 1 hour. After the incubation was complete, BMG LRBTECH microplate reader was used for FP detection, with Excitation at 485 nm and Emission at 520 nm. FP was usually represented by mP value.

$$mP = \left(\frac{I_{II} - G(I_{\perp})}{I_{II} + G(I_{\perp})}\right) x\ 1000$$

$I_{//}$ (S520): fluorescence intensity in parallel direction
I⊥ (P520): fluorescence intensity in perpendicular direction
G: G factor = 1

Calculation of inhibition rate (% Inhibition):

% Inhibition = $[1 - (mP_{(sample)} - mP_{(negative\ control)})/(mP_{(positive}\ control) - mP_{(negative}\ control))] \times 100\%$

$mP_{(sample)}$: mP of the test compound in a reaction well
$mP_{(negative\ control)}$: mP in the negative control well
$mP_{(positive\ control)}$: mP in the positive control well

According to the calculated inhibition rate at each concentration, the $IC_{50}$ value of each compound was calculated by GraphPad Prism 8 software.

The $IC_{50}$ values of some specific compounds of the present invention are as shown in Table 5 below, wherein A < 10 nM, 10 nM ≤ B < 50 nM, and 50 nM ≤ C < 100 nM.

Table 5. PDE4D2 activity

| Compound | PDE4D2 $IC_{50}$ (nM) | Compound | PDE4D2 $IC_{50}$ (nM) |
|---|---|---|---|
| Compound 1 | A | Compound 60 | B |
| Compound 4 | B | Compound 61-1 | A |
| Compound 5 | A | Compound 61-2 | B |
| Compound 6 | A | Compound 62-1 | A |
| Compound 8 | B | Compound 62-2 | B |
| Compound 9 | B | Compound 63 | A |
| Compound 10 | B | Compound 64 | B |
| Compound 13 | A | Compound 65 | A |
| Compound 14 | A | Compound 66 | A |
| Compound 19 | B | Compound 67-1 | B |
| Compound 20 | A | Compound 67-2 | C |
| Compound 22 | B | Compound 68-1 | B |
| Compound 25 | A | Compound 68-2 | B |
| Compound 26 | A | Compound 69 | B |
| Compound 27 | A | Compound 70 | A |
| Compound 28 | A | Compound 71 | B |
| Compound 29 | A | Compound 74-1 | A |
| Compound 30 | A | Compound 75-1 | B |
| Compound 31 | A | Compound 75-2 | C |
| Compound 32 | B | Compound 76 | A |
| Compound 34 | A | Compound 77-1 | A |
| Compound 35 | A | Compound 77-2 | B |
| Compound 37 | A | Compound 79 | B |
| Compound 40-1 | A | Compound 80-1 | B |
| Compound 40-2 | A | Compound 80-2 | B |
| Compound 42-1 | A | Compound 81-1 | A |
| Compound 42-2 | A | Compound 81-2 | A |
| Compound 43 | B | Compound 82 | A |
| Compound 46-1 | A | Compound 83 | A |
| Compound 46-2 | A | | |
| Compound 47-2 | A | | |
| Compound 48-1 | A | | |
| Compound 48-2 | B | | |
| Compound 50 | B | | |
| Compound 51 | B | | |
| Compound 52 | B | | |
| Compound 53 | A | | |
| Compound 54 | A | | |
| Compound 55 | A | | |

(continued)

| Compound | PDE4D2 $IC_{50}$ (nM) | Compound | PDE4D2 $IC_{50}$ (nM) |
|---|---|---|---|
| Compound 56 | A | | |
| Compound 57 | B | | |
| Compound 58 | A | | |
| Compound 59 | B | | |

**5. Pharmacokinetic test in mice**

**5.1 Experimental animals:** Male ICR mice, 20-25 g, 6 mice/compound. Purchased from Chengdu Ddossy Experimental Animals Co., Ltd.

**5.2 Experimental design:** On the day of the experiment, 42 ICR mice were randomly grouped according to their body weights. The animals were fasted with water available for 12-14 h one day before administration and fed 4 h after administration.

Table 6. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | **Compound 37** | 5 | 1 | 5 | Plasma | Intravenous |
| G2 | 3 | | 10 | 1 | 10 | Plasma | Intragastric |
| G3 | 3 | **Compound 28** | 5 | 1 | 5 | Plasma | Intravenous |
| G4 | 3 | | 10 | 1 | 10 | Plasma | Intragastric |
| G5 | 3 | **Compound 42-1** | 5 | 1 | 5 | Plasma | Intravenous |
| G6 | 3 | | 10 | 1 | 10 | Plasma | Intragastric |
| G7 | 3 | **Compound 5** | 5 | 1 | 5 | Plasma | Intravenous |
| G8 | 3 | | 10 | 1 | 10 | Plasma | Intragastric |
| G9 | 3 | **Compound 1** | 5 | 1 | 5 | Plasma | Intravenous |
| G10 | 3 | | 10 | 1 | 10 | Plasma | Intragastric |
| G11 | 3 | **Compound 67-1** | 5 | 1 | 5 | Plasma | Intravenous |
| G12 | 3 | | 10 | 1 | 10 | Plasma | Intragastric |
| G13 | 3 | **Compound 80-1** | 5 | 1 | 5 | Plasma | Intravenous |
| G14 | 3 | | 10 | 1 | 10 | Plasma | Intragastric |
| Note: Vehicle I for intravenous administration: 5% DMA + 5% Solutol + 90% Saline, and vehicle II for intravenous administration: 10% DMA + 10% Solutol + 80% Saline; vehicle for intragastric administration: 0.5% MC (DMA: dimethylacetamide; Solutol: polyethylene glycol-15-hydroxystearate; Saline: physiological saline; MC: methylcellulose) | | | | | | | |

Before and after administration, 0.06 mL of blood was taken from the orbit under isoflurane anaesthesia and placed in an EDTAK2 centrifuge tube for centrifugation at 5000 rpm at 4°C for 10 min, followed by plasma collection. The blood collection time points for the intravenous group and intragastric group were both 0 min, 5 min, 15 min, 30 min, 1 h, 2 h, 4 h, 6 h, 8 h, and 24 h. Before analysis and detection, all the samples were stored at -80°C, and the samples were subjected to quantitative analysis using LC-MS/MS.

Table 7. Pharmacokinetic parameters of test compounds in plasma of mice

| Test compound | Mode of administration | CL (mL/min/kg) | $Vd_{ss}$ (L/kg) | $AUC_{0-t}$ (hr*ng/mL) | T1/2 (h) | F (%) |
|---|---|---|---|---|---|---|
| **Compound 37** | i.v. (5 mg/kg) | 7.03 ± 1.1 | 1.30 ± 0.086 | 6000 ± 1086 | 3.34 ± 0.30 | - |
| | i.g. (10 mg/kg) | - | - | 26929 ± 3912 | 4.30 ± 0.69 | ≥ 98 |
| **Compound 28** | i.v. (5 mg/kg) | 4.67 ± 1.2 | 1.31 ± 0.28 | 9211 ± 2066 | 3.59 ± 0.19 | - |
| | i.g. (10 mg/kg) | - | - | 26287 ± 4150 | 7.04 ± 1.8 | 71.4 ± 11 |
| **Compound 42-1** | i.v. (5 mg/kg) | 7.00 ± 0.89 | 1.08 ± 0.22 | 2760 ± 350 | 2.06 ± 0.59 | - |
| | i.g. (10 mg/kg) | - | - | 37722 ± 3009 | 3.44 ± 0.65 | ≥ 98 |
| **Compound 5** | i.v. (5 mg/kg) | 5.71 ± 0.86 | 1.31 ± 0.12 | 7395 ± 1110 | 2.72 ± 0.23 | - |
| | i.g. (10 mg/kg) | - | - | 27184 ± 2232 | 3.42 ± 0.16 | 91.9 ± 7.5 |
| **Compound 1** | i.v. (5 mg/kg) | 4.55 ± 0.46 | 2.07 ± 0.20 | 8655 ± 759 | 6.17 ± 0.71 | |
| | i.g. (10 mg/kg) | - | - | 26389 ± 1635 | 15.7 ± 4.1 | 76.2 ± 4.7 |
| **Compound 67-1** | i.v. (5 mg/kg) | 9.04 ± 0.75 | 0.986 ± 0.076 | 4552 ± 393 | 1.27 ± 0.053 | |
| | i.g. (10 mg/kg) | - | - | 41862 ± 676 | 2.66 ± 0.14 | ≥ 98 |
| **Compound 80-1** | i.v. (5 mg/kg) | 6.34 ± 0.27 | 0.968 ± 0.076 | 6156 ± 249 | 1.78 ± 0.17 | - |
| | i.g. (10 mg/kg) | - | - | 35989 ± 3265 | 2.67 ± 0.62 | ≥ 98 |
| -: not applicable. | | | | | | |

Conclusion: The compounds of the present invention, such as the examples, exhibit excellent pharmacokinetic properties in the PK test in mice.

**6. Pharmacokinetic test in beagle dogs**

**6.1 Experimental animals:** male beagle dogs, about 8-11 kg, 6 beagle dogs/compound, purchased from Beijing Marshall Biotechnology Co., Ltd.

**6.2 Experimental method:** On the day of the experiment, 18 beagle dogs were randomly grouped according to their body weights. The animals were fasted with water available for 12-14 h one day before administration and fed 4 h after administration. Administration was carried out according to Table 8.

Table 8. Administration information

| Group | Number | Administration information | | | | | |
|---|---|---|---|---|---|---|---|
| | Male | Test compound | Administr ation dosage (mg/kg) | Administr ation concentra tion (mg/mL) | Administr ation volume (mL/kg) | Collected sample | Mode of administrati on |
| G1 | 3 | **Compound 31** | 1 | 1 | 1 | Plasma | Intravenous |
| G2 | 3 | | 5 | 1 | 5 | Plasma | Intragastric |
| G3 | 3 | **Compound 5** | 1 | 1 | 1 | Plasma | Intravenous |
| G4 | 3 | | 5 | 1 | 5 | Plasma | Intragastric |

(continued)

| Group | Number | Administration information | | | | | |
|---|---|---|---|---|---|---|---|
| | Male | Test compound | Administr ation dosage (mg/kg) | Administr ation concentra tion (mg/mL) | Administr ation volume (mL/kg) | Collected sample | Mode of administrati on |
| G5 | | **Compound 67-1** | 1 | 1 | 1 | Plasma | Intravenous |
| G6 | | | 5 | 1 | 5 | Plasma | Intragastric |
| Note: Vehicle I for intravenous administration: 5% DMA + 5% Solutol + 90% Saline, and vehicle II for intravenous administration: 10% DMA + 10% Solutol + 80% Saline; vehicle for intragastric administration: 0.5% MC (DMA: dimethylacetamide; Solutol: polyethylene glycol-15-hydroxystearate; Saline: physiological saline; MC: Methylcellulose solution) | | | | | | | |

Before and after administration, 1 ml of blood was taken from the jugular vein or limb vein and placed in an EDTAK2 centrifuge tube. Centrifugation was carried out at 5000 rpm at 4°C for 10 min, and plasma was collected. The blood collection time points for the intravenous group and intragastric group were both 0 min, 5 min, 15 min, 30 min, 1 h, 2 h, 4 h, 6 h, 8 h, 10 h, 12 h, 24 h, 48 h, and 72 h. Before analysis and detection, all the samples were stored at -80°C, and the samples were subjected to quantitative analysis using LC-MS/MS.

Table 9. Pharmacokinetic parameters of test compounds in plasma of beagle dogs

| Test compound | Mode of administration | CL (mL/min/kg) | $Vd_{ss}$ (L/kg) | $AUC_{0-t}$ (hr*ng/mL) | T1/2 (h) | F (%) |
|---|---|---|---|---|---|---|
| **Compound 31** | i.v. (1 mg/kg) | 2.69 ± 1.7 | 0.957 ± 0.37 | 7070 ± 3250 | 5.85 ± 0.63 | - |
| | i.g. (5 mg/kg) | - | - | 26113 ± 11945 | 7.38 ± 1.2 | ≥ 98 |
| **Compound 5** | i.v. (1 mg/kg) | 13.8 ± 2.3 | 4.16 ± 0.76 | 1202 ± 210 | 3.91 ± 0.037 | - |
| | i.g. (5 mg/kg) | - | - | 5022 ± 1696 | 6.32 ± 2.1 | ≥ 98 |
| **Compound 67-1** | i.v. (1 mg/kg) | 3.24 ± 0.27 | 1.09 ± 0.091 | 5154 ± 436 | 6.20 ± 0.35 | - |
| | i.g. (5 mg/kg) | - | - | 9080 ± 1987 | 6.39 ± 0.54 | 58.7 ± 13 |
| -: not applicable. | | | | | | |

**Conclusion:** The compounds of the present invention, such as the examples, exhibit excellent pharmacokinetic properties in the PK test in beagle dogs.

**7. Pharmacokinetic test in monkeys**

**Experimental animals:** Male cynomolgus monkeys, 3-5 kg, 3-6 years old, 6 monkeys/compound. Purchased from Suzhou Xishan Biotechnology Inc.

**Experimental method:** On the day of the experiment, 12 monkeys were randomly grouped according to their body weights. The animals were fasted with water available for 14-18 h one day before administration and fed 4 h after administration.

Table 10. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound 31 | 1 | 0.5 | 2 | Plasma | Intravenous |
| G2 | 3 | Compound 31 | 3 | 1 | 3 | Plasma | Intragastric |
| G3 | 3 | Compound 67-1 | 1 | 0.5 | 2 | Plasma | Intravenous |
| G4 | 3 | Compound 67-1 | 5 | 1 | 5 | Plasma | Intragastric |
| Note: Vehicle for intravenous administration: 5% DMA + 5% Solutol + 90% Saline;<br>* Dosage is calculated based on free base. | | | | | | | |

Before and after administration, 1.0 mL of blood sample was taken from the limb vein and placed in an EDTAK2 centrifuge tube. Centrifugation was carried out at 5000 rpm at 4°C for 10 min, and plasma was collected. The blood collection time points for the intravenous group were 0 min, 5 min, 15 min, 30 min, 1 h, 2 h, 4 h, 6 h, 8 h, 10 h, 12 h, and 24 h. Before analysis and detection, all the samples were stored at -80°C, and the samples were subjected to quantitative analysis using LC-MS/MS.

Table 11. Pharmacokinetic parameters of test compounds in plasma of monkeys

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 31 | i.v. (1 mg/kg) | 1.95 ± 0.15 | 0.888 ± 0.16 | 8546 ± 664 | - |
| Compound 31 | i.g. (3 mg/kg) | - | - | 7928 ± 3236 | 30.9 ± 13 |
| Compound 67-1 | i.v. (1 mg/kg) | 5.70 ± 0.63 | 1.06 ± 0.024 | 4391 ± 592 | - |
| Compound 67-1 | i.g. (5 mg/kg) | - | - | 7662 ± 1168 | 34.9 ± 53 |

Conclusion: The compounds of the present invention, such as the examples, exhibit excellent pharmacokinetic properties in the PK test in cynomolgus monkeys.

**8. Test for hERG potassium ion channel**

Experimental platform: electrophysiological manual patch-clamp system
Cell line: Chinese hamster ovary (CHO) cell line stably expressing hERG potassium ion channel
Experimental method: CHO (Chinese Hamster Ovary) cells stably expressing hERG potassium channel were subjected to a whole cell patch-clamp technique to record the hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.
Data processing: Data analysis processing was carried out by using pClamp 10, GraphPad Prism 5, and Excel software. The degree of inhibition on the hERG potassium current (peak value of hERG tail current induced at -50 mV) at different compound concentrations was calculated by the following formula:

$$\text{Inhibition \%} = [1-(I / Io)] \times 100\%$$

where Inhibition % represents the percentage inhibition of the compound on the hERG potassium current, and *I* and *I*o

represent the amplitude of the hERG potassium current after and before administration, respectively.

Compound $IC_{50}$ was calculated using GraphPad Prism 5 software by fitting according to the following equation:

$$Y = Bottom + (Top - Bottom)/(1 + 10^{\wedge}((LogIC50\text{-}X)*HillSlope))$$

where X represents the Log value of the tested concentration of the test sample, Y represents the percentage inhibition at the corresponding concentration, and Bottom and Top represent the minimum and maximum percentage inhibitions, respectively.

**Experimental results:** The $IC_{50}$ values for the inhibitory effect of the test compounds on the hERG potassium channel current are shown in Table 12 below:

Table 12. Inhibitory activity of test compounds on hERG potassium channel current

| Test compound | $IC_{50}$ (μM) |
|---|---|
| **Compound 37** | 13.3 |
| **Compound 31** | 12.2 |
| **Compound 40-2** | > 20 |
| **Compound 34** | 13.6 |
| **Compound 80-1** | 18.7 |

Conclusion: The compounds of the present invention, such as the examples, have no obvious inhibitory effect on the hERG potassium channel current.

**9. Detection of inhibitory activity of compounds on the release of tumour necrosis factor-a (TNF-α) from human peripheral blood mononuclear cells induced by lipopolysaccharide (LPS) *in vitro***

Normal human peripheral blood which was anticoagulated (citric acid anticoagulation) was collected, and hPBMCs were prepared by Ficoll-Paque PLUS (Cytiva, Cat#17144002, density 1.077 g/mL). The concentration of hPBMC cells was adjusted to 0.25 x $10^6$ cells/mL with RPMI1640 medium and inoculated into a 96-well plate in an amount of 50000 cells per well. Subsequently, different concentrations of drugs were added for pre-incubation for 1 h (the DMSO final concentration was 0.1%, and the positive and negative control wells had the same volume of RPMI1640 containing 0.1% DMSO). After the pre-incubation was complete, 100 ng/mL LPS (SIGMA, L2630) was added to the compound and positive control wells, and the negative control well had the same volume of RPMI1640 containing 0.1% DMSO. Incubation was carried out for 4 h in an incubator at 37°C and 5% $CO_2$. The cell supernatant was collected, and the content of TNF-α in the supernatant sample was detected by human TNF-α Elisa quantitative test kit (Sino Biological, Cat#KIT10602). The $IC_{50}$ value was calculated using GraphPad Prism software. The $IC_{50}$ values of some specific compounds of the present invention are as shown in Table 13 below, wherein A < 10 nM, 10 nM ≤ B < 50 nM, and 50 nM ≤ C < 100 nM.

Table 13. Inhibitory activity of test compounds on TNF-α induced by LPS *in vitro*

| Test compound | **Re** $IC_{50}$ (nM) | **Max inhibition (%)** |
|---|---|---|
| **Compound 31** | C | 75.37 |
| **Compound 37** | A | / |
| **Compound 54** | B | 78.4 |
| **Compound 55** | B | 67.23 |
| **Compound 62-1** | B | 75.83 |
| **Compound 64** | A | 70.83 |
| **Compound 65** | B | 82 |
| **Compound 66** | B | 79.33 |
| **Compound 67-1** | B | / |
| **Compound 67-2** | B | / |

(continued)

| Test compound | **Re** $IC_{50}$ (nM) | **Max inhibition (%)** |
|---|---|---|
| **Compound 68-1** | B | / |
| **Compound 68-2** | C | / |
| **Compound 69** | C | 74.38 |
| **Compound 70** | A | 70.54 |
| **Compound 73-1** | C | 66.38 |
| **Compound 74-1** | B | 72.39 |
| **Compound 75-1** | A | 63.52 |
| **Compound 75-2** | B | 72.44 |
| **Compound 76** | A | 73.75 |
| **Compound 77-1** | A | 75.70 |
| **Compound 77-2** | A | 73.99 |
| **Compound 78-1** | A | 61.16 |
| **Compound 78-2** | B | 58.51 |
| **Compound 79** | A | 61.49 |
| **Compound 80-1** | A | 70.26 |
| **Compound 80-2** | B | 63.03 |
| **Compound 83** | A | 77.65 |
| Re $IC_{50}$: relative $IC_{50}$. | | |

## Claims

**1.** A compound as represented by formula (I) or a stereoisomer or pharmaceutically acceptable salt thereof,

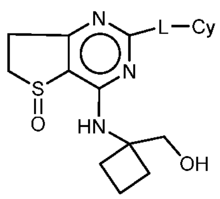

I

**characterized in that** Cy is selected from Cy1, Cy2, a 5-membered monocyclic heteroaryl, or an 8- to 10-membered bicyclic heteroaryl, wherein the monocyclic heteroaryl and the bicyclic heteroaryl are optionally further substituted with 1-3 R;

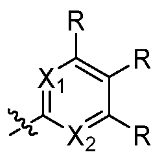

Cy1 ,

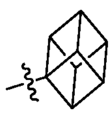

Cy2 ;

Cy2 is optionally substituted with 1-3 R;
L is -$(L_1)$n-$(L_2)_m$-;

$L_1$ is $-NR_1-$, $-CR_2=N-$, $-CR_2=CR_2-$, or $-CR_2R_2-$;

$L_2$ is 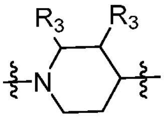, 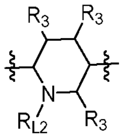, 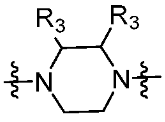, 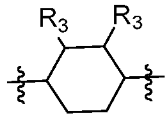, 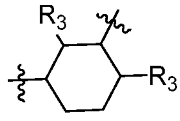,

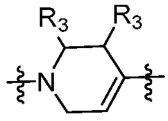, 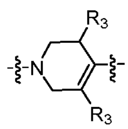, 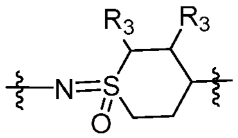, 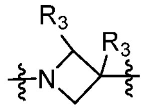,

$C_{1-4}$ alkylene, $C_{2-4}$ alkenylene, $C_{2-4}$ alkynylene, CO, O, $NR_{L2}$, $C_{3-5}$ cycloalkylene, 6-9-membered arylene, 5- or 6-membered heteroarylene containing 1-3 heteroatoms selected from N, S and O, 5-membered monoheterocyclic alkylene containing 1-3 heteroatoms selected from N, S and O, 7- or 8-membered monoheterocyclic alkylene containing 1-3 heteroatoms selected from N, S and O, 5- to 10-membered fused-heterocyclic alkylene containing 1-3 heteroatoms selected from N, S and O, 6- to 10-membered bridged-heterocyclic alkylene containing 1-3 heteroatoms selected from N, S and O, and 7- to 12-membered spiro-heterocyclic alkylene containing 1-3 heteroatoms selected from N, S and O, wherein the alkylene, alkenylene, alkynylene, cycloalkylene, arylene, heteroarylene, monoheterocyclic alkylene, fused-heterocyclic alkylene, bridged-heterocyclic alkylene, and spiro-heterocyclic alkylene are optionally substituted with 1-3 groups selected from halogen, =O, CN, $C_{1-4}$ alkyl, $C_{1-4}$ alkoxy, OH, and $NH_2$;

$R_{L2}$ is H, $C_{1-4}$ alkyl, or $C_{3-6}$ cycloalkyl;

$X_1$ and $X_2$ are independently $CR_{X1}$ or N;

each $R_{X1}$ is independently H, halogen, $C_{1-4}$ alkyl, $C_{1-4}$ haloalkyl, $C_{2-4}$ alkenyl, $C_{2-4}$ alkynyl, or $-SO_2NH_2$, or $R_{X1}$ and $R_1$, $R_{X1}$ and $R_2$, or $R_{X1}$ and $R_3$, together with the atoms to which they are attached, form $C_{3-8}$ cycloalkyl, 5- to 10-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

each R is independently H, halogen, cyano, $C_{1-4}$ alkyl, $C_{2-4}$ alkenyl, $C_{2-4}$ alkynyl, $C_{3-8}$ cycloalkyl, 4- to 7-membered heterocycloalkyl containing 1-3 heteroatoms selected from N, S, O, Si and P, $-SO_2NH_2$, $-NHSO_2NH_2$, $-NHCONH_2$, $-NHCOC_{1-4}$ alkyl, $-CONHC_{1-4}$ alkyl, $-NHSO_2C_{1-4}$ alkyl, $-SO_2NHC_{1-4}$ alkyl,$-SO_2C_{1-4}$ alkyl, $-P(O)(C_{1-4}\ alkyl)_2$, $-SCF_3$, $-SF_5$, or $-C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, $-C(O)C_{1-4}$ alkyl, and $NH_2$; or two adjacent R, or R and $R_{X1}$ together with the atoms to which they are attached form $C_{3-8}$ cycloalkyl, 5- to 10-membered heterocycloalkyl containing 1-3 heteroatoms selected from N, S, O, P and B, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, $-C(O)C_{1-4}$ alkyl, and $NH_2$;

$R_1$ is H, $C_{1-4}$ alkyl, $C_{1-4}$ haloalkyl, or $C_{3-6}$ cycloalkyl;

$R_2$ is H, $C_{1-4}$ alkyl, $C_{1-4}$ haloalkyl, $C_{1-4}$ alkoxy, $-NHC_{1-4}$ alkyl, or $C_{3-6}$ cycloalkyl;

$R_3$ is H, halogen, =O, CN, $C_{1-4}$ alkyl, or $C_{1-4}$ alkoxy;

n is 0, 1, 2, 3, or 4;

m is 0, 1, 2, or 3;

provided that

the compound is not

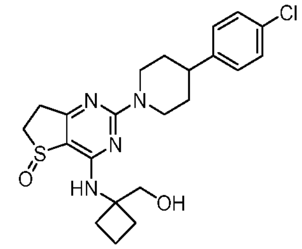 or 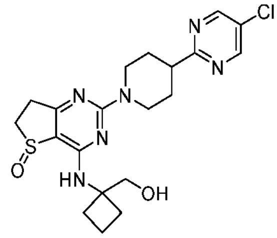 .

2. The compound or the stereoisomer or pharmaceutically acceptable salt thereof according to claim 1,

**characterized in that** each $L_2$ is independently selected from CO, O, $NR_{L2}$, $C_{1-4}$ alkylene, $C_{2-4}$ alkenylene, $C_{2-4}$ alkynylene,

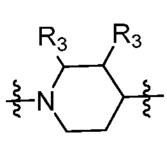,
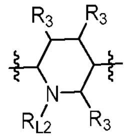,
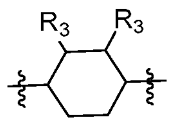,

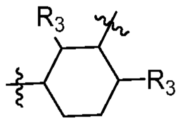,
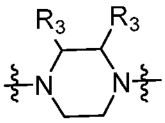,
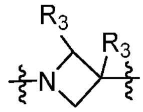,

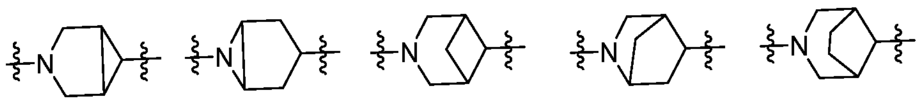,

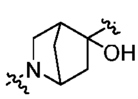,
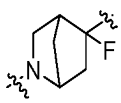,

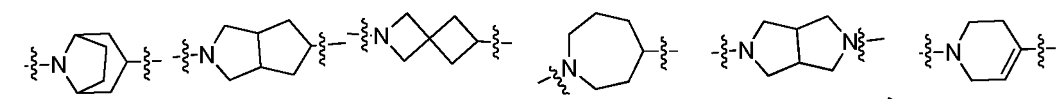
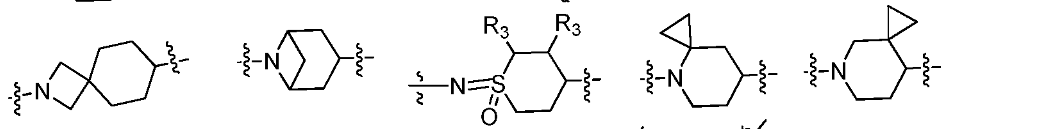
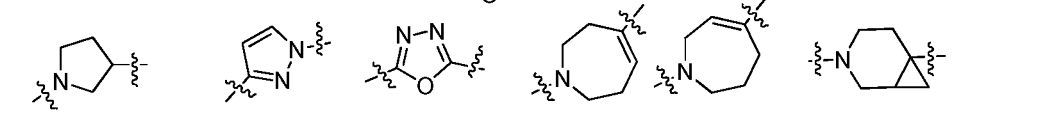
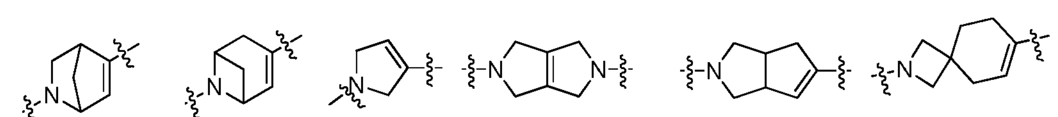
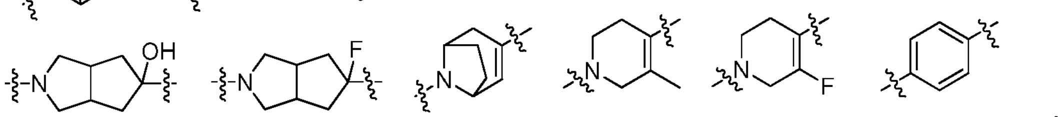,

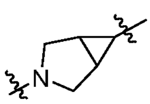,
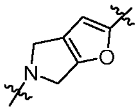,
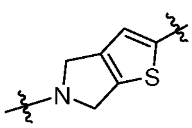,
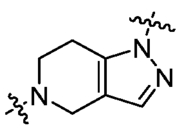, and
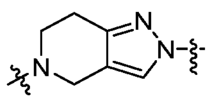;

$R_{L2}$ is H, $C_{1-4}$ alkyl, or $C_{3-6}$ cycloalkyl;
each $R_{X1}$ is independently H, halogen, $C_{1-4}$ alkyl, or $C_{1-4}$ haloalkyl, or $R_{X1}$ and $R_1$, $R_{X1}$ and $R_2$, or $R_{X1}$ and $R_3$ together with the atoms to which they are attached form $C_{3-6}$ cycloalkyl, 5- to 7-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;

each R is independently H, halogen, cyano, $C_{1-4}$ alkyl, $C_{2-4}$ alkenyl, $C_{2-4}$ alkynyl, $C_{3-6}$ cycloalkyl, 4- to 6-membered heterocycloalkyl containing 1-3 heteroatoms selected from N, S, O, Si and P, $-SO_2NH_2$, $-NHSO_2NH_2$, $-NHSO_2C_{1-4}$ alkyl, $-SO_2NHC_{1-4}$ alkyl, $SO_2C_{1-4}$ alkyl, $-P(O)(C_{1-2}$ alkyl$)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH,$-C(O)C_{1-4}$ alkyl, and $NH_2$; or two adjacent R, or R and $R_{X1}$ together with the atoms to which they are attached form $C_{3-6}$ cycloalkyl, or 5- to 10-membered heterocycloalkyl containing 1-3 heteroatoms selected from N, S, O, P and B, wherein the cycloalkyl and heterocycloalkyl are optionally substituted with 1-3 groups selected from halogen, =O, CN, $C_{1-4}$ alkyl, $C_{1-4}$ alkoxy, OH, $-C(O)C_{1-4}$ alkyl and $NH_2$;
$R_1$ is H, $C_{1-4}$ alkyl, or $C_{1-4}$ haloalkyl;
$R_2$ is H, $C_{1-4}$ alkyl, $C_{1-4}$ haloalkyl, or $C_{1-4}$ alkoxy;
$R_3$ is H, halogen, =O, CN, or $C_{1-4}$ alkyl;
n is 0, 1, 2, 3, or 4;
m is 0, 1, or 2.

3. The compound or the stereoisomer or pharmaceutically acceptable salt thereof according to claim 2, **characterized in that** the compound has a structure of the following formula II:

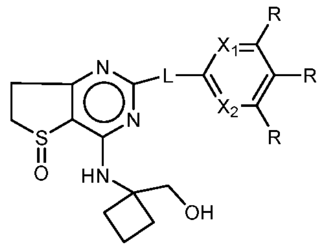

II

wherein L is selected from one of the following structures:

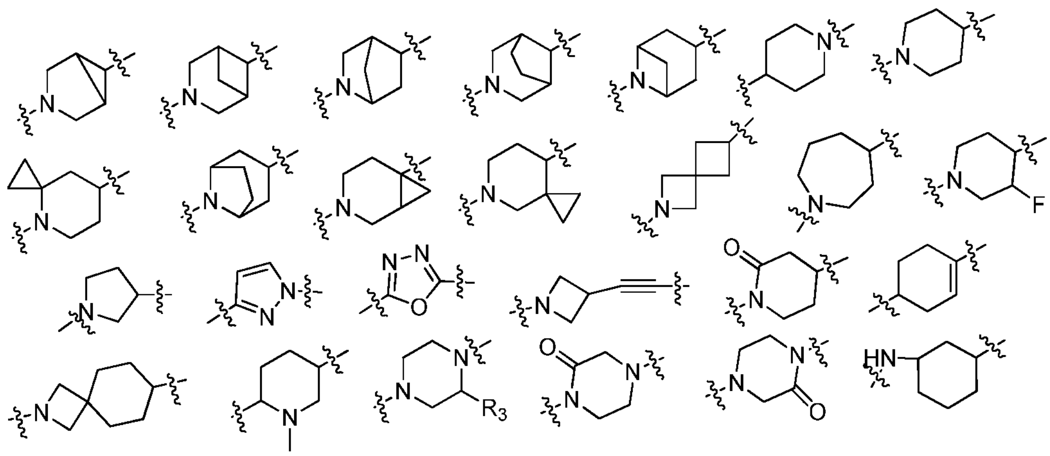

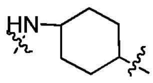 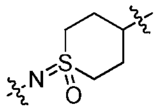 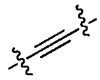 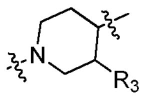 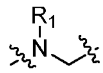 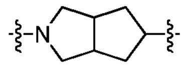
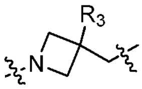 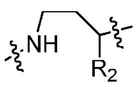 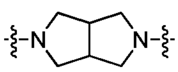 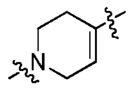 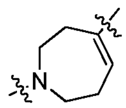 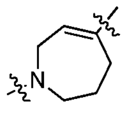
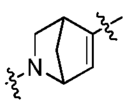 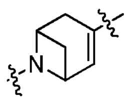 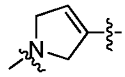 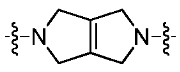 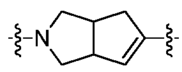 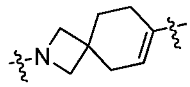
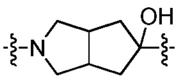 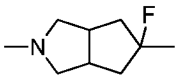 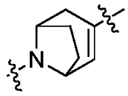 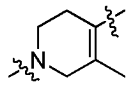 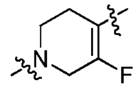 ,

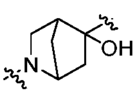 , 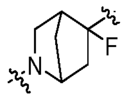 , 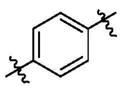 , 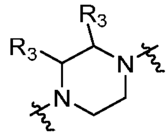 , 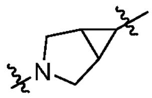 , 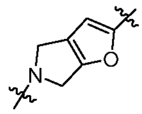 ,

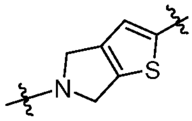 , 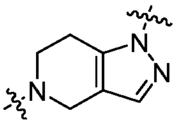 , 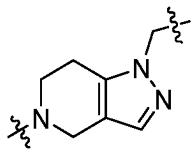 , and 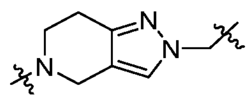 ;

$X_1$ and $X_2$ are independently $CR_{X1}$ or N;
each $R_{X1}$ is independently H, halogen, $C_{1-4}$ alkyl, or $C_{1-4}$ haloalkyl, or $R_{X1}$ and $R_1$, $R_{X1}$ and $R_2$, or $R_{X1}$ and $R_3$ together with the atoms to which they are attached form $C_{3-6}$ cycloalkyl, 5- to 7-membered heterocycloalkyl containing 1-3 heteroatoms selected from N, S and O, or 5- or 6-membered heteroaryl containing 1-3 heteroatoms selected from N, S and O, wherein the cycloalkyl, heterocycloalkyl, and heteroaryl are optionally substituted with 1-3 groups selected from halogen, =O, CN, $C_{1-4}$ alkyl, $C_{1-4}$ alkoxy, OH, and $NH_2$;
each R is independently H, halogen, cyano, $C_{1-4}$ alkyl, $C_{2-4}$ alkynyl, $C_{3-6}$ cycloalkyl, 4- to 6-membered heterocycloalkyl containing 1-3 heteroatoms selected from N, S, O, Si and P, $-SO_2NH_2$, $-NHSO_2C_{1-4}$ alkyl, $-SO_2NHC_{1-4}$ alkyl, $SO_2C_{1-4}$ alkyl, $-P(O)(CH_3)_2$, $SCF_3$, $SF_5$, or $C_{1-4}$ haloalkyl, wherein the cycloalkyl and heterocycloalkyl are optionally substituted with 1-3 groups selected from halogen, =O, CN, $C_{1-4}$ alkyl, $C_{1-4}$ alkoxy, OH, $-C(O)C_{1-4}$ alkyl, and $NH_2$; or two adjacent R, or R and $R_{X1}$ together with the atoms to which they are attached form $C_{3-6}$ cycloalkyl, or 5- to 10-membered heterocycloalkyl containing 1-3 heteroatoms selected from N, S, O, P and B, wherein the cycloalkyl and heterocycloalkyl are optionally substituted with 1-3 groups selected from halogen, =O, CN, $C_{1-4}$ alkyl, $C_{1-4}$ alkoxy, OH, $-C(O)C_{1-4}$ alkyl and $NH_2$;
$R_1$ is H, or $C_{1-4}$ alkyl, or $C_{1-4}$ haloalkyl;
$R_2$ is H, $C_{1-4}$ alkyl, or $C_{1-4}$ haloalkyl; and
$R_3$ is H, halogen, =O, CN, or $C_{1-4}$ alkyl.

**4.** The compound or the stereoisomer or pharmaceutically acceptable salt thereof according to claim 1, **characterized in that**

Cy is selected from the following groups that are optionally further substituted with 1-3 R: 5-membered monocyclic heteroaryl, 5-membered heteroaryl fused benzene ring, 5-membered heteroaryl fused 5-membered heteroaryl, 5-membered heteroaryl fused 6-membered heteroaryl, 5-membered heteroaryl fused 4-membered cycloalkyl, 5-membered heteroaryl fused 5-membered cycloalkyl, 5-membered heteroaryl fused 6-membered cycloalkyl, 5-membered heteroaryl fused 4-membered heterocycloalkyl, 5-membered heteroaryl fused 5-membered heterocycloalkyl, 5-membered heteroaryl fused 6-membered heterocycloalkyl, 6-membered heteroaryl fused benzene ring, 6-membered heteroaryl fused 5-membered heteroaryl, 6-membered heteroaryl fused 6-membered heteroaryl, 6-membered heteroaryl fused 4-membered cycloalkyl, 6-membered heteroaryl fused 5-membered cycloalkyl, 6-membered heteroaryl fused 6-membered cycloalkyl, 6-membered heteroaryl fused 4-membered heterocycloalkyl, 6-membered heteroaryl fused 5-membered heterocycloalkyl, and 6-membered heteroaryl

fused 6-membered heterocycloalkyl;
or Cy is selected from the following structures that are optionally further substituted with 1-3 R:

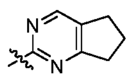 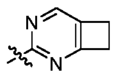 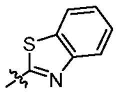 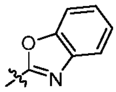 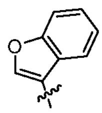 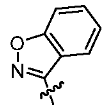 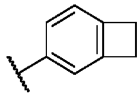 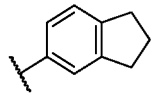

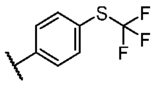 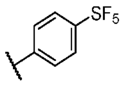 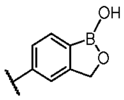 .

5. The compound or the stereoisomer or pharmaceutically acceptable salt thereof according to claim 3, **characterized in that**

L is selected from

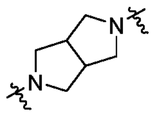, 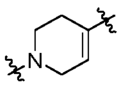, 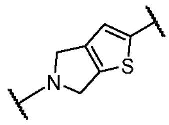, 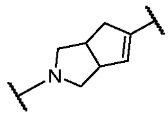,

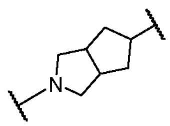, 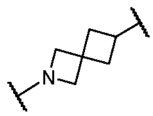, 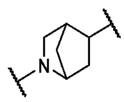, 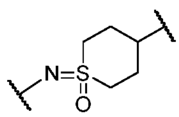, 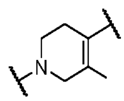, 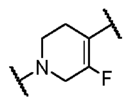,

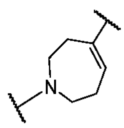, 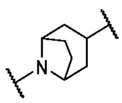, 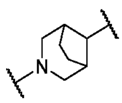, 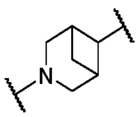, 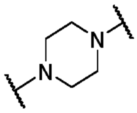, 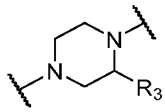, 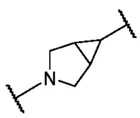,

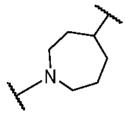, 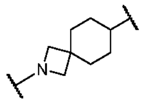, 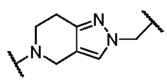, and 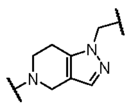;

$X_1$ and $X_2$ are independently $CR_{X1}$ or N;
each $R_{X1}$ is independently H, F, Cl, methyl, or ethyl, or $R_{X1}$ and $R_3$ together with the atoms to which they are attached form $C_5$ cycloalkyl, $C_6$ cycloalkyl, 5-membered heterocycloalkyl containing 1-3 heteroatoms selected from N, S and O, or 6-membered heterocycloalkyl containing 1-3 heteroatoms selected from N, S and O;
$R_3$ is H, F, Cl, =O, CN, or $C_{1-3}$ alkyl; and
each R is independently H, F, Cl, methyl, ethyl, acetylene, or propyne, or two adjacent R together with the atoms to which they are attached form cyclopropenyl, cyclobutenyl, cyclopentenyl, or cyclohexenyl, wherein the cyclopropenyl, cyclobutenyl, cyclopentenyl, and cyclohexenyl are optionally substituted with 1-3 groups selected from F, Cl, =O, CN, methyl, ethyl, methoxy, ethoxy, OH, and $NH_2$.

6. The compound or the stereoisomer or pharmaceutically acceptable salt thereof according to claim 1, **characterized in that**

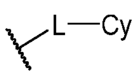

is selected from the following structures:

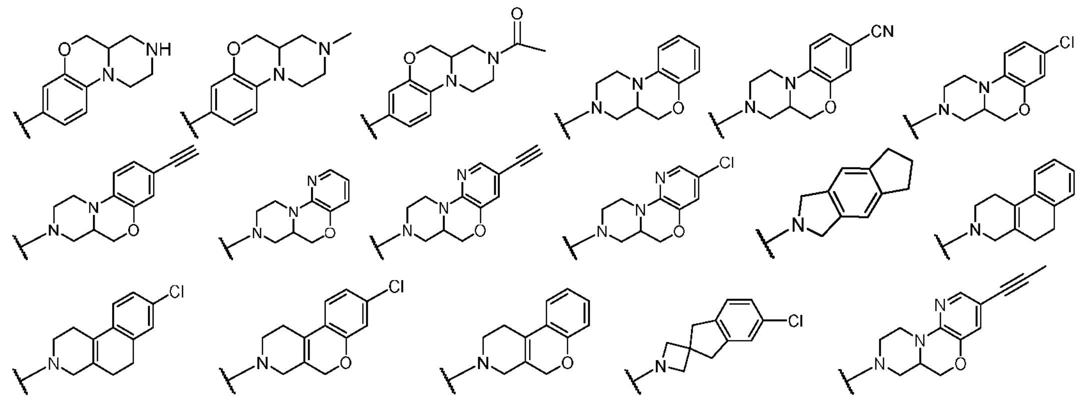

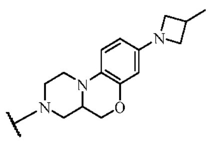

.

7. A compound described in Table 1 of the description or a stereoisomer or pharmaceutically acceptable salt thereof.

8. The compound or the stereoisomer or pharmaceutically acceptable salt thereof according to claim 7, **characterized in that** the compound is selected from one of structures in Table 2.

9. A pharmaceutical composition or pharmaceutical preparation, **characterized in that** the pharmaceutical composition or pharmaceutical preparation comprises the compound or the stereoisomer or pharmaceutically acceptable salt thereof according to any one of claims 1-8, and a pharmaceutically acceptable carrier and/or auxiliary material.

10. The pharmaceutical composition or pharmaceutical preparation according to claim 9, **characterized in that** the pharmaceutical composition or pharmaceutical preparation comprises 1-1500 mg of the compound or the stereoisomer or pharmaceutically acceptable salt thereof according to any one of claims 1-8, and a pharmaceutically acceptable carrier and/or auxiliary material.

11. Use of the compound or the stereoisomer or pharmaceutically acceptable salt thereof according to any one of claims 1-8 in the preparation of a medicament for treating/preventing a PDE4B-mediated disease.

12. A method for treating a disease in a mammal, **characterized in that** the method comprises administering to a subject a therapeutically effective amount of the compound or the stereoisomer or pharmaceutically acceptable salt thereof according to any one of claims 1-8, the therapeutically effective amount is preferably 1-1500 mg, and the disease is preferably cancer, COPD, idiopathic pulmonary fibrosis, or interstitial lung disease.

**INTERNATIONAL SEARCH REPORT**

International application No. **PCT/CN2023/097944**

**A. CLASSIFICATION OF SUBJECT MATTER**

C07D495/04(2006.01)i；C07D519/00(2006.01)i；A61K31/519(2006.01)i；A61P11/00(2006.01)i

According to International Patent Classification (IPC) or to both national classification and IPC

**B. FIELDS SEARCHED**

Minimum documentation searched (classification system followed by classification symbols)

IPC: C07D,A61K,A61P

Documentation searched other than minimum documentation to the extent that such documents are included in the fields searched

Electronic data base consulted during the international search (name of data base and, where practicable, search terms used)

CNTXT, WPABS, CAPLUS(STN), REGISTRY(STN), CNKI: 海思科, 磷酸二酯酶, 癌, 肿瘤, PDE4+, PDE4, COPD, 肺纤维化, 噻吩 6W 嘧啶, Phosphodiesterase, Lung Fibrosis, cancer, tumor, tumour, thieno 6w pyrimidin+, 结构式检索, search for structural formula.

**C. DOCUMENTS CONSIDERED TO BE RELEVANT**

| Category* | Citation of document, with indication, where appropriate, of the relevant passages | Relevant to claim No. |
|---|---|---|
| X | CN 101827853 A (BOEHRINGER INGELHEIM INTERNATIONAL GMBH) 08 September 2010 (2010-09-08)<br>claims 1-3, 6, 11-14 and 17-23, description, pages 46-50 and 54 | 1-5, 7-12 |
| X | US 2011046096 A1 (BOEHRINGER INGELHEIM INTERNATIONAL GMBH) 24 February 2011 (2011-02-24)<br>claims 1-3, 7, 12-18 and 21-27, description, pages 33-36 and 49 | 1-5, 7-12 |
| X | CN 101827852 A (BOEHRINGER INGELHEIM INTERNATIONAL GMBH) 08 September 2010 (2010-09-08)<br>claims 1-3, 6, 12-20 and 25-30, description, page 15, compound 2, page 16, compounds 5-6 and 8, page 17, compounds 7-8, 10 and 12-13, page 18, compounds 1-2 and 4-7, page 22, compounds 2, 4-5, 7-8 and 11, page 23 compounds 1-7 and 9-11 | 1-4, 7-12 |
| X | US 2013225609 A1 (BOEHRINGER INGELHEIM INTERNATIONAL GMBH) 29 August 2013 (2013-08-29)<br>claims 4-5, 8, 14-22 and 24-27 | 1-4, 7-12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 August 2023** | **15 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/097944**

**C. DOCUMENTS CONSIDERED TO BE RELEVANT**

| Category* | Citation of document, with indication, where appropriate, of the relevant passages | Relevant to claim No. |
|---|---|---|
| X | CN 111278442 A (BOEHRINGER INGELHEIM INTERNATIONAL GMBH) 12 June 2020 (2020-06-12) claims 7, 11 and 31 | 1-4, 7-12 |
| X | CN 103889970 A (BOEHRINGER INGELHEIM INTERNATIONAL GMBH) 25 June 2014 (2014-06-25) claims 1, 4-7 and 17-24 | 1-4, 7-12 |
| X | WO 2014124860 A1 (BOEHRINGER INGELHEIM INTERNATIONAL GMBH) 21 August 2014 (2014-08-21) claim 1, description, pages 8 and 47-48 | 1-4, 7-12 |
| X | CN 111712502 A (LEO PHARMA A/S) 25 September 2020 (2020-09-25) claims 1, 7-10, description, paragraph [0306] | 1-4, 9-12 |
| A | CN 101827853 A (BOEHRINGER INGELHEIM INTERNATIONAL GMBH) 08 September 2010 (2010-09-08) claims 1-3, 6, 11-14 and 17-23, description, pages 46-50 and 54 | 6 |

Form PCT/ISA/210 (second sheet) (July 2022)

**INTERNATIONAL SEARCH REPORT**

International application No.
**PCT/CN2023/097944**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:

The subject matter of claim 12 is a method for treating diseases in a mammal, which relates to a living human or animal body, and falls within methods for treatment of diseases. The present report is provided on the basis of the pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/097944**

| Patent document cited in search report | | | Publication date (day/month/year) | Patent family member(s) | | | Publication date (day/month/year) |
|---|---|---|---|---|---|---|---|
| CN | 101827853 | A | 08 September 2010 | US | 2014107103 | A1 | 17 April 2014 |
| | | | | US | 9115142 | B2 | 25 August 2015 |
| | | | | EP | 2205609 | A2 | 14 July 2010 |
| | | | | EP | 2205609 | B1 | 29 March 2017 |
| | | | | MX | 2010003346 | A | 09 April 2010 |
| | | | | AR | 069076 | A1 | 30 December 2009 |
| | | | | BRPI | 0817781 | A2 | 24 September 2019 |
| | | | | NZ | 585348 | A | 24 February 2012 |
| | | | | ZA | 201001621 | B | 27 October 2010 |
| | | | | PE | 20091081 | A1 | 24 August 2009 |
| | | | | AU | 2008313742 | A1 | 23 April 2009 |
| | | | | AU | 2008313742 | B2 | 28 November 2013 |
| | | | | RU | 2010119645 | A | 27 January 2012 |
| | | | | RU | 2500681 | C2 | 10 December 2013 |
| | | | | US | 2010305102 | A1 | 02 December 2010 |
| | | | | US | 8637519 | B2 | 28 January 2014 |
| | | | | KR | 20100075930 | A | 05 July 2010 |
| | | | | CA | 2702524 | A1 | 23 April 2009 |
| | | | | TW | 200918073 | A | 01 May 2009 |
| | | | | TWI | 445535 | B | 21 July 2014 |
| | | | | CL | 2008003097 | A1 | 22 January 2010 |
| | | | | UY | 31402 | A1 | 29 May 2009 |
| | | | | WO | 2009050242 | A2 | 23 April 2009 |
| | | | | WO | 2009050242 | A3 | 30 July 2009 |
| | | | | JP | 2011500639 | A | 06 January 2011 |
| | | | | JP | 5341899 | B2 | 13 November 2013 |
| US | 2011046096 | A1 | 24 February 2011 | EP | 2215093 | A1 | 11 August 2010 |
| | | | | EP | 2215093 | B1 | 14 December 2011 |
| | | | | AT | 537175 | T | 15 December 2011 |
| | | | | CA | 2702518 | A1 | 23 April 2009 |
| | | | | WO | 2009050236 | A1 | 23 April 2009 |
| | | | | US | 8486948 | B2 | 16 July 2013 |
| | | | | US | 2013274264 | A1 | 17 October 2013 |
| | | | | US | 9090626 | B2 | 28 July 2015 |
| | | | | JP | 2011500638 | A | 06 January 2011 |
| | | | | JP | 5563466 | B2 | 30 July 2014 |
| CN | 101827852 | A | 08 September 2010 | MX | 2010004026 | A | 30 April 2010 |
| | | | | PT | 2610258 | E | 24 October 2014 |
| | | | | WO | 2009050248 | A1 | 23 April 2009 |
| | | | | UY | 31405 | A1 | 29 May 2009 |
| | | | | BRPI | 0818006 | A2 | 22 December 2015 |
| | | | | BRPI | 0818006 | B1 | 22 October 2019 |
| | | | | BRPI | 0818006 | B8 | 25 May 2021 |
| | | | | CL | 2008003096 | A1 | 12 February 2010 |
| | | | | CY | 1112703 | T1 | 10 February 2016 |
| | | | | CA | 2705414 | A1 | 23 April 2009 |
| | | | | CA | 2705414 | C | 24 May 2016 |
| | | | | MA | 31845 | B1 | 01 November 2010 |
| | | | | TW | 200918074 | A | 01 May 2009 |
| | | | | TWI | 421077 | B | 01 January 2014 |

Form PCT/ISA/210 (patent family annex) (July 2022)

**INTERNATIONAL SEARCH REPORT**
**Information on patent family members**

International application No.
**PCT/CN2023/097944**

| Patent document cited in search report | Publication date (day/month/year) | Patent family member(s) | Publication date (day/month/year) |
|---|---|---|---|
| | | AU 2008313660 A1 | 23 April 2009 |
| | | AU 2008313660 B2 | 07 November 2013 |
| | | PE 20091386 A1 | 17 October 2009 |
| | | ES 2524910 T3 | 15 December 2014 |
| | | JP 2013064001 A | 11 April 2013 |
| | | JP 5615889 B2 | 29 October 2014 |
| | | ES 2381452 T3 | 28 May 2012 |
| | | EP 2215092 A1 | 11 August 2010 |
| | | EP 2215092 B1 | 25 January 2012 |
| | | UA 99309 C2 | 10 August 2012 |
| | | HRP 20141153 T1 | 13 February 2015 |
| | | ECSP 10010156 A | 29 June 2010 |
| | | SI 2610258 T1 | 31 December 2014 |
| | | SI 2215092 T1 | 30 April 2012 |
| | | NZ 585346 A | 30 September 2011 |
| | | DK 2610258 T3 | 10 November 2014 |
| | | PL 2215092 T3 | 31 July 2012 |
| | | PL 2610258 T3 | 27 February 2015 |
| | | TN 2010000175 A1 | 11 November 2011 |
| | | EP 2610258 A1 | 03 July 2013 |
| | | EP 2610258 B1 | 27 August 2014 |
| | | RS 52271 B | 31 October 2012 |
| | | DK 2215092 T3 | 07 May 2012 |
| | | EA 201000609 A1 | 29 October 2010 |
| | | EA 019480 B1 | 30 April 2014 |
| | | HRP 20120334 T1 | 31 May 2012 |
| | | AR 069075 A1 | 30 December 2009 |
| | | CY 1115858 T1 | 25 January 2017 |
| | | ME 01330 B | 20 December 2013 |
| | | EP 2380891 A1 | 26 October 2011 |
| | | EP 2380891 B1 | 11 December 2013 |
| | | ZA 201001683 B | 27 October 2010 |
| | | AT 542825 T | 15 February 2012 |
| | | JP 2011500640 A | 06 January 2011 |
| | | JP 5150728 B2 | 27 February 2013 |
| | | KR 20100100807 A | 15 September 2010 |
| | | KR 101548975 B1 | 01 September 2015 |
| | | PE 20131463 A1 | 23 December 2013 |
| | | US 2011021501 A1 | 27 January 2011 |
| | | US 8754073 B2 | 17 June 2014 |
| | | MY 153979 A | 30 April 2015 |
| | | PT 2215092 E | 10 April 2012 |
| US 2013225609 A1 | 29 August 2013 | US 2014350035 A1 | 27 November 2014 |
| | | WO 2011124525 A1 | 13 October 2011 |
| | | EP 2555774 A1 | 13 February 2013 |
| | | EP 2555774 B1 | 21 October 2015 |
| | | JP 2013523792 A | 17 June 2013 |
| CN 111278442 A | 12 June 2020 | IL 273169 A | 30 April 2020 |
| | | US 2019134043 A1 | 09 May 2019 |
| | | US 11406638 B2 | 09 August 2022 |

Form PCT/ISA/210 (patent family annex) (July 2022)

**INTERNATIONAL SEARCH REPORT**
**Information on patent family members**

International application No.
**PCT/CN2023/097944**

| Patent document cited in search report | Publication date (day/month/year) | Patent family member(s) | Publication date (day/month/year) |
|---|---|---|---|
| | | MX 2020004173 A | 03 August 2020 |
| | | CL 2020000627 A1 | 21 August 2020 |
| | | TW 201929858 A | 01 August 2019 |
| | | TWI 700088 B | 01 August 2020 |
| | | EP 3700529 A1 | 02 September 2020 |
| | | BR 112020003973 A2 | 01 September 2020 |
| | | AU 2018357775 A1 | 19 March 2020 |
| | | PH 12020550452 A1 | 15 March 2021 |
| | | JP 2021500362 A | 07 January 2021 |
| | | JP 7196169 B2 | 26 December 2022 |
| | | WO 2019081235 A1 | 02 May 2019 |
| | | MA 50441 A | 02 September 2020 |
| | | US 2022378793 A1 | 01 December 2022 |
| | | CA 3079299 A1 | 02 May 2019 |
| | | KR 20200075864 A | 26 June 2020 |
| | | EA 202090977 A1 | 04 September 2020 |
| CN 103889970 A | 25 June 2014 | IL 230592 A0 | 31 March 2014 |
| | | AR 125150 A2 | 14 June 2023 |
| | | AP 2014007401 A0 | 31 January 2014 |
| | | BR 112014003378 A2 | 01 March 2017 |
| | | BR 112014003378 B1 | 17 May 2022 |
| | | CO 6900138 A2 | 20 March 2014 |
| | | US 2013059866 A1 | 07 March 2013 |
| | | PE 20141532 A1 | 31 October 2014 |
| | | PL 2748156 T3 | 29 July 2016 |
| | | ECSP 14013254 A | 30 April 2014 |
| | | WO 2013026797 A1 | 28 February 2013 |
| | | CA 2846183 A1 | 28 February 2013 |
| | | CA 2846183 C | 28 April 2020 |
| | | ZA 201400792 B | 25 August 2021 |
| | | UY 34285 A | 28 February 2013 |
| | | HUE 028531 T2 | 28 December 2016 |
| | | EA 201400252 A1 | 30 December 2014 |
| | | EA 024033 B1 | 31 August 2016 |
| | | AP 201407401 D0 | 31 January 2014 |
| | | TW 201323427 A | 16 June 2013 |
| | | TWI 597283 B | 01 September 2017 |
| | | JP 2014524452 A | 22 September 2014 |
| | | JP 5851038 B2 | 03 February 2016 |
| | | CL 2014000296 A1 | 22 August 2014 |
| | | ES 2563028 T3 | 10 March 2016 |
| | | AR 087655 A1 | 09 April 2014 |
| | | EP 2748156 A1 | 02 July 2014 |
| | | EP 2748156 B1 | 16 December 2015 |
| | | NZ 620199 A | 30 October 2015 |
| | | US 2015148322 A1 | 28 May 2015 |
| | | US 9150586 B2 | 06 October 2015 |
| | | TN 2014000081 A1 | 01 July 2015 |
| | | AU 2012298599 A1 | 13 February 2014 |
| | | AU 2012298599 B2 | 22 September 2016 |

Form PCT/ISA/210 (patent family annex) (July 2022)

**INTERNATIONAL SEARCH REPORT**
**Information on patent family members**

International application No.
**PCT/CN2023/097944**

| Patent document cited in search report | | | Publication date (day/month/year) | Patent family member(s) | | | Publication date (day/month/year) |
|---|---|---|---|---|---|---|---|
| | | | | US | 2013079359 | A1 | 28 March 2013 |
| | | | | US | 8609670 | B2 | 17 December 2013 |
| | | | | DK | 2748156 | T3 | 07 March 2016 |
| | | | | MX | 2014002025 | A | 27 March 2014 |
| | | | | MX | 358651 | B | 30 August 2018 |
| | | | | BR | 122022002799 | B1 | 30 August 2022 |
| | | | | KR | 20140060504 | A | 20 May 2014 |
| | | | | KR | 101959194 | B1 | 19 March 2019 |
| WO | 2014124860 | A1 | 21 August 2014 | None | | | |
| CN | 111712502 | A | 25 September 2020 | US | 2021070769 | A1 | 11 March 2021 |
| | | | | US | 11292799 | B2 | 05 April 2022 |
| | | | | EP | 3724194 | A1 | 21 October 2020 |
| | | | | JP | 2021506805 | A | 22 February 2021 |
| | | | | JP | 7198820 | B2 | 04 January 2023 |
| | | | | WO | 2019115774 | A1 | 20 June 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013026797 A1
- WO 2013026797 A
- WO 2021158829 A


### Non-patent literature cited in the description


- *Chemistry-A European Journal*, 2019, vol. 25 (14), 3521-3524